# NEW EUROPEAN PATENT SPECIFICATION

(11) **EP 2 785 374 B2**
(45) Date of publication and mention of the opposition decision: **17.08.2022**
(45) Mention of the grant of the patent: 09.01.2019
(21) Application number: 12805843.5
(22) Date of filing: 29.11.2012
(51) Int. Cl.: A61K 39/12

(54) **RECOMBINANT GALLID HERPESVIRUS 3 (MDV SEROTYPE 2) VECTORS EXPRESSING ANTIGENS OF AVIAN PATHOGENS AND USES THEREOF**
REKOMBINANTE GALLID-HERPESVIRUS 3 (MDV-SEROTYP 2)-VEKTOREN, DIE ANTIGENE VON GEFLÜGELPATHOGENEN EXPRIMIEREN UND DEREN VERWENDUNGEN
VECTEURS RECOMBINANTS DE GALLID HERPÈSVIRUS 3 (MDV SÉROTYPE 2) EXPRIMANT DES ANTIGÈNES DE PATHOGÈNES AVIAIRES ET LEURS UTILISATIONS

(30) Priority: 30.11.2011 US 201161564877 P; 30.08.2012 US 201261694957 P
(43) Date of publication of application: 08.10.2014
(73) Proprietor: Boehringer Ingelheim Animal Health USA Inc., Duluth, GA 30096 (US)
(72) Inventor: BUBLOT, Michel, 69630 Chaponost (FR); MEBATSION, Teshome, Watkinsville, GA 30677 (US); PRITCHARD, Joyce, Gainesville, GA 30504 (US); LINZ, Perry, Jefferson, GA 30549 (US)
(74) Representative: D Young & Co LLP
(86) International application number: PCT/US2012/067123
(87) International publication number: WO 2013/082317

(56) References cited:
- EP-A2- 1 298 139
- US-A- 5 980 906
- PETHERBRIDGE L ET AL: "Cloning of Gallid herpesvirus 3 (Marek's disease virus serotype-2) genome as infectious bacterial artificial chromosomes for analysis of viral gene functions", JOURNAL OF VIROLOGICAL METHODS, ELSEVIER BV, NL, vol. 158, no. 1-2, 1 June 2009 (2009-06-01), pages 11-17, XP026022528, ISSN: 0166-0934, DOI: 10.1016/J.JVIROMET.2009.01.009 [retrieved on 2009-01-31]
- SLACUM ET AL: "The compatibility of HVT recombinants with other Marek's disease vaccines", 58TH WESTERN POULTRY DISEASE CONFERENCE, SACRAMENTO, CA, USA, MARCH 23-25 2009,, 1 January 2009 (2009-01-01), page 84, XP009167868,

### SEQUENCE LISTING

<110> Merial Limited Bublot, Michel Mebatsion, Teshome Pritchard, Nikki Linz, Perry
<120> RECOMBINANT GALLID HERPESVIRUS 3 (MDV SEROTYPE 2) VECTORS EXPRESSING ANTIGENS OF AVIAN PATHOGENS AND USES THEREOF
<130> MER 11-178
<150> 61/564,877
   <151> 2011-11-30
<150> 61/694,957
   <151> 2012-08-30
<160> 64
<170> PatentIn version 3.5
<210> 1
   <211> 1665
   <212> DNA
   <213> artificial sequence
<220>
   <223> NDV-F codon-optimized gene from modified wt VIId
<400> 1
   atgggcagca agcccagcac aagaatccca gcccccctga tgctgatcac ccgcatcatg      60
   ctgatcctgg gctgcatcag acccacaagc tccctggatg gacgccccct ggccgctgcc      120
   ggcatcgtgg tgaccggcga caaggccgtg aacgtgtaca ccagcagcca gaccggcagc      180
   atcatcgtga agctgctgcc caacatgccc agagacaaag aggcctgcgc caaggccccc      240
   ctggaagcct acaacagaac cctgaccacc ctgctgaccc ccctgggcga cagcatcaga      300
   aagatccagg gctccgtgag cacaagcggc ggaggaaagc agggcagact gatcggcgcc      360
   gtgatcggca gcgtggccct gggagtggct acagctgccc agattaccgc tgcagccgcc      420
   ctgatccagg ccaaccagaa cgccgccaac atcctgagac tgaaagagag cattgccgcc      480
   accaacgagg ccgtgcacga agtgaccgac ggcctgagcc agctgtccgt ggccgtgggc      540
   aagatgcagc agttcgtgaa cgaccagttc aacaacaccg ccagagagct ggactgcatc      600
   aagatcaccc agcaggtggg cgtggagctg aacctgtacc tgaccgagct gaccacagtg      660
   ttcggccccc agatcacaag cccagccctg acacagctga ccatccaggc cctgtacaac      720
   ctggctggcg gcaacatgga ctatctgctg acaaagctgg gaatcggcaa caaccagctg      780
   tccagcctga tcggaagcgg cctgatcacc ggctacccca tcctgtacga cagccagaca      840
   cagctgctgg gcatccaggt gaacctgccc agcgtgggca acctgaacaa catgcgcgcc      900
   acctacctgg aaaccctgag cgtgtccacc accaagggct acgccagcgc cctggtgccc      960
   aaggtggtga cacaggtggg cagcgtgatc gaggaactgg acaccagcta ctgcatcgag     1020
   agcgacctgg acctgtactg caccagaatc gtgaccttcc caatgagccc cggcatctac     1080
   agctgcctga gcggcaacac cagcgcctgc atgtacagca agaccgaagg cgcactgaca     1140
   acaccctaca tggccctgaa gggaagcgtg atcgccaact gcaagatcac cacctgcaga     1200
   tgcaccgacc ccccaggcat catcagccag aactacggcg aggccgtgag cctgatcgat     1260
   cgccattcct gtaacgtgct gtccctggac ggcatcacac tgagactgag cggcgagttc     1320
   gatgccacct accagaagaa catcagcatc ctggacagcc aggtgatcgt gaccggcaac     1380
   ctggacatca gcaccgagct gggcaacgtg aataacagca tcagcaacgc cctggacaga     1440
   ctggccgaga gcaacagcaa gctggaaaaa gtgaacgtgc gcctgacatc cacttccgct     1500
   ctgatcacct acatcgtgct gaccgtgatc agcctggtgt tcggcgccct gagcctggtg     1560
   ctggcctgct acctgatgta caagcagaag gcccagcaga aaaccctgct gtggctgggc     1620
   aacaacaccc tggaccagat gagagccacc accagagcct gatga                     1665
<210> 2
   <211> 553
   <212> PRT
   <213> artificial sequence
<220>
   <223> NDV-F protein of modified wt VIId of codon-optimized gene
<400> 2
   Met Gly Ser Lys Pro Ser Thr Arg Ile Pro Ala Pro Leu Met Leu Ile
   1               5                   10                  15
   Thr Arg Ile Met Leu Ile Leu Gly Cys Ile Arg Pro Thr Ser Ser Leu
               20                  25                  30
   Asp Gly Arg Pro Leu Ala Ala Ala Gly Ile Val Val Thr Gly Asp Lys
           35                  40                  45
   Ala Val Asn Val Tyr Thr Ser Ser Gln Thr Gly Ser Ile Ile Val Lys
       50                  55                  60
   Leu Leu Pro Asn Met Pro Arg Asp Lys Glu Ala Cys Ala Lys Ala Pro
   65                  70                  75                  80
   Leu Glu Ala Tyr Asn Arg Thr Leu Thr Thr Leu Leu Thr Pro Leu Gly
                   85                  90                  95
   Asp Ser Ile Arg Lys Ile Gln Gly Ser Val Ser Thr Ser Gly Gly Gly
               100                 105                 110
   Lys Gln Gly Arg Leu Ile Gly Ala Val Ile Gly Ser Val Ala Leu Gly
           115                 120                 125
   Val Ala Thr Ala Ala Gln Ile Thr Ala Ala Ala Ala Leu Ile Gln Ala
       130                 135                 140
   Asn Gln Asn Ala Ala Asn Ile Leu Arg Leu Lys Glu Ser Ile Ala Ala
   145                 150                 155                 160
   Thr Asn Glu Ala Val His Glu Val Thr Asp Gly Leu Ser Gln Leu Ser
                   165                 170                 175
   Val Ala Val Gly Lys Met Gln Gln Phe Val Asn Asp Gln Phe Asn Asn
               180                 185                 190
   Thr Ala Arg Glu Leu Asp Cys Ile Lys Ile Thr Gln Gln Val Gly Val
           195                 200                 205
   Glu Leu Asn Leu Tyr Leu Thr Glu Leu Thr Thr Val Phe Gly Pro Gln
       210                 215                 220
   Ile Thr Ser Pro Ala Leu Thr Gln Leu Thr Ile Gln Ala Leu Tyr Asn
   225                 230                 235                 240
   Leu Ala Gly Gly Asn Met Asp Tyr Leu Leu Thr Lys Leu Gly Ile Gly
                   245                 250                 255
   Asn Asn Gln Leu Ser Ser Leu Ile Gly Ser Gly Leu Ile Thr Gly Tyr
               260                 265                 270
   Pro Ile Leu Tyr Asp Ser Gln Thr Gln Leu Leu Gly Ile Gln Val Asn
           275                 280                 285
   Leu Pro Ser Val Gly Asn Leu Asn Asn Met Arg Ala Thr Tyr Leu Glu
       290                 295                 300
   Thr Leu Ser Val Ser Thr Thr Lys Gly Tyr Ala Ser Ala Leu Val Pro
   305                 310                 315                 320
   Lys Val Val Thr Gln Val Gly Ser Val Ile Glu Glu Leu Asp Thr Ser
                   325                 330                 335
   Tyr Cys Ile Glu Ser Asp Leu Asp Leu Tyr Cys Thr Arg Ile Val Thr
               340                 345                 350
   Phe Pro Met Ser Pro Gly Ile Tyr Ser Cys Leu Ser Gly Asn Thr Ser
               355                 360                 365
   Ala Cys Met Tyr Ser Lys Thr Glu Gly Ala Leu Thr Thr Pro Tyr Met
       370                 375                 380
   Ala Leu Lys Gly Ser Val Ile Ala Asn Cys Lys Ile Thr Thr Cys Arg
   385                 390                 395                 400
   Cys Thr Asp Pro Pro Gly Ile Ile Ser Gln Asn Tyr Gly Glu Ala Val
                   405                 410                 415
   Ser Leu Ile Asp Arg His Ser Cys Asn Val Leu Ser Leu Asp Gly Ile
               420                 425                 430
   Thr Leu Arg Leu Ser Gly Glu Phe Asp Ala Thr Tyr Gln Lys Asn Ile
           435                 440                 445
   Ser Ile Leu Asp Ser Gln Val Ile Val Thr Gly Asn Leu Asp Ile Ser
       450                 455                 460
   Thr Glu Leu Gly Asn Val Asn Asn Ser Ile Ser Asn Ala Leu Asp Arg
   465                 470                 475                 480
   Leu Ala Glu Ser Asn Ser Lys Leu Glu Lys Val Asn Val Arg Leu Thr
                   485                 490                 495
   Ser Thr Ser Ala Leu Ile Thr Tyr Ile Val Leu Thr Val Ile Ser Leu
               500                 505                 510
   Val Phe Gly Ala Leu Ser Leu Val Leu Ala Cys Tyr Leu Met Tyr Lys
           515                 520                 525
   Gln Lys Ala Gln Gln Lys Thr Leu Leu Trp Leu Gly Asn Asn Thr Leu
       530                 535                 540
   Asp Gln Met Arg Ala Thr Thr Arg Ala
   545                 550
<210> 3
   <211> 1662
   <212> DNA
   <213> artificial sequence
<220>
   <223> NDV-F DAN wt VIId
<400> 3
   atgggctcca aaccttctac caggatccca gcacctctga tgctgatcac ccggattatg 60
   ctgatattgg gctgtatccg tccgacaagc tctcttgacg gcaggcctct tgcagctgca 120
   ggaattgtag taacaggaga taaggcagtc aatgtataca cttcgtctca gacagggtca 180
   atcatagtca agttgctccc gaatatgccc agggataagg aggcgtgtgc aaaagcccca 240
   ttagaggcat ataacagaac actgactact ttgctcactc ctcttggcga ctccatccgc 300
   aagatccaag ggtctgtgtc cacatctgga ggaggcaagc aaggccgcct gataggtgct 360
   gttattggca gtgtagctct tggggttgca acagcggcac agataacagc agctgcggcc 420
   ctaatacaag ccaaccagaa tgccgccaac atcctccggc ttaaggagag cattgctgca 480
   accaatgaag ctgtgcatga agtcaccgac ggattatcac aactatcagt ggcagttggg 540
   aagatgcagc agtttgtcaa tgaccagttt aataatacgg cgcgagaatt ggactgtata 600
   aaaatcacac aacaggttgg tgtagaactc aacctatacc taactgaatt gactacagta 660
   ttcgggccac agatcacctc ccctgcatta actcagctga ccatccaggc actttataat 720
   ttagctggtg gcaatatgga ttacttatta actaagttag gtatagggaa caatcaactc 780
   agctcgttaa ttggtagcgg cctgatcact ggttacccta tactgtatga ctcacagact 840
   caactcttgg gcatacaagt gaatttaccc tcagtcggga acttaaataa tatgcgtgcc 900
   acctatttgg agaccttatc tgtaagtaca accaaaggat atgcctcagc acttgtcccg 960
   aaagtagtga cacaagtcgg ttccgtgata gaagagcttg acacctcata ctgtatagag 1020
   tccgatctgg atttatattg tactagaata gtgacattcc ccatgtcccc aggtatttat 1080
   tcctgtttga gcggcaacac atcagcttgc atgtattcaa agactgaagg cgcactcact 1140
   acgccgtata tggcccttaa aggctcagtt attgccaatt gtaaaataac aacatgtaga 1200
   tgtacagacc ctcctggtat catatcgcaa aattatggag aagctgtatc cctgatagat 1260
   agacattcgt gcaatgtctt atcattagac gggataactc taaggctcag tggggaattt 1320
   gatgcaactt atcaaaagaa catctcaata ctagattctc aagtcatcgt gacaggcaat 1380
   cttgatatat caactgaact tggaaacgtc aacaattcaa tcagcaatgc cttggatagg 1440
   ttggcagaaa gcaacagcaa gctagaaaaa gtcaatgtca gactaaccag cacatctgct 1500
   ctcattacct atattgttct aactgtcatt tctctagttt tcggtgcact tagtctggtg 1560
   ttagcgtgtt acctgatgta caaacagaag gcacaacaaa agaccttgct atggcttggg 1620
   aataataccc tcgatcagat gagagccact acaagagcat ga 1662
<210> 4
   <211> 1695
   <212> DNA
   <213> artificial sequence
<220>
   <223> NDV-F DNA with GenBank accession No. AY337464.1
<400> 4
   ctggatcccg gttggctcat tcaggacgca atatgggctc caaaccttct accaggatcc      60
   cagcacctct gatgctgatc acccggatta tgctgatatt gggctgtatc cgtccgacaa     120
   gctctcttga cggcaggcct cttgcagctg caggaattgt agtaacagga gataaggcag     180
   tcaatgtata cacttcgtct cagacagggt caatcatagt caagttgctc ccgaatatgc     240
   ccagggataa ggaggcgtgt gcaaaagccc cattagaggc atataacaga acactgacta     300
   ctttgctcac tcctcttggc gactccatcc gcaagatcca agggtctgtg tccacatctg     360
   gaggaaggag acaaaaacgc tttataggtg ctgttattgg cagtgtagct cttggggttg     420
   caacagcggc acagataaca gcagctgcgg ccctaataca agccaaccag aatgccgcca     480
   acatcctccg gcttaaggag agcattgctg caaccaatga agctgtgcat gaagtcaccg     540
   acggattatc acaactatca gtggcagttg ggaagatgca gcagtttgtc aatgaccagt     600
   ttaataatac ggcgcgagaa ttggactgta taaaaatcac acaacaggtt ggtgtagaac     660
   tcaacctata cctaactgaa ttgactacag tattcgggcc acagatcacc tcccctgcat     720
   taactcagct gaccatccag gcactttata atttagctgg tggcaatatg gattacttat     780
   taactaagtt aggtataggg aacaatcaac tcagctcgtt aattggtagc ggcctgatca     840
   ctggttaccc tatactgtat gactcacaga ctcaactctt gggcatacaa gtgaatttac     900
   cctcagtcgg gaacttaaat aatatgcgtg ccacctattt ggagacctta tctgtaagta     960
   caaccaaagg atatgcctca gcacttgtcc cgaaagtagt gacacaagtc ggttccgtga    1020
   tagaagagct tgacacctca tactgtatag agtccgatct ggatttatat tgtactagaa    1080
   tagtgacatt ccccatgtcc ccaggtattt attcctgttt gagcggcaac acatcagctt    1140
   gcatgtattc aaagactgaa ggcgcactca ctacgccgta tatggccctt aaaggctcag    1200
   ttattgccaa ttgtaggata acaacatgta gatgtacaga ccctcctggt atcatatcgc    1260
   aaaattatgg agaagctgta tccctgatag atagacattc gtgcaatgtc ttatcattag    1320
   acgggataac tctaaggctc agtggggaat ttgatgcaac ttatcaaaag aacatctcaa    1380
   tactagattc tcaagtcatc gtgacaggca atcttgatat atcaactgaa cttggaaacg    1440
   tcaacaattc aatcagcaat gccttggata ggttggcaga aagcaacagc aagctagaaa    1500
   aagtcaatgt cagactaacc agcacatctg ctctcattac ctatattgtt ctaactgtca    1560
   tttctctagt tttcggtgca cttagtctgg gtttagcgtg ttacctgatg tacaaacaga    1620
   aggcacaaca aaagaccttg ctatggcttg ggaataatac cctcgatcag atgagagcca    1680
   ctacaagagc atgaa                                                     1695
<210> 5
   <211> 553
   <212> PRT
   <213> artificial sequence
<220>
   <223> NDV-F protein with GenBank accession No. AAP97877.1
<400> 5
   Met Gly Ser Lys Pro Ser Thr Arg Ile Pro Ala Pro Leu Met Leu Ile
   1               5                   10                  15
   Thr Arg Ile Met Leu Ile Leu Gly Cys Ile Arg Pro Thr Ser Ser Leu
               20                  25                  30
   Asp Gly Arg Pro Leu Ala Ala Ala Gly Ile Val Val Thr Gly Asp Lys
           35                  40                  45
   Ala Val Asn Val Tyr Thr Ser Ser Gln Thr Gly Ser Ile Ile Val Lys
       50                  55                  60
   Leu Leu Pro Asn Met Pro Arg Asp Lys Glu Ala Cys Ala Lys Ala Pro
   65                  70                  75                  80
   Leu Glu Ala Tyr Asn Arg Thr Leu Thr Thr Leu Leu Thr Pro Leu Gly
                   85                  90                  95
   Asp Ser Ile Arg Lys Ile Gln Gly Ser Val Ser Thr Ser Gly Gly Arg
               100                 105                 110
   Arg Gln Lys Arg Phe Ile Gly Ala Val Ile Gly Ser Val Ala Leu Gly
           115                 120                 125
   Val Ala Thr Ala Ala Gln Ile Thr Ala Ala Ala Ala Leu Ile Gln Ala
       130                 135                 140
   Asn Gln Asn Ala Ala Asn Ile Leu Arg Leu Lys Glu Ser Ile Ala Ala
   145                 150                 155                 160
   Thr Asn Glu Ala Val His Glu Val Thr Asp Gly Leu Ser Gln Leu Ser
                   165                 170                 175
   Val Ala Val Gly Lys Met Gln Gln Phe Val Asn Asp Gln Phe Asn Asn
               180                 185                 190
   Thr Ala Arg Glu Leu Asp Cys Ile Lys Ile Thr Gln Gln Val Gly Val
           195                 200                 205
   Glu Leu Asn Leu Tyr Leu Thr Glu Leu Thr Thr Val Phe Gly Pro Gln
       210                 215                 220
   Ile Thr Ser Pro Ala Leu Thr Gln Leu Thr Ile Gln Ala Leu Tyr Asn
   225 230 235 240
   Leu Ala Gly Gly Asn Met Asp Tyr Leu Leu Thr Lys Leu Gly Ile Gly
   245 250 255
   Asn Asn Gln Leu Ser Ser Leu Ile Gly Ser Gly Leu Ile Thr Gly Tyr
   260 265 270
   Pro Ile Leu Tyr Asp Ser Gln Thr Gln Leu Leu Gly Ile Gln Val Asn
   275 280 285
   Leu Pro Ser Val Gly Asn Leu Asn Asn Met Arg Ala Thr Tyr Leu Glu
   290 295 300
   Thr Leu Ser Val Ser Thr Thr Lys Gly Tyr Ala Ser Ala Leu Val Pro
   305 310 315 320
   Lys Val Val Thr Gln Val Gly Ser Val Ile Glu Glu Leu Asp Thr Ser
   325 330 335
   Tyr Cys Ile Glu Ser Asp Leu Asp Leu Tyr Cys Thr Arg Ile Val Thr
   340 345 350
   Phe Pro Met Ser Pro Gly Ile Tyr Ser Cys Leu Ser Gly Asn Thr Ser
   355 360 365
   Ala Cys Met Tyr Ser Lys Thr Glu Gly Ala Leu Thr Thr Pro Tyr Met
   370 375 380
   Ala Leu Lys Gly Ser Val Ile Ala Asn Cys Arg Ile Thr Thr Cys Arg
   385 390 395 400
   Cys Thr Asp Pro Pro Gly Ile Ile Ser Gln Asn Tyr Gly Glu Ala Val
   405 410 415
   Ser Leu Ile Asp Arg His Ser Cys Asn Val Leu Ser Leu Asp Gly Ile
   420 425 430
   Thr Leu Arg Leu Ser Gly Glu Phe Asp Ala Thr Tyr Gln Lys Asn Ile
   435 440 445
   Ser Ile Leu Asp Ser Gln Val Ile Val Thr Gly Asn Leu Asp Ile Ser
   450 455 460
   Thr Glu Leu Gly Asn Val Asn Asn Ser Ile Ser Asn Ala Leu Asp Arg
   465 470 475 480
   Leu Ala Glu Ser Asn Ser Lys Leu Glu Lys Val Asn Val Arg Leu Thr
                   485                 490                 495
   Ser Thr Ser Ala Leu Ile Thr Tyr Ile Val Leu Thr Val Ile Ser Leu
               500                 505                 510
   Val Phe Gly Ala Leu Ser Leu Gly Leu Ala Cys Tyr Leu Met Tyr Lys
           515                 520                 525
   Gln Lys Ala Gln Gln Lys Thr Leu Leu Trp Leu Gly Asn Asn Thr Leu
       530                 535                 540
   Asp Gln Met Arg Ala Thr Thr Arg Ala
   545                 550
<210> 6
   <211> 1662
   <212> DNA
   <213> artificial sequence
<220>
   <223> NDV-F DNA wildtype V (CA02 strain) with GenBank accession No. EF520718
<400> 6
   atgggctcca aaccttctac ctggatctca gtaactctga tgctgatcac tcggaccatg 60
   cttatactta gctgtatctg tccgacaagc tctcttgacg gtagacctct cgcagccgca 120
   gggattgtgg tgacgggaga taaagcagtc aatatataca cttcatctca aacagggtca 180
   atcatcatca agttactccc aaatatgccc aaggataaag aagcgtgcgc aaaagcccca 240
   ttggaagcat acaatagaac actgaccact ttactcaccc ctcttggtga ctctatccgc 300
   agaatacaag ggtctgcgac tacatctgga ggaaggagac agaaacgctt tgtaggtgcc 360
   attatcggca gtgtagctct tggggttgca acagctgcac agataacagc agccgcagct 420
   ctgatacaag ccaaccaaaa tgctgccaac atcctccggc ttaaggagag cattgctgca 480
   accaatgacg ctgtacacga ggtcactaac ggattatcac aactagcggt ggcggtcggg 540
   aagatgcagc agtttgttaa taaccagttt aataatacgg cgcgagaatt ggactgcata 600
   aaaattgcac aacaagtggg cgtcgaactc aatttgtatc taactgaatt gaccacagtg 660
   ttcgggccac aaatcacctc ccctgcttta actcagctga ctatccaggc actttataat 720
   ttagccggtg gcaatatgga ttacctgttg actaagttgg gtgtagggaa taatcaactc 780
   agttcgttaa ttggtagtgg cttgataact ggcaacccta tactatatga ctcacagacc 840
   caactcttag gcatacagat aaatttaccc tcagtcggga gcctaaataa tatgcgtgcc 900
   acctacttgg agaccttatc cgtaagcacg accaaagggt tcgcctcagc acttgtcccg 960
   aaagttgtga cgcaagtcgg ctctgtgata gaagaacttg acacctcata ttgtatagaa 1020
   tccgatatag atctatattg tacaagggta gtgacattcc ccatgtctcc tggtatttac 1080
   tcctgtctga gcggcaatac gtcagcttgt atgtattcaa agaccgaagg tgcactcact 1140
   acaccataca tggccctcaa aggctcagtt attgccaatt gcaagatgac tacatgcaga 1200
   tgcgcagatc ccccaggtat catatcacag aattatgggg aagctgtgtc tctaatagat 1260
   aaacattcat gcagtgtctt gtccctagac gggataactc tgaggctcag tggggaattt 1320
   gatgcgacct atcaaaagaa catctcaata ctagattctc aagtcatcgt gacaggaaat 1380
   ctcgatatat caactgagct tgggaatgtt aacaactcga taagcagtac cctggacaaa 1440
   ttagcagaaa gcaacaacaa gctaaacaag gtcaatgtaa acctaaccag cacatctgct 1500
   ctcatcactt atattgtctt agctatcgta tctcttgctt tcggcgtaat tagcctggtt 1560
   ctagcatgct acctgatgta taaacaaaga gcacaacaaa agaccttact atggctcggg 1620
   aacaacaccc ttgatcagat gagagccacc acaagaacct ga 1662
<210> 7
   <211> 553
   <212> PRT
   <213> artificial sequence
<220>
   <223> NDV-F protein wildtype V (CA02 strain) with GenBank accession No. ABS84266
<400> 7
   Met Gly Ser Lys Pro Ser Thr Trp Ile Ser Val Thr Leu Met Leu Ile
   1               5                   10                  15
   Thr Arg Thr Met Leu Ile Leu Ser Cys Ile Cys Pro Thr Ser Ser Leu
               20                  25                  30
   Asp Gly Arg Pro Leu Ala Ala Ala Gly Ile Val Val Thr Gly Asp Lys
           35                  40                  45
   Ala Val Asn Ile Tyr Thr Ser Ser Gln Thr Gly Ser Ile Ile Ile Lys
       50                  55                  60
   Leu Leu Pro Asn Met Pro Lys Asp Lys Glu Ala Cys Ala Lys Ala Pro
   65                  70                  75                  80
   Leu Glu Ala Tyr Asn Arg Thr Leu Thr Thr Leu Leu Thr Pro Leu Gly
                   85                  90                  95
   Asp Ser Ile Arg Arg Ile Gln Gly Ser Ala Thr Thr Ser Gly Gly Arg
               100                 105                 110
   Arg Gln Lys Arg Phe Val Gly Ala Ile Ile Gly Ser Val Ala Leu Gly
           115                 120                 125
   Val Ala Thr Ala Ala Gln Ile Thr Ala Ala Ala Ala Leu Ile Gln Ala
       130                 135                 140
   Asn Gln Asn Ala Ala Asn Ile Leu Arg Leu Lys Glu Ser Ile Ala Ala
   145                 150                 155                 160
   Thr Asn Asp Ala Val His Glu Val Thr Asn Gly Leu Ser Gln Leu Ala
                   165                 170                 175
   Val Ala Val Gly Lys Met Gln Gln Phe Val Asn Asn Gln Phe Asn Asn
               180                 185                 190
   Thr Ala Arg Glu Leu Asp Cys Ile Lys Ile Ala Gln Gln Val Gly Val
           195                 200                 205
   Glu Leu Asn Leu Tyr Leu Thr Glu Leu Thr Thr Val Phe Gly Pro Gln
       210                 215                 220
   Ile Thr Ser Pro Ala Leu Thr Gln Leu Thr Ile Gln Ala Leu Tyr Asn
   225                 230                 235                 240
   Leu Ala Gly Gly Asn Met Asp Tyr Leu Leu Thr Lys Leu Gly Val Gly
                   245                 250                 255
   Asn Asn Gln Leu Ser Ser Leu Ile Gly Ser Gly Leu Ile Thr Gly Asn
               260                 265                 270
   Pro Ile Leu Tyr Asp Ser Gln Thr Gln Leu Leu Gly Ile Gln Ile Asn
           275                 280                 285
   Leu Pro Ser Val Gly Ser Leu Asn Asn Met Arg Ala Thr Tyr Leu Glu
       290                 295                 300
   Thr Leu Ser Val Ser Thr Thr Lys Gly Phe Ala Ser Ala Leu Val Pro
   305                 310                 315                 320
   Lys Val Val Thr Gln Val Gly Ser Val Ile Glu Glu Leu Asp Thr Ser
                   325                 330                 335
   Tyr Cys Ile Glu Ser Asp Ile Asp Leu Tyr Cys Thr Arg Val Val Thr
               340                 345                 350
   Phe Pro Met Ser Pro Gly Ile Tyr Ser Cys Leu Ser Gly Asn Thr Ser
           355                 360                 365
   Ala Cys Met Tyr Ser Lys Thr Glu Gly Ala Leu Thr Thr Pro Tyr Met
       370                 375                 380
   Ala Leu Lys Gly Ser Val Ile Ala Asn Cys Lys Met Thr Thr Cys Arg
   385                 390                 395                 400
   Cys Ala Asp Pro Pro Gly Ile Ile Ser Gln Asn Tyr Gly Glu Ala Val
                   405                 410                 415
   Ser Leu Ile Asp Lys His Ser Cys Ser Val Leu Ser Leu Asp Gly Ile
               420                 425                 430
   Thr Leu Arg Leu Ser Gly Glu Phe Asp Ala Thr Tyr Gln Lys Asn Ile
           435                 440                 445
   Ser Ile Leu Asp Ser Gln Val Ile Val Thr Gly Asn Leu Asp Ile Ser
       450                 455                 460
   Thr Glu Leu Gly Asn Val Asn Asn Ser Ile Ser Ser Thr Leu Asp Lys
   465                 470                 475                 480
   Leu Ala Glu Ser Asn Asn Lys Leu Asn Lys Val Asn Val Asn Leu Thr
                   485                 490                 495
   Ser Thr Ser Ala Leu Ile Thr Tyr Ile Val Leu Ala Ile Val Ser Leu
               500                 505                 510
   Ala Phe Gly Val Ile Ser Leu Val Leu Ala Cys Tyr Leu Met Tyr Lys
           515                 520                 525
   Gln Arg Ala Gln Gln Lys Thr Leu Leu Trp Leu Gly Asn Asn Thr Leu
       530                 535                 540
   Asp Gln Met Arg Ala Thr Thr Arg Thr
   545                 550
<210> 8
   <211> 1665
   <212> DNA
   <213> artificial sequence
<220>
   <223> NDV-F codon-optimized gene from modified wildtype V (CA02 strain)
<400> 8
   atgggcagca agcccagcac ctggatcagc gtgaccctga tgctgatcac cagaaccatg 60
   ctgatcctga gctgcatctg ccccacaagc agcctggacg gcagacccct ggccgctgcc 120
   ggcatcgtgg tgaccggcga caaggccgtg aacatctaca ccagcagcca gaccggcagc 180
   atcatcatca agctgctgcc caacatgccc aaggacaaag aggcctgcgc caaggccccc 240
   ctggaagcct acaacagaac cctgaccacc ctgctgaccc ccctgggcga cagcatcaga 300
   agaatccagg gcagcgccac cacaagcggc ggaggaaagc agggcagact ggtgggcgct 360
   atcatcggga gcgtggccct gggcgtggcc acagctgccc agattaccgc tgcagccgcc 420
   ctgattcagg ccaatcagaa cgccgccaac atcctgagac tgaaagagag cattgccgcc 480
   accaacgacg ccgtgcacga agtgacaaac ggactgtccc agctggctgt cgctgtcggc 540
   aagatgcagc agttcgtgaa caaccagttc aacaacaccg ccagagagct ggactgcatc 600
   aagatcgccc agcaggtggg cgtggagctg aacctgtacc tgaccgagct gaccacagtg 660
   ttcggccccc agatcacaag ccccgctctg acccagctga caatccaggc cctgtacaac 720
   ctggctggcg gcaacatgga ctatctgctg actaagctgg gagtgggcaa caaccagctg 780
   tccagcctga tcgggtccgg gctgatcaca ggcaacccca tcctgtacga cagccagaca 840
   cagctgctgg gcatccagat caacctgcca tccgtgggaa gcctgaacaa catgagagcc 900
   acctacctgg aaaccctgag cgtgtccacc accaagggct tcgccagcgc cctggtgccc 960
   aaggtggtga cacaggtggg cagcgtgatc gaggaactgg acaccagcta ctgcatcgag 1020
   agcgacatcg acctgtactg caccagagtg gtgaccttcc caatgagccc cggcatctac 1080
   agctgcctga gcggcaacac cagcgcctgc atgtacagca agaccgaagg agcactgaca 1140
   acaccctaca tggccctgaa gggaagcgtg atcgccaact gcaagatgac cacctgcaga 1200
   tgcgccgacc ccccaggcat catcagccag aactacggcg aggccgtgag cctgatcgac 1260
   aaacattcct gtagcgtgct gtccctggat ggcatcacac tgagactgag cggcgagttc 1320
   gacgccacct accagaagaa catcagcatc ctggacagcc aggtgatcgt gaccggcaac 1380
   ctggacatca gcaccgagct gggcaacgtg aacaacagca tcagcagcac cctggacaag 1440
   ctggccgagt ccaacaacaa gctgaacaaa gtgaacgtga acctgaccag cacaagcgcc 1500
   ctgatcacct acatcgtgct ggccatcgtg tccctggcct tcggcgtgat cagcctggtg 1560
   ctggcctgct acctgatgta caagcagaga gcccagcaga aaaccctgct gtggctgggc 1620
   aataacaccc tggaccagat gagggccacc accagaacct gatga 1665
<210> 9
   <211> 553
   <212> PRT
   <213> artificial sequence
<220>
   <223> NDV-F protein of codon-optimized NDV-F gene of modified wildtype V (CA02 strain)
<400> 9
   Met Gly Ser Lys Pro Ser Thr Trp Ile Ser Val Thr Leu Met Leu Ile
   1               5                   10                  15
   Thr Arg Thr Met Leu Ile Leu Ser Cys Ile Cys Pro Thr Ser Ser Leu
               20                  25                  30
   Asp Gly Arg Pro Leu Ala Ala Ala Gly Ile Val Val Thr Gly Asp Lys
           35                  40                  45
   Ala Val Asn Ile Tyr Thr Ser Ser Gln Thr Gly Ser Ile Ile Ile Lys
       50                  55                  60
   Leu Leu Pro Asn Met Pro Lys Asp Lys Glu Ala Cys Ala Lys Ala Pro
   65                  70                  75                  80
   Leu Glu Ala Tyr Asn Arg Thr Leu Thr Thr Leu Leu Thr Pro Leu Gly
                   85                  90                  95
   Asp Ser Ile Arg Arg Ile Gln Gly Ser Ala Thr Thr Ser Gly Gly Gly
               100                 105                 110
   Lys Gln Gly Arg Leu Val Gly Ala Ile Ile Gly Ser Val Ala Leu Gly
           115                 120                 125
   Val Ala Thr Ala Ala Gln Ile Thr Ala Ala Ala Ala Leu Ile Gln Ala
       130                 135                 140
   Asn Gln Asn Ala Ala Asn Ile Leu Arg Leu Lys Glu Ser Ile Ala Ala
   145                 150                 155                 160
   Thr Asn Asp Ala Val His Glu Val Thr Asn Gly Leu Ser Gln Leu Ala
                   165                 170                 175
   Val Ala Val Gly Lys Met Gln Gln Phe Val Asn Asn Gln Phe Asn Asn
               180                 185                 190
   Thr Ala Arg Glu Leu Asp Cys Ile Lys Ile Ala Gln Gln Val Gly Val
           195                 200                 205
   Glu Leu Asn Leu Tyr Leu Thr Glu Leu Thr Thr Val Phe Gly Pro Gln
       210                 215                 220
   Ile Thr Ser Pro Ala Leu Thr Gln Leu Thr Ile Gln Ala Leu Tyr Asn
   225                 230                 235                 240
   Leu Ala Gly Gly Asn Met Asp Tyr Leu Leu Thr Lys Leu Gly Val Gly
                   245                 250                 255
   Asn Asn Gln Leu Ser Ser Leu Ile Gly Ser Gly Leu Ile Thr Gly Asn
               260                 265                 270
   Pro Ile Leu Tyr Asp Ser Gln Thr Gln Leu Leu Gly Ile Gln Ile Asn
           275                 280                 285
   Leu Pro Ser Val Gly Ser Leu Asn Asn Met Arg Ala Thr Tyr Leu Glu
       290                 295                 300
   Thr Leu Ser Val Ser Thr Thr Lys Gly Phe Ala Ser Ala Leu Val Pro
   305                 310                 315                 320
   Lys Val Val Thr Gln Val Gly Ser Val Ile Glu Glu Leu Asp Thr Ser
                   325                 330                 335
   Tyr Cys Ile Glu Ser Asp Ile Asp Leu Tyr Cys Thr Arg Val Val Thr
               340                 345                 350
   Phe Pro Met Ser Pro Gly Ile Tyr Ser Cys Leu Ser Gly Asn Thr Ser
           355                 360                 365
   Ala Cys Met Tyr Ser Lys Thr Glu Gly Ala Leu Thr Thr Pro Tyr Met
       370                 375                 380
   Ala Leu Lys Gly Ser Val Ile Ala Asn Cys Lys Met Thr Thr Cys Arg
   385                 390                 395                 400
   Cys Ala Asp Pro Pro Gly Ile Ile Ser Gln Asn Tyr Gly Glu Ala Val
                   405                 410                 415
   Ser Leu Ile Asp Lys His Ser Cys Ser Val Leu Ser Leu Asp Gly Ile
               420                 425                 430
   Thr Leu Arg Leu Ser Gly Glu Phe Asp Ala Thr Tyr Gln Lys Asn Ile
           435                 440                 445
   Ser Ile Leu Asp Ser Gln Val Ile Val Thr Gly Asn Leu Asp Ile Ser
       450                 455                 460
   Thr Glu Leu Gly Asn Val Asn Asn Ser Ile Ser Ser Thr Leu Asp Lys
   465                 470                 475                 480
   Leu Ala Glu Ser Asn Asn Lys Leu Asn Lys Val Asn Val Asn Leu Thr
                   485                 490                 495
   Ser Thr Ser Ala Leu Ile Thr Tyr Ile Val Leu Ala Ile Val Ser Leu
               500                 505                 510
   Ala Phe Gly Val Ile Ser Leu Val Leu Ala Cys Tyr Leu Met Tyr Lys
   515 520 525
   Gln Arg Ala Gln Gln Lys Thr Leu Leu Trp Leu Gly Asn Asn Thr Leu
   530 535 540
   Asp Gln Met Arg Ala Thr Thr Arg Thr
   545 550
<210> 10
   <211> 1411
   <212> DNA
   <213> artificial sequence
<220>
   <223> MCMV IE promoter
<400> 10
   aattcaatag tggatccccc aactccgccc gttttatgac tagaaccaat agtttttaat      60
   gccaaatgca ctgaaatccc ctaatttgca aagccaaacg ccccctatgt gagtaatacg     120
   gggacttttt acccaatttc ccacgcggaa agccccctaa tacactcata tggcatatga     180
   atcagcacgg tcatgcactc taatggcggc ccatagggac tttccacata gggggcgttc     240
   accatttccc agcatagggg tggtgactca atggccttta cccaagtaca ttgggtcaat     300
   gggaggtaag ccaatgggtt tttcccatta ctggcaagca cactgagtca aatgggactt     360
   tccactgggt tttgcccaag tacattgggt caatgggagg tgagccaatg ggaaaaaccc     420
   attgctgcca agtacactga ctcaataggg actttccaat gggtttttcc attgttggca     480
   agcatataag gtcaatgtgg gtgagtcaat agggactttc cattgtattc tgcccagtac     540
   ataaggtcaa tagggggtga atcaacagga aagtcccatt ggagccaagt acactgcgtc     600
   aatagggact ttccattggg ttttgcccag tacataaggt caatagggga tgagtcaatg     660
   ggaaaaaccc attggagcca agtacactga ctcaataggg actttccatt gggttttgcc     720
   cagtacataa ggtcaatagg gggtgagtca acaggaaagt tccattggag ccaagtacat     780
   tgagtcaata gggactttcc aatgggtttt gcccagtaca taaggtcaat gggaggtaag     840
   ccaatgggtt tttcccatta ctggcacgta tactgagtca ttagggactt tccaatgggt     900
   tttgcccagt acataaggtc aataggggtg aatcaacagg aaagtcccat tggagccaag     960
   tacactgagt caatagggac tttccattgg gttttgccca gtacaaaagg tcaatagggg    1020
   gtgagtcaat gggtttttcc cattattggc acgtacataa ggtcaatagg ggtgagtcat    1080
   tgggtttttc cagccaattt aattaaaacg ccatgtactt tcccaccatt gacgtcaatg    1140
   ggctattgaa actaatgcaa cgtgaccttt aaacggtact ttcccatagc tgattaatgg    1200
   gaaagtaccg ttctcgagcc aatacacgtc aatgggaagt gaaagggcag ccaaaacgta 1260
   acaccgcccc ggttttcccc tggaaattcc atattggcac gcattctatt ggctgagctg 1320
   cgttctacgt gggtataaga ggcgcgacca gcgtcggtac cgtcgcagtc ttcggtctga 1380
   ccaccgtaga acgcagagct cctcgctgca g 1411
<210> 11
   <211> 217
   <212> DNA
   <213> artificial sequence
<220>
   <223> SV40 PolyA
<400> 11
   ggggatccag acatgataag atacattgat gagtttggac aaaccacaac tagaatgcag 60
   tgaaaaaaat gctttatttg tgaaatttgt gatgctattg ctttatttgt aaccattata 120
   agctgcaata aacaagttaa caacaacaat tgcattgatt ttatgtttca ggttcagggg 180
   gaggtgtggg aggttttttc ggatcctcta gagtcga 217
<210> 12
   <211> 368
   <212> DNA
   <213> artificial sequence
<220>
   <223> SV40 promoter
<400> 12
   caattcgagc tcggtacagc ttggctgtgg aatgtgtgtc agttagggtg tggaaagtcc 60
   ccaggctccc cagcaggcag aagtatgcaa agcatgcatc tcaattagtc agcaaccagg 120
   tgtggaaagt ccccaggctc cccagcaggc agaagtatgc aaagcatgca tctcaattag 180
   tcagcaacca tagtcccgcc cctaactccg cccatcccgc ccctaactcc gcccagttcc 240
   gcccattctc cgccccatgg ctgactaatt ttttttattt atgcagaggc cgaggccgcc 300
   tcggcctctg agctattcca gaagtagtga ggaggctttt ttggaggcct aggcttttgc 360
   aaaaagct 368
<210> 13
   <211> 154
   <212> DNA
   <213> artificial sequence
<220>
   <223> Synthetic PolyA
<400> 13
   aataaaatat ctttattttc attacatctg tgtgttggtt ttttgtgtga atcgatagta        60
   ctaacatacg ctctccatca aaacaaaacg aaacaaaaca aactagcaaa ataggctgtc       120
   cccagtgcaa gtgcaggtgc cagaacattt ctct                                   154
<210> 14
   <211> 165994
   <212> DNA
   <213> artificial sequence
<220>
   <223> SB-1 genome HQ840738.1
<400> 14
<210> 15
   <211> 22
   <212> DNA
   <213> artificial sequence
<220>
   <223> MB080 primer
<400> 15
   cgaacaaact tcatcgctat gc 22
<210> 16
   <211> 22
   <212> DNA
   <213> artificial sequence
<220>
   <223> MB081 primer
<400> 16
   taactcaaat gcgaagcgtt gc 22
<210> 17
   <211> 21
   <212> DNA
   <213> artificial sequence
<220>
   <223> SB-1 US10 primer
<400> 17
   tcaacgtgcg acaatcgtct g 21
<210> 18
   <211> 21
   <212> DNA
   <213> artificial sequence
<220>
   <223> SB-1 SORF4 primer
<400> 18
   atgtggagga acgatcctat a 21
<210> 19
   <211> 18
   <212> DNA
   <213> artificial sequence
<220>
   <223> ALLNDVFprimer
<400> 19
   atggcttggg aataatac 18
<210> 20
   <211> 18
   <212> DNA
   <213> artificial sequence
<220>
   <223> mCMVF primer
<400> 20
   aactccgccc gttttatg 18
<210> 21
   <211> 18
   <212> DNA
   <213> artificial sequence
<220>
   <223> SV40tailR primer
<400> 21
   tcgactctag aggatccg 18
<210> 22
   <211> 21
   <212> DNA
   <213> artificial sequence
<220>
   <223> newSB-1 UL55R primer
<400> 22
   atggctatag agggactgtg t 21
<210> 23
   <211> 21
   <212> DNA
   <213> artificial sequence
<220>
   <223> New SB-1 ORF5F primer
<400> 23
   gatctcaacg ctataccggc g 21
<210> 24
   <211> 21
   <212> DNA
   <213> artificial sequence
<220>
   <223> OptF primer
<400> 24
   actgacaaca ccctacatgg c 21
<210> 25
   <211> 19
   <212> DNA
   <213> artificial sequence
<220>
   <223> VIIoptF RP primer
<400> 25
   gccagcacca ggctcaggg 19
<210> 26
   <211> 18
   <212> DNA
   <213> artificial sequence
<220>
   <223> SV40promoterF primer
<400> 26
   agcttggctg tggaatgt 18
<210> 27
   <211> 21
   <212> DNA
   <213> artificial sequence
<220>
   <223> SB1 43.F primer
<400> 27
   gctctcggag acgcggctcg c 21
<210> 28
   <211> 21
   <212> DNA
   <213> artificial sequence
<220>
   <223> SB1 45.R primer
<400> 28
   gctcttgtaa catcgcggac g 21
<210> 29
   <211> 18
   <212> DNA
   <213> artificial sequence
<220>
   <223> SV40 promoter F primer
<400> 29
   agcttggctg tggaatgt 18
<210> 30
   <211> 20
   <212> DNA
   <213> artificial sequence
<220>
   <223> HVTUS10 FP primer
<400> 30
   ccggcaacat acataatgtg 20
<210> 31
   <211> 18
   <212> DNA
   <213> artificial sequence
<220>
   <223> HVTUS10 RP primer
<400> 31
   ggcactatcc 18
<210> 32
   <211> 19
   <212> DNA
   <213> artificial sequence
<220>
   <223> CaoptF RP primer
<400> 32
   gccagcacca ggctcatca 19
<210> 33
   <211> 18
   <212> DNA
   <213> artificial sequence
<220>
   <223> SynTailR primer
<400> 33
   atgttctggc acctgcac 18
<210> 34
   <211> 1437
   <212> DNA
   <213> artificial sequence
<220>
   <223> DNA sequence encoding glycoprotein C of SB-1 strain with GenBank accession No. HQ840738
<400> 34
   atgcacgcgt cacgcgcgtt gcgagctttg gggtggacga gactcttatt tgtcgtttta      60
   ttttcgggcc gcgtcctaag cgctagcatt aaccccgatc tagctacacc cccggtcatt    120
   gctttcaacc cgtcaagtat tccggccgat gatgggcctt tggccaaagt tcctgcatcc     180
   ccgccggcag gggagaaaga ggagagccac aagaatgcaa gcgacgcgcg taggatgcct     240
   agtatagttt gcgataaaga agaagttttc gttttcctga acaagaccgg gcgtttcgtg     300
   tgcactctta agatcgcccc tccctccgac aacgaatggt cgaactttgc tctggacctt      360
   attttcaatc cgatcgaata ccatgctaat gagaagaacg tggaagcagc gcgtattgct      420
   ggcctctatg gggtgcccgg atcagattac gcctacccgc gtccttctga attaatctct      480
   tctattcggc gagaccccca agggaccttt tggacaagcc catcggcaca tggagacaag      540
   tacttcatat ggctaaacaa aacgacgaat acgatgggcg tggaaattag gaacgtcgac      600
   tacgcagaca acggttacat ccaagttgcc atgcgggatc ctttcaatcg gcctttacta      660
   gataagcacg tgtacatccg cgtgtgtcaa cgacccgcct cggtcgacgt tctagccccc      720
   cccgtcctca gtggcgataa gtacaaggct tcatgcatcg ttaggcattt ttatccaccg      780
   ggctccgtct atgtgttctg gaggcaagat gggaatatcg ttacaccacg taaggacacg      840
   gacggaagtt tttggtggtt tgaatcagcc cggggagcca ccctggtatc tacgataacg      900
   ctgggcaact cggccatcga ccctcctccc aagatttcat gtctggtagc ctggaagcag      960
   ggaaatatga tgagtactac gaacgccact gcaatcccga ccgtatatca tcatccccgg     1020
   atatccctgg ctttcaaaga tgggtatgca atatgtacta cgcaatgtgt gccgttcgga     1080
   attaccatac gatggttagt acacgatgaa cccaaaccta atacaactta tgatactgtg     1140
   gttacaggtc tttgcaggac cctcaagcgg catagaaata tcatcagccg aatattactc     1200
   caagatgact ggcagaaaac aaagtataca tgtcgtctca tcggctatcc tttcgacgaa     1260
   gacaaatttc aagctttcga ttacttcgac gcgacgccat cgacgagggg gtcccccatg     1320
   gttctcgcga tagcggctgt tgtgggacta gctttgattt tgggaatggg tacactcctg     1380
   acggctctgt gtttctacgc ctccgggaaa aaatacatat tactttcgtc cgtctag        1437
<210> 35
   <211> 478
   <212> PRT
   <213> artificial sequence
<220>
   <223> glycoportein C of SB-1 strain with GenBank accession No. AEI00252
<400> 35
   Met His Ala Ser Arg Ala Leu Arg Ala Leu Gly Trp Thr Arg Leu Leu
   1               5                   10                  15
   Phe Val Val Leu Phe Ser Gly Arg Val Leu Ser Ala Ser Ile Asn Pro
               20                  25                  30
   Asp Leu Ala Thr Pro Pro Val Ile Ala Phe Asn Pro Ser Ser Ile Pro
           35                  40                  45
   Ala Asp Asp Gly Pro Leu Ala Lys Val Pro Ala Ser Pro Pro Ala Gly
       50                  55                  60
   Glu Lys Glu Glu Ser His Lys Asn Ala Ser Asp Ala Arg Arg Met Pro
   65 70 75 80
   Ser Ile Val Cys Asp Lys Glu Glu Val Phe Val Phe Leu Asn Lys Thr
   85 90 95
   Gly Arg Phe Val Cys Thr Leu Lys Ile Ala Pro Pro Ser Asp Asn Glu
   100 105 110
   Trp Ser Asn Phe Ala Leu Asp Leu Ile Phe Asn Pro Ile Glu Tyr His
   115 120 125
   Ala Asn Glu Lys Asn Val Glu Ala Ala Arg Ile Ala Gly Leu Tyr Gly
   130 135 140
   Val Pro Gly Ser Asp Tyr Ala Tyr Pro Arg Pro Ser Glu Leu Ile Ser
   145 150 155 160
   Ser Ile Arg Arg Asp Pro Gln Gly Thr Phe Trp Thr Ser Pro Ser Ala
   165 170 175
   His Gly Asp Lys Tyr Phe Ile Trp Leu Asn Lys Thr Thr Asn Thr Met
   180 185 190
   Gly Val Glu Ile Arg Asn Val Asp Tyr Ala Asp Asn Gly Tyr Ile Gln
   195 200 205
   Val Ala Met Arg Asp Pro Phe Asn Arg Pro Leu Leu Asp Lys His Val
   210 215 220
   Tyr Ile Arg Val Cys Gln Arg Pro Ala Ser Val Asp Val Leu Ala Pro
   225 230 235 240
   Pro Val Leu Ser Gly Asp Lys Tyr Lys Ala Ser Cys Ile Val Arg His
   245 250 255
   Phe Tyr Pro Pro Gly Ser Val Tyr Val Phe Trp Arg Gln Asp Gly Asn
   260 265 270
   Ile Val Thr Pro Arg Lys Asp Thr Asp Gly Ser Phe Trp Trp Phe Glu
   275 280 285
   Ser Ala Arg Gly Ala Thr Leu Val Ser Thr Ile Thr Leu Gly Asn Ser
   290 295 300
   Ala Ile Asp Pro Pro Pro Lys Ile Ser Cys Leu Val Ala Trp Lys Gln
   305                 310                 315                 320
   Gly Asn Met Met Ser Thr Thr Asn Ala Thr Ala Ile Pro Thr Val Tyr
                   325                 330                 335
   His His Pro Arg Ile Ser Leu Ala Phe Lys Asp Gly Tyr Ala Ile Cys
               340                 345                 350
   Thr Thr Gln Cys Val Pro Phe Gly Ile Thr Ile Arg Trp Leu Val His
           355                 360                 365
   Asp Glu Pro Lys Pro Asn Thr Thr Tyr Asp Thr Val Val Thr Gly Leu
       370                 375                 380
   Cys Arg Thr Leu Lys Arg His Arg Asn Ile Ile Ser Arg Ile Leu Leu
   385                 390                 395                 400
   Gln Asp Asp Trp Gln Lys Thr Lys Tyr Thr Cys Arg Leu Ile Gly Tyr
                   405                 410                 415
   Pro Phe Asp Glu Asp Lys Phe Gln Ala Phe Asp Tyr Phe Asp Ala Thr
               420                 425                 430
   Pro Ser Thr Arg Gly Ser Pro Met Val Leu Ala Ile Ala Ala Val Val
           435                 440                 445
   Gly Leu Ala Leu Ile Leu Gly Met Gly Thr Leu Leu Thr Ala Leu Cys
       450                 455                 460
   Phe Tyr Ala Ser Gly Lys Lys Tyr Ile Leu Leu Ser Ser Val
   465                 470                 475
<210> 36
   <211> 4734
   <212> DNA
   <213> artificial sequence
<220>
   <223> plasmid pSB1 44cds for gC deletion
<400> 36
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgaatt cgagctcggt acctcgcgaa 420
   tgcatctaga tgccaatgaa ttcgcgatag cttcgattag aattttcgca tgagtaaagt 480
   cgacaagctt gctgttatgt cgggagcacg tatctagggc ttctagctcg gtagcgttgc 540
   tccccatcgc gcgcatacaa ctatagccta aaccgatcca ttcgtgagga catgtaccgg 600
   actccaggga catgtaagag aatgcaaaca gcgaagctag cacgaccgta gctgtgatga 660
   cgaacacagt aagagcgatc ccgatcgtac aaccgcataa caaccggggg catcctgaac 720
   gcgaacgttt gtgtcgctca gatgtggtct cgtccatcat ccggagcagc ccacgtactg 780
   cgaccaagtc gcggtcgtct tcgggagtag gggacatcat acttacatat ttaaggaccg 840
   agtagctata tagagaccgt atttcgcgat tgtacggtac tcgcagtcat attcgatttc 900
   taaaattgtt accgacgtat tcgtcggtag gcgcgcagcc acttttcaac ggacccgcgg 960
   ggtccgacaa ccttatcccg caaacgacgc ttccgttaaa ctctgttcgt gcgtggctgg 1020
   ggtcgcgtgt acctcgtccc ttagctttcc agccgtggaa caaagaaggc ctaggttcac 1080
   tgacacttat ctactctaga ggtttgcttt ttaggcgctg ccgagggaat aattccggtc 1140
   caaacgaagt tcgagagaac ctgtgtagtg tacgttattt agtctgtgcc gtgtggaaaa 1200
   ggatctacct ttttttagaa ataccgtact gctagagggt tacatactat tcctcatcac 1260
   ctgaggagtc taaagctgaa gttttccggt ggatatatgc cctaccgact gtaagcaggg 1320
   ccggatgtac ctatacggtt gacatgccgc ttttgcattt gagatgtgcc tactacggaa 1380
   atcgcgtccg cgatgttaca agagctcggg gcatataatg agccagatca atgtcaccgc 1440
   aaacctgcag ggatatcggg ccccctgcag gggggaaaat atttcagctg gcgtgcgagc 1500
   cgcgtctccg agagcgtata tggagtggcc tctgcctgcg caacttagct ttaaatcggc 1560
   cctttcatgt tttcgacagc gcataaaccc acgataggcc cctttagtag ccacgaaagt 1620
   tttattcgca gttcgaaaga acgctatgag cacatgatat aaaaattaat cgctccgcga 1680
   tcaggaatat tgtattaaca cgcaaaccaa catgctcgag atggcagttc ctaatatgca 1740
   tttaccagct gcggcggccg ccaattgctt tattccgcta ttcgcgttgc gtacatttat 1800
   acaagtgtag cagcacagaa ccgcggctgt cgcggaaaac aatgtcggcc cgagaagtaa 1860
   accgaagccg gctaaggtag tagccattgc cccgaatatt aacagtaata caagaattgg 1920
   acggaacaga acctcgtggt agcgcgacgg aaggaatatc gcgacacttg tacccaccaa 1980
   gaaaccgcat atactgcgta acgtgtcagt caatgttgac tggtgcttag ttgcgttcga 2040
   catgtacttt tgaaagctgg ctatggctgg aatcgagatg accgctatga ggctcagaat 2100
   tatcgtagga atattccccc ccagcgtatc gtaactggat acgtcttctt cgcgcgtcgc 2160
   ctcggcgtcc gtgctgggcg ggagacgcgc gcgaccgagg cgatatgcac cgttcacagt 2220
   aatttcccga cgtgcacggt agtagcacgt gttcattaga gaccgactgg cgtctctgac 2280
   cacgtgaata gctaacgccg tatacgccga aagaaagccg gctagaggag aaatgtctcc 2340
   atacggagcc gtgatgccgg caatcgtccc tgccgccaca actaaaaacc cggtcttcca 2400
   cgtcacgtct ccagacgttg ctatgcagac gtaatgataa gatgcgatca gagctccgag 2460
   catgacaaaa gcccgactgc agaggcctgc atgcaagctt ggcgtaatca tggtcatagc 2520
   tgtttcctgt gtgaaattgt tatccgctca caattccaca caacatacga gccggaagca 2580
   taaagtgtaa agcctggggt gcctaatgag tgagctaact cacattaatt gcgttgcgct 2640
   cactgcccgc tttccagtcg ggaaacctgt cgtgccagct gcattaatga atcggccaac 2700
   gcgcggggag aggcggtttg cgtattgggc gctcttccgc ttcctcgctc actgactcgc 2760
   tgcgctcggt cgttcggctg cggcgagcgg tatcagctca ctcaaaggcg gtaatacggt 2820
   tatccacaga atcaggggat aacgcaggaa agaacatgtg agcaaaaggc cagcaaaagg 2880
   ccaggaaccg taaaaaggcc gcgttgctgg cgtttttcca taggctccgc ccccctgacg 2940
   agcatcacaa aaatcgacgc tcaagtcaga ggtggcgaaa cccgacagga ctataaagat 3000
   accaggcgtt tccccctgga agctccctcg tgcgctctcc tgttccgacc ctgccgctta 3060
   ccggatacct gtccgccttt ctcccttcgg gaagcgtggc gctttctcat agctcacgct 3120
   gtaggtatct cagttcggtg taggtcgttc gctccaagct gggctgtgtg cacgaacccc 3180
   ccgttcagcc cgaccgctgc gccttatccg gtaactatcg tcttgagtcc aacccggtaa 3240
   gacacgactt atcgccactg gcagcagcca ctggtaacag gattagcaga gcgaggtatg 3300
   taggcggtgc tacagagttc ttgaagtggt ggcctaacta cggctacact agaagaacag 3360
   tatttggtat ctgcgctctg ctgaagccag ttaccttcgg aaaaagagtt ggtagctctt 3420
   gatccggcaa acaaaccacc gctggtagcg gtggtttttt tgtttgcaag cagcagatta 3480
   cgcgcagaaa aaaaggatct caagaagatc ctttgatctt ttctacgggg tctgacgctc 3540
   agtggaacga aaactcacgt taagggattt tggtcatgag attatcaaaa aggatcttca 3600
   cctagatcct tttaaattaa aaatgaagtt ttaaatcaat ctaaagtata tatgagtaaa 3660
   cttggtctga cagttaccaa tgcttaatca gtgaggcacc tatctcagcg atctgtctat 3720
   ttcgttcatc catagttgcc tgactccccg tcgtgtagat aactacgata cgggagggct 3780
   taccatctgg ccccagtgct gcaatgatac cgcgagaccc acgctcaccg gctccagatt 3840
   tatcagcaat aaaccagcca gccggaaggg ccgagcgcag aagtggtcct gcaactttat 3900
   ccgcctccat ccagtctatt aattgttgcc gggaagctag agtaagtagt tcgccagtta 3960
   atagtttgcg caacgttgtt gccattgcta caggcatcgt ggtgtcacgc tcgtcgtttg 4020
   gtatggcttc attcagctcc ggttcccaac gatcaaggcg agttacatga tcccccatgt 4080
   tgtgcaaaaa agcggttagc tccttcggtc ctccgatcgt tgtcagaagt aagttggccg 4140
   cagtgttatc actcatggtt atggcagcac tgcataattc tcttactgtc atgccatccg      4200
   taagatgctt ttctgtgact ggtgagtact caaccaagtc attctgagaa tagtgtatgc      4260
   ggcgaccgag ttgctcttgc ccggcgtcaa tacgggataa taccgcgcca catagcagaa      4320
   ctttaaaagt gctcatcatt ggaaaacgtt cttcggggcg aaaactctca aggatcttac      4380
   cgctgttgag atccagttcg atgtaaccca ctcgtgcacc caactgatct tcagcatctt      4440
   ttactttcac cagcgtttct gggtgagcaa aaacaggaag gcaaaatgcc gcaaaaaagg      4500
   gaataagggc gacacggaaa tgttgaatac tcatactctt cctttttcaa tattattgaa      4560
   gcatttatca gggttattgt ctcatgagcg gatacatatt tgaatgtatt tagaaaaata      4620
   aacaaatagg ggttccgcgc acatttcccc gaaaagtgcc acctgacgtc taagaaacca      4680
   ttattatcat gacattaacc tataaaaata ggcgtatcac gaggcccttt cgtc            4734
<210> 37
   <211> 4085
   <212> DNA
   <213> artificial sequence
<220>
   <223> partial plasmid sequence of pSB1 44cds SV FCAopt for vSB1-009
<400> 37
   cttttgtcat gctcggagct ctgatcgcat cttatcatta cgtctgcata gcaacgtctg      60
   gagacgtgac gtggaagacc gggtttttag ttgtggcggc agggacgatt gccggcatca     120
   cggctccgta tggagacatt tctcctctag ccggctttct ttcggcgtat acggcgttag     180
   ctattcacgt ggtcagagac gccagtcggt ctctaatgaa cacgtgctac taccgtgcac     240
   gtcgggaaat tactgtgaac ggtgcatatc gcctcggtcg cgcgcgtctc ccgcccagca     300
   cggacgccga ggcgacgcgc gaagaagacg tatccagtta cgatacgctg ggggggaata     360
   ttcctacgat aattctgagc ctcatagcgg tcatctcgat tccagccata gccagctttc     420
   aaaagtacat gtcgaacgca actaagcacc agtcaacatt gactgacacg ttacgcagta     480
   tatgcggttt cttggtgggt acaagtgtcg cgatattcct tccgtcgcgc taccacgagg     540
   ttctgttccg tccaattctt gtattactgt taatattcgg ggcaatggct actaccttag     600
   ccggcttcgg tttacttctc gggccgacat tgttttccgc gacagccgcg gttctgtgct     660
   gctacacttg tataaatgta cgcaacgcga atagcggaat aaagcaattg gcggccgccg     720
   cagctggtaa atgcatatta ggaactgcca tctcgagcat gttggtttgc gtgttaatac     780
   aatattcctg atcgcggagc gattaatttt tatatcatgt gctcatagcg ttctttcgaa     840
   ctgcgaataa aactttcgtg gctactaaag gggcctatcg tgggtttatg cgctgtcgaa      900
   aacatgaaag ggccgattta aagctaagtt gcgcaggcag aggccactcc atatacgctc     960
   tcggagacgc ggctcgcacg ccagctgaaa tattttcccc cctgcaggtc gacccaattc 1020
   gagctcggta cagcttggct gtggaatgtg tgtcagttag ggtgtggaaa gtccccaggc 1080
   tccccagcag gcagaagtat gcaaagcatg catctcaatt agtcagcaac caggtgtgga 1140
   aagtccccag gctccccagc aggcagaagt atgcaaagca tgcatctcaa ttagtcagca 1200
   accatagtcc cgcccctaac tccgcccatc ccgcccctaa ctccgcccag ttccgcccat 1260
   tctccgcccc atggctgact aatttttttt atttatgcag aggccgaggc cgcctcggcc 1320
   tctgagctat tccagaagta gtgaggaggc ttttttggag gcctaggctt ttgcaaaaag 1380
   ctcccggggc ggccgccacc atgggcagca agcccagcac ctggatcagc gtgaccctga 1440
   tgctgatcac cagaaccatg ctgatcctga gctgcatctg ccccacaagc agcctggacg 1500
   gcagacccct ggccgctgcc ggcatcgtgg tgaccggcga caaggccgtg aacatctaca 1560
   ccagcagcca gaccggcagc atcatcatca agctgctgcc caacatgccc aaggacaaag 1620
   aggcctgcgc caaggccccc ctggaagcct acaacagaac cctgaccacc ctgctgaccc 1680
   ccctgggcga cagcatcaga agaatccagg gcagcgccac cacaagcggc ggaggaaagc 1740
   agggcagact ggtgggcgct atcatcggga gcgtggccct gggcgtggcc acagctgccc 1800
   agattaccgc tgcagccgcc ctgattcagg ccaatcagaa cgccgccaac atcctgagac 1860
   tgaaagagag cattgccgcc accaacgacg ccgtgcacga agtgacaaac ggactgtccc 1920
   agctggctgt cgctgtcggc aagatgcagc agttcgtgaa caaccagttc aacaacaccg 1980
   ccagagagct ggactgcatc aagatcgccc agcaggtggg cgtggagctg aacctgtacc 2040
   tgaccgagct gaccacagtg ttcggccccc agatcacaag ccccgctctg acccagctga 2100
   caatccaggc cctgtacaac ctggctggcg gcaacatgga ctatctgctg actaagctgg 2160
   gagtgggcaa caaccagctg tccagcctga tcgggtccgg gctgatcaca ggcaacccca 2220
   tcctgtacga cagccagaca cagctgctgg gcatccagat caacctgcca tccgtgggaa 2280
   gcctgaacaa catgagagcc acctacctgg aaaccctgag cgtgtccacc accaagggct 2340
   tcgccagcgc cctggtgccc aaggtggtga cacaggtggg cagcgtgatc gaggaactgg 2400
   acaccagcta ctgcatcgag agcgacatcg acctgtactg caccagagtg gtgaccttcc 2460
   caatgagccc cggcatctac agctgcctga gcggcaacac cagcgcctgc atgtacagca 2520
   agaccgaagg agcactgaca acaccctaca tggccctgaa gggaagcgtg atcgccaact 2580
   gcaagatgac cacctgcaga tgcgccgacc ccccaggcat catcagccag aactacggcg 2640
   aggccgtgag cctgatcgac aaacattcct gtagcgtgct gtccctggat ggcatcacac 2700
   tgagactgag cggcgagttc gacgccacct accagaagaa catcagcatc ctggacagcc 2760
   aggtgatcgt gaccggcaac ctggacatca gcaccgagct gggcaacgtg aacaacagca 2820
   tcagcagcac cctggacaag ctggccgagt ccaacaacaa gctgaacaaa gtgaacgtga 2880
   acctgaccag cacaagcgcc ctgatcacct acatcgtgct ggccatcgtg tccctggcct    2940
   tcggcgtgat cagcctggtg ctggcctgct acctgatgta caagcagaga gcccagcaga    3000
   aaaccctgct gtggctgggc aataacaccc tggaccagat gagggccacc accagaacct    3060
   gatgagcggc cgcgatacct gcaggtttgc ggtgacattg atctggctca ttatatgccc    3120
   cgagctcttg taacatcgcg gacgcgattt ccgtagtagg cacatctcaa atgcaaaagc    3180
   ggcatgtcaa ccgtataggt acatccggcc ctgcttacag tcggtagggc atatatccac    3240
   cggaaaactt cagctttaga ctcctcaggt gatgaggaat agtatgtaac cctctagcag    3300
   tacggtattt ctaaaaaaag gtagatcctt ttccacacgg cacagactaa ataacgtaca    3360
   ctacacaggt tctctcgaac ttcgtttgga ccggaattat tccctcggca gcgcctaaaa    3420
   agcaaacctc tagagtagat aagtgtcagt gaacctaggc cttctttgtt ccacggctgg    3480
   aaagctaagg gacgaggtac acgcgacccc agccacgcac gaacagagtt taacggaagc    3540
   gtcgtttgcg ggataaggtt gtcggacccc gcgggtccgt tgaaaagtgg ctgcgcgcct    3600
   accgacgaat acgtcggtaa caattttaga aatcgaatat gactgcgagt accgtacaat    3660
   cgcgaaatac ggtctctata tagctactcg gtccttaaat atgtaagtat gatgtcccct    3720
   actcccgaag acgaccgcga cttggtcgca gtacgtgggc tgctccggat gatggacgag    3780
   accacatctg agcgacacaa acgttcgcgt tcaggatgcc cccggttgtt atgcggttgt    3840
   acgatcggga tcgctcttac tgtgttcgtc atcacagcta cggtcgtgct agcttcgctg    3900
   tttgcattct cttacatgtc cctggagtcc ggtacatgtc ctcacgaatg gatcggttta    3960
   ggctatagtt gtatgcgcgc gatggggagc aacgctaccg agctagaagc cctagatacg    4020
   tgctcccgac ataacagcaa gcttgtcgac tttactcatg cgaaaattct aatcgaagct    4080
   atcgc                                                                4085
<210> 38
   <211> 4344
   <212> DNA
   <213> artificial sequence
<220>
   <223> partial plasmid sequence of pHM103+Fopt for vHVT114
<400> 38
   gagctcaggg tatgatactc agctgttatt gtggccgacc aggaggactc caatgcttag      60
   cattcataag aacgctagag atgctattta acgatgtgct gtcgtctaaa gaatttgtgc     120
   atttagcctt taaatgtaaa accaatgacg cattcactac gctcgtgcgt gcaatttctg     180
   ggccagggta tgcatattcc ataacagaaa tcgacacttg agaagaggat ctgactgttt     240
   gggataaagg tcgtttgggt ctgtcctagc gatataattt atatgacgat atacattaaa     300
   catctgtgtg cagtacttag gtatttaatc atgtcgatga aatgttatgt gtaaatatcg 360
   gacaatatag ataacgggca cgctgctatt gtaacgtgcg cccgcgcgct agtgctgact 420
   aatagtgtgg atgatgtata cagtatatta caaacggaaa tgatacgtaa taaattatgt 480
   actcttattg atttataaaa acatacatgc agtgttgcta tgtcacataa ttagcctcgc 540
   ccgtctacgc tccactgaag ataatgggct cccgctgttc aaaaaaatca gcgtgcgtcg 600
   ataagacttt ggtgcagtct cttcggggtc gcaatttaga tttgccgcat ggagggtatc 660
   tggggatttt tgccaatgct ggagcgacga ctgtacgatt cgtcccatcg ggatctagca 720
   gaccaatgat gttgacacac atcggccatg catgtacgga cggtctattg cgcgagtttg 780
   ttattttcga aggacaagat ggaagtgtat atggaaccga caataatgtt agtttgcatt 840
   tcttagggcg gaatctacat gatatcttat ccaagcgggg tatgagccag agagatgtga 900
   tggtcataaa gggtaaattt tttagatctg aaataacgca gttgcccaaa caacgatcgc 960
   gattaaaaga aaaatcggat ggttcaatta ggacatgcat ggattctgtg cgcataaacc 1020
   ataaccgcag cactgttggg cacttcggta actcaaatgc gaagcgttgc acgtctgcga 1080
   taactacgcc tactatgcac attgttactc ctgcatctta aaaatatatc ctgtagtaat 1140
   tttcacagca atgtcataac atcatctcgc taaagaatga cctgggattg gagaagtaat 1200
   gaatatttgc aaccaatgca ttgaataaac taacattaaa cgaattcgag ctcggtacag 1260
   cttggctgtg gaatgtgtgt cagttagggt gtggaaagtc cccaggctcc ccagcaggca 1320
   gaagtatgca aagcatgcat ctcaattagt cagcaaccag gtgtggaaag tccccaggct 1380
   ccccagcagg cagaagtatg caaagcatgc atctcaatta gtcagcaacc atagtcccgc 1440
   ccctaactcc gcccatcccg cccctaactc cgcccagttc cgcccattct ccgccccatg 1500
   gctgactaat tttttttatt tatgcagagg ccgaggccgc ctcggcctct gagctattcc 1560
   agaagtagtg aggaggcttt tttggaggcc taggcttttg caaaaagctg cggccgccac 1620
   catgggcagc aagcccagca caagaatccc agcccccctg atgctgatca cccgcatcat 1680
   gctgatcctg ggctgcatca gacccacaag ctccctggat ggacgccccc tggccgctgc 1740
   cggcatcgtg gtgaccggcg acaaggccgt gaacgtgtac accagcagcc agaccggcag 1800
   catcatcgtg aagctgctgc ccaacatgcc cagagacaaa gaggcctgcg ccaaggcccc 1860
   cctggaagcc tacaacagaa ccctgaccac cctgctgacc cccctgggcg acagcatcag 1920
   aaagatccag ggctccgtga gcacaagcgg cggaggaaag cagggcagac tgatcggcgc 1980
   cgtgatcggc agcgtggccc tgggagtggc tacagctgcc cagattaccg ctgcagccgc 2040
   cctgatccag gccaaccaga acgccgccaa catcctgaga ctgaaagaga gcattgccgc 2100
   caccaacgag gccgtgcacg aagtgaccga cggcctgagc cagctgtccg tggccgtggg 2160
   caagatgcag cagttcgtga acgaccagtt caacaacacc gccagagagc tggactgcat 2220
   caagatcacc cagcaggtgg gcgtggagct gaacctgtac ctgaccgagc tgaccacagt 2280
   gttcggcccc cagatcacaa gcccagccct gacacagctg accatccagg ccctgtacaa 2340
   cctggctggc ggcaacatgg actatctgct gacaaagctg ggaatcggca acaaccagct 2400
   gtccagcctg atcggaagcg gcctgatcac cggctacccc atcctgtacg acagccagac 2460
   acagctgctg ggcatccagg tgaacctgcc cagcgtgggc aacctgaaca acatgcgcgc 2520
   cacctacctg gaaaccctga gcgtgtccac caccaagggc tacgccagcg ccctggtgcc 2580
   caaggtggtg acacaggtgg gcagcgtgat cgaggaactg gacaccagct actgcatcga 2640
   gagcgacctg gacctgtact gcaccagaat cgtgaccttc ccaatgagcc ccggcatcta 2700
   cagctgcctg agcggcaaca ccagcgcctg catgtacagc aagaccgaag gcgcactgac 2760
   aacaccctac atggccctga agggaagcgt gatcgccaac tgcaagatca ccacctgcag 2820
   atgcaccgac cccccaggca tcatcagcca gaactacggc gaggccgtga gcctgatcga 2880
   tcgccattcc tgtaacgtgc tgtccctgga cggcatcaca ctgagactga gcggcgagtt 2940
   cgatgccacc taccagaaga acatcagcat cctggacagc caggtgatcg tgaccggcaa 3000
   cctggacatc agcaccgagc tgggcaacgt gaataacagc atcagcaacg ccctggacag 3060
   actggccgag agcaacagca agctggaaaa agtgaacgtg cgcctgacat ccacttccgc 3120
   tctgatcacc tacatcgtgc tgaccgtgat cagcctggtg ttcggcgccc tgagcctggt 3180
   gctggcctgc tacctgatgt acaagcagaa ggcccagcag aaaaccctgc tgtggctggg 3240
   caacaacacc ctggaccaga tgagagccac caccagagcc tgatgagcgg ccgcggggat 3300
   ccagacatga taagatacat tgatgagttt ggacaaacca caactagaat gcagtgaaaa 3360
   aaatgcttta tttgtgaaat ttgtgatgct attgctttat ttgtaaccat tataagctgc 3420
   aataaacaag ttaacaacaa caattgcatt gattttatgt ttcaggttca gggggaggtg 3480
   tgggaggttt tttcggatcc tctagagtcg acaattattt tatttaataa catatagccc 3540
   aaagacctct atgaacattt agtttcccgt atactcaacg gcgcgtgtac acacgcatct 3600
   ctttgcatag cgatgaagtt tgttcggcag cagaaaatgc agatatccaa caatctggag 3660
   aaaacttatc atcacagtgg cagtggaaac ataccccctc tatattcatg gtataattat 3720
   cgtctacagc gtccaggata gtggcgtgag aaaatggaga tctgcagccc tcctttccat 3780
   ggcatgccgc tttattgttc attaaacgca caatggtctc aacgccagat atgggcatag 3840
   attctgaaga acccgttgac aatccgaaga agaaggcgtg caggtctttg gaagactcgc 3900
   acgttggtct tataatgtat gatcgagatg tcaccctaat gccacatggt acaggcttat 3960
   cgcggtcatg gcgatcggac ttgtaatttg caacgatggg caaaggatcg acgacatgcc 4020
   aaacattctg aacccgtaga gatgttaacg atgacgagga tgaatatccc atgctcgctg 4080
   ccatagtatc aagtacaccg cgaataagga cgcgtccaac atcgttatat gcacacaatg 4140
   ggctacacgt gactaacacc cccgaatatt agtcatatgt gagtttcagt ctggctccca 4200
   tatagcctgt agactatttg tggtttaagt gtgaacgagg cgctgtgaac gagactcggg 4260
   ccgattgtaa gaacaagcaa atgcactttc catttaacaa gaagtgtaga gagaatactc 4320
   aacctctttg gatgtatcct cgag 4344
<210> 39
   <211> 1362
   <212> DNA
   <213> artificial sequence
<220>
   <223> IBDV DNA encoding VP2 protein
<400> 39
   atgacaaacc tgcaagatca aacccaacag attgttccgt tcatacggag ccttctgatg 60
   ccaacaaccg gaccggcgtc cattccggac gacaccctgg agaagcacac tctcaggtca 120
   gagacctcga cctacaattt gactgtgggg gacacagggt cagggctaat tgtctttttc 180
   cctggattcc ctggctcaat tgtgggtgct cactacacac tgcagagcaa tgggaactac 240
   aagttcgatc agatgctcct gactgcccag aacctaccgg ccagctacaa ctactgcaga 300
   ctagtgagtc ggagtctcac agtgaggtca agcacactcc ctggtggcgt ttatgcacta 360
   aacggcacca taaacgccgt gaccttccaa ggaagcctga gtgaactgac agatgttagc 420
   tacaatgggt tgatgtctgc aacagccaac atcaacgaca aaattgggaa tgtcctggta 480
   ggggaagggg tcactgtcct cagcctaccc acatcatatg atcttgggta tgtgaggctt 540
   ggtgacccca ttcccgctat agggcttgac ccaaaaatgg tagctacatg cgacagcagt 600
   gacaggccca gagtctacac cataactgca gccgatgatt accaattctc atcacagtac 660
   caaccaggtg gggtaacaat cacactgttc tcagccaaca ttgatgctat cacaagcctc 720
   agcattgggg gagagctcgt gtttcaaaca agcgtccaag gccttgtact gggcgccacc 780
   atctacctta taggctttga tgggactgcg gtaatcacca gagctgtagc cgcagataat 840
   gggctgacgg ccggcaccga caatcttatg ccattcaatc ttgtcattcc aaccaatgag 900
   ataacccagc caatcacatc catcaaactg gagatagtga cctccaaaag tggtggtcag 960
   gcaggggatc agatgtcatg gtcggcaagt gggagcctag cagtgacgat ccatggtggc 1020
   aactatccag gggccctccg tcccgtcaca ctagtagcct acgaaagagt ggcaacagga 1080
   tccgtcgtta cggtcgctgg ggtgagtaac ttcgagctga ttccaaatcc tgaactagca 1140
   aagaacctgg ttacagaata cggccgattt gacccaggag ccatgaacta cacaaaattg 1200
   atactgagtg agagggaccg tcttggcatc aagaccgtct ggccaacaag ggagtacact 1260
   gattttcgtg agtacttcat ggaggtggcc gacctcaact ctcccctgaa gattgcagga 1320
   gcatttggct tcaaagacat aatccgggct ataaggaggt aa 1362
<210> 40
   <211> 453
   <212> PRT
   <213> artificial sequence
<220>
   <223> IBDV VP2 protein
<400> 40
   Met Thr Asn Leu Gln Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Ser Asn Gly Asn Tyr
   65                  70                  75                  80
   Lys Phe Asp Gln Met Leu Leu Thr Ala Gln Asn Leu Pro Ala Ser Tyr
                   85                  90                  95
   Asn Tyr Cys Arg Leu Val Ser Arg Ser Leu Thr Val Arg Ser Ser Thr
               100                 105                 110
   Leu Pro Gly Gly Val Tyr Ala Leu Asn Gly Thr Ile Asn Ala Val Thr
           115                 120                 125
   Phe Gln Gly Ser Leu Ser Glu Leu Thr Asp Val Ser Tyr Asn Gly Leu
       130                 135                 140
   Met Ser Ala Thr Ala Asn Ile Asn Asp Lys Ile Gly Asn Val Leu Val
   145                 150                 155                 160
   Gly Glu Gly Val Thr Val Leu Ser Leu Pro Thr Ser Tyr Asp Leu Gly
                   165                 170                 175
   Tyr Val Arg Leu Gly Asp Pro Ile Pro Ala Ile Gly Leu Asp Pro Lys
               180                 185                 190
   Met Val Ala Thr Cys Asp Ser Ser Asp Arg Pro Arg Val Tyr Thr Ile
           195                 200                 205
   Thr Ala Ala Asp Asp Tyr Gln Phe Ser Ser Gln Tyr Gln Pro Gly Gly
       210                 215                 220
   Val Thr Ile Thr Leu Phe Ser Ala Asn Ile Asp Ala Ile Thr Ser Leu
   225                 230                 235                 240
   Ser Ile Gly Gly Glu Leu Val Phe Gln Thr Ser Val Gln Gly Leu Val
                   245                 250                 255
   Leu Gly Ala Thr Ile Tyr Leu Ile Gly Phe Asp Gly Thr Ala Val Ile
               260                 265                 270
   Thr Arg Ala Val Ala Ala Asp Asn Gly Leu Thr Ala Gly Thr Asp Asn
           275                 280                 285
   Leu Met Pro Phe Asn Leu Val Ile Pro Thr Asn Glu Ile Thr Gln Pro
       290                 295                 300
   Ile Thr Ser Ile Lys Leu Glu Ile Val Thr Ser Lys Ser Gly Gly Gln
   305                 310                 315                 320
   Ala Gly Asp Gln Met Ser Trp Ser Ala Ser Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg
   450
<210> 41
   <211> 5178
   <212> DNA
   <213> artificial sequence
<220>
   <223> partial plasmid sequence of SB-1 US10mFwt SbfI for vSB1-004
<400> 41
<210> 42
   <211> 4226
   <212> DNA
   <213> artificial sequence
<220>
   <223> partial plasmid sequence of SB1 UL55 SVFopt syn tail SbfI for vSB1-006
<400> 42
   gtcctcagcc ttgcgctagt aagctgtaat acagagaacg gggcacgaac gtgcatcgaa      60
   acattatttt cctttggagg tgccgtgctg gaaacaaaca ttctgattca tgcaattaac     120
   caactgcttc ctttgtgcga tgtcatggcg gtttccggaa taagtagact tgctatcata     180
   gaatcaatcc ccgaactcgg aacggttcca tacaggtgcg ttttacaagc gacgcctcat     240
   attacagcgt gtctcacccc caaggtagtc agatgcatga attatgggat actactatct     300
   cactgggagg aatgcgtcgt gcgggattcg aaatacatcg taaacgtacc cacgcaaaca     360
   cagtttgtca catccttaac ccgttgtccg cacgggccag tcgccaacga ttggtacctt     420
   ggattcttct tcccatttaa ggggttcagg gtaataaccg tcagaacaag gcgctggctg     480
   gcagcatata cctacagatt tcgaaagtgg ttgcaatggg aagagggttc cccccactac     540
   gtcgctttga ggcgtctggt cccactatgt gattgctact tgatggatgc ttgcatgaca     600
   aacaactttt ttgcctgcgg aatgctattc cacttacact gcgtcccaat acccgcgcgc     660
   gaaaaacgca ttgtggcgat actggcgaga gcgattgatg acgcgcaaac atatgcggga     720
   acctcactgc acgtagatac gagtgaacag tagagcgtta gtatgtggtt ttctttaggt     780
   gttactacca cttacattcc tacaaaatca ctactcccgt attagatctc aacgctatac     840
   cggcgtaggc gaacctgagg gtgcgcccct agctctcgca attggtatgc gtaaatgaat     900
   tgttatcgcg ggttgccata agattttgta tacatacgaa cccgagaatg tataaaccaa     960
   taaatgctga aatggtagac ctgcaggtcg acccaattcg agctcggtac agcttggctg    1020
   tggaatgtgt gtcagttagg gtgtggaaag tccccaggct ccccagcagg cagaagtatg    1080
   caaagcatgc atctcaatta gtcagcaacc aggtgtggaa agtccccagg ctccccagca    1140
   ggcagaagta tgcaaagcat gcatctcaat tagtcagcaa ccatagtccc gcccctaact    1200
   ccgcccatcc cgcccctaac tccgcccagt tccgcccatt ctccgcccca tggctgacta    1260
   atttttttta tttatgcaga ggccgaggcc gcctcggcct ctgagctatt ccagaagtag    1320
   tgaggaggct tttttggagg cctaggcttt tgcaaaaagc tcccggggcg gccgccacca    1380
   tgggcagcaa gcccagcaca agaatcccag cccccctgat gctgatcacc cgcatcatgc    1440
   tgatcctggg ctgcatcaga cccacaagct ccctggatgg acgccccctg gccgctgccg    1500
   gcatcgtggt gaccggcgac aaggccgtga acgtgtacac cagcagccag accggcagca    1560
   tcatcgtgaa gctgctgccc aacatgccca gagacaaaga ggcctgcgcc aaggcccccc    1620
   tggaagccta caacagaacc ctgaccaccc tgctgacccc cctgggcgac agcatcagaa 1680
   agatccaggg ctccgtgagc acaagcggcg gaggaaagca gggcagactg atcggcgccg 1740
   tgatcggcag cgtggccctg ggagtggcta cagctgccca gattaccgct gcagccgccc 1800
   tgatccaggc caaccagaac gccgccaaca tcctgagact gaaagagagc attgccgcca 1860
   ccaacgaggc cgtgcacgaa gtgaccgacg gcctgagcca gctgtccgtg gccgtgggca 1920
   agatgcagca gttcgtgaac gaccagttca acaacaccgc cagagagctg gactgcatca 1980
   agatcaccca gcaggtgggc gtggagctga acctgtacct gaccgagctg accacagtgt 2040
   tcggccccca gatcacaagc ccagccctga cacagctgac catccaggcc ctgtacaacc 2100
   tggctggcgg caacatggac tatctgctga caaagctggg aatcggcaac aaccagctgt 2160
   ccagcctgat cggaagcggc ctgatcaccg gctaccccat cctgtacgac agccagacac 2220
   agctgctggg catccaggtg aacctgccca gcgtgggcaa cctgaacaac atgcgcgcca 2280
   cctacctgga aaccctgagc gtgtccacca ccaagggcta cgccagcgcc ctggtgccca 2340
   aggtggtgac acaggtgggc agcgtgatcg aggaactgga caccagctac tgcatcgaga 2400
   gcgacctgga cctgtactgc accagaatcg tgaccttccc aatgagcccc ggcatctaca 2460
   gctgcctgag cggcaacacc agcgcctgca tgtacagcaa gaccgaaggc gcactgacaa 2520
   caccctacat ggccctgaag ggaagcgtga tcgccaactg caagatcacc acctgcagat 2580
   gcaccgaccc cccaggcatc atcagccaga actacggcga ggccgtgagc ctgatcgatc 2640
   gccattcctg taacgtgctg tccctggacg gcatcacact gagactgagc ggcgagttcg 2700
   atgccaccta ccagaagaac atcagcatcc tggacagcca ggtgatcgtg accggcaacc 2760
   tggacatcag caccgagctg ggcaacgtga ataacagcat cagcaacgcc ctggacagac 2820
   tggccgagag caacagcaag ctggaaaaag tgaacgtgcg cctgacatcc acttccgctc 2880
   tgatcaccta catcgtgctg accgtgatca gcctggtgtt cggcgccctg agcctggtgc 2940
   tggcctgcta cctgatgtac aagcagaagg cccagcagaa aaccctgctg tggctgggca 3000
   acaacaccct ggaccagatg agagccacca ccagagcctg atgagcggcc gcgatatcaa 3060
   taaaatatct ttattttcat tacatctgtg tgttggtttt ttgtgtgaat cgatagtact 3120
   aacatacgct ctccatcaaa acaaaacgaa acaaaacaaa ctagcaaaat aggctgtccc 3180
   cagtgcaagt gcaggtgcca gaacatttct cttctagacc tgcaggtcaa atcatgattc 3240
   gtttttattc agtgtccgta ctcgcgaata gctctgcact gcgatgcgat tttttcggca 3300
   gcgctaaaag ccgatctcca acactcgagc gaaaacttat tttcgcagtg gcaatgaaaa 3360
   cacagtccct ctatagccat gctgtagttt tcatccagtg agtcgatgat cgtcgcatga 3420
   gaaaaaggcg actcgcattg gtcgcctgtc aatcgaccag tccgtaactt cttgttcatt 3480
   ctatacgcga tatacgaggc accgcattgg gacatgttct cagacggatc cgtcgccaat 3540
   ccaaagaaga acgcgtggaa agtcgtcgac gatttacacg ctgcccgtat gacgtacgaa      3600
   cgaggaataa cggatgcggc acatgacaca tgttcgcccg tagctagttg agtagaggcg      3660
   gacggcacgt caattgctaa cggagcgatc acttgccaaa cgtacggaat tcgtaccggt      3720
   gaagctgacg gttcagctgt actttccgcc attggacact gtagaactga ggatctgcca      3780
   ccgctgggcg cacgtggttt ctgccgggtg ttatataagt ttttccacgc ccactacgtg      3840
   ttacctacac actcacggca cgtggtacgt ggacgtaagg gatggccgta atgaaaaatg      3900
   caccgggcga ctagttgtcc cgtctagcat gaccatgcat tgtagaccgc aggtccagcg      3960
   ctgcgccaat tacgactcta catcggacgc tccaacccag acgggcttcc caatgtggct      4020
   caccccgaga gaactctcac cgactaacgt ttatggaata gtcgctagac cggtactcct      4080
   cacgcacgcc gggggcccaa taaacagtgt actgacggga ggttggaaag ggagaatggt      4140
   cacggcaacg tcgtctatct gcgaaaggat actgctttgg gtagcggata cgcagcgttc      4200
   ttctccgagc accggatcgt ttgacc                                           4226
<210> 43
   <211> 4085
   <212> DNA
   <213> artificial sequence
<220>
   <223> partial plasmid sequence of pSB1 44cds SVOptF for vSB1-007
<400> 43
   gcgatagctt cgattagaat tttcgcatga gtaaagtcga caagcttgct gttatgtcgg      60
   gagcacgtat ctagggcttc tagctcggta gcgttgctcc ccatcgcgcg catacaacta     120
   tagcctaaac cgatccattc gtgaggacat gtaccggact ccagggacat gtaagagaat     180
   gcaaacagcg aagctagcac gaccgtagct gtgatgacga acacagtaag agcgatcccg     240
   atcgtacaac cgcataacaa ccgggggcat cctgaacgcg aacgtttgtg tcgctcagat     300
   gtggtctcgt ccatcatccg gagcagccca cgtactgcga ccaagtcgcg gtcgtcttcg     360
   ggagtagggg acatcatact tacatattta aggaccgagt agctatatag agaccgtatt     420
   tcgcgattgt acggtactcg cagtcatatt cgatttctaa aattgttacc gacgtattcg     480
   tcggtaggcg cgcagccact tttcaacgga cccgcggggt ccgacaacct tatcccgcaa     540
   acgacgcttc cgttaaactc tgttcgtgcg tggctggggt cgcgtgtacc tcgtccctta     600
   gctttccagc cgtggaacaa agaaggccta ggttcactga cacttatcta ctctagaggt     660
   ttgcttttta ggcgctgccg agggaataat tccggtccaa acgaagttcg agagaacctg     720
   tgtagtgtac gttatttagt ctgtgccgtg tggaaaagga tctacctttt tttagaaata     780
   ccgtactgct agagggttac atactattcc tcatcacctg aggagtctaa agctgaagtt     840
   ttccggtgga tatatgccct accgactgta agcagggccg gatgtaccta tacggttgac 900
   atgccgcttt tgcatttgag atgtgcctac tacggaaatc gcgtccgcga tgttacaaga 960
   gctcggggca tataatgagc cagatcaatg tcaccgcaaa cctgcaggtc gacccaattc 1020
   gagctcggta cagcttggct gtggaatgtg tgtcagttag ggtgtggaaa gtccccaggc 1080
   tccccagcag gcagaagtat gcaaagcatg catctcaatt agtcagcaac caggtgtgga 1140
   aagtccccag gctccccagc aggcagaagt atgcaaagca tgcatctcaa ttagtcagca 1200
   accatagtcc cgcccctaac tccgcccatc ccgcccctaa ctccgcccag ttccgcccat 1260
   tctccgcccc atggctgact aatttttttt atttatgcag aggccgaggc cgcctcggcc 1320
   tctgagctat tccagaagta gtgaggaggc ttttttggag gcctaggctt ttgcaaaaag 1380
   ctcccggggc ggccgccacc atgggcagca agcccagcac aagaatccca gcccccctga 1440
   tgctgatcac ccgcatcatg ctgatcctgg gctgcatcag acccacaagc tccctggatg 1500
   gacgccccct ggccgctgcc ggcatcgtgg tgaccggcga caaggccgtg aacgtgtaca 1560
   ccagcagcca gaccggcagc atcatcgtga agctgctgcc caacatgccc agagacaaag 1620
   aggcctgcgc caaggccccc ctggaagcct acaacagaac cctgaccacc ctgctgaccc 1680
   ccctgggcga cagcatcaga aagatccagg gctccgtgag cacaagcggc ggaggaaagc 1740
   agggcagact gatcggcgcc gtgatcggca gcgtggccct gggagtggct acagctgccc 1800
   agattaccgc tgcagccgcc ctgatccagg ccaaccagaa cgccgccaac atcctgagac 1860
   tgaaagagag cattgccgcc accaacgagg ccgtgcacga agtgaccgac ggcctgagcc 1920
   agctgtccgt ggccgtgggc aagatgcagc agttcgtgaa cgaccagttc aacaacaccg 1980
   ccagagagct ggactgcatc aagatcaccc agcaggtggg cgtggagctg aacctgtacc 2040
   tgaccgagct gaccacagtg ttcggccccc agatcacaag cccagccctg acacagctga 2100
   ccatccaggc cctgtacaac ctggctggcg gcaacatgga ctatctgctg acaaagctgg 2160
   gaatcggcaa caaccagctg tccagcctga tcggaagcgg cctgatcacc ggctacccca 2220
   tcctgtacga cagccagaca cagctgctgg gcatccaggt gaacctgccc agcgtgggca 2280
   acctgaacaa catgcgcgcc acctacctgg aaaccctgag cgtgtccacc accaagggct 2340
   acgccagcgc cctggtgccc aaggtggtga cacaggtggg cagcgtgatc gaggaactgg 2400
   acaccagcta ctgcatcgag agcgacctgg acctgtactg caccagaatc gtgaccttcc 2460
   caatgagccc cggcatctac agctgcctga gcggcaacac cagcgcctgc atgtacagca 2520
   agaccgaagg cgcactgaca acaccctaca tggccctgaa gggaagcgtg atcgccaact 2580
   gcaagatcac cacctgcaga tgcaccgacc ccccaggcat catcagccag aactacggcg 2640
   aggccgtgag cctgatcgat cgccattcct gtaacgtgct gtccctggac ggcatcacac 2700
   tgagactgag cggcgagttc gatgccacct accagaagaa catcagcatc ctggacagcc 2760
   aggtgatcgt gaccggcaac ctggacatca gcaccgagct gggcaacgtg aataacagca     2820
   tcagcaacgc cctggacaga ctggccgaga gcaacagcaa gctggaaaaa gtgaacgtgc     2880
   gcctgacatc cacttccgct ctgatcacct acatcgtgct gaccgtgatc agcctggtgt     2940
   tcggcgccct gagcctggtg ctggcctgct acctgatgta caagcagaag gcccagcaga     3000
   aaaccctgct gtggctgggc aacaacaccc tggaccagat gagagccacc accagagcct     3060
   gatgagcggc cgcgatacct gcagggggga aaatatttca gctggcgtgc gagccgcgtc     3120
   tccgagagcg tatatggagt ggcctctgcc tgcgcaactt agctttaaat cggccctttc     3180
   atgttttcga cagcgcataa acccacgata ggccccttta gtagccacga aagttttatt     3240
   cgcagttcga aagaacgcta tgagcacatg atataaaaat taatcgctcc gcgatcagga     3300
   atattgtatt aacacgcaaa ccaacatgct cgagatggca gttcctaata tgcatttacc     3360
   agctgcggcg gccgccaatt gctttattcc gctattcgcg ttgcgtacat ttatacaagt     3420
   gtagcagcac agaaccgcgg ctgtcgcgga aaacaatgtc ggcccgagaa gtaaaccgaa     3480
   gccggctaag gtagtagcca ttgccccgaa tattaacagt aatacaagaa ttggacggaa     3540
   cagaacctcg tggtagcgcg acggaaggaa tatcgcgaca cttgtaccca ccaagaaacc     3600
   gcatatactg cgtaacgtgt cagtcaatgt tgactggtgc ttagttgcgt tcgacatgta     3660
   cttttgaaag ctggctatgg ctggaatcga gatgaccgct atgaggctca gaattatcgt     3720
   aggaatattc ccccccagcg tatcgtaact ggatacgtct tcttcgcgcg tcgcctcggc     3780
   gtccgtgctg ggcgggagac gcgcgcgacc gaggcgatat gcaccgttca cagtaatttc     3840
   ccgacgtgca cggtagtagc acgtgttcat tagagaccga ctggcgtctc tgaccacgtg     3900
   aatagctaac gccgtatacg ccgaaagaaa gccggctaga ggagaaatgt ctccatacgg     3960
   agccgtgatg ccggcaatcg tccctgccgc cacaactaaa aacccggtct tccacgtcac     4020
   gtctccagac gttgctatgc agacgtaatg ataagatgcg atcagagctc cgagcatgac     4080
   aaaag                                                                 4085
<210> 44
   <211> 4226
   <212> DNA
   <213> artificial sequence
<220>
   <223> partial plasmid sequence of SB-1 UL55 CAFopt syn tail SbfI for vSB1-008
<400> 44
   gtcctcagcc ttgcgctagt aagctgtaat acagagaacg gggcacgaac gtgcatcgaa        60
   acattatttt cctttggagg tgccgtgctg gaaacaaaca ttctgattca tgcaattaac       120
   caactgcttc ctttgtgcga tgtcatggcg gtttccggaa taagtagact tgctatcata       180
   gaatcaatcc ccgaactcgg aacggttcca tacaggtgcg ttttacaagc gacgcctcat 240
   attacagcgt gtctcacccc caaggtagtc agatgcatga attatgggat actactatct 300
   cactgggagg aatgcgtcgt gcgggattcg aaatacatcg taaacgtacc cacgcaaaca 360
   cagtttgtca catccttaac ccgttgtccg cacgggccag tcgccaacga ttggtacctt 420
   ggattcttct tcccatttaa ggggttcagg gtaataaccg tcagaacaag gcgctggctg 480
   gcagcatata cctacagatt tcgaaagtgg ttgcaatggg aagagggttc cccccactac 540
   gtcgctttga ggcgtctggt cccactatgt gattgctact tgatggatgc ttgcatgaca 600
   aacaactttt ttgcctgcgg aatgctattc cacttacact gcgtcccaat acccgcgcgc 660
   gaaaaacgca ttgtggcgat actggcgaga gcgattgatg acgcgcaaac atatgcggga 720
   acctcactgc acgtagatac gagtgaacag tagagcgtta gtatgtggtt ttctttaggt 780
   gttactacca cttacattcc tacaaaatca ctactcccgt attagatctc aacgctatac 840
   cggcgtaggc gaacctgagg gtgcgcccct agctctcgca attggtatgc gtaaatgaat 900
   tgttatcgcg ggttgccata agattttgta tacatacgaa cccgagaatg tataaaccaa 960
   taaatgctga aatggtagac ctgcaggtcg acccaattcg agctcggtac agcttggctg 1020
   tggaatgtgt gtcagttagg gtgtggaaag tccccaggct ccccagcagg cagaagtatg 1080
   caaagcatgc atctcaatta gtcagcaacc aggtgtggaa agtccccagg ctccccagca 1140
   ggcagaagta tgcaaagcat gcatctcaat tagtcagcaa ccatagtccc gcccctaact 1200
   ccgcccatcc cgcccctaac tccgcccagt tccgcccatt ctccgcccca tggctgacta 1260
   atttttttta tttatgcaga ggccgaggcc gcctcggcct ctgagctatt ccagaagtag 1320
   tgaggaggct tttttggagg cctaggcttt tgcaaaaagc tcccggggcg gccgccacca 1380
   tgggcagcaa gcccagcacc tggatcagcg tgaccctgat gctgatcacc agaaccatgc 1440
   tgatcctgag ctgcatctgc cccacaagca gcctggacgg cagacccctg gccgctgccg 1500
   gcatcgtggt gaccggcgac aaggccgtga acatctacac cagcagccag accggcagca 1560
   tcatcatcaa gctgctgccc aacatgccca aggacaaaga ggcctgcgcc aaggcccccc 1620
   tggaagccta caacagaacc ctgaccaccc tgctgacccc cctgggcgac agcatcagaa 1680
   gaatccaggg cagcgccacc acaagcggcg gaggaaagca gggcagactg gtgggcgcta 1740
   tcatcgggag cgtggccctg ggcgtggcca cagctgccca gattaccgct gcagccgccc 1800
   tgattcaggc caatcagaac gccgccaaca tcctgagact gaaagagagc attgccgcca 1860
   ccaacgacgc cgtgcacgaa gtgacaaacg gactgtccca gctggctgtc gctgtcggca 1920
   agatgcagca gttcgtgaac aaccagttca acaacaccgc cagagagctg gactgcatca 1980
   agatcgccca gcaggtgggc gtggagctga acctgtacct gaccgagctg accacagtgt 2040
   tcggccccca gatcacaagc cccgctctga cccagctgac aatccaggcc ctgtacaacc 2100
   tggctggcgg caacatggac tatctgctga ctaagctggg agtgggcaac aaccagctgt 2160
   ccagcctgat cgggtccggg ctgatcacag gcaaccccat cctgtacgac agccagacac 2220
   agctgctggg catccagatc aacctgccat ccgtgggaag cctgaacaac atgagagcca 2280
   cctacctgga aaccctgagc gtgtccacca ccaagggctt cgccagcgcc ctggtgccca 2340
   aggtggtgac acaggtgggc agcgtgatcg aggaactgga caccagctac tgcatcgaga 2400
   gcgacatcga cctgtactgc accagagtgg tgaccttccc aatgagcccc ggcatctaca 2460
   gctgcctgag cggcaacacc agcgcctgca tgtacagcaa gaccgaagga gcactgacaa 2520
   caccctacat ggccctgaag ggaagcgtga tcgccaactg caagatgacc acctgcagat 2580
   gcgccgaccc cccaggcatc atcagccaga actacggcga ggccgtgagc ctgatcgaca 2640
   aacattcctg tagcgtgctg tccctggatg gcatcacact gagactgagc ggcgagttcg 2700
   acgccaccta ccagaagaac atcagcatcc tggacagcca ggtgatcgtg accggcaacc 2760
   tggacatcag caccgagctg ggcaacgtga acaacagcat cagcagcacc ctggacaagc 2820
   tggccgagtc caacaacaag ctgaacaaag tgaacgtgaa cctgaccagc acaagcgccc 2880
   tgatcaccta catcgtgctg gccatcgtgt ccctggcctt cggcgtgatc agcctggtgc 2940
   tggcctgcta cctgatgtac aagcagagag cccagcagaa aaccctgctg tggctgggca 3000
   ataacaccct ggaccagatg agggccacca ccagaacctg atgagcggcc gcgatatcaa 3060
   taaaatatct ttattttcat tacatctgtg tgttggtttt ttgtgtgaat cgatagtact 3120
   aacatacgct ctccatcaaa acaaaacgaa acaaaacaaa ctagcaaaat aggctgtccc 3180
   cagtgcaagt gcaggtgcca gaacatttct cttctagacc tgcaggtcaa atcatgattc 3240
   gtttttattc agtgtccgta ctcgcgaata gctctgcact gcgatgcgat tttttcggca 3300
   gcgctaaaag ccgatctcca acactcgagc gaaaacttat tttcgcagtg gcaatgaaaa 3360
   cacagtccct ctatagccat gctgtagttt tcatccagtg agtcgatgat cgtcgcatga 3420
   gaaaaaggcg actcgcattg gtcgcctgtc aatcgaccag tccgtaactt cttgttcatt 3480
   ctatacgcga tatacgaggc accgcattgg gacatgttct cagacggatc cgtcgccaat 3540
   ccaaagaaga acgcgtggaa agtcgtcgac gatttacacg ctgcccgtat gacgtacgaa 3600
   cgaggaataa cggatgcggc acatgacaca tgttcgcccg tagctagttg agtagaggcg 3660
   gacggcacgt caattgctaa cggagcgatc acttgccaaa cgtacggaat tcgtaccggt 3720
   gaagctgacg gttcagctgt actttccgcc attggacact gtagaactga ggatctgcca 3780
   ccgctgggcg cacgtggttt ctgccgggtg ttatataagt ttttccacgc ccactacgtg 3840
   ttacctacac actcacggca cgtggtacgt ggacgtaagg gatggccgta atgaaaaatg 3900
   caccgggcga ctagttgtcc cgtctagcat gaccatgcat tgtagaccgc aggtccagcg 3960
   ctgcgccaat tacgactcta catcggacgc tccaacccag acgggcttcc caatgtggct 4020
   caccccgaga gaactctcac cgactaacgt ttatggaata gtcgctagac cggtactcct 4080
   cacgcacgcc gggggcccaa taaacagtgt actgacggga ggttggaaag ggagaatggt 4140
   cacggcaacg tcgtctatct gcgaaaggat actgctttgg gtagcggata cgcagcgttc 4200
   ttctccgagc accggatcgt ttgacc 4226
<210> 45
   <211> 4335
   <212> DNA
   <213> artificial sequence
<220>
   <223> partial plasmid sequence of pHVT US2 SV-Fopt-synPA for vHVT306
<400> 45
   taaaatggga tctatcatta cattcgttaa gagtctggat aattttactg tttgccagct      60
   tcgatcttgg aacgtactgt ggatagtgcc ttacttggaa tcgtgaaaat ttgaaacgtc     120
   cattatttgg atatcttccg gttgtcccat atcccgccct ggtaccgctc ggataccttg     180
   cccgtatgga ttcgtattga cagtcgcgca atcggggacc aacaacgcgt gggtccacac     240
   tcattcggaa attttccgat gattctgaat atttattgcc gctcgttacg agtcgttgga     300
   catatctgta atacatttct tcttctgaag gatcgctgca catttgatct atacattggc     360
   caggatgttc aagtctcaga tgttgcattc tggcacagca caactttatg gcatttccga     420
   tgtaatcgtc cggcagccct gggggagttc tatattcgca tattgggatg gtaaggacaa     480
   tagcagatct cgcaacctcc agggaggcta taataacgtt tttaaaggat ggatttctca     540
   taaaaatctg tcgcaaatta cactgagaat atcctttact agcgccgatt gagagcatcg     600
   tcgtccaatt ttctaaatgg aaagaaaaca aggcgggcaa gagtgttcca aacattttca     660
   ttttcggcga atctctcaaa tcccatggcg tgcaattgat tgcaaaattg gcacttccgt     720
   tcacgtttgt atctccaaac tctaagacac ttttaattga aaaactacgt tctagtgtgg     780
   aaagaaacct ataggcagac catagaacta tttgacacca catatctttt tgtatgtcaa     840
   actgaccatg atcgtatgtt gctgaatgca ctagggcaat tcgctcgcgc gactccatac     900
   attgaataat tccacacgtc agctcatcgg ttagcaaggt ccagtagttg aagtcattta     960
   tttttccccg cggctggcca aatctacctc tgggaatatc caagttgtcg aatatgatcg    1020
   caccggctct ggtcatggtg aaggaacttg tagcataaag acgcaggtat cataggggta    1080
   atattttttt attcactcac atactaaaag taacgcatat tagcaccatg tatgggctat    1140
   caattgacat ttgcgtagca ctacatcacg attatgtaca acataatggg acaacatatg    1200
   cctgcaggtc gacccaattc gagctcggta cagcttggct gtggaatgtg tgtcagttag    1260
   ggtgtggaaa gtccccaggc tccccagcag gcagaagtat gcaaagcatg catctcaatt 1320
   agtcagcaac caggtgtgga aagtccccag gctccccagc aggcagaagt atgcaaagca 1380
   tgcatctcaa ttagtcagca accatagtcc cgcccctaac tccgcccatc ccgcccctaa 1440
   ctccgcccag ttccgcccat tctccgcccc atggctgact aatttttttt atttatgcag 1500
   aggccgaggc cgcctcggcc tctgagctat tccagaagta gtgaggaggc ttttttggag 1560
   gcctaggctt ttgcaaaaag ctcccggggc ggccgccacc atgggcagca agcccagcac 1620
   aagaatccca gcccccctga tgctgatcac ccgcatcatg ctgatcctgg gctgcatcag 1680
   acccacaagc tccctggatg gacgccccct ggccgctgcc ggcatcgtgg tgaccggcga 1740
   caaggccgtg aacgtgtaca ccagcagcca gaccggcagc atcatcgtga agctgctgcc 1800
   caacatgccc agagacaaag aggcctgcgc caaggccccc ctggaagcct acaacagaac 1860
   cctgaccacc ctgctgaccc ccctgggcga cagcatcaga aagatccagg gctccgtgag 1920
   cacaagcggc ggaggaaagc agggcagact gatcggcgcc gtgatcggca gcgtggccct 1980
   gggagtggct acagctgccc agattaccgc tgcagccgcc ctgatccagg ccaaccagaa 2040
   cgccgccaac atcctgagac tgaaagagag cattgccgcc accaacgagg ccgtgcacga 2100
   agtgaccgac ggcctgagcc agctgtccgt ggccgtgggc aagatgcagc agttcgtgaa 2160
   cgaccagttc aacaacaccg ccagagagct ggactgcatc aagatcaccc agcaggtggg 2220
   cgtggagctg aacctgtacc tgaccgagct gaccacagtg ttcggccccc agatcacaag 2280
   cccagccctg acacagctga ccatccaggc cctgtacaac ctggctggcg gcaacatgga 2340
   ctatctgctg acaaagctgg gaatcggcaa caaccagctg tccagcctga tcggaagcgg 2400
   cctgatcacc ggctacccca tcctgtacga cagccagaca cagctgctgg gcatccaggt 2460
   gaacctgccc agcgtgggca acctgaacaa catgcgcgcc acctacctgg aaaccctgag 2520
   cgtgtccacc accaagggct acgccagcgc cctggtgccc aaggtggtga cacaggtggg 2580
   cagcgtgatc gaggaactgg acaccagcta ctgcatcgag agcgacctgg acctgtactg 2640
   caccagaatc gtgaccttcc caatgagccc cggcatctac agctgcctga gcggcaacac 2700
   cagcgcctgc atgtacagca agaccgaagg cgcactgaca acaccctaca tggccctgaa 2760
   gggaagcgtg atcgccaact gcaagatcac cacctgcaga tgcaccgacc ccccaggcat 2820
   catcagccag aactacggcg aggccgtgag cctgatcgat cgccattcct gtaacgtgct 2880
   gtccctggac ggcatcacac tgagactgag cggcgagttc gatgccacct accagaagaa 2940
   catcagcatc ctggacagcc aggtgatcgt gaccggcaac ctggacatca gcaccgagct 3000
   gggcaacgtg aataacagca tcagcaacgc cctggacaga ctggccgaga gcaacagcaa 3060
   gctggaaaaa gtgaacgtgc gcctgacatc cacttccgct ctgatcacct acatcgtgct 3120
   gaccgtgatc agcctggtgt tcggcgccct gagcctggtg ctggcctgct acctgatgta 3180
   caagcagaag gcccagcaga aaaccctgct gtggctgggc aacaacaccc tggaccagat 3240
   gagagccacc accagagcct gatgagcggc cgcgatatca ataaaatatc tttattttca 3300
   ttacatctgt gtgttggttt tttgtgtgaa tcgatagtac taacatacgc tctccatcaa 3360
   aacaaaacga aacaaaacaa actagcaaaa taggctgtcc ccagtgcaag tgcaggtgcc 3420
   agaacatttc tcttctagac ctgcaggccc gggcaagtag atgcaatttc ctcacactag 3480
   ttgggtttat ctactattga attttcccct atctgtgata cacttgggag cctctacaag 3540
   catattgcca tcatgtacgt ttttatctac tgtcttaacg cccatgggaa cggaggcgtc 3600
   gtcgtcatgt attggacggc aacataggca gcaacacaaa ttgcgtttag gtggggtgca 3660
   tgtggactcg ataccaagcc cctgcagctg gggaacgtct ggtggagagc cgataatttg 3720
   atatacgcac gccatattac tgtcgttgaa gtacgcctta tcttctatgt tttcaaattt 3780
   aggttcccaa gtggacgtga gaagtgtttg tatctcacat ggaatggccc aaggcattcc 3840
   agcccaggtg cctggtactt taatggcaaa caaacgtttt ggtagaggta ttgattctat 3900
   tgcagttctg cagatatctg cagccccgag tatccacagg ctatacgata cgttatcgga 3960
   ggcctccgat tctagcatta catagccggt cagtagatcc tgccattcgg tagcgcaacc 4020
   ggctacatct tcaaacagtc tcacaataaa tgcatctctc gttcctgcca atccggaacc 4080
   gggcatacca ctcccgcctg ccgatttaat tctcacaatt gggcgatgcc ggcggggcaa 4140
   aacgaatgtg gatttggcaa accgacacag gtctgctgta cggactaata tgggcacacc 4200
   cacatcattc ttcagatgct ccatgcattg ttctatgaga aagatccata gggtggaggc 4260
   agcgtcacga gatcgcccag gcaatcgatc gcattcgtct agtaaagtga cgagagttat 4320
   catgcacaca cccat 4335
<210> 46
   <211> 5381
   <212> DNA
   <213> artificial sequence
<220>
   <223> Partial plasmid pCD046+NDV-F VII YZCQ sequence for vHVT112
<400> 46
   gagctcaggg tatgatactc agctgttatt gtggccgacc aggaggactc caatgcttag 60
   cattcataag aacgctagag atgctattta acgatgtgct gtcgtctaaa gaatttgtgc 120
   atttagcctt taaatgtaaa accaatgacg cattcactac gctcgtgcgt gcaatttctg 180
   ggccagggta tgcatattcc ataacagaaa tcgacacttg agaagaggat ctgactgttt 240
   gggataaagg tcgtttgggt ctgtcctagc gatataattt atatgacgat atacattaaa 300
   catctgtgtg cagtacttag gtatttaatc atgtcgatga aatgttatgt gtaaatatcg 360
   gacaatatag ataacgggca cgctgctatt gtaacgtgcg cccgcgcgct agtgctgact 420
   aatagtgtgg atgatgtata cagtatatta caaacggaaa tgatacgtaa taaattatgt 480
   actcttattg atttataaaa acatacatgc agtgttgcta tgtcacataa ttagcctcgc 540
   ccgtctacgc tccactgaag ataatgggct cccgctgttc aaaaaaatca gcgtgcgtcg 600
   ataagacttt ggtgcagtct cttcggggtc gcaatttaga tttgccgcat ggagggtatc 660
   tggggatttt tgccaatgct ggagcgacga ctgtacgatt cgtcccatcg ggatctagca 720
   gaccaatgat gttgacacac atcggccatg catgtacgga cggtctattg cgcgagtttg 780
   ttattttcga aggacaagat ggaagtgtat atggaaccga caataatgtt agtttgcatt 840
   tcttagggcg gaatctacat gatatcttat ccaagcgggg tatgagccag agagatgtga 900
   tggtcataaa gggtaaattt tttagatctg aaataacgca gttgcccaaa caacgatcgc 960
   gattaaaaga aaaatcggat ggttcaatta ggacatgcat ggattctgtg cgcataaacc 1020
   ataaccgcag cactgttggg cacttcggta actcaaatgc gaagcgttgc acgtctgcga 1080
   taactacgcc tactatgcac attgttactc ctgcatctta aaaatatatc ctgtagtaat 1140
   tttcacagca atgtcataac atcatctcgc taaagaatga cctgggattg gagaagtaat 1200
   gaatatttgc aaccaatgca ttgaataaac taacattaaa cgaattcaat agtggatccc 1260
   ccaactccgc ccgttttatg actagaacca atagttttta atgccaaatg cactgaaatc 1320
   ccctaatttg caaagccaaa cgccccctat gtgagtaata cggggacttt ttacccaatt 1380
   tcccacgcgg aaagccccct aatacactca tatggcatat gaatcagcac ggtcatgcac 1440
   tctaatggcg gcccataggg actttccaca tagggggcgt tcaccatttc ccagcatagg 1500
   ggtggtgact caatggcctt tacccaagta cattgggtca atgggaggta agccaatggg 1560
   tttttcccat tactggcaag cacactgagt caaatgggac tttccactgg gttttgccca 1620
   agtacattgg gtcaatggga ggtgagccaa tgggaaaaac ccattgctgc caagtacact 1680
   gactcaatag ggactttcca atgggttttt ccattgttgg caagcatata aggtcaatgt 1740
   gggtgagtca atagggactt tccattgtat tctgcccagt acataaggtc aatagggggt 1800
   gaatcaacag gaaagtccca ttggagccaa gtacactgcg tcaataggga ctttccattg 1860
   ggttttgccc agtacataag gtcaataggg gatgagtcaa tgggaaaaac ccattggagc 1920
   caagtacact gactcaatag ggactttcca ttgggttttg cccagtacat aaggtcaata 1980
   gggggtgagt caacaggaaa gttccattgg agccaagtac attgagtcaa tagggacttt 2040
   ccaatgggtt ttgcccagta cataaggtca atgggaggta agccaatggg tttttcccat 2100
   tactggcacg tatactgagt cattagggac tttccaatgg gttttgccca gtacataagg 2160
   tcaatagggg tgaatcaaca ggaaagtccc attggagcca agtacactga gtcaataggg 2220
   actttccatt gggttttgcc cagtacaaaa ggtcaatagg gggtgagtca atgggttttt 2280
   cccattattg gcacgtacat aaggtcaata ggggtgagtc attgggtttt tccagccaat 2340
   ttaattaaaa cgccatgtac tttcccacca ttgacgtcaa tgggctattg aaactaatgc 2400
   aacgtgacct ttaaacggta ctttcccata gctgattaat gggaaagtac cgttctcgag 2460
   ccaatacacg tcaatgggaa gtgaaagggc agccaaaacg taacaccgcc ccggttttcc 2520
   cctggaaatt ccatattggc acgcattcta ttggctgagc tgcgttctac gtgggtataa 2580
   gaggcgcgac cagcgtcggt accgtcgcag tcttcggtct gaccaccgta gaacgcagag 2640
   ctcctcgctg caggcggccg catgggctct aaaccttcta ccaggatccc agcacctctg 2700
   atgctgatca cccggattat gctgatattg gactgtatcc gtccgacaag ctctcttgac 2760
   ggcaggcctc ttgcagctgc aggaattgta gtaacaggag ataaggcagt caatgtatat 2820
   acctcgtctc agacagggtc aatcatagtc aagttgctcc cgaatatgcc caaggataag 2880
   gaggcgtgtg cgaaagaccc attagaggca tataacagaa cactgactac tttgctcact 2940
   cctcttggcg aatccatccg caagatccaa gggtctgtgt ccacgtctgg aggaggcaag 3000
   caaggccgcc tgataggtgc tgttattggt agtgtagctc ttggggttgc aacagcggca 3060
   caaataacag cagctgcggc cctaatacaa gccaaccaga atgctgccaa catccttcgg 3120
   cttaaggaga gcattgctgc aaccaatgaa gctgtgcatg aagtcaccga cggattatca 3180
   caactatcag tggcagttgg gaagatgcag cagtttgtca atgaccagtt taataataca 3240
   gcgcgagaat tggactgtat aaaaatcaca caacaggttg gtgtagaact caacctatac 3300
   ctaactgaat tgactacagt attcgggcca cagatcacct cccctgcatt aactcagctg 3360
   accatccagg cactttataa tttagctggt ggcaatatgg attacttatt aactaagtta 3420
   ggtataggga acaatcaact cagctcatta attggcagcg gcctgatcac tggttaccct 3480
   atattgtatg actcacagac tcaactcttg ggcatacaag tgaatttgcc ctcagtcggg 3540
   aacttaaata atatgcgtgc cacctattta gagaccttat ctgtaagtac agccaaagga 3600
   tatgcctcag cacttgttcc aaaagtagtg acacaagtcg gttctgtgat agaagagctt 3660
   gacacctcat actgtataga gtccgatctg gatttatatt gtactagaat agtgacattc 3720
   cccatgtccc caggtattta ttcctgttta agcggcaaca catcagcttg catgtattca 3780
   aagactgaag gcgcactcac tacgccgtat atggccctta aaggctcagt tattgccaat 3840
   tgtaagataa caacatgtag atgtacagac cctcctggta tcatatcgca aaattatgga 3900
   gaagctgtat ccctgataga tagacattcg tgcaatgtct tatcattaga cgggataact 3960
   ctgaggctca gtggagaatt tgatgcaact tatcaaaaga acatctcaat actagattct 4020
   caagtcatcg tgacaggcaa tcttgatata tcaactgaac ttggaaacgt caacaattca 4080
   atcagcaatg ccttggataa gttggcaaaa agcaacagca agctagaaaa agtcaatgtc 4140
   agactaacca gcacatccgc tctcattacc tatattgttc tgactgtcat ttctctagtt 4200
   ttcggtgcac taagtctggg tttaacatgt tacctgatgt acaaacaaaa ggcacaacaa 4260
   aagaccttgc tatggcttgg gaataatacc ctcgatcaga tgagagccac tacaagagca 4320
   tgagcggccg cggggatcca gacatgataa gatacattga tgagtttgga caaaccacaa 4380
   ctagaatgca gtgaaaaaaa tgctttattt gtgaaatttg tgatgctatt gctttatttg 4440
   taaccattat aagctgcaat aaacaagtta acaacaacaa ttgcattgat tttatgtttc 4500
   aggttcaggg ggaggtgtgg gaggtttttt cggatcctct agagtcgaca attattttat 4560
   ttaataacat atagcccaaa gacctctatg aacatttagt ttcccgtata ctcaacggcg 4620
   cgtgtacaca cgcatctctt tgcatagcga tgaagtttgt tcggcagcag aaaatgcaga 4680
   tatccaacaa tctggagaaa acttatcatc acagtggcag tggaaacata ccccctctat 4740
   attcatggta taattatcgt ctacagcgtc caggatagtg gcgtgagaaa atggagatct 4800
   gcagccctcc tttccatggc atgccgcttt attgttcatt aaacgcacaa tggtctcaac 4860
   gccagatatg ggcatagatt ctgaagaacc cgttgacaat ccgaagaaga aggcgtgcag 4920
   gtctttggaa gactcgcacg ttggtcttat aatgtatgat cgagatgtca ccctaatgcc 4980
   acatggtaca ggcttatcgc ggtcatggcg atcggacttg taatttgcaa cgatgggcaa 5040
   aggatcgacg acatgccaaa cattctgaac ccgtagagat gttaacgatg acgaggatga 5100
   atatcccatg ctcgctgcca tagtatcaag tacaccgcga ataaggacgc gtccaacatc 5160
   gttatatgca cacaatgggc tacacgtgac taacaccccc gaatattagt catatgtgag 5220
   tttcagtctg gctcccatat agcctgtaga ctatttgtgg tttaagtgtg aacgaggcgc 5280
   tgtgaacgag actcgggccg attgtaagaa caagcaaatg cactttccat ttaacaagaa 5340
   gtgtagagag aatactcaac ctctttggat gtatcctcga g 5381
<210> 47
   <211> 5381
   <212> DNA
   <213> artificial sequence
<220>
   <223> Partial plasmid pCD046+NDV Texas F sequence for vHVT113
<400> 47
   gagctcaggg tatgatactc agctgttatt gtggccgacc aggaggactc caatgcttag      60
   cattcataag aacgctagag atgctattta acgatgtgct gtcgtctaaa gaatttgtgc     120
   atttagcctt taaatgtaaa accaatgacg cattcactac gctcgtgcgt gcaatttctg     180
   ggccagggta tgcatattcc ataacagaaa tcgacacttg agaagaggat ctgactgttt     240
   gggataaagg tcgtttgggt ctgtcctagc gatataattt atatgacgat atacattaaa     300
   catctgtgtg cagtacttag gtatttaatc atgtcgatga aatgttatgt gtaaatatcg     360
   gacaatatag ataacgggca cgctgctatt gtaacgtgcg cccgcgcgct agtgctgact      420
   aatagtgtgg atgatgtata cagtatatta caaacggaaa tgatacgtaa taaattatgt      480
   actcttattg atttataaaa acatacatgc agtgttgcta tgtcacataa ttagcctcgc      540
   ccgtctacgc tccactgaag ataatgggct cccgctgttc aaaaaaatca gcgtgcgtcg      600
   ataagacttt ggtgcagtct cttcggggtc gcaatttaga tttgccgcat ggagggtatc      660
   tggggatttt tgccaatgct ggagcgacga ctgtacgatt cgtcccatcg ggatctagca      720
   gaccaatgat gttgacacac atcggccatg catgtacgga cggtctattg cgcgagtttg      780
   ttattttcga aggacaagat ggaagtgtat atggaaccga caataatgtt agtttgcatt      840
   tcttagggcg gaatctacat gatatcttat ccaagcgggg tatgagccag agagatgtga      900
   tggtcataaa gggtaaattt tttagatctg aaataacgca gttgcccaaa caacgatcgc      960
   gattaaaaga aaaatcggat ggttcaatta ggacatgcat ggattctgtg cgcataaacc     1020
   ataaccgcag cactgttggg cacttcggta actcaaatgc gaagcgttgc acgtctgcga     1080
   taactacgcc tactatgcac attgttactc ctgcatctta aaaatatatc ctgtagtaat     1140
   tttcacagca atgtcataac atcatctcgc taaagaatga cctgggattg gagaagtaat     1200
   gaatatttgc aaccaatgca ttgaataaac taacattaaa cgaattcaat agtggatccc     1260
   ccaactccgc ccgttttatg actagaacca atagttttta atgccaaatg cactgaaatc     1320
   ccctaatttg caaagccaaa cgccccctat gtgagtaata cggggacttt ttacccaatt     1380
   tcccacgcgg aaagccccct aatacactca tatggcatat gaatcagcac ggtcatgcac     1440
   tctaatggcg gcccataggg actttccaca tagggggcgt tcaccatttc ccagcatagg     1500
   ggtggtgact caatggcctt tacccaagta cattgggtca atgggaggta agccaatggg     1560
   tttttcccat tactggcaag cacactgagt caaatgggac tttccactgg gttttgccca     1620
   agtacattgg gtcaatggga ggtgagccaa tgggaaaaac ccattgctgc caagtacact     1680
   gactcaatag ggactttcca atgggttttt ccattgttgg caagcatata aggtcaatgt     1740
   gggtgagtca atagggactt tccattgtat tctgcccagt acataaggtc aatagggggt     1800
   gaatcaacag gaaagtccca ttggagccaa gtacactgcg tcaataggga ctttccattg     1860
   ggttttgccc agtacataag gtcaataggg gatgagtcaa tgggaaaaac ccattggagc     1920
   caagtacact gactcaatag ggactttcca ttgggttttg cccagtacat aaggtcaata     1980
   gggggtgagt caacaggaaa gttccattgg agccaagtac attgagtcaa tagggacttt     2040
   ccaatgggtt ttgcccagta cataaggtca atgggaggta agccaatggg tttttcccat     2100
   tactggcacg tatactgagt cattagggac tttccaatgg gttttgccca gtacataagg     2160
   tcaatagggg tgaatcaaca ggaaagtccc attggagcca agtacactga gtcaataggg     2220
   actttccatt gggttttgcc cagtacaaaa ggtcaatagg gggtgagtca atgggttttt 2280
   cccattattg gcacgtacat aaggtcaata ggggtgagtc attgggtttt tccagccaat 2340
   ttaattaaaa cgccatgtac tttcccacca ttgacgtcaa tgggctattg aaactaatgc 2400
   aacgtgacct ttaaacggta ctttcccata gctgattaat gggaaagtac cgttctcgag 2460
   ccaatacacg tcaatgggaa gtgaaagggc agccaaaacg taacaccgcc ccggttttcc 2520
   cctggaaatt ccatattggc acgcattcta ttggctgagc tgcgttctac gtgggtataa 2580
   gaggcgcgac cagcgtcggt accgtcgcag tcttcggtct gaccaccgta gaacgcagag 2640
   ctcctcgctg caggcggccg catgggctcc agatcttcta ccaggatccc ggtacctcta 2700
   atgctgatca tccgaaccgc gctgacactg agctgtatcc gtctgacaag ctctcttgat 2760
   ggcaggcctc ttgcggctgc agggatcgtg gtaacaggag ataaagcagt caacatatac 2820
   acctcatccc agacagggtc aatcatagtt aagttactcc cgaatatgcc caaggacaaa 2880
   gaggtgtgtg caaaagcccc attggaggca tacaacagga cactgactac tttactcacc 2940
   ccccttggtg attctatccg caggatacaa gagtctgtga ctacttccgg aggaggcaag 3000
   caaggccgcc tgataggtgc cattatcggc agtgtagctc ttggggttgc gacagctgca 3060
   cagataacag cagcttcggc cctgatacaa gccaaccaga atgctgccaa catcctccgg 3120
   cttaaagaga gcattgctgc aaccaatgaa gctgtgcacg aggtcactga cggattatca 3180
   caactagcag tggcagtagg gaagatgcaa cagtttgtca atgaccagtt caataataca 3240
   gcgcaagaat tggactgtat aaaaattgca cagcaggtcg gtgtagaact caacttgtac 3300
   ctaactgaat tgactacagt atttgggcca caaatcactt cccctgcctt aactcagctg 3360
   actatccaag cgctttacaa tctagctggt ggtaatatgg attacttgct gactaagtta 3420
   ggtgtaggga acaaccaact cagctcatta attggtagcg gcttgatcac cggcaaccct 3480
   attctgtacg actcacagac tcagatcttg ggtatacagg taactttgcc ttcagttggg 3540
   aacctgaata atatgcgtgc cacctacctg gagaccttat ctgtaagcac aaccaaggga 3600
   tttgcctcag cacttgtccc aaaagtggtg acacaggtcg gttccgtgat agaagaactt 3660
   gacacctcat actgtatagg gaccgacttg gatttatact gtacaagaat agtgacattc 3720
   cctatgtctc ctggtattta ttcttgtctg agcggtaata catcggcttg catgtattca 3780
   aagactgaag gcgcacttac tacgccatat atggctctca aaggctcagt tattgccaat 3840
   tgcaagctga caacatgtag atgtgcagat cccccaggta tcatatcgca aaattatgga 3900
   gaagctgtgt ccttaataga taggcactca tgcaacgtct tatccttaga cgggataact 3960
   ctgaggctca gtggggaatt tgatgcaacc tatcaaaaga atatctctat actagattct 4020
   caagttatag tgacaggcaa tcttgatata tcaactgagc ttgggaatgt caacaactca 4080
   ataagtaatg ccctgaataa gttagaggaa agcaacagca aactagacaa agtcaatgtc 4140
   aaactgacca gcacatctgc tctcattacc tacatcgttt taactgtcat atctcttgtt 4200
   tttggtgtac ttagcctggt tctagcatgc tacctgatgt acaagcaaaa ggcacaacaa 4260
   aagaccttgt tatggcttgg gaataatacc cttgatcaga tgagagccac tacaaaaata 4320
   tgagcggccg cggggatcca gacatgataa gatacattga tgagtttgga caaaccacaa 4380
   ctagaatgca gtgaaaaaaa tgctttattt gtgaaatttg tgatgctatt gctttatttg 4440
   taaccattat aagctgcaat aaacaagtta acaacaacaa ttgcattgat tttatgtttc 4500
   aggttcaggg ggaggtgtgg gaggtttttt cggatcctct agagtcgaca attattttat 4560
   ttaataacat atagcccaaa gacctctatg aacatttagt ttcccgtata ctcaacggcg 4620
   cgtgtacaca cgcatctctt tgcatagcga tgaagtttgt tcggcagcag aaaatgcaga 4680
   tatccaacaa tctggagaaa acttatcatc acagtggcag tggaaacata ccccctctat 4740
   attcatggta taattatcgt ctacagcgtc caggatagtg gcgtgagaaa atggagatct 4800
   gcagccctcc tttccatggc atgccgcttt attgttcatt aaacgcacaa tggtctcaac 4860
   gccagatatg ggcatagatt ctgaagaacc cgttgacaat ccgaagaaga aggcgtgcag 4920
   gtctttggaa gactcgcacg ttggtcttat aatgtatgat cgagatgtca ccctaatgcc 4980
   acatggtaca ggcttatcgc ggtcatggcg atcggacttg taatttgcaa cgatgggcaa 5040
   aggatcgacg acatgccaaa cattctgaac ccgtagagat gttaacgatg acgaggatga 5100
   atatcccatg ctcgctgcca tagtatcaag tacaccgcga ataaggacgc gtccaacatc 5160
   gttatatgca cacaatgggc tacacgtgac taacaccccc gaatattagt catatgtgag 5220
   tttcagtctg gctcccatat agcctgtaga ctatttgtgg tttaagtgtg aacgaggcgc 5280
   tgtgaacgag actcgggccg attgtaagaa caagcaaatg cactttccat ttaacaagaa 5340
   gtgtagagag aatactcaac ctctttggat gtatcctcga g 5381
<210> 48
   <211> 4600
   <212> DNA
   <213> artificial sequence
<220>
   <223> Partial plasmid pHM119 sequence for vHVT039
<400> 48
   gagctcaggg tatgatactc agctgttatt gtggccgacc aggaggactc caatgcttag        60
   cattcataag aacgctagag atgctattta acgatgtgct gtcgtctaaa gaatttgtgc       120
   atttagcctt taaatgtaaa accaatgacg cattcactac gctcgtgcgt gcaatttctg       180
   ggccagggta tgcatattcc ataacagaaa tcgacacttg agaagaggat ctgactgttt       240
   gggataaagg tcgtttgggt ctgtcctagc gatataattt atatgacgat atacattaaa       300
   catctgtgtg cagtacttag gtatttaatc atgtcgatga aatgttatgt gtaaatatcg 360
   gacaatatag ataacgggca cgctgctatt gtaacgtgcg cccgcgcgct agtgctgact 420
   aatagtgtgg atgatgtata cagtatatta caaacggaaa tgatacgtaa taaattatgt 480
   actcttattg atttataaaa acatacatgc agtgttgcta tgtcacataa ttagcctcgc 540
   ccgtctacgc tccactgaag ataatgggct cccgctgttc aaaaaaatca gcgtgcgtcg 600
   ataagacttt ggtgcagtct cttcggggtc gcaatttaga tttgccgcat ggagggtatc 660
   tggggatttt tgccaatgct ggagcgacga ctgtacgatt cgtcccatcg ggatctagca 720
   gaccaatgat gttgacacac atcggccatg catgtacgga cggtctattg cgcgagtttg 780
   ttattttcga aggacaagat ggaagtgtat atggaaccga caataatgtt agtttgcatt 840
   tcttagggcg gaatctacat gatatcttat ccaagcgggg tatgagccag agagatgtga 900
   tggtcataaa gggtaaattt tttagatctg aaataacgca gttgcccaaa caacgatcgc 960
   gattaaaaga aaaatcggat ggttcaatta ggacatgcat ggattctgtg cgcataaacc 1020
   ataaccgcag cactgttggg cacttcggta actcaaatgc gaagcgttgc acgtctgcga 1080
   taactacgcc tactatgcac attgttactc ctgcatctta aaaatatatc ctgtagtaat 1140
   tttcacagca atgtcataac atcatctcgc taaagaatga cctgggattg gagaagtaat 1200
   gaatatttgc aaccaatgca ttgaataaac taacattaaa cgaattccga tgtttagtca 1260
   cgatagacat cggttcgccc agccgtcgaa tacagcatta tattttagtg ttgaaaatgt 1320
   agggctgctt cctcacttaa aggaggaaat ggctcgattc atgtttcata gcagtagaaa 1380
   aacagattgg accgtcagta agtttagagg gttttatgac tttagcacta tagataatgt 1440
   aactgcggcc catcgcatgg cttggaaata tatcaaagaa ctgatttttg caacagcttt 1500
   attttcttct gtatttaaat gtggcgaatt gcacatctgt cgtgccgaca gtttgcagat 1560
   caacagcaat ggagactatg tatggaaaaa tggaatatat ataacatatg aaaccgaata 1620
   tccacttata atgattctgg ggtcagaatc aagcacttca gaaacgcaaa atatgactgc 1680
   aattattgat acagatgttt tttcgttgct ttattctatt ttgcagtata tggcccccgt 1740
   tacggcagat caggtgcgag tagaacagat taccaacagc cacgccccca tctgacccgt 1800
   ccaatattct tgtgtccctg cattttatct cacacaattt atgaacagca tcattaagat 1860
   catctcactg cggccgcaag atgggctcca gatcttctac caggatcccg gtacctctaa 1920
   tgctgatcat ccgaaccgcg ctgacactga gctgtatccg tctgacaagc tctcttgatg 1980
   gcaggcctct tgcggctgca gggatcgtgg taacaggaga taaagcagtc aacatataca 2040
   cctcatccca gacagggtca atcatagtta agttactccc gaatatgccc aaggacaaag 2100
   aggtgtgtgc aaaagcccca ttggaggcat acaacaggac actgactact ttactcaccc 2160
   cccttggtga ttctatccgc aggatacaag agtctgtgac tacttccgga ggaaggagac 2220
   agagacgctt tataggtgcc attatcggca gtgtagctct tggggttgcg acagctgcac 2280
   agataacagc agcttcggcc ctgatacaag ccaaccagaa tgctgccaac atcctccggc 2340
   ttaaagagag cattgctgca accaatgaag ctgtgcacga ggtcactgac ggattatcac 2400
   aactagcagt ggcagtaggg aagatgcaac agtttgtcaa tgaccagttc aataatacag 2460
   cgcaagaatt ggactgtata aaaattgcac agcaggtcgg tgtagaactc aacttgtacc 2520
   taactgaatt gactacagta tttgggccac aaatcacttc ccctgcctta actcagctga 2580
   ctatccaagc gctttacaat ctagctggtg gtaatatgga ttacttgctg actaagttag 2640
   gtgtagggaa caaccaactc agctcattaa ttggtagcgg cttgatcacc ggcaacccta 2700
   ttctgtacga ctcacagact cagatcttgg gtatacaggt aactttgcct tcagttggga 2760
   acctgaataa tatgcgtgcc acctacctgg agaccttatc tgtaagcaca accaagggat 2820
   ttgcctcagc acttgtccca aaagtggtga cacaggtcgg ttccgtgata gaagaacttg 2880
   acacctcata ctgtataggg accgacttgg atttatactg tacaagaata gtgacattcc 2940
   ctatgtctcc tggtatttat tcttgtctga gcggtaatac atcggcttgc atgtattcaa 3000
   agactgaagg cgcacttact acgccatata tggctctcaa aggctcagtt attgccaatt 3060
   gcaagctgac aacatgtaga tgtgcagatc ccccaggtat catatcgcaa aattatggag 3120
   aagctgtgtc cttaatagat aggcactcat gcaacgtctt atccttagac gggataactc 3180
   tgaggctcag tggggaattt gatgcaacct atcaaaagaa tatctctata ctagattctc 3240
   aagttatagt gacaggcaat cttgatatat caactgagct tgggaatgtc aacaactcaa 3300
   taagtaatgc cctgaataag ttagaggaaa gcaacagcaa actagacaaa gtcaatgtca 3360
   aactgaccag cacatctgct ctcattacct acatcgtttt aactgtcata tctcttgttt 3420
   ttggtgtact tagcctggtt ctagcatgct acctgatgta caagcaaaag gcacaacaaa 3480
   agaccttgtt atggcttggg aataataccc ttgatcagat gagagccact acaaaaatat 3540
   gagcggccgc ggggatccag acatgataag atacattgat gagtttggac aaaccacaac 3600
   tagaatgcag tgaaaaaaat gctttatttg tgaaatttgt gatgctattg ctttatttgt 3660
   aaccattata agctgcaata aacaagttaa caacaacaat tgcattcatt ttatgtttca 3720
   ggttcagggg gaggtgtggg aggttttttc ggatcctcta gagtcgacaa ttattttatt 3780
   taataacata tagcccaaag acctctatga acatttagtt tcccgtatac tcaacggcgc 3840
   gtgtacacac gcatctcttt gcatagcgat gaagtttgtt cggcagcaga aaatgcagat 3900
   atccaacaat ctggagaaaa cttatcatca cagtggcagt ggaaacatac cccctctata 3960
   ttcatggtat aattatcgtc tacagcgtcc aggatagtgg cgtgagaaaa tggagatctg 4020
   cagccctcct ttccatggca tgccgcttta ttgttcatta aacgcacaat ggtctcaacg 4080
   ccagatatgg gcatagattc tgaagaaccc gttgacaatc cgaagaagaa ggcgtgcagg    4140
   tctttggaag actcgcacgt tggtcttata atgtatgatc gagatgtcac cctaatgcca    4200
   catggtacag gcttatcgcg gtcatggcga tcggacttgt aatttgcaac gatgggcaaa    4260
   ggatcgacga catgccaaac attctgaacc cgtagagatg ttaacgatga cgaggatgaa    4320
   tatcccatgc tcgctgccat agtatcaagt acaccgcgaa taaggacgcg tccaacatcg    4380
   ttatatgcac acaatgggct acacgtgact aacacccccg aatattagtc atatgtgagt    4440
   ttcagtctgg ctcccatata gcctgtagac tatttgtggt ttaagtgtga acgaggcgct    4500
   gtgaacgaga ctcgggccga ttgtaagaac aagcaaatgc actttccatt taacaagaag    4560
   tgtagagaga atactcaacc tctttggatg tatcctcgag                          4600
<210> 49
   <211> 1662
   <212> DNA
   <213> artificial sequence
<220>
   <223> NDV Texas F gene (wild type non-modified)
<400> 49
   atgggctcca gatcttctac caggatcccg gtacctctaa tgctgatcat ccgaaccgcg 60
   ctgacactga gctgtatccg tctgacaagc tctcttgatg gcaggcctct tgcggctgca 120
   gggatcgtgg taacaggaga taaagcagtc aacatataca cctcatccca gacagggtca 180
   atcatagtta agttactccc gaatatgccc aaggacaaag aggtgtgtgc aaaagcccca 240
   ttggaggcat acaacaggac actgactact ttactcaccc cccttggtga ttctatccgc 300
   aggatacaag agtctgtgac tacttccgga ggaaggagac agagacgctt tataggtgcc 360
   attatcggca gtgtagctct tggggttgcg acagctgcac agataacagc agcttcggcc 420
   ctgatacaag ccaaccagaa tgctgccaac atcctccggc ttaaagagag cattgctgca 480
   accaatgaag ctgtgcacga ggtcactgac ggattatcac aactagcagt ggcagtaggg 540
   aagatgcaac agtttgtcaa tgaccagttc aataatacag cgcaagaatt ggactgtata 600
   aaaattgcac agcaggtcgg tgtagaactc aacttgtacc taactgaatt gactacagta 660
   tttgggccac aaatcacttc ccctgcctta actcagctga ctatccaagc gctttacaat 720
   ctagctggtg gtaatatgga ttacttgctg actaagttag gtgtagggaa caaccaactc 780
   agctcattaa ttggtagcgg cttgatcacc ggcaacccta ttctgtacga ctcacagact 840
   cagatcttgg gtatacaggt aactttgcct tcagttggga acctgaataa tatgcgtgcc 900
   acctacctgg agaccttatc tgtaagcaca accaagggat ttgcctcagc acttgtccca 960
   aaagtggtga cacaggtcgg ttccgtgata gaagaacttg acacctcata ctgtataggg 1020
   accgacttgg atttatactg tacaagaata gtgacattcc ctatgtctcc tggtatttat 1080
   tcttgtctga gcggtaatac atcggcttgc atgtattcaa agactgaagg cgcacttact 1140
   acgccatata tggctctcaa aggctcagtt attgccaatt gcaagctgac aacatgtaga 1200
   tgtgcagatc ccccaggtat catatcgcaa aattatggag aagctgtgtc cttaatagat 1260
   aggcactcat gcaacgtctt atccttagac gggataactc tgaggctcag tggggaattt 1320
   gatgcaacct atcaaaagaa tatctctata ctagattctc aagttatagt gacaggcaat 1380
   cttgatatat caactgagct tgggaatgtc aacaactcaa taagtaatgc cctgaataag 1440
   ttagaggaaa gcaacagcaa actagacaaa gtcaatgtca aactgaccag cacatctgct 1500
   ctcattacct acatcgtttt aactgtcata tctcttgttt ttggtgtact tagcctggtt 1560
   ctagcatgct acctgatgta caagcaaaag gcacaacaaa agaccttgtt atggcttggg 1620
   aataataccc ttgatcagat gagagccact acaaaaatat ga 1662
<210> 50
   <211> 553
   <212> PRT
   <213> artificial sequence
<220>
   <223> NDV Texas F protein (wild type non-modified; cleavage site underlined)
<400> 50
   Met Gly Ser Arg Ser Ser Thr Arg Ile Pro Val Pro Leu Met Leu Ile
   1               5                   10                  15
   Ile Arg Thr Ala Leu Thr Leu Ser Cys Ile Arg Leu Thr Ser Ser Leu
               20                  25                  30
   Asp Gly Arg Pro Leu Ala Ala Ala Gly Ile Val Val Thr Gly Asp Lys
           35                  40                  45
   Ala Val Asn Ile Tyr Thr Ser Ser Gln Thr Gly Ser Ile Ile Val Lys
       50                  55                  60
   Leu Leu Pro Asn Met Pro Lys Asp Lys Glu Val Cys Ala Lys Ala Pro
   65                  70                  75                  80
   Leu Glu Ala Tyr Asn Arg Thr Leu Thr Thr Leu Leu Thr Pro Leu Gly
                   85                  90                  95
   Asp Ser Ile Arg Arg Ile Gln Glu Ser Val Thr Thr Ser Gly Gly Arg
               100                 105                 110
   Arg Gln Arg Arg Phe Ile Gly Ala Ile Ile Gly Ser Val Ala Leu Gly
           115                 120                 125
   Val Ala Thr Ala Ala Gln Ile Thr Ala Ala Ser Ala Leu Ile Gln Ala
       130                 135                 140
   Asn Gln Asn Ala Ala Asn Ile Leu Arg Leu Lys Glu Ser Ile Ala Ala
   145                 150                 155                 160
   Thr Asn Glu Ala Val His Glu Val Thr Asp Gly Leu Ser Gln Leu Ala
                   165                 170                 175
   Val Ala Val Gly Lys Met Gln Gln Phe Val Asn Asp Gln Phe Asn Asn
               180                 185                 190
   Thr Ala Gln Glu Leu Asp Cys Ile Lys Ile Ala Gln Gln Val Gly Val
           195                 200                 205
   Glu Leu Asn Leu Tyr Leu Thr Glu Leu Thr Thr Val Phe Gly Pro Gln
       210                 215                 220
   Ile Thr Ser Pro Ala Leu Thr Gln Leu Thr Ile Gln Ala Leu Tyr Asn
   225                 230                 235                 240
   Leu Ala Gly Gly Asn Met Asp Tyr Leu Leu Thr Lys Leu Gly Val Gly
                   245                 250                 255
   Asn Asn Gln Leu Ser Ser Leu Ile Gly Ser Gly Leu Ile Thr Gly Asn
               260                 265                 270
   Pro Ile Leu Tyr Asp Ser Gln Thr Gln Ile Leu Gly Ile Gln Val Thr
           275                 280                 285
   Leu Pro Ser Val Gly Asn Leu Asn Asn Met Arg Ala Thr Tyr Leu Glu
       290                 295                 300
   Thr Leu Ser Val Ser Thr Thr Lys Gly Phe Ala Ser Ala Leu Val Pro
   305                 310                 315                 320
   Lys Val Val Thr Gln Val Gly Ser Val Ile Glu Glu Leu Asp Thr Ser
                   325                 330                 335
   Tyr Cys Ile Gly Thr Asp Leu Asp Leu Tyr Cys Thr Arg Ile Val Thr
               340                 345                 350
   Phe Pro Met Ser Pro Gly Ile Tyr Ser Cys Leu Ser Gly Asn Thr Ser
           355                 360                 365
   Ala Cys Met Tyr Ser Lys Thr Glu Gly Ala Leu Thr Thr Pro Tyr Met
       370                 375                 380
   Ala Leu Lys Gly Ser Val Ile Ala Asn Cys Lys Leu Thr Thr Cys Arg
   385                 390                 395                 400
   Cys Ala Asp Pro Pro Gly Ile Ile Ser Gln Asn Tyr Gly Glu Ala Val
                   405                 410                 415
   Ser Leu Ile Asp Arg His Ser Cys Asn Val Leu Ser Leu Asp Gly Ile
               420                 425                 430
   Thr Leu Arg Leu Ser Gly Glu Phe Asp Ala Thr Tyr Gln Lys Asn Ile
           435                 440                 445
   Ser Ile Leu Asp Ser Gln Val Ile Val Thr Gly Asn Leu Asp Ile Ser
       450                 455                 460
   Thr Glu Leu Gly Asn Val Asn Asn Ser Ile Ser Asn Ala Leu Asn Lys
   465                 470                 475                 480
   Leu Glu Glu Ser Asn Ser Lys Leu Asp Lys Val Asn Val Lys Leu Thr
                   485                 490                 495
   Ser Thr Ser Ala Leu Ile Thr Tyr Ile Val Leu Thr Val Ile Ser Leu
               500                 505                 510
   Val Phe Gly Val Leu Ser Leu Val Leu Ala Cys Tyr Leu Met Tyr Lys
           515                 520                 525
   Gln Lys Ala Gln Gln Lys Thr Leu Leu Trp Leu Gly Asn Asn Thr Leu
       530                 535                 540
   Asp Gln Met Arg Ala Thr Thr Lys Ile
   545                 550
<210> 51
   <211> 1662
   <212> DNA
   <213> artificial sequence
<220>
   <223> NDV-F YZCQ wildtype DNA
<400> 51
   atgggctcca gatcttctac caggatcccg gtacctctaa tgctgatcat ccgaaccgcg 60
   ctgacactga gctgtatccg tctgacaagc tctcttgatg gcaggcctct tgcggctgca 120
   gggatcgtgg taacaggaga taaagcagtc aacatataca cctcatccca gacagggtca 180
   atcatagtta agttactccc gaatatgccc aaggacaaag aggtgtgtgc aaaagcccca 240
   ttggaggcat acaacaggac actgactact ttactcaccc cccttggtga ttctatccgc 300
   aggatacaag agtctgtgac tacttccgga ggaggcaagc aaggccgcct gataggtgcc 360
   attatcggca gtgtagctct tggggttgcg acagctgcac agataacagc agcttcggcc 420
   ctgatacaag ccaaccagaa tgctgccaac atcctccggc ttaaagagag cattgctgca 480
   accaatgaag ctgtgcacga ggtcactgac ggattatcac aactagcagt ggcagtaggg 540
   aagatgcaac agtttgtcaa tgaccagttc aataatacag cgcaagaatt ggactgtata 600
   aaaattgcac agcaggtcgg tgtagaactc aacttgtacc taactgaatt gactacagta 660
   tttgggccac aaatcacttc ccctgcctta actcagctga ctatccaagc gctttacaat 720
   ctagctggtg gtaatatgga ttacttgctg actaagttag gtgtagggaa caaccaactc 780
   agctcattaa ttggtagcgg cttgatcacc ggcaacccta ttctgtacga ctcacagact 840
   cagatcttgg gtatacaggt aactttgcct tcagttggga acctgaataa tatgcgtgcc 900
   acctacctgg agaccttatc tgtaagcaca accaagggat ttgcctcagc acttgtccca 960
   aaagtggtga cacaggtcgg ttccgtgata gaagaacttg acacctcata ctgtataggg 1020
   accgacttgg atttatactg tacaagaata gtgacattcc ctatgtctcc tggtatttat 1080
   tcttgtctga gcggtaatac atcggcttgc atgtattcaa agactgaagg cgcacttact 1140
   acgccatata tggctctcaa aggctcagtt attgccaatt gcaagctgac aacatgtaga 1200
   tgtgcagatc ccccaggtat catatcgcaa aattatggag aagctgtgtc cttaatagat 1260
   aggcactcat gcaacgtctt atccttagac gggataactc tgaggctcag tggggaattt 1320
   gatgcaacct atcaaaagaa tatctctata ctagattctc aagttatagt gacaggcaat 1380
   cttgatatat caactgagct tgggaatgtc aacaactcaa taagtaatgc cctgaataag 1440
   ttagaggaaa gcaacagcaa actagacaaa gtcaatgtca aactgaccag cacatctgct 1500
   ctcattacct acatcgtttt aactgtcata tctcttgttt ttggtgtact tagcctggtt 1560
   ctagcatgct acctgatgta caagcaaaag gcacaacaaa agaccttgtt atggcttggg 1620
   aataataccc ttgatcagat gagagccact acaaaaatat ga 1662
<210> 52
   <211> 553
   <212> PRT
   <213> artificial sequence
<220>
   <223> NDV-F protein from wildtype YZCQ strain (Amino Acid Sequence of NDV-F of Texas strain with lentogenic cleavage site sequence)
<400> 52
   Met Gly Ser Arg Ser Ser Thr Arg Ile Pro Val Pro Leu Met Leu Ile
   1               5                   10                  15
   Ile Arg Thr Ala Leu Thr Leu Ser Cys Ile Arg Leu Thr Ser Ser Leu
   20 25 30
   Asp Gly Arg Pro Leu Ala Ala Ala Gly Ile Val Val Thr Gly Asp Lys
   35 40 45
   Ala Val Asn Ile Tyr Thr Ser Ser Gln Thr Gly Ser Ile Ile Val Lys
   50 55 60
   Leu Leu Pro Asn Met Pro Lys Asp Lys Glu Val Cys Ala Lys Ala Pro
   65 70 75 80
   Leu Glu Ala Tyr Asn Arg Thr Leu Thr Thr Leu Leu Thr Pro Leu Gly
   85 90 95
   Asp Ser Ile Arg Arg Ile Gln Glu Ser Val Thr Thr Ser Gly Gly Gly
   100 105 110
   Lys Gln Gly Arg Leu Ile Gly Ala Ile Ile Gly Ser Val Ala Leu Gly
   115 120 125
   Val Ala Thr Ala Ala Gln Ile Thr Ala Ala Ser Ala Leu Ile Gln Ala
   130 135 140
   Asn Gln Asn Ala Ala Asn Ile Leu Arg Leu Lys Glu Ser Ile Ala Ala
   145 150 155 160
   Thr Asn Glu Ala Val His Glu Val Thr Asp Gly Leu Ser Gln Leu Ala
   165 170 175
   Val Ala Val Gly Lys Met Gln Gln Phe Val Asn Asp Gln Phe Asn Asn
   180 185 190
   Thr Ala Gln Glu Leu Asp Cys Ile Lys Ile Ala Gln Gln Val Gly Val
   195 200 205
   Glu Leu Asn Leu Tyr Leu Thr Glu Leu Thr Thr Val Phe Gly Pro Gln
   210 215 220
   Ile Thr Ser Pro Ala Leu Thr Gln Leu Thr Ile Gln Ala Leu Tyr Asn
   225 230 235 240
   Leu Ala Gly Gly Asn Met Asp Tyr Leu Leu Thr Lys Leu Gly Val Gly
   245 250 255
   Asn Asn Gln Leu Ser Ser Leu Ile Gly Ser Gly Leu Ile Thr Gly Asn
               260                 265                 270
   Pro Ile Leu Tyr Asp Ser Gln Thr Gln Ile Leu Gly Ile Gln Val Thr
           275                 280                 285
   Leu Pro Ser Val Gly Asn Leu Asn Asn Met Arg Ala Thr Tyr Leu Glu
       290                 295                 300
   Thr Leu Ser Val Ser Thr Thr Lys Gly Phe Ala Ser Ala Leu Val Pro
   305                 310                 315                 320
   Lys Val Val Thr Gln Val Gly Ser Val Ile Glu Glu Leu Asp Thr Ser
                   325                 330                 335
   Tyr Cys Ile Gly Thr Asp Leu Asp Leu Tyr Cys Thr Arg Ile Val Thr
               340                 345                 350
   Phe Pro Met Ser Pro Gly Ile Tyr Ser Cys Leu Ser Gly Asn Thr Ser
           355                 360                 365
   Ala Cys Met Tyr Ser Lys Thr Glu Gly Ala Leu Thr Thr Pro Tyr Met
       370                 375                 380
   Ala Leu Lys Gly Ser Val Ile Ala Asn Cys Lys Leu Thr Thr Cys Arg
   385                 390                 395                 400
   Cys Ala Asp Pro Pro Gly Ile Ile Ser Gln Asn Tyr Gly Glu Ala Val
                   405                 410                 415
   Ser Leu Ile Asp Arg His Ser Cys Asn Val Leu Ser Leu Asp Gly Ile
               420                 425                 430
   Thr Leu Arg Leu Ser Gly Glu Phe Asp Ala Thr Tyr Gln Lys Asn Ile
           435                 440                 445
   Ser Ile Leu Asp Ser Gln Val Ile Val Thr Gly Asn Leu Asp Ile Ser
       450                 455                 460
   Thr Glu Leu Gly Asn Val Asn Asn Ser Ile Ser Asn Ala Leu Asn Lys
   465                 470                 475                 480
   Leu Glu Glu Ser Asn Ser Lys Leu Asp Lys Val Asn Val Lys Leu Thr
                   485                 490                 495
   Ser Thr Ser Ala Leu Ile Thr Tyr Ile Val Leu Thr Val Ile Ser Leu
               500                 505                 510
   Val Phe Gly Val Leu Ser Leu Val Leu Ala Cys Tyr Leu Met Tyr Lys
           515                 520                 525
   Gln Lys Ala Gln Gln Lys Thr Leu Leu Trp Leu Gly Asn Asn Thr Leu
       530                 535                 540
   Asp Gln Met Arg Ala Thr Thr Lys Ile
   545                 550
<210> 53
   <211> 1662
   <212> DNA
   <213> artificial sequence
<220>
   <223> NDV-F Texas wildtype DNA
<400> 53
   atgggctcta aaccttctac caggatccca gcacctctga tgctgatcac ccggattatg 60
   ctgatattgg actgtatccg tccgacaagc tctcttgacg gcaggcctct tgcagctgca 120
   ggaattgtag taacaggaga taaggcagtc aatgtatata cctcgtctca gacagggtca 180
   atcatagtca agttgctccc gaatatgccc aaggataagg aggcgtgtgc gaaagaccca 240
   ttagaggcat ataacagaac actgactact ttgctcactc ctcttggcga atccatccgc 300
   aagatccaag ggtctgtgtc cacgtctgga ggaggcaagc aaggccgcct gataggtgct 360
   gttattggta gtgtagctct tggggttgca acagcggcac aaataacagc agctgcggcc 420
   ctaatacaag ccaaccagaa tgctgccaac atccttcggc ttaaggagag cattgctgca 480
   accaatgaag ctgtgcatga agtcaccgac ggattatcac aactatcagt ggcagttggg 540
   aagatgcagc agtttgtcaa tgaccagttt aataatacag cgcgagaatt ggactgtata 600
   aaaatcacac aacaggttgg tgtagaactc aacctatacc taactgaatt gactacagta 660
   ttcgggccac agatcacctc ccctgcatta actcagctga ccatccaggc actttataat 720
   ttagctggtg gcaatatgga ttacttatta actaagttag gtatagggaa caatcaactc 780
   agctcattaa ttggcagcgg cctgatcact ggttacccta tattgtatga ctcacagact 840
   caactcttgg gcatacaagt gaatttgccc tcagtcggga acttaaataa tatgcgtgcc 900
   acctatttag agaccttatc tgtaagtaca gccaaaggat atgcctcagc acttgttcca 960
   aaagtagtga cacaagtcgg ttctgtgata gaagagcttg acacctcata ctgtatagag 1020
   tccgatctgg atttatattg tactagaata gtgacattcc ccatgtcccc aggtatttat 1080
   tcctgtttaa gcggcaacac atcagcttgc atgtattcaa agactgaagg cgcactcact 1140
   acgccgtata tggcccttaa aggctcagtt attgccaatt gtaagataac aacatgtaga 1200
   tgtacagacc ctcctggtat catatcgcaa aattatggag aagctgtatc cctgatagat 1260
   agacattcgt gcaatgtctt atcattagac gggataactc tgaggctcag tggagaattt 1320
   gatgcaactt atcaaaagaa catctcaata ctagattctc aagtcatcgt gacaggcaat 1380
   cttgatatat caactgaact tggaaacgtc aacaattcaa tcagcaatgc cttggataag 1440
   ttggcaaaaa gcaacagcaa gctagaaaaa gtcaatgtca gactaaccag cacatccgct 1500
   ctcattacct atattgttct gactgtcatt tctctagttt tcggtgcact aagtctgggt 1560
   ttaacatgtt acctgatgta caaacaaaag gcacaacaaa agaccttgct atggcttggg 1620
   aataataccc tcgatcagat gagagccact acaagagcat ga 1662
<210> 54
   <211> 553
   <212> PRT
   <213> artificial sequence
<220>
   <223> NDV-F protein from wildtype Texas strain (Amino Acid Sequence of NDV-F VIId wt YZCQ with lentogenic cleavage site sequence)
<400> 54
   Met Gly Ser Lys Pro Ser Thr Arg Ile Pro Ala Pro Leu Met Leu Ile
   1               5                   10                  15
   Thr Arg Ile Met Leu Ile Leu Asp Cys Ile Arg Pro Thr Ser Ser Leu
               20                  25                  30
   Asp Gly Arg Pro Leu Ala Ala Ala Gly Ile Val Val Thr Gly Asp Lys
           35                  40                  45
   Ala Val Asn Val Tyr Thr Ser Ser Gln Thr Gly Ser Ile Ile Val Lys
       50                  55                  60
   Leu Leu Pro Asn Met Pro Lys Asp Lys Glu Ala Cys Ala Lys Asp Pro
   65                  70                  75                  80
   Leu Glu Ala Tyr Asn Arg Thr Leu Thr Thr Leu Leu Thr Pro Leu Gly
                   85                  90                  95
   Glu Ser Ile Arg Lys Ile Gln Gly Ser Val Ser Thr Ser Gly Gly Gly
               100                 105                 110
   Lys Gln Gly Arg Leu Ile Gly Ala Val Ile Gly Ser Val Ala Leu Gly
           115                 120                 125
   Val Ala Thr Ala Ala Gln Ile Thr Ala Ala Ala Ala Leu Ile Gln Ala
       130                 135                 140
   Asn Gln Asn Ala Ala Asn Ile Leu Arg Leu Lys Glu Ser Ile Ala Ala
   145                 150                 155                 160
   Thr Asn Glu Ala Val His Glu Val Thr Asp Gly Leu Ser Gln Leu Ser
                   165                 170                 175
   Val Ala Val Gly Lys Met Gln Gln Phe Val Asn Asp Gln Phe Asn Asn
               180                 185                 190
   Thr Ala Arg Glu Leu Asp Cys Ile Lys Ile Thr Gln Gln Val Gly Val
           195                 200                 205
   Glu Leu Asn Leu Tyr Leu Thr Glu Leu Thr Thr Val Phe Gly Pro Gln
       210                 215                 220
   Ile Thr Ser Pro Ala Leu Thr Gln Leu Thr Ile Gln Ala Leu Tyr Asn
   225                 230                 235                 240
   Leu Ala Gly Gly Asn Met Asp Tyr Leu Leu Thr Lys Leu Gly Ile Gly
                   245                 250                 255
   Asn Asn Gln Leu Ser Ser Leu Ile Gly Ser Gly Leu Ile Thr Gly Tyr
               260                 265                 270
   Pro Ile Leu Tyr Asp Ser Gln Thr Gln Leu Leu Gly Ile Gln Val Asn
           275                 280                 285
   Leu Pro Ser Val Gly Asn Leu Asn Asn Met Arg Ala Thr Tyr Leu Glu
       290                 295                 300
   Thr Leu Ser Val Ser Thr Ala Lys Gly Tyr Ala Ser Ala Leu Val Pro
   305                 310                 315                 320
   Lys Val Val Thr Gln Val Gly Ser Val Ile Glu Glu Leu Asp Thr Ser
                   325                 330                 335
   Tyr Cys Ile Glu Ser Asp Leu Asp Leu Tyr Cys Thr Arg Ile Val Thr
               340                 345                 350
   Phe Pro Met Ser Pro Gly Ile Tyr Ser Cys Leu Ser Gly Asn Thr Ser
           355                 360                 365
   Ala Cys Met Tyr Ser Lys Thr Glu Gly Ala Leu Thr Thr Pro Tyr Met
       370                 375                 380
   Ala Leu Lys Gly Ser Val Ile Ala Asn Cys Lys Ile Thr Thr Cys Arg
   385                 390                 395                 400
   Cys Thr Asp Pro Pro Gly Ile Ile Ser Gln Asn Tyr Gly Glu Ala Val
                   405                 410                 415
   Ser Leu Ile Asp Arg His Ser Cys Asn Val Leu Ser Leu Asp Gly Ile
               420                 425                 430
   Thr Leu Arg Leu Ser Gly Glu Phe Asp Ala Thr Tyr Gln Lys Asn Ile
           435                 440                 445
   Ser Ile Leu Asp Ser Gln Val Ile Val Thr Gly Asn Leu Asp Ile Ser
       450                 455                 460
   Thr Glu Leu Gly Asn Val Asn Asn Ser Ile Ser Asn Ala Leu Asp Lys
   465                 470                 475                 480
   Leu Ala Lys Ser Asn Ser Lys Leu Glu Lys Val Asn Val Arg Leu Thr
                   485                 490                 495
   Ser Thr Ser Ala Leu Ile Thr Tyr Ile Val Leu Thr Val Ile Ser Leu
               500                 505                 510
   Val Phe Gly Ala Leu Ser Leu Gly Leu Thr Cys Tyr Leu Met Tyr Lys
           515                 520                 525
   Gln Lys Ala Gln Gln Lys Thr Leu Leu Trp Leu Gly Asn Asn Thr Leu
       530                 535                 540
   Asp Gln Met Arg Ala Thr Thr Arg Ala
   545                 550
<210> 55
   <211> 622
   <212> DNA
   <213> artificial sequence
<220>
   <223> MDV gB promoter
<400> 55
   cgatgtttag tcacgataga catcggttcg cccagccgtc gaatacagca ttatatttta 60
   gtgttgaaaa tgtagggctg cttcctcact taaaggagga aatggctcga ttcatgtttc 120
   atagcagtag aaaaacagat tggaccgtca gtaagtttag agggttttat gactttagca 180
   ctatagataa tgtaactgcg gcccatcgca tggcttggaa atatatcaaa gaactgattt 240
   ttgcaacagc tttattttct tctgtattta aatgtggcga attgcacatc tgtcgtgccg 300
   acagtttgca gatcaacagc aatggagact atgtatggaa aaatggaata tatataacat 360
   atgaaaccga atatccactt ataatgattc tggggtcaga atcaagcact tcagaaacgc 420
   aaaatatgac tgcaattatt gatacagatg ttttttcgtt gctttattct attttgcagt 480
   atatggcccc cgttacggca gatcaggtgc gagtagaaca gattaccaac agccacgccc 540
   ccatctgacc cgtccaatat tcttgtgtcc ctgcatttta tctcacacaa tttatgaaca 600
   gcatcattaa gatcatctca ct 622
<210> 56
   <211> 4850
   <212> DNA
   <213> artificial sequence
<220>
   <223> Partial plasmid HVT SORF3-US2 gpVar-Ewtsyn sequence for vHVT202
<400> 56
   taaaatggga tctatcatta cattcgttaa gagtctggat aattttactg tttgccagct      60
   tcgatcttgg aacgtactgt ggatagtgcc ttacttggaa tcgtgaaaat ttgaaacgtc     120
   cattatttgg atatcttccg gttgtcccat atcccgccct ggtaccgctc ggataccttg     180
   cccgtatgga ttcgtattga cagtcgcgca atcggggacc aacaacgcgt gggtccacac     240
   tcattcggaa attttccgat gattctgaat atttattgcc gctcgttacg agtcgttgga     300
   catatctgta atacatttct tcttctgaag gatcgctgca catttgatct atacattggc     360
   caggatgttc aagtctcaga tgttgcattc tggcacagca caactttatg gcatttccga     420
   tgtaatcgtc cggcagccct gggggagttc tatattcgca tattgggatg gtaaggacaa     480
   tagcagatct cgcaacctcc agggaggcta taataacgtt tttaaaggat ggatttctca     540
   taaaaatctg tcgcaaatta cactgagaat atcctttact agcgccgatt gagagcatcg     600
   tcgtccaatt ttctaaatgg aaagaaaaca aggcgggcaa gagtgttcca aacattttca     660
   ttttcggcga atctctcaaa tcccatggcg tgcaattgat tgcaaaattg gcacttccgt     720
   tcacgtttgt atctccaaac tctaagacac ttttaattga aaaactacgt tctagtgtgg     780
   aaagaaacct ataggcagac catagaacta tttgacacca catatctttt tgtatgtcaa     840
   actgaccatg atcgtatgtt gctgaatgca ctagggcaat tcgctcgcgc gactccatac     900
   attgaataat tccacacgtc agctcatcgg ttagcaaggt ccagtagttg aagtcattta     960
   tttttccccg cggctggcca aatctacctc tgggaatatc caagttgtcg aatatgatcg    1020
   caccggctct ggtcatggtg aaggaacttg tagcataaag acgcaggtat cataggggta     1080
   atattttttt attcactcac atactaaaag taacgcatat tagcaccatg tatgggctat    1140
   caattgacat ttgcgtagca ctacatcacg attatgtaca acataatggg acaacatatg    1200
   cctgcaggtt agtcatatgt tacttggcag aggccgcatg gaaagtccct ggacgtggga    1260
   catctgatta atacgtgagg aggtcagcca tgttcttttt ggcaaaggac tacggtcatt    1320
   ggacgtttga ttggcatggg atagggtcag ccagagttaa cagtgttctt ttggcaaagg    1380
   gatacgtgga aagtcccggg ccatttacag taaactgata cggggacaaa gcacagccat 1440
   atttagtcat gtattgcttg gcagagggtc tatggaaagt ccctggacgt gggacgtctg 1500
   attaatatga aagaaggtca gccagaggta gctgtgtcct ttttggcaaa gggatacggt 1560
   tatgggacgt ttgattggac tgggataggg tcagccagag ttaacagtgt tcttttggca 1620
   aaggaaacgt ggaaagtccc gggccattta cagtaaactg atactgggac aaagtacacc 1680
   catatttagt catgttcttt ttggcaaaga gcatctggaa agtcccgggc agcattatag 1740
   tcacttggca gagggaaagg gtcactcaga gttaagtaca tctttccagg gccaatattc 1800
   cagtaaatta cacttagttt tatgcaaatc agccacaaag ggatttttcc cggtcaatta 1860
   tgactttttc cttagtcatg cggtatccaa ttactgccaa attggcagta catactaggt 1920
   gattcactga catttggccg tcctctggaa agtccctgga aaccgctcaa gtactgtatc 1980
   atggtgactt tgcatttttg gagagcacgc cccactccac cattggtcca cgtaccctat 2040
   gggggagtgg tttatgagta tataaggggc tccggtttag aagccgggca gagcggccgc 2100
   atgacaaacc tgcaagatca aacccaacag attgttccgt tcatacggag ccttctgatg 2160
   ccaacaaccg gaccggcgtc cattccggac gacaccctgg agaagcacac tctcaggtca 2220
   gagacctcga cctacaattt gactgtgggg gacacagggt cagggctaat tgtctttttc 2280
   cctggattcc ctggctcaat tgtgggtgct cactacacac tgcagagcaa tgggaactac 2340
   aagttcgatc agatgctcct gactgcccag aacctaccgg ccagctacaa ctactgcagg 2400
   ctagtgagtc ggagtctcac agtaaggtca agcacactcc ctggtggcgt ttatgcacta 2460
   aacggcacca taaacgccgt gaccttccaa ggaagcctga gtgaactgac agatgttagc 2520
   tacaacgggt tgatgtctgc aacagccaac atcaacgaca aaattgggaa cgtcctagta 2580
   ggggaagggg taaccgtcct cagcttaccc acatcatatg atcttgggta tgtgaggctt 2640
   ggtgacccca tacccgctat agggcttgac ccaaaaatgg tagcaacatg tgacagcagt 2700
   gacaggccca gagtctacac cataactgca gccgataatt accaattctc atcacagtac 2760
   caaacaggtg gggtaacaat cacactgttc tcagccaaca ttgatgccat cacaagtctc 2820
   agcgttgggg gagagctcgt gttcaaaaca agcgtccaaa gccttgtact gggcgccacc 2880
   atctacctta taggctttga tgggactgcg gtaatcacca gagctgtggc cgcaaacaat 2940
   gggctgacgg ccggcatcga caatcttatg ccattcaatc ttgtgattcc aaccaatgag 3000
   ataacccagc caatcacatc catcaaactg gagatagtga cctccaaaag tgatggtcag 3060
   gcaggggaac agatgtcatg gtcggcaagt gggagcctag cagtgacgat ccatggtggc 3120
   aactatccag gagccctccg tcccgtcaca ctagtggcct acgaaagagt ggcaacagga 3180
   tctgtcgtta cggtcgctgg ggtgagcaac ttcgagctga tcccaaatcc tgaactagca 3240
   aagaacctgg ttacagaata tggccgattt gacccaggag ccatgaacta cacgaaattg 3300
   atactgagtg agagggaccg ccttggcatc aagaccgtct ggccaacaag ggagtacact 3360
   gactttcgtg agtacttcat ggaggtggcc gacctcaact ctcccctgaa gattgcagga 3420
   gcatttggct tcaaagacat aatccgggcc ataaggaggt gagcggccgc gatatcaata 3480
   aaatatcttt attttcatta catctgtgtg ttggtttttt gtgtgaatcg atagtactaa 3540
   catacgctct ccatcaaaac aaaacgaaac aaaacaaact agcaaaatag gctgtcccca 3600
   gtgcaagtgc aggtgccaga acatttctct tctagacctg caggcccggg gcaagtagat 3660
   gcaatttcct cacactagtt gggtttatct actattgaat tttcccctat ctgtgataca 3720
   cttgggagcc tctacaagca tattgccatc atgtacgttt ttatctactg tcttaacgcc 3780
   catgggaacg gaggcgtcgt cgtcatgtat tggacggcaa cataggcagc aacacaaatt 3840
   gcgtttaggt ggggtgcatg tggactcgat accaagcccc tgcagctggg gaacgtctgg 3900
   tggagagccg ataatttgat atacgcacgc catattactg tcgttgaagt acgccttatc 3960
   ttctatgttt tcaaatttag gttcccaagt ggacgtgaga agtgtttgta tctcacatgg 4020
   aatggcccaa ggcattccag cccaggtgcc tggtacttta atggcaaaca aacgttttgg 4080
   tagaggtatt gattctattg cagttctgca gatatctgca gccccgagta tccacaggct 4140
   atacgatacg ttatcggagg cctccgattc tagcattaca tagccggtca gtagatcctg 4200
   ccattcggta gcgcaaccgg ctacatcttc aaacagtctc acaataaatg catctctcgt 4260
   tcctgccaat ccggaaccgg gcataccact cccgcctgcc gatttaattc tcacaattgg 4320
   gcgatgccgg cggggcaaaa cgaatgtgga tttggcaaac cgacacaggt ctgctgtacg 4380
   gactaatatg ggcacaccca catcattctt cagatgctcc atgcattgtt ctatgagaaa 4440
   gatccatagg gtggaggcag cgtcacgaga tcgcccaggc aatcgatcgc attcgtctag 4500
   taaagtgacg agagttatca tgcacacacc catgcccacg ccttccgaat aactggagct 4560
   gtggaagatc ggaaacgtct ttttgactgc cggtctcgta ctactttcgc acaggtgtat 4620
   acccggacgc gtactatata ttttatatca tccaacgtcc cgaaattaca tacgtggcgg 4680
   cgatggaagt agatgttgag tcttcgaaag taagtgcctc gaatatgggt attgtctgtg 4740
   aaaatatcga aagcggtacg acggttgcag aaccgtcgat gtcgccagat actagtaaca 4800
   atagcttcga taacgaagac ttccgtgggc ctgaatacga tgtggagata 4850
<210> 57
   <211> 4943
   <212> DNA
   <213> artificial sequence
<220>
   <223> Partial plasmid SB1US2 gpVIIdwtsyn sequence for vSB1-010
<400> 57
   tctcgtctaa aacgctccag tgctttacag ttcgataatc tggacctggg gacgcgtata 60
   ggatcgttcc tccacatgcg ctgctgtcgg tatctcgaat ccccggtatt cagttgaatc 120
   gttggcggag tgtcctcctg gactctgcaa tgttccctag ccgtcttcac tatctcgtgc 180
   aaggctctat aatacagttc ctctgcagac ccgtcgttgc tcttcccttc tgcgtcgtta 240
   gttatttctg taggctccag acgatttgcc tgcatttgtg cgcaacataa tctgattgca 300
   ttccctatct cgtcttccgg taatcccata ggtgttcggt attcgcagat aggtagagaa 360
   agcaccactg caaatcgtgc aatttccatt gccccaacca atattttttt taagaacggc 420
   atcgccgtta atgtacctcg ggcattgtga cgatcgaaac ccttatggat gcctaaagag 480
   agcattgcgg tccagttctc caggtgaaaa gagaatagcg cgggtagaaa cgggccgatt 540
   agttttatct tcgccgcgtc cctaatatcc caagttctgc agtataactt ccatcgtccg 600
   ttttcgacaa ggtccggcgc gacatagttt gaaatgtcat ctatcagaaa catctcgccc 660
   atcgtagaaa aaaacctgta cgcagaccat aaaaccattc ggtaccacat atccttgtgt 720
   atatcaaacg atatgttggt tatgtcgttg gcggatgttg tatgaaatag agctaagcgt 780
   tctctggatt ccacgcactg aacgattccg ttagtcaatt catctgctaa cataggccaa 840
   aagtttattc gtgttacttt tctcggcggt ttggcaaaac gcccccttgg cacatccatg 900
   tcattaaata cagcggcata actcctactc atgtgttcca tagcccaggt ttctgttcgg 960
   tctgctacta cgatcagatc agtggcgcga tcagatgcgt gggatgaatg aagtgtatcc 1020
   gaaagcagtt ttgagatata cgctaaactg tacgacgatt gtggcactaa acgaagcttt 1080
   gcgcgacccc catcccacgc cctgcaggtt agtcatatgt tacttggcag aggccgcatg 1140
   gaaagtccct ggacgtggga catctgatta atacgtgagg aggtcagcca tgttcttttt 1200
   ggcaaaggac tacggtcatt ggacgtttga ttggcatggg atagggtcag ccagagttaa 1260
   cagtgttctt ttggcaaagg gatacgtgga aagtcccggg ccatttacag taaactgata 1320
   cggggacaaa gcacagccat atttagtcat gtattgcttg gcagagggtc tatggaaagt 1380
   ccctggacgt gggacgtctg attaatatga aagaaggtca gccagaggta gctgtgtcct 1440
   ttttggcaaa gggatacggt tatgggacgt ttgattggac tgggataggg tcagccagag 1500
   ttaacagtgt tcttttggca aaggaaacgt ggaaagtccc gggccattta cagtaaactg 1560
   atactgggac aaagtacacc catatttagt catgttcttt ttggcaaaga gcatctggaa 1620
   agtcccgggc agcattatag tcacttggca gagggaaagg gtcactcaga gttaagtaca 1680
   tctttccagg gccaatattc cagtaaatta cacttagttt tatgcaaatc agccacaaag 1740
   gggattttcc cggtcaatta tgactttttc cttagtcatg cggtatccaa ttactgccaa 1800
   attggcagta catactaggt gattcactga catttggccg tcctctggaa agtccctgga 1860
   aaccgctcaa gtactgtatc atggtgactt tgcatttttg gagagcacgc cccactccac 1920
   cattggtcca cgtaccctat gggggagtgg tttatgagta tataaggggc tccggtttag 1980
   aagccgggca gagcggccgc atgggctcca aaccttctac caggatccca gcacctctga 2040
   tgctgatcac ccggattatg ctgatattgg gctgtatccg tccgacaagc tctcttgacg 2100
   gcaggcctct tgcagctgca ggaattgtag taacaggaga taaggcagtc aatgtataca 2160
   cttcgtctca gacagggtca atcatagtca agttgctccc gaatatgccc agggataagg 2220
   aggcgtgtgc aaaagcccca ttagaggcat ataacagaac actgactact ttgctcactc 2280
   ctcttggcga ctccatccgc aagatccaag ggtctgtgtc cacatctgga ggaggcaagc 2340
   aaggccgcct gataggtgct gttattggca gtgtagctct tggggttgca acagcggcac 2400
   agataacagc agctgcggcc ctaatacaag ccaaccagaa tgccgccaac atcctccggc 2460
   ttaaggagag cattgctgca accaatgaag ctgtgcatga agtcaccgac ggattatcac 2520
   aactatcagt ggcagttggg aagatgcagc agtttgtcaa tgaccagttt aataatacgg 2580
   cgcgagaatt ggactgtata aaaatcacac aacaggttgg tgtagaactc aacctatacc 2640
   taactgaatt gactacagta ttcgggccac agatcacctc ccctgcatta actcagctga 2700
   ccatccaggc actttataat ttagctggtg gcaatatgga ttacttatta actaagttag 2760
   gtatagggaa caatcaactc agctcgttaa ttggtagcgg cctgatcact ggttacccta 2820
   tactgtatga ctcacagact caactcttgg gcatacaagt gaatttaccc tcagtcggga 2880
   acttaaataa tatgcgtgcc acctatttgg agaccttatc tgtaagtaca accaaaggat 2940
   atgcctcagc acttgtcccg aaagtagtga cacaagtcgg ttccgtgata gaagagcttg 3000
   acacctcata ctgtatagag tccgatctgg atttatattg tactagaata gtgacattcc 3060
   ccatgtcccc aggtatttat tcctgtttga gcggcaacac atcagcttgc atgtattcaa 3120
   agactgaagg cgcactcact acgccgtata tggcccttaa aggctcagtt attgccaatt 3180
   gtaaaataac aacatgtaga tgtacagacc ctcctggtat catatcgcaa aattatggag 3240
   aagctgtatc cctgatagat agacattcgt gcaatgtctt atcattagac gggataactc 3300
   taaggctcag tggggaattt gatgcaactt atcaaaagaa catctcaata ctagattctc 3360
   aagtcatcgt gacaggcaat cttgatatat caactgaact tggaaacgtc aacaattcaa 3420
   tcagcaatgc cttggatagg ttggcagaaa gcaacagcaa gctagaaaaa gtcaatgtca 3480
   gactaaccag cacatctgct ctcattacct atattgttct aactgtcatt tctctagttt 3540
   tcggtgcact tagtctggtg ttagcgtgtt acctgatgta caaacagaag gcacaacaaa 3600
   agaccttgct atggcttggg aataataccc tcgatcagat gagagccact acaagagcat 3660
   gagcggccgc gatatcaata aaatatcttt attttcatta catctgtgtg ttggtttttt 3720
   gtgtgaatcg atagtactaa catacgctct ccatcaaaac aaaacgaaac aaaacaaact 3780
   agcaaaatag gctgtcccca gtgcaagtgc aggtgccaga acatttctct tctagacctg 3840
   caggggagtc tgtgcaaggt taatgaccct cgcagttcat tcggaagtta taactgccgc 3900
   cttcgcacat ttctttttgt cctgttttgt attgccataa cagataggaa ttgaaacctg 3960
   atcctcctgt tttttgcagc atggccagca acagaatact ttgtcggatc gactacttgc 4020
   gcgagatggt tccgttcttg gaggtttcgg cgggtcgggt ggagaaccta ttattttata 4080
   cacacacgtc ataccgttgt cgcgaaaatg ttctttgtct tctgccgtct cgaacgtcgg 4140
   ttcccacgta gacgttagga gcgttggaat ggtatcagga agagcccacg gcatgccgga 4200
   ccaagtaccc gctactttga ccgcgagcag tctcttcggt aatgggatgt attccagagc 4260
   agcgcggcag agatcagcgg cccccactat ccacagactg tatgaagtgt tttctgaaac 4320
   atcggactcc aacatcaaat atccagacat aacatcttgc cattcggaag cacatccgcc 4380
   gacatcttca aatagcctaa ctataaacga gtctctagtt cctgctaacc cagtacctcg 4440
   aatgccagtc ccatccggtg ggttcgtcct gataatcggt ctctgacgcc gaggaagaac 4500
   taaaaggggt ctggaaaagc ggaacagatc tgcagaccga acgactacag acacgcccac 4560
   atcatcatgt atctgttcca tgcattgctt tatgagaaaa atccataagg ccgaggcggc 4620
   atctctagat ctcccgggga gtctctcgca ctcatctagg agagtgacga cagttatcat 4680
   agacacgccc atttgtgcac caaacgaaaa gttcctgtac tggtggagcg tcggcgcggg 4740
   aatcggtccg tgctctgaaa ccagtgtcta gacagaagac catccggtaa attctggtgt 4800
   atgaactgac ggtctccaga cgaacgtcga agacattaac gatggaaact aacgagcttt 4860
   cttcaaaagt gtctgattac aacgctaata gaccttacga aactatacgc agcgatacca 4920
   gtgacacaga tccgtcggtg tcg 4943
<210> 58
   <211> 1362
   <212> DNA
   <213> artificial sequence
<220>
   <223> IBDV DNA encoding VP2 protein of IBDV E strain
<400> 58
<210> 59
   <211> 453
   <212> PRT
   <213> artificial sequence
<220>
   <223> IBDV VP2 protein of IBDV E strain
<400> 59
   Met Thr Asn Leu Gln Asp Gln Thr Gln Gln Ile Val Pro Phe Ile Arg
   1               5                   10                  15
   Ser Leu Leu Met Pro Thr Thr Gly Pro Ala Ser Ile Pro Asp Asp Thr
               20                  25                  30
   Leu Glu Lys His Thr Leu Arg Ser Glu Thr Ser Thr Tyr Asn Leu Thr
           35                  40                  45
   Val Gly Asp Thr Gly Ser Gly Leu Ile Val Phe Phe Pro Gly Phe Pro
       50                  55                  60
   Gly Ser Ile Val Gly Ala His Tyr Thr Leu Gln Ser Asn Gly Asn Tyr
   65                  70                  75                  80
   Lys Phe Asp Gln Met Leu Leu Thr Ala Gln Asn Leu Pro Ala Ser Tyr
   85 90 95
   Asn Tyr Cys Arg Leu Val Ser Arg Ser Leu Thr Val Arg Ser Ser Thr
   100 105 110
   Leu Pro Gly Gly Val Tyr Ala Leu Asn Gly Thr Ile Asn Ala Val Thr
   115 120 125
   Phe Gln Gly Ser Leu Ser Glu Leu Thr Asp Val Ser Tyr Asn Gly Leu
   130 135 140
   Met Ser Ala Thr Ala Asn Ile Asn Asp Lys Ile Gly Asn Val Leu Val
   145 150 155 160
   Gly Glu Gly Val Thr Val Leu Ser Leu Pro Thr Ser Tyr Asp Leu Gly
   165 170 175
   Tyr Val Arg Leu Gly Asp Pro Ile Pro Ala Ile Gly Leu Asp Pro Lys
   180 185 190
   Met Val Ala Thr Cys Asp Ser Ser Asp Arg Pro Arg Val Tyr Thr Ile
   195 200 205
   Thr Ala Ala Asp Asn Tyr Gln Phe Ser Ser Gln Tyr Gln Thr Gly Gly
   210 215 220
   Val Thr Ile Thr Leu Phe Ser Ala Asn Ile Asp Ala Ile Thr Ser Leu
   225 230 235 240
   Ser Val Gly Gly Glu Leu Val Phe Lys Thr Ser Val Gln Ser Leu Val
   245 250 255
   Leu Gly Ala Thr Ile Tyr Leu Ile Gly Phe Asp Gly Thr Ala Val Ile
   260 265 270
   Thr Arg Ala Val Ala Ala Asn Asn Gly Leu Thr Ala Gly Ile Asp Asn
   275 280 285
   Leu Met Pro Phe Asn Leu Val Ile Pro Thr Asn Glu Ile Thr Gln Pro
   290 295 300
   Ile Thr Ser Ile Lys Leu Glu Ile Val Thr Ser Lys Ser Asp Gly Gln
   305 310 315 320
   Ala Gly Glu Gln Met Ser Trp Ser Ala Ser Gly Ser Leu Ala Val Thr
                   325                 330                 335
   Ile His Gly Gly Asn Tyr Pro Gly Ala Leu Arg Pro Val Thr Leu Val
               340                 345                 350
   Ala Tyr Glu Arg Val Ala Thr Gly Ser Val Val Thr Val Ala Gly Val
           355                 360                 365
   Ser Asn Phe Glu Leu Ile Pro Asn Pro Glu Leu Ala Lys Asn Leu Val
       370                 375                 380
   Thr Glu Tyr Gly Arg Phe Asp Pro Gly Ala Met Asn Tyr Thr Lys Leu
   385                 390                 395                 400
   Ile Leu Ser Glu Arg Asp Arg Leu Gly Ile Lys Thr Val Trp Pro Thr
                   405                 410                 415
   Arg Glu Tyr Thr Asp Phe Arg Glu Tyr Phe Met Glu Val Ala Asp Leu
               420                 425                 430
   Asn Ser Pro Leu Lys Ile Ala Gly Ala Phe Gly Phe Lys Asp Ile Ile
           435                 440                 445
   Arg Ala Ile Arg Arg
       450
<210> 60
   <211> 884
   <212> DNA
   <213> artificial sequence
<220>
   <223> Guinea pig CMV promoter
<400> 60
   ttagtcatat gttacttggc agaggccgca tggaaagtcc ctggacgtgg gacatctgat      60
   taatacgtga ggaggtcagc catgttcttt ttggcaaagg actacggtca ttggacgttt     120
   gattggcatg ggatagggtc agccagagtt aacagtgttc ttttggcaaa gggatacgtg     180
   gaaagtcccg ggccatttac agtaaactga tacggggaca aagcacagcc atatttagtc     240
   atgtattgct tggcagaggg tctatggaaa gtccctggac gtgggacgtc tgattaatat     300
   gaaagaaggt cagccagagg tagctgtgtc ctttttggca aagggatacg gttatgggac     360
   gtttgattgg actgggatag ggtcagccag agttaacagt gttcttttgg caaaggaaac     420
   gtggaaagtc ccgggccatt tacagtaaac tgatactggg acaaagtaca cccatattta     480
   gtcatgttct ttttggcaaa gagcatctgg aaagtcccgg gcagcattat agtcacttgg     540
   cagagggaaa gggtcactca gagttaagta catctttcca gggccaatat tccagtaaat     600
   tacacttagt tttatgcaaa tcagccacaa aggggatttt cccggtcaat tatgactttt     660
   tccttagtca tgcggtatcc aattactgcc aaattggcag tacatactag gtgattcact     720
   gacatttggc cgtcctctgg aaagtccctg gaaaccgctc aagtactgta tcatggtgac     780
   tttgcatttt tggagagcac gccccactcc accattggtc cacgtaccct atgggggagt     840
   ggtttatgag tatataaggg gctccggttt agaagccggg caga                      884
<210> 61
   <211> 30
   <212> DNA
   <213> artificial sequence
<220>
   <223> primer HM101
<400> 61
   ccggaattcc gatgtttagt cacgatagac 30
<210> 62
   <211> 35
   <212> DNA
   <213> artificial sequence
<220>
   <223> Primer HM102
<400> 62
   ataagagcgg ccgcagtgag atgatcttaa tgatg 35
<210> 63
   <211> 38
   <212> DNA
   <213> artificial sequence
<220>
   <223> primer F-ATG
<400> 63
   tatagcggcc gcaagatggg ctccagatct tctaccag 38
<210> 64
   <211> 34
   <212> DNA
   <213> artificial sequence
<220>
   <223> Primer F-STOP
<400> 64
   cgaggcggcc gctcatattt ttgtagtggc tctc 34

## Description

### CROSS-REFERENCE TO RELATED APPLICATIONS

This application claims priority to U.S. provisional application 61/564,877 filed on November 30, 2011 and U.S. provisional application 61/694,957 filed on August 30, 2012.

### FIELD OF THE INVENTION

The invention relates to recombinant viral vectors for the insertion and expression of foreign genes for use as safe immunization vehicles to protect against Newcastle Disease Virus (NDV). It also relates to multivalent composition or vaccine comprising one or more recombinant viral vectors for protection against NDV. Also described herein are methods of making and using the recombinant viral vectors.

### BACKGROUND OF THE INVENTION

Poultry vaccination is widely used to protect poultry flocks against devastating diseases including Newcastle disease (ND), infectious bursal disease (IBD), Marek's disease (MD), infectious bronchitis (IB), infectious laryngotracheitis (ILT) and avian influenza (AI). ND is caused by the avian paramyxovirus 1 (APMV-1) also designated ND virus (NDV) belonging to the *Paramyxoviridae* family. MD is caused by Gallid herpesvirus 2 (*Herpesviridae* family) also designated as MD virus serotype 1 (MDV1). IB is caused by IB virus (IBV) belonging to the *Coronaviridae* family, ILT is caused by Gallid herpesvirus 1 (*Herpesviridae* family) also designated ILT virus (ILTV) and AI is caused by AI virus (AIV) belonging to the *Orthomyxoviridae* family.

A number of recombinant avian viral vectors have been proposed with a view to vaccinating birds against these avian pathogens. The viral vectors used comprise avipox viruses, especially fowlpox (EP-A-0,517,292), Marek's virus, such as serotypes 2 and 3 (HVT) (WO-A-87/04463), or alternatively the ITLV, NDV and avian adenovirus. When some of these recombinant avian viral vectors were used for vaccination, they display variable levels of protection.

Several recombinant herpesvirus of turkeys (HVT, also designated Meleagrid herpesvirus 1 or MDV serotype 3) vectors expressing antigens from various pathogens (US patent Nos. 5,980,906, 5,853,733, 6,183,753, 5,187,087) including IBDV, NDV, ILTV and AIV have been developed and licensed. Of particular interest is a HVT vector-expressing IBDV VP2 protective gene that has shown clear advantages over classical IBD vaccines (Bublot et al J.Comp. Path.2007, Vol.137, S81-S84). Other HVT vectors of interest are those expressing either NDV (Morgan et al 1992, Avian dis. 36, 858-70) or ILTV (Johnson et al, 2010 Avian Dis 54, 1251-1259) protective gene(s). One of the practical problems of using several HVT-based recombinant vaccines together is their interference. Lower protection is induced at least against one of the disease when two HVT recombinants expressing different antigens are mixed (Rudolf Heine 2011; Issues of the Poultry Recombinant Viral Vector Vaccines which May Cause an Effect on the Economic Benefits of those Vaccines; paper presented at the XVII World Veterinary Poultry Association (WVPA) Congress in Cancún, Mexico, August 14-18, 2011; Slacum G, Hein R. and Lynch P., 2009, The compatibility of HVT recombinants with other Marek's disease vaccines, 58th Western Poultry Disease Conference, Sacramento, CA, USA, March 23rd-25th, p 84).

The combination of HVT and SB-1, a Gallid herpesvirus 3 (MDV serotype 2 or MDV-2) vaccine strain, has shown a synergistic effect on MD protection (Witter and Lee, 1984, Avian Pathology 13, 75-92). To address the interference problem, it is of interest to evaluate the SB-1 virus as a vaccine vector to express protective antigen(s) that could be compatible with HVT vector and improve MD protection.

The SB-1 genome was cloned and characterized in bacterial artificial chromosome (BAC) (Petherbridge, et al.,J. Virol. Methods 158, 11-17, 2009; Singh et al., Research in Veterinary Science 89, 140-145, 2010). The MDV2 SB-1 sequence was recently obtained and analyzed (Spatz and Schat, Virus Gene 42, 331-338, 2011). A glycoprotein E deletion of SB-1 virus was described by Petherbridge et al. (J. Virol. Methods 158, 11-17, 2009). However, no research has been reported using SB-1 as a viral vector expressing foreign protective genes.

It has been shown that both U_{L}13 protein kinase and glycoprotein C (U_{L}44) genes individually are essential for horizontal transmission of MDV in chickens (Jarosinski, et al., J. of Virology 81, 10575-10587, 2007; Jarosinski, et al., J. of Virology 84, 7911-7916, 2010).
EP1298139 discloses Avian Herpes virus-based recombinant vaccines, teaching various positions for inserting heterologous antigens and methods to determine non-essential regions. Also disclosed is a recombinant vector containing the heterologous gene of VP2 of IBDV, which can be mixed with a vaccine consisting mainly of MDV-3. However, EP1298139 neither teaches the use of SB-1 to express an NDV-F transgene, nor the medical use of an MDV-2 vector.

Considering the potential effect of animal pathogens, such as NDV and IBDV on veterinary public health and the economy, efficient methods of preventing infection and protecting animals are needed. There is a need for a solution of combined effective vector vaccines and a suitable method for making the vaccine that could alleviate the problem of interference observed between 2 HVT-based vector vaccines.

### SUMMARY OF THE INVENTION

In a first embodiment, the invention is a composition or vaccine for use in a method of inducing an immunogenic or protective response in an animal against NDV, said composition or vaccine comprising a recombinant Gallid herpesvirus 3 (MDV-2) SB-1 strain vector, said vector comprising one or more heterologous polynucleotides coding for and expressing at least one antigen of an avian pathogen, wherein the heterologous polynucleotide encodes the Newcastle Disease Virus protein NDV-F, wherein said method comprises at least one administration of said composition or vaccine.

In a second embodiment, the invention is a recombinant Gallid herpesvirus 3 (MDV-2) SB-1 strain vector comprising a heterologous polynucleotide encoding a Newcastle Disease Virus protein NDV-F, a promoter, and a polyadenylation signal; wherein
(a) the heterologous polynucleotide is a wild-type NDV-F VIId polynucleotide, the promoter is a mouse cytomegalovirus IE (mCMV IE) promoter and the polyadenylation signal is an Simian virus 40 (SV40) polyadenylation signal, further wherein the heterologous polynucleotide encoding NDV-F is inserted into the region between ORF SORF4 and ORF US10 of the Gallid herpesvirus 3 (MDV-2) SB-1 strain vector; or
(b) the heterologous polynucleotide is a codon-optimized NDV-F VIId polynucleotide, the promoter is a SV40 promoter and the polyadenylation signal is SEQ ID NO: 13, further wherein the heterologous polynucleotide encoding NDV-F is inserted into the region between ORF UL55 and ORF LORF5 in the unique long (UL) region of the Gallid herpesvirus 3 (MDV-2) SB-1 strain vector; or
(c) the heterologous polynucleotide is a codon-optimized NDV-F VIId polynucleotide, the promoter is a SV40 promoter and the polyadenylation signal is endogenous originating from the glycoprotein C (gC) gene, further wherein the heterologous polynucleotide encoding NDV-F is inserted into the region coding for glycoprotein C (UL44) of the Gallid herpesvirus 3 (MDV-2) SB-1 strain vector; or
(d) the heterologous polynucleotide is a codon-optimized NDV-F V (CA02 strain) polynucleotide, the promoter is a SV40 promoter and the polyadenylation signal is SEQ ID NO: 13, further wherein the heterologous polynucleotide encoding NDV-F is inserted into the region between ORF UL55 and ORF LORF5 in the unique long (UL) region of the Gallid herpesvirus 3 (MDV-2) SB-1 strain vector; or
(e) the heterologous polynucleotide is a codon-optimized NDV-F V (CA02 strain) polynucleotide, the promoter is a SV40 promoter and the polyadenylation signal is endogenous originating from the glycoprotein C (gC) gene, further wherein the heterologous polynucleotide encoding NDV-F is inserted into the region coding for glycoprotein C (UL44) of the Gallid herpesvirus 3 (MDV-2) SB-1 strain vector.

The present invention demonstrated for the first time a recombinant Gallid Herpesvirus-3 (MDV-2) viral vector protecting against a poultry pathogen beyond Marek's disease virus.

The present invention showed surprising result when multivalent vaccines were used to protect animals against a variety of avian pathogens.

The present invention relates to a recombinant Gallid Herpesvirus-3 (MDV-2) vector comprising one or more heterologous polynucleotides coding for and expressing at least one antigen of an avian pathogen. Described herein is a recombinant Gallid Herpesvirus-3 (MDV-2) vector comprising a mutated glycoprotein C (gC) gene.

Described herein is a composition or vaccine comprising one or more recombinant Gallid Herpesvirus-3 (MDV-2) vectors comprising one or more heterologous polynucleotides coding for and expressing at least one antigen of an avian pathogen. Further described herein is a composition for vaccine comprising one or more Gallid Herpesvirus-3 (MDV-2) vectors comprising a mutated glycoprotein C (gC) gene.

Described herein is a polyvalent composition or vaccine comprising: i) a recombinant Gallid Herpesvirus-3 (MDV-2) vector comprising heterologous polynucleotides coding for and expressing at least one antigen of an avian pathogen, or comprising a mutated glycoprotein C (gC) gene; and ii) at least one of: a recombinant HVT vector (or MDV-3 or Meleagrid herpesvirus-1) comprising heterologous polynucleotides coding for and expressing at least one antigen of an avian pathogen; or wild type HVT (MDV-3); or recombinant MDV serotype 1 vector (*i.e.,* MDV-1, Gallid herpesvirus-2) comprising heterologous polynucleotides coding for and expressing at least one antigen of an avian pathogen; or any wild type MDV-1.

Described herein is a method of vaccinating an animal, or inducing an immunogenic or protective response in an animal, comprising at least one administration of the composition or vector of the present invention.

The present invention further provides specific insertion loci for the introduction of one or more isolated polynucleotide into nonessential regions of the SB-1 genome.

### BRIEF DESCRIPTION OF THE DRAWINGS

The following detailed description, given by way of example, and which is not intended to limit the invention to specific embodiments described, may be understood in conjunction with the accompanying figures, in which:

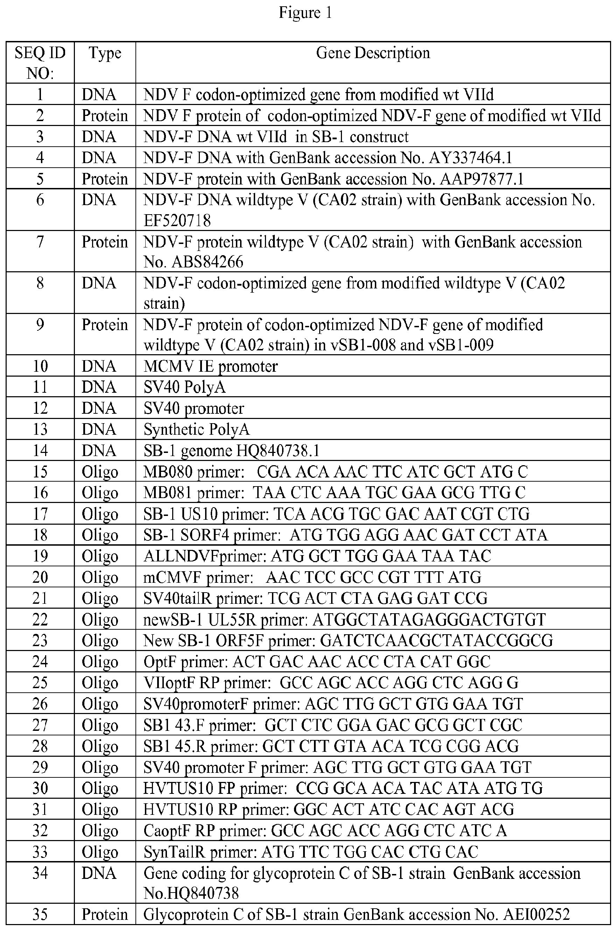

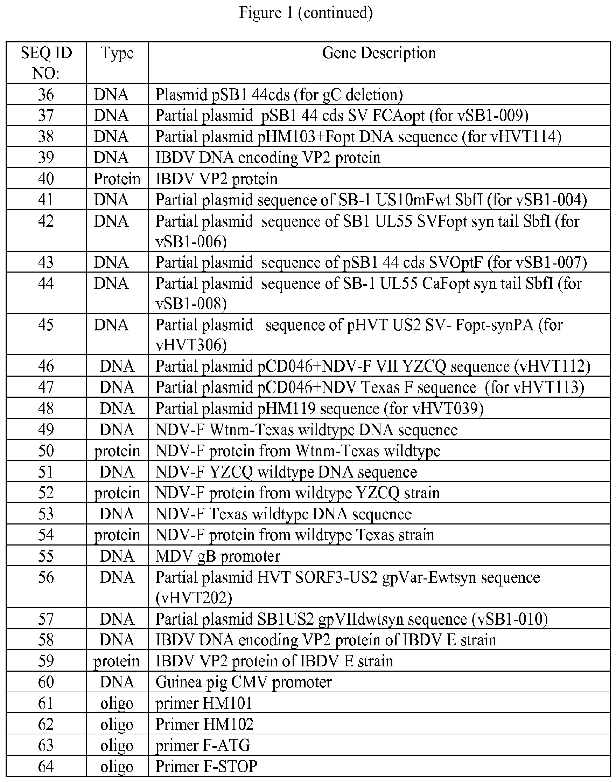

Figure 1 is a table showing the SEQ ID NO assigned to each DNA and protein sequence.
Figure 2 depicts a schematic diagram of SB-1 genome organization.
Figure 3 depicts the immunofluorescent staining of recombinant vSB 1-004 virus expressing NDV-F protein.
Figure 4 depicts the schematic representation of primer binding sites.
Figure 5 shows the PCR results of identifying vSB1-004.
Figure 6 shows the immunofluorescent staining of recombinant vSB 1-006 virus expressing NDV-F protein.
Figure 7 depicts the schematic representation of primer binding sites on vSB1-006.
Figure 8 shows the PCR results of vSB 1-006.
Figure 9 depicts the immunofluorescent staining of recombinant SB1-007 virus expressing NDV-F protein.
Figure 10 depicts the schematic diagram of primer location on pSB1 44 cds SVOptF donor plasmid.
Figure 11 shows the PCR results of vSB1-007.
Figure 12 depicts the immunofluorescent staining of recombinant SB1-008 virus expressing NDV-F protein.
Figure 13 depicts the schematic representation of primer binding sites.
Figure 14 shows the PCR results of vSB1-008.
Figure 15 depicts the Western blot analysis of immunoprecipitated sample from vSB 1-009 infected cells.
Figure 16 depicts the Immunoprecipitation and Western Blot of vHVT114.
Figure 17 depicts the clinical analysis (percentage of birds shedding challenge virus) of the recombinants against CA02 and ZJ1 NDV challenge.
Figure 18 depicts the clinical analysis (oropharyngeal shedding) of the recombinants against NDV challenge.

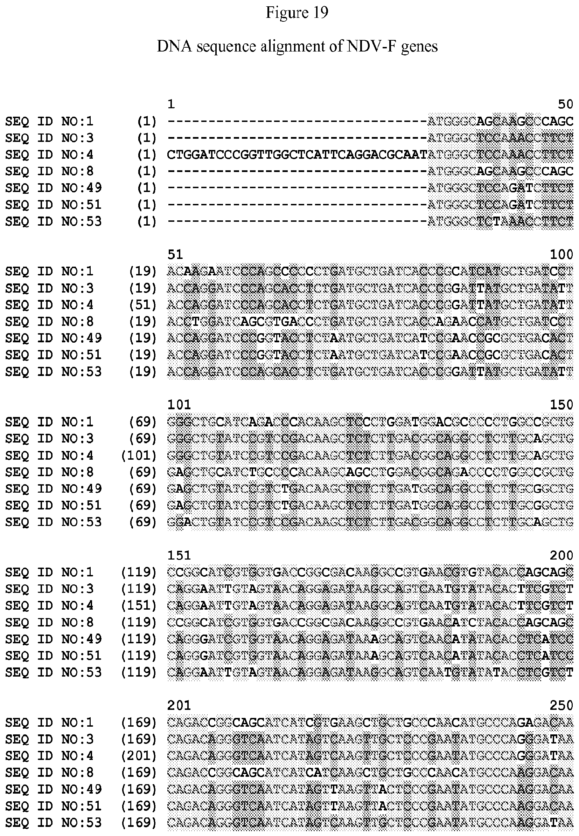

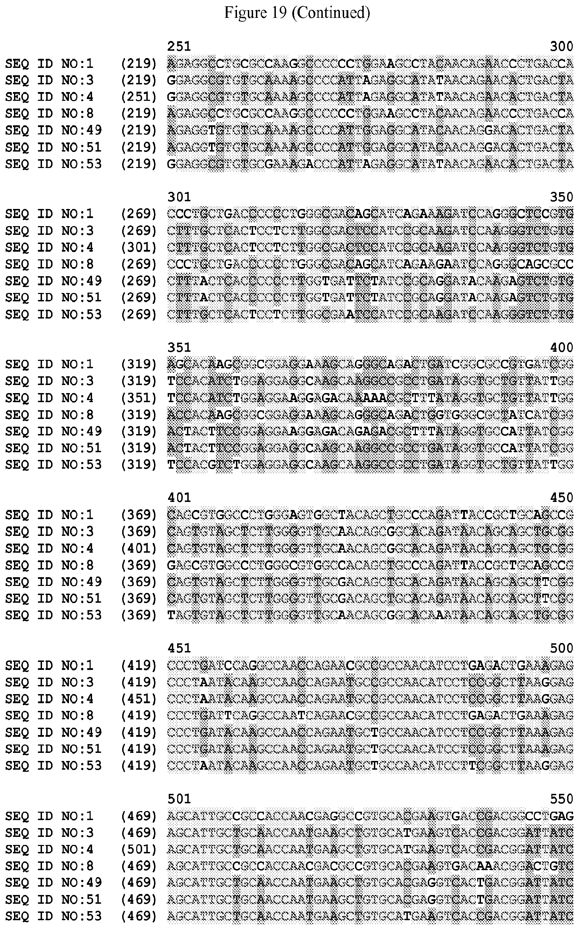

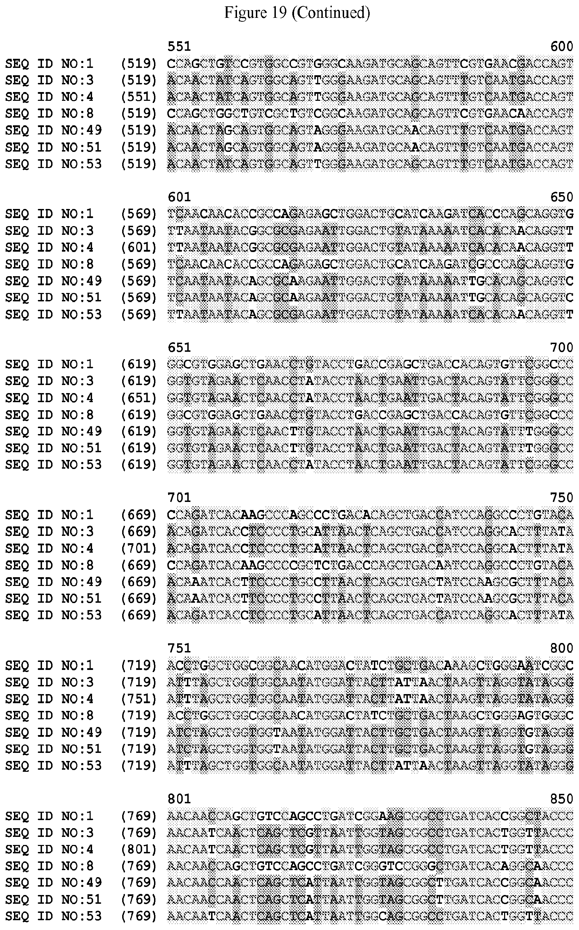

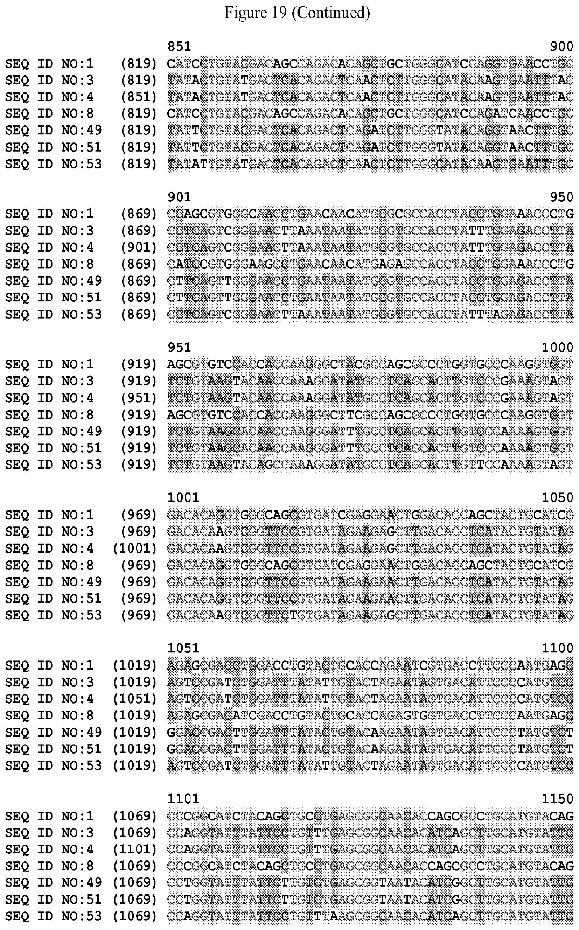

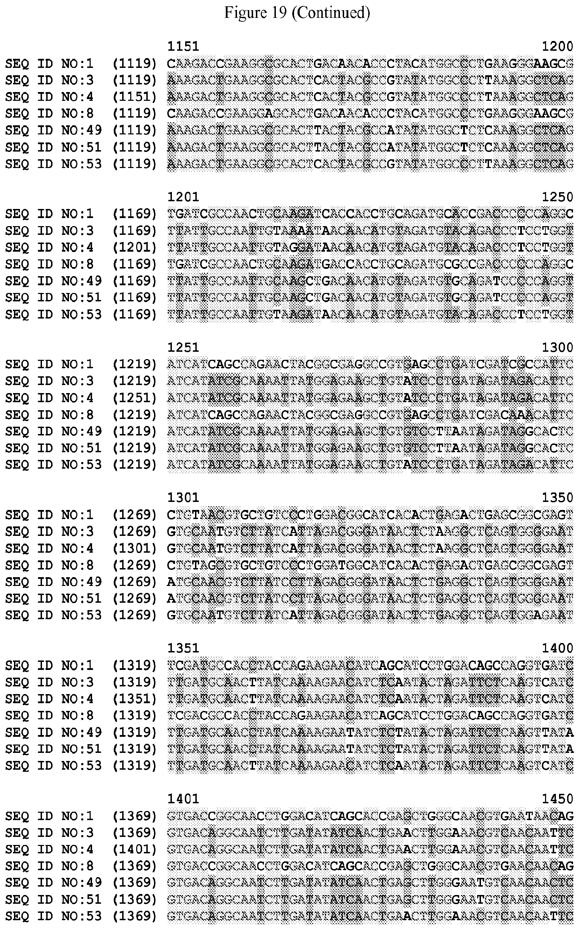

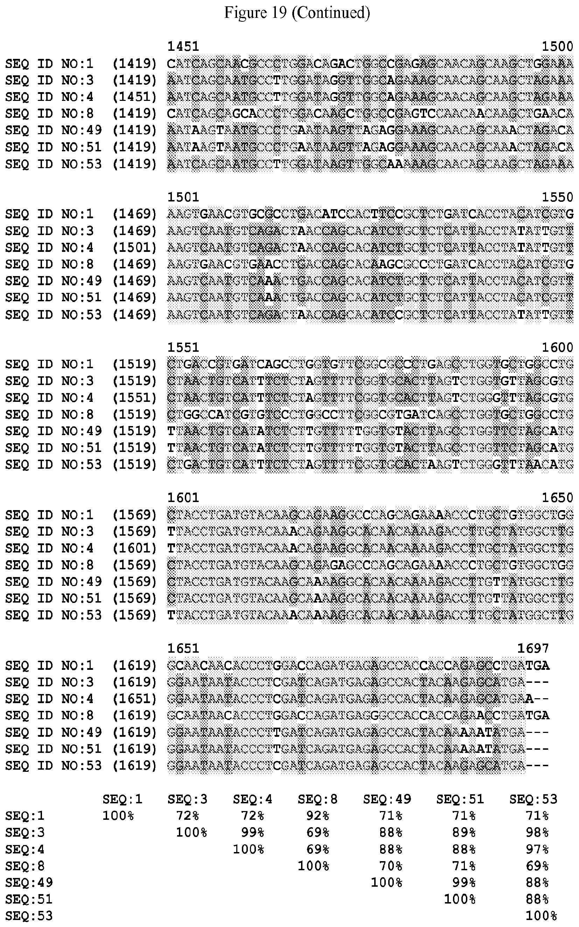

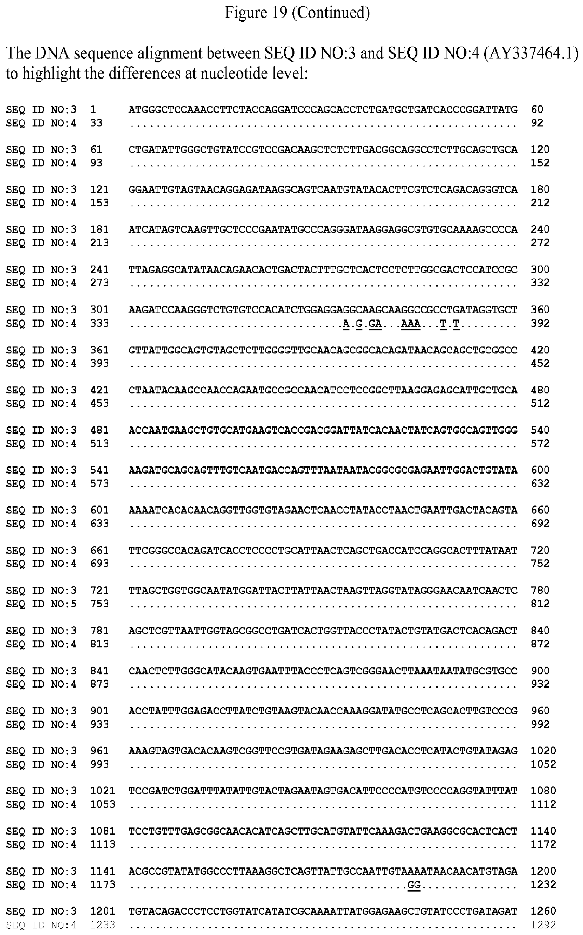

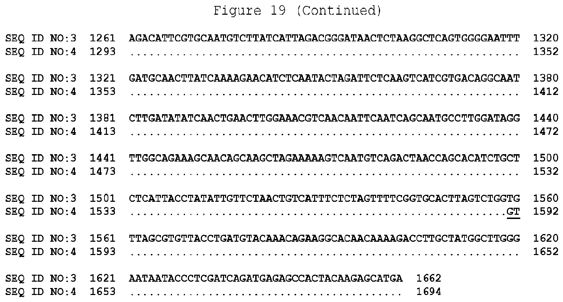

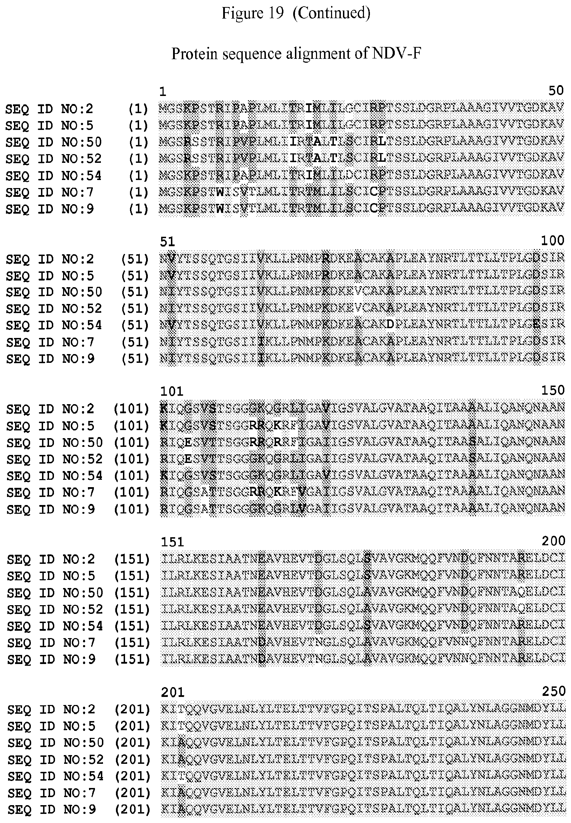

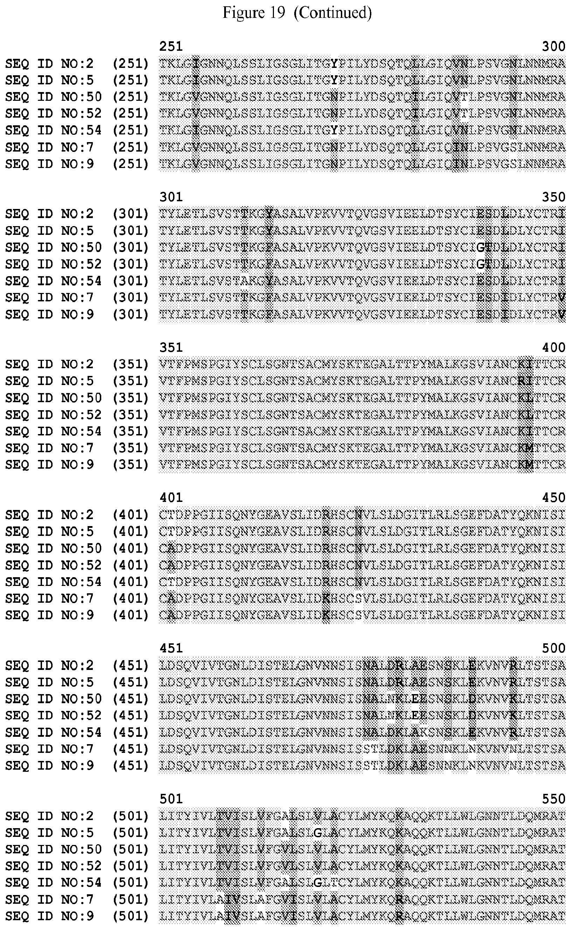

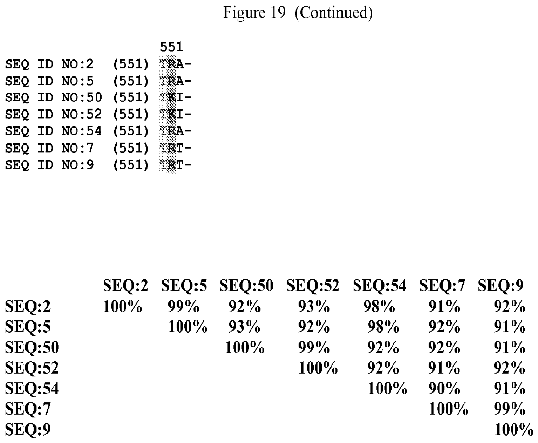

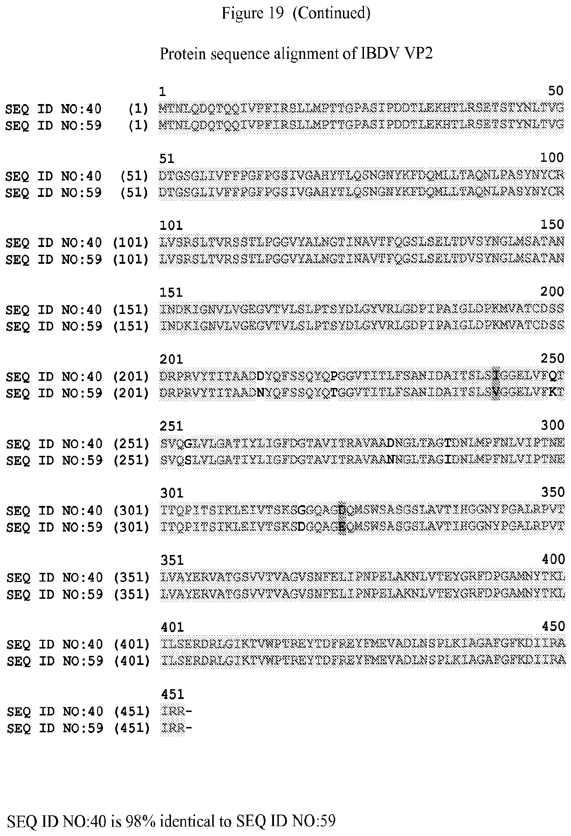

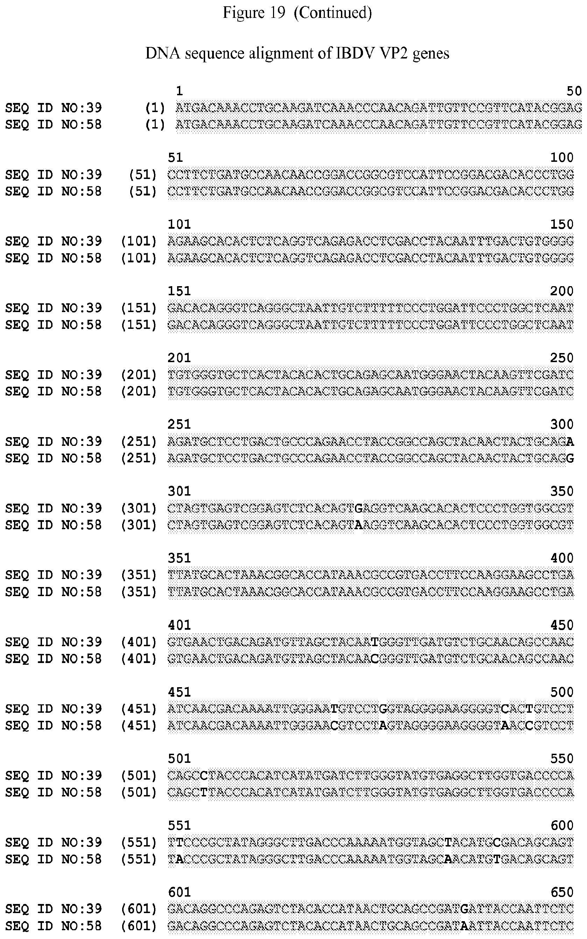

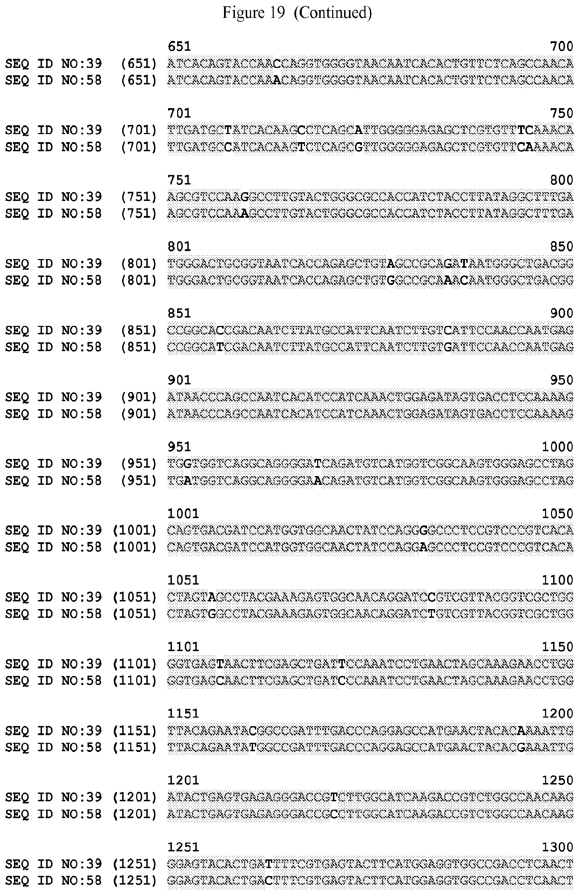

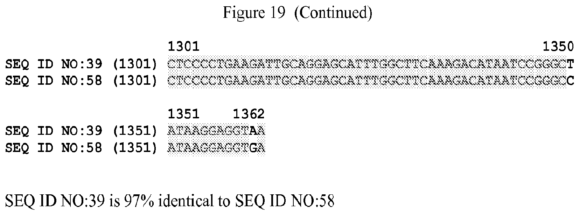

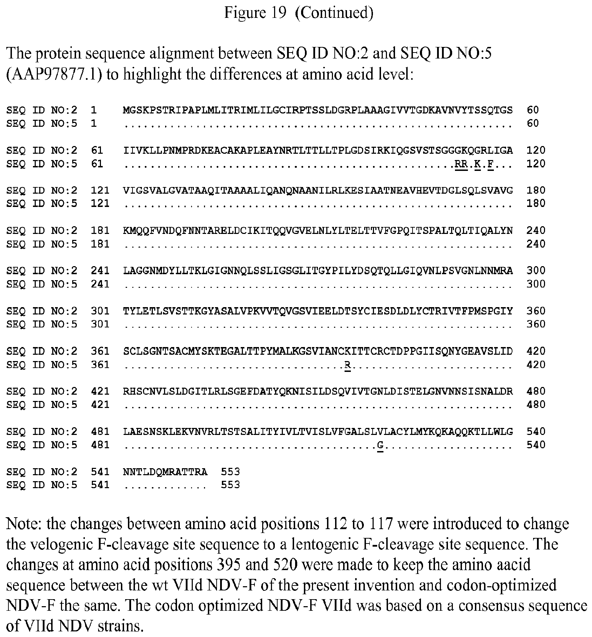

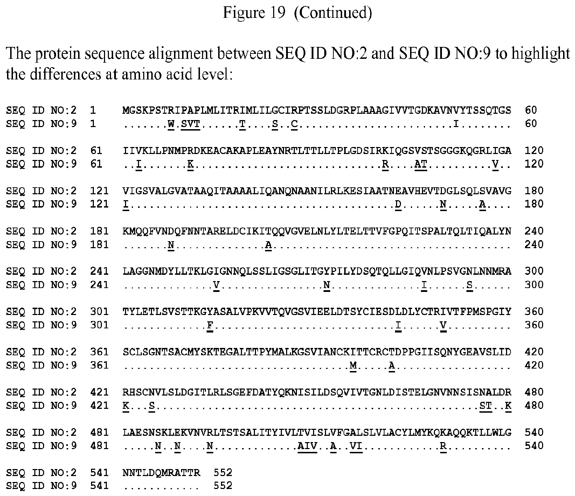

Figure 19 shows the sequence alignment and sequence identity percentage.
Figure 20 shows the DNA and protein sequences.

### DETAILED DESCRIPTION OF THE INVENTION

It is noted that in this disclosure and particularly in the claims, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

Unless otherwise noted, technical terms are used according to conventional usage. Definitions of common terms in molecular biology may be found in Benjamin Lewin, Genes V. published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8). The singular terms "a," "an," and "the" include plural referents unless context clearly indicates otherwise. Similarly, the word "or" is intended to include "and" unless the context clearly indicate otherwise. The word "or" means any one member of a particular list and also includes any combination of members of that list.

The term "animal" is used herein to include all mammals, birds and fish. The animal as used herein may be selected from the group consisting of equine (e.g., horse), canine (e.g., dogs, wolves, foxes, coyotes, jackals), feline (e.g., lions, tigers, domestic cats, wild cats, other big cats, and other felines including cheetahs and lynx), bovine (e.g., cattle), porcine (e.g., pig), ovine (e.g., sheep, goats, lamas, bisons), avian (e.g., chicken, duck, goose, turkey, quail, pheasant, parrot, finches, hawk, crow, ostrich, emu and cassowary), primate (e.g., prosimian, tarsier, monkey, gibbon, ape), humans, and fish. The term "animal" also includes an individual animal in all stages of development, including embryonic and fetal stages.

The terms "polypeptide" and "protein" are used interchangeably herein to refer to a polymer of consecutive amino acid residues.

The term "nucleic acid", "nucleotide", and "polynucleotide" are used interchangeably and refer to RNA, DNA, cDNA, or cRNA and derivatives thereof, such as those containing modified backbones. It should be appreciated that the invention provides polynucleotides comprising sequences complementary to those described herein. The "polynucleotide" contemplated in the present invention includes both the forward strand (5' to 3') and reverse complementary strand (3' to 5'). Polynucleotides according to the invention can be prepared in different ways (e.g. by chemical synthesis, by gene cloning etc.) and can take various forms (e.g. linear or branched, single or double stranded, or a hybrid thereof, primers, probes etc.).

The term "genomic DNA", or "genome" is used interchangeably and refers to the heritable genetic information of a host organism. The genomic DNA comprises the DNA of the nucleus (also referred to as chromosomal DNA) but also the DNA of the plastids (e.g., chloroplasts) and other cellular organelles (e.g., mitochondria). The genomic DNA or genome contemplated in the present invention also refers to the RNA of a virus. The RNA may be a positive strand or a negative strand RNA. The term "genomic DNA" contemplated in the present invention includes the genomic DNA containing sequences complementary to those described herein. The term "genomic DNA" also refers to messenger RNA (mRNA), complementary DNA (cDNA), and complementary RNA (cRNA).

The term "gene" is used broadly to refer to any segment of polynucleotide associated with a biological function. Thus, genes or polynucleotides include introns and exons as in genomic sequence, or just the coding sequences as in cDNAs , such as an open reading frame (ORF), starting from the start codon (methionine codon) and ending with a termination signal (stop codon). Genes and polynucleotides can also include regions that regulate their expression, such as transcription initiation, translation and transcription termination. Thus, also included are promoters and ribosome binding regions (in general these regulatory elements lie approximately between 60 and 250 nucleotides upstream of the start codon of the coding sequence or gene; Doree S M *et al.*; Pandher K *et al.*; Chung J Y *et al*.), transcription terminators (in general the terminator is located within approximately 50 nucleotides downstream of the stop codon of the coding sequence or gene; Ward C K *et al*.). Gene or polynucleotide also refers to a nucleic acid fragment that expresses mRNA or functional RNA, or encodes a specific protein, and which includes regulatory sequences.

The term "heterologous DNA" as used herein refers to the DNA derived from a different organism, such as a different cell type or a different species from the recipient. The term also refers to a DNA or fragment thereof on the same genome of the host DNA wherein the heterologous DNA is inserted into a region of the genome which is different from its original location.

As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a polypeptide, an epitope, a hapten, or any combination thereof. Alternately, the immunogen or antigen may comprise a toxin or antitoxin.

The term "immunogenic protein or peptide" as used herein includes polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the invention comprises or consists essentially of or consists of at least one epitope or antigenic determinant. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, e.g., Epitope Mapping Protocols in Methods in Molecular Biology, Vol. 66 (Glenn E. Morris, Ed., 1996). For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Pat. No. 4,708,871. Similarly, conformational epitopes are readily identified by determining spatial conformation of amino acids such as by, e.g., x-ray crystallography and 2-dimensional nuclear magnetic resonance. See, e.g., Epitope Mapping Protocols, supra.

The term "immunogenic protein or peptide" further contemplates deletions, additions and substitutions to the sequence, so long as the polypeptide functions to produce an immunological response as defined herein. The term "conservative variation" denotes the replacement of an amino acid residue by another biologically similar residue, or the replacement of a nucleotide in a nucleic acid sequence such that the encoded amino acid residue does not change or is another biologically similar residue. In this regard, particularly preferred substitutions will generally be conservative in nature, i.e., those substitutions that take place within a family of amino acids. For example, amino acids are generally divided into four families: (1) acidic--aspartate and glutamate; (2) basiclysine, arginine, histidine; (3) non-polar--alanine, valine, leucine, isoleucine, proline, phenylalanine, methionine, tryptophan; and (4) uncharged polar--glycine, asparagine, glutamine, cysteine, serine, threonine, tyrosine. Phenylalanine, tryptophan, and tyrosine are sometimes classified as aromatic amino acids. Examples of conservative variations include the substitution of one hydrophobic residue such as isoleucine, valine, leucine or methionine for another hydrophobic residue, or the substitution of one polar residue for another polar residue, such as the substitution of arginine for lysine, glutamic acid for aspartic acid, or glutamine for asparagine; or a similar conservative replacement of an amino acid with a structurally related amino acid that will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide. All of the polypeptides produced by these modifications are included herein. The term "conservative variation" also includes the use of a substituted amino acid in place of an unsubstituted parent amino acid provided that antibodies raised to the substituted polypeptide also immunoreact with the unsubstituted polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The terms "recombinant" and "genetically modified" are used interchangeably and refer to any modification, alteration or engineering of a polynucleotide or protein in its native form or structure, or any modification, alteration or engineering of a polynucleotide or protein in its native environment or surrounding. The modification, alteration or engineering of a polynucleotide or protein may include, but is not limited to, deletion of one or more nucleotides or amino acids, deletion of an entire gene, codon-optimization of a gene, conservative substitution of amino acids, insertion of one or more heterologous polynucleotides.

The terms "polyvalent vaccine or composition", "combination or combo vaccine or composition" and "multivalent vaccine or composition" are used interchangeably to refer to a composition or vaccine containing more than one composition or vaccines. The polyvalent vaccine or composition may contain two, three, four or more compositions or vaccines. The polyvalent vaccine or composition may comprise recombinant viral vectors, active or attenuated or killed wild-type viruses, or a mixture of recombinant viral vectors and wild-type viruses in active or attenuated or killed forms.

Described herein is a recombinant Gallid herpesvirus 3 (MDV-2) vector that comprises a mutated Glycoprotein C (gC or UL44) gene. The term "mutated gC gene" refers to the gC gene of Gallid herpesvirus 3 (MDV-2) that is altered or engineered which results in a non-functional gC protein upon expression. The alteration or engineering of the gC gene includes mutation or deletion of a segment of the gC gene which is essential for the expression of a functional gC protein. The term "mutated gC gene" also includes deletion of the entire gC gene of Gallid herpesvirus 3 (MDV-2) wherein gC protein is not expressed. Further described herein is a recombinant Gallid herpesvirus 3 (MDV-2) wherein the Glycoprotein C (gC) gene in the native (wild-type) Gallid herpesvirus 3 (MDV-2) genome encoding the gC protein is deleted. The term "Glycoprotein C (gC) gene" includes any gene or polynucleotide that encodes the Glycoprotein C (gC) of Gallid herpesvirus 3 (MDV-2), and homologs, fragments or variants thereof. The gC gene may encode a gC protein having at least 75%, 80%, 85%, 90%, 95%, 95%, 96%, 97%, 98%, 99%, 99.1%, 99.2%, 99.3%, 99.4%, 99.5%, 99.6%, 99.7%, 99.8% or 99.9% sequence identity to SEQ ID NO: 35, or a variant thereof. The gC gene having at least 75%, 80%, 85%, 90%, 95%, 95%, 96%, 97%, 98%, 99%, 99.1%, 99.2%, 99.3%, 99.4%, 99.5%, 99.6%, 99.7%, 99.8% or 99.9% sequence identity to SEQ ID NO:34 is also described herein.

Another embodiment of the invention provides a recombinant Gallid herpesvirus 3 (MDV-2) viral vector comprising one or more heterologous polynucleotides coding for and expressing at least one antigen or polypeptide of an avian pathogen. The Gallid herpesvirus 3 (MDV-2) strains used for the recombinant viral vector may be any SB-1 strains, including, but not limited to, the commercial Marek's Disease Vaccine (SB-1 vaccine) (Merial Select Inc., Gainesville, GA 30503, USA), the SB-1 strain having the genome sequence as defined by GenBank Accession Number HQ840738.1. The Gallid herpesvirus 3 (MDV-2) strains used for the recombinant viral vector described herein may be any other Gallid herpesvirus 3 isolate including the HPRS24 strain having the genome sequence as defined by GenBank Accession Number AB049735.1, or the HPRS24 strain having the genome sequence as defined by GenBank Accession Number NC_002577.1. The genomes of HPRS24 and SB-1 share 98.4% sequence identity (Spatz and Schat, 2011; Virus Gene 42, 331-338). The Gallid herpesvirus 3 (MDV-2) strains used for the recombinant viral vector described herein may be the 301B/1 isolate described by Witter (1987 Avian Dis 31, 752-765) or by Witter et al. (1987 Avian Dis 31, 829-840). The Gallid herpesvirus 3 (MDV-2) strains described hereinmay be any Gallid herpesvirus 3 (MDV-2) strains comprising the genome sequence having at least 90%, 91%, 92%, 93%, 94%, 95%, 96%, 97%, 98%, or 99% sequence identity to the sequence as defined in GenBank Accession Number HQ840738.1 (SEQ ID NO:14), AB049735.1, or NC_002577.1.

The genes coding for antigen or polypeptide described herein may be those coding for Newcastle Disease Virus fusion protein (NDV-F), Newcastle Disease Virus hemagglutinin neuraminidase (NDV-HN), Marek's Disease Virus glycoprotein C (gC), Marek's Disease Virus glycoprotein B (gB), Marek's Disease Virus glycoprotein E (gE), Marek's Disease Virus glycoprotein I (gI), Marek's Disease Virus glycoprotein H (gH) or Marek's Disease Virus glycoprotein L (gL), IBDV VP2, IBDV VPX, IBDV VP3, IBDV VP4, ILTV glycoprotein B, ILTV glycoprotein I, ILTV UL32, ILTV glycoprotein D, ILTV glycoprotein E, ILTV glycoprotein C, influenza hemaglutinin (HA), influenza neuraminidase (NA), protective genes derived from Mycoplasma gallisepticum (MG), or Mycoplasma synoviae (MS), or combinations thereof. The antigen or polypeptide may be any antigen from the poultry pathogen selected form the group consisting of avian encephalomyelitis virus, avian reovirus, avian paramyxovirus, avian metapneumovirus, avian influenza virus, avian adenovirus, fowl pox virus, avian coronavirus, avian rotavirus, chick anemia virus, avian astrovirus, avian parvovirus, coccidiosis (*Eimeria sp.), Campylobacter sp., Salmonella sp., Pasteurella sp., Avibacterium sp., Mycoplasma gallisepticum, Mycoplasma synoviae, Clostridium sp.,* and *E. coli.*

Moreover, homologs of aforementioned antigen or polynucleotides are intended to be within the scope of the present description. As used herein, the term "homologs" includes orthologs, analogs and paralogs. The term "analogs" refers to two polynucleotides or polypeptides that have the same or similar function, but that have evolved separately in unrelated organisms. The term "orthologs" refers to two polynucleotides or polypeptides from different species, but that have evolved from a common ancestral gene by speciation. Normally, orthologs encode polypeptides having the same or similar functions. The term "paralogs" refers to two polynucleotides or polypeptides that are related by duplication within a genome. Paralogs usually have different functions, but these functions may be related. Analogs, orthologs, and paralogs of a wild-type polypeptide can differ from the wild-type polypeptide by post-translational modifications, by amino acid sequence differences, or by both. In particular, homologs described herein will generally exhibit at least 80-85%, 85-90%, 90-95%, or 95%, 96%, 97%, 98%, 99% sequence identity, with all or part of the polynucleotide or polypeptide sequences of antigens described above, and will exhibit a similar function.

In one embodiment, the present invention provides a recombinant Gallid Herpesvirus-3 (MDV-2) viral vector comprising one, two or more heterologous polynucleotides coding for and expressing the NDV-F antigen or polypeptide. In one aspect of the embodiment, the NDV-F antigen or polypeptide has at least 70%, 75%, 80%, 85%, 90%, 95%, 96%, 97%, 98% or 99% sequence identity to a polypeptide having the sequence as set forth in SEQ ID NO:2, 9, 50, 52, or 54, or a conservative variant, an allelic variant, a homolog or an immunogenic fragment comprising at least eight or at least ten consecutive amino acids of one of these polypeptides, or a combination of these polypeptides. In another aspect of the embodiment, the heterologous polynucleotide encoding an NDV-F antigen or polypeptide has at least 70%, 75%, 80%, 85%, 90%, 95%, 96%, 97%, 98% or 99% sequence identity to a polypeptide having the sequence as set forth in SEQ ID NO:2, 9, 50, 52, or 54. In yet another aspect of the embodiment, the heterologous polynucleotide has at least 70%, 75%, 80%, 85%, 90%, 95%, 96%, 97%, 98% or 99% sequence identity to a polynucleotide having the sequence as set forth in SEQ ID NO:1, 8, 49, 51, or 53.

Variants include allelic variants. The term "allelic variant" refers to a polynucleotide or a polypeptide containing polymorphisms that lead to changes in the amino acid sequences of a protein and that exist within a natural population (e.g., a virus species or variety). Such natural allelic variations can typically result in 1- 5% variance in a polynucleotide or a polypeptide. Allelic variants can be identified by sequencing the nucleic acid sequence of interest in a number of different species, which can be readily carried out by using hybridization probes to identify the same gene genetic locus in those species. Any and all such nucleic acid variations and resulting amino acid polymorphisms or variations that are the result of natural allelic variation and that do not alter the functional activity of gene of interest, are intended to be within the scope of the invention.

The term "identity" with respect to sequences can refer to, for example, the number of positions with identical nucleotides or amino acids divided by the number of nucleotides or amino acids in the shorter of the two sequences wherein alignment of the two sequences can be determined in accordance with the Wilbur and Lipman algorithm (Wilbur and Lipman). The sequence identity or sequence similarity of two amino acid sequences, or the sequence identity between two nucleotide sequences can be determined using Vector NTI software package (Invitrogen, 1600 Faraday Ave., Carlsbad, CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

The polynucleotides of the disclosure include sequences that are degenerate as a result of the genetic code, e.g., optimized codon usage for a specific host. As used herein, "optimized" refers to a polynucleotide that is genetically engineered to increase its expression in a given species. To provide optimized polynucleotides coding for NDV-F polypeptides, the DNA sequence of the NDV-F protein gene can be modified to 1) comprise codons preferred by highly expressed genes in a particular species; 2) comprise an A+T or G+C content in nucleotide base composition to that substantially found in said species; 3) form an initiation sequence of said species; or 4) eliminate sequences that cause destabilization, inappropriate polyadenylation, degradation and termination of RNA, or that form secondary structure hairpins or RNA splice sites. Increased expression of NDV F protein in said species can be achieved by utilizing the distribution frequency of codon usage in eukaryotes and prokaryotes, or in a particular species. The term "frequency of preferred codon usage" refers to the preference exhibited by a specific host cell in usage of nucleotide codons to specify a given amino acid. There are 20 natural amino acids, most of which are specified by more than one codon. Therefore, all degenerate nucleotide sequences are included in the disclosure as long as the amino acid sequence of the NDV-F polypeptide encoded by the nucleotide sequence is functionally unchanged.

Further described herein is a method for producing a recombinant Gallid Herpesvirus-3 or SB-1 viral vector comprising the introduction into the SB-1 genome of one, two or more isolated polynucleotides in a nonessential region of the SB-1 genome. Further described herein is a method for producing a recombinant Gallid Herpesvirus-3 or SB-1 viral vector comprising the steps of altering, engineering, or deleting the gC gene from the SB-1 genome. The term "nonessential region" refers to a region of a virus genome which is not essential for replication and propagation of the virus in tissue culture or in chickens. Any nonessential region or portion thereof can be deleted from the SB-1 genome or a foreign sequence can be inserted in it, and the viability and stability of the recombinant Gallid Herpesvirus-3 or SB-1 vector resulting from the deletion or insertion can be used to ascertain whether a deleted region or portion thereof is indeed nonessential. In one aspect of the embodiment, the non-essential regions are located in the unique long (UL) and unique short (US) regions of the SB-1 genome (see Spatz et al., Virus Genes 42:331-338, 2011). The UL region of SB-1 is about 109,744 bp to about 109,932 bp in length and may extend from positions 12,209 to 121,952 of SEQ ID NO:14 (GenBank accession No, HQ840738.1) or equivalent positions of other SB1-genomes, for example, from 11,826 bp to 121,757 bp of HPRS24 genome. The US region of SB-1 is about 12,109 bp to about 12,910 bp in
length and may extend from positions 143,514 to 156,423 of SEQ ID NO:14 (GenBank accession No, HQ840738.1) or equivalent positions of other SB1-genomes, for example from 142,681bp to 154,789 bp of HPRS24 genome (Spatz et al., 2011). Described herein, the non-essential region is between ORF of UL55 and ORF of LORF5 in the unique long (UL) region of SB-1. Described herein, the polynucleotide is inserted into or to replace SB-1 glycoprotein C gene (also designated UL44). The use of the gC locus may allow the generation of recombinant virus unable to produce a functional gC protein and unable to be transmitted horizontally. Described herein, the nonessential region may be in the intergenic regions between UL7 and UL8, between UL 21 and UL22, between UL40 and UL41, between UL50 and UL51, between UL54 and LORF4, between US10 and SORF4, or within the UL43, US2, US 10 or US6 (coding for gD) gene (see GenBank accession No, HQ840738.1). Described herein, the nonessential regions may be in the region of nucleotide positions 118057-118306 (intergenic UL55-LORF5), 98595-100031 (gC or UL44), 25983-26038 (intergenic UL7-UL8), 49865-50033 (intergenic UL21-UL22), 75880-75948 (intergenic UL35-UL36), 93928-93990 (intergenic UL40-UL41), 109777-109847 (intergenic UL50-UL51), 116466-116571 (intergenic UL54-LORF4), 146548-146697 (intergenic US10-SORF4), 97141-98385 (UL43), 147857-148672 (US2), 145853..146548 (US10) or 150322-151479 (gD or US6) of SEQ ID NO:14.

Construction of recombinant virus is well known in the art as described in, e.g., US patent Nos. 4,769,330, 4,722,848, 4,603, 112, 5,174, 993, and 5,756,103, 6,719,979. Specifically, a recombinant Gallid Herpesvirus-3 (MDV-2) viral vector may be constructed in two steps. First, the Gallid Herpesvirus-3 (MDV-2) or SB-1 genomic regions flanking the locus of insertion are cloned into an *E.coli* plasmid construct; unique(s) restriction site(s) is (are) placed between the two flanking regions (insertion plasmid) in order to allow the insertion of the donor expression cassette DNA. Separately, the cDNA or DNA gene sequence to be inserted is preceded by a promoter region (gene start region) and a terminator (or poly-adenylation,poly-A) sequence which is specific for the Gallid Herpesvirus-3 (MDV-2) or SB-1 vector and/or eukaryotic cells. The whole expression cassette (promoter-foreign gene-poly-A) is then cloned into the unique(s) restriction site(s) of the insertion plasmid to construct the "donor plasmid" which contains the expression cassette flanked by Gallid Herpesvirus-3 (MDV-2) or SB-1 "arms" flanking the insertion locus. The resulting donor plasmid construct is then amplified by growth within *E.coli* bacteria and plasmid DNA is extracted. This plasmid is then linearized using a restriction enzyme that cut the plasmid backbone (outside the Gallid Herpesvirus-3 (MDV-2) or SB-1 arms and expression cassette). Chicken embryo fibroblasts are then co-transfected with parental Gallid Herpesvirus-3 (MDV-2) or SB-1 DNA and linearized donor plasmid DNA. The resulting virus population is then cloned by multiple limiting dilution steps where viruses expressing the foreign gene are isolated from the non-expressing viral population. Similarly, another foreign cassette can be inserted in another locus of insertion to create a double Gallid Herpesvirus-3 (MDV-2) or SB-1 recombinant expressing two genes. The second cassette can also be inserted into the same locus. The Gallid Herpesvirus-3 (MDV-2) or SB-1 recombinant is produced in primary chicken embryo fibroblasts similarly to the parental Gallid Herpesvirus-3 (MDV-2) or SB-1 MD vaccine. After incubation, infected cells are harvested, mixed with a freezing medium allowing survival of infected cells, and frozen usually in cryovial or glass ampoules and stored in liquid nitrogen.

Successful expression of the inserted cDNA genetic sequence by the modified infectious virus requires two conditions. First, the insertion must be introduced into a region of the genome of the virus in order that the modified virus remains viable. The second condition for expression of inserted cDNA is the presence of a regulatory sequences allowing expression of the gene in the viral background (for instance: promoter, enhancer, donor and acceptor splicing sites and intron, Kozak translation initiation consensus sequence, polyadenylation signals, untranslated sequence elements).

In general, it is advantageous to employ a strong promoter functional in eukaryotic cells. The promoters include, but are not limited to, an immediate early cytomegalovirus (CMV) promoter, guinea pig CMV promoter, an SV40 promoter, Pseudorabies Virus promoters such as that of glycoprotein X promoter, Herpes Simplex Virus-1 such as the alpha 4 promoter, Marek's Disease Viruses (including MDV-1, MDV-2 and HVT) promoters such as those driving glycoproteins gC, gB, gE, or gI expression, Infectious Laryngotracheitis Virus promoters such as those of glycoprotein gB, gE, gI, gD genes, or other herpesvirus promoters. When the insertion locus consists of a SB-1 gene (for instance, gC, gD, US2 or US10 genes), the foreign gene can be inserted into the vector with no additional promoter sequence since the promoter of the deleted gene of the vector will drive the transcription of the inserted foreign gene.

Described herein is a pharmaceutical composition or vaccine comprising one or more recombinant Gallid Herpesvirus-3 (MDV-2) rival vectors of the present invention and a pharmaceutically or veterinarily acceptable carrier, excipient, vehicle or adjuvant. The Gallid herpesvirus 3 (MDV-2) strains used for the recombinant Gallid Herpesvirus-3 viral vector may be any SB-1 strains, the HPSR24 strains, or the 301B/1 strains. The Gallid Herpesvirus-3 (MDV-2) strains may also include those described in Witter et al (Avian Diseases 34, 944-957; 1990), Witter (Avian Pathology 21, 601-614, 1992) and Witter (Avian Pathology 24, 665-678, 1995): 280-5/1, 281MI/1, 287C/1, 298B/1, 301A/1, 401/1, 437A/1, 437B/1, 468A/1, 468A/2, 468B/1, 471B/1, or HN-1/1.

In another embodiment, the present invention provides a composition or vaccine comprising: i) a recombinant Gallid Herpesvirus-3 vector (MDV-2) comprising heterologous polynucleotides coding for and expressing at least one antigen of an avian pathogen; and ii) at least one of: a recombinant HVT vector (or MDV-3 or Meleagrid Herpesvirus-1) comprising heterologous polynucleotides coding for and expressing at least one antigen of an avian pathogen; or wild type MDV-3; or recombinant MDV-1 vector (or Gallid herpesvirus-2) comprising heterologous polynucleotides coding for and expressing at least one antigen of an avian pathogen; or wild type MDV-1. The composition or vaccine may further comprise a pharmaceutically or veterinarily acceptable carrier, excipient, vehicle or adjuvant. This composition may further contain a recombinant fowlpox vector comprising heterologous polynucleotides coding for and expressing at least one antigen of an avian pathogen; or wild type fowlpox.

In one aspect of the embodiment, the composition or vaccine comprises one (or more) recombinant Gallid Herpesvirus-3 (MDV-2) vectors and one or more wild type HVT (MDV-3). In another aspect, the composition or vaccine comprises one (or more) recombinant Gallid Herpesvirus-3 (MDV-2) vectors and one or more recombinant HVT (MDV-3). In another aspect, the composition or vaccine comprises one or more recombinant Gallid Herpesvirus-3 (MDV-2) vectors and one or more wild type or genetically modified MDV-1. In another aspect, the composition or vaccine comprises one or more recombinant Gallid Herpesvirus-3 (MDV-2) vectors and one or more recombinant MDV-1. In another aspect, the composition or vaccine comprises one or more recombinant Gallid Herpesvirus-3 (MDV-2) vectors, one or more wild type HVT (MDV-3) and one or more wild type MDV-1. In another aspect, the composition or vaccine comprises one or more recombinant Gallid Herpesvirus-3 (MDV-2) vectors, one or more recombinant HVT (MDV-3) and one or more wild type MDV-1. In another aspect, the composition or vaccine comprises one or more recombinant Gallid Herpesvirus-3 (MDV-2) vectors, one or more wild type HVT (MDV-3) and one or more recombinant MDV-1. In yet another aspect, the composition or vaccine comprises one or more recombinant Gallid Herpesvirus-3 (MDV-2) vectors, one or more recombinant HVT (MDV-3) and one or more recombinant MDV-1. The wild type HVT (MDV-3) or wild type MDV-1 may be live, attenuated or genetically modified. The heterologous polynucleotides in recombinant Gallid Herpesvirus-3 (MDV-2) vectors, recombinant HVT (MDV-3) vectors, and recombinant MDV-1 vectors may encode same or different antigens from the same or different avian pathogens.

The pharmaceutically or veterinarily acceptable carriers or adjuvant or vehicles or excipients are well known to the one skilled in the art. For example, a pharmaceutically or veterinarily acceptable carrier or adjuvant or vehicle or excipient can be Marek's disease vaccine diluent used for MD vaccines. Other pharmaceutically or veterinarily acceptable carrier or adjuvant or vehicle or excipients that can be used for methods of this invention include, but are not limited to, 0.9% NaCl (e.g., saline) solution or a phosphate buffer, poly-(L-glutamate) or polyvinylpyrrolidone. The pharmaceutically or veterinarily acceptable carrier or vehicle or excipients may be any compound or combination of compounds facilitating the administration of the vector (or protein expressed from an inventive vector *in vitro*), or facilitating transfection or infection and/or improve preservation of the vector (or protein). Doses and dose volumes are herein discussed in the general description and can also be determined by the skilled artisan from this disclosure read in conjunction with the knowledge in the art, without any undue experimentation.

Optionally other compounds may be added as pharmaceutically or veterinarily acceptable carriers or adjuvant or vehicles or excipients, including, but not limited to, alum; CpG oligonucleotides (ODN), in particular ODN 2006, 2007, 2059, or 2135 (Pontarollo R.A. et al., Vet. Immunol. Immunopath, 2002, 84: 43-59; Wernette C.M. et al., Vet. Immunol. Immunopath, 2002, 84: 223-236; Mutwiri G. et al., Vet. Immunol. Immunopath, 2003, 91: 89-103); polyA-polyU, dimethyldioctadecylammonium bromide (DDA) ("Vaccine Design The Subunit and Adjuvant Approach", edited by Michael F. Powell and Mark J. Newman, Pharmaceutical Biotechnology, 6: p.03, p.157); N,N-dioctadecyl-N',N'-bis(2-hydroxyethyl) propanediamine (such as AVRIDINE^{®}) (*Ibid,* p. 148); carbomer, chitosan (*see* US Patent Serial No. 5,980,912 for example).

The pharmaceutical compositions and vaccines according to the invention may comprise or consist essentially of one or more adjuvants. Suitable adjuvants for use in the practice of the present invention are (1) polymers of acrylic or methacrylic acid, maleic anhydride and alkenyl derivative polymers, (2) immunostimulating sequences (ISS), such as oligodeoxyribonucleotide sequences having one or more non-methylated CpG units (Klinman et al., 1996; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, e.g., DDA (5) cytokines, (6) aluminum hydroxide or aluminum phosphate, (7) saponin or (8) other adjuvants discussed in any document cited in the instant application, or (9) any combinations or mixtures thereof.

Described herein is a method for inducing an immunological response in an animal against one or more antigens or a protective response in an animal against one or more avian pathogens, which method comprises inoculating the animal at least once with the vaccine or pharmaceutical composition of the present invention. Further described herein is to a method for inducing an immunological response in an animal to one or more antigens or a protective response in an animal against one or more avian pathogens in a prime-boost administration regimen, which is comprised of at least one primary administration and at least one booster administration using at least one booster administration using at least one common polypeptide, antigen, epitope or immunogen. The immunological composition or vaccine used in primary administration may be same, may be different in nature from those used as a booster.

The avian pathogens may be Newcastle Disease Virus (NDV), Infectious Bursal Disease Virus (*i.e*., IBDV or Gumboro Disease virus), Marek's Disease Virus (MDV), Infectious Laryngotracheitis Virus (ILTV), avian encephalomyelitis virus and other picornavirus, avian reovirus, avian paramyxovirus, avian metapneumovirus, avian influenza virus, avian adenovirus, fowl pox virus, avian coronavirus, avian rotavirus, avian parvovirus, avian astrovirus and chick anemia virus, coccidiosis (*Eimeria sp.)*, *Campylobacter sp., Salmonella sp., Mycoplasma gallisepticum, Mycoplasma synoviae, Pasteurella sp., Avibacterium sp., E. coli* or *Clostridium sp.*

Usually, one administration of the vaccine is performed either at one day-of-age by the subcutaneous or intramuscular route or *in ovo* in 17-19 day-old embryo. A second administration can be done within the first 10 days of age. The animals are preferably at least 17-day-embryo or one day old at the time of the first administration.

A variety of administration routes in day-old chicks may be used such as subcutaneously or intramuscularly, intradermally, transdermally. The *in ovo* vaccination can be performed in the amniotic sac and/or the embryo. Commercially available *in ovo* and SC administration devices can be used for vaccination.

The invention will now be further described by way of the following non-limiting examples.

### EXAMPLES

Construction of DNA inserts, plasmids and recombinant viral vectors was carried out using the standard molecular biology techniques described by J. Sambrook et al. (Molecular Cloning: A Laboratory Manual, 2nd Edition, Cold Spring Harbor Laboratory, Cold Spring Harbor, New York, 1989).

### Example 1 Construction of recombinant vSB1-004 expressing NDV-F

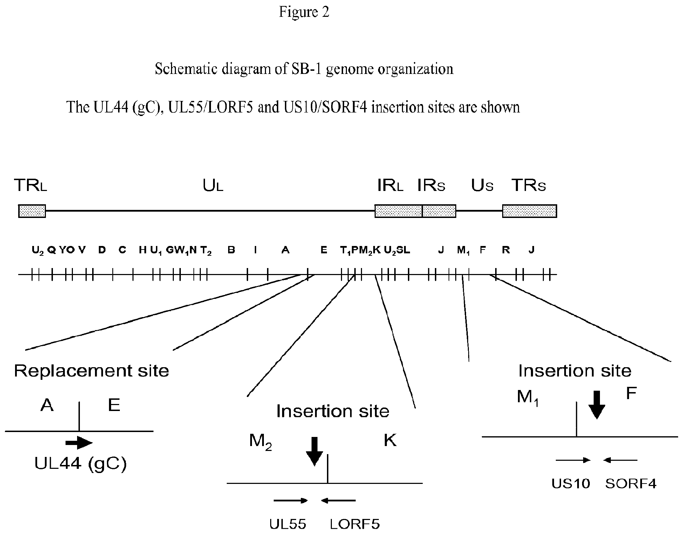

The aim of the work is to construct a recombinant SB-1 virus in which an expression cassette containing mouse cytomegalovirus (mCMV) promoter, Newcastle disease virus fusion protein (NDV-F), and Simian virus 40 (SV40) poly A tail is inserted into the intergenic site between US10 and SORF4 site of SB-1 virus (Table 1 and Fig. 2).

**Table 1 Characteristics of vSB1-004**

| Name | Parental virus | Promoter | gene | Poly-A | Locus |
|---|---|---|---|---|---|
| vSB1-004 | SB-1* | mCMVIE | Wt-NDV-F of VIId | SV40 | SORF4/US10 |
| SB-1*: Merial's commercial Marek's Disease Vaccine SB-1(Merial Select Inc., Gainesville, GA 30503, USA). Vaccine Lot# JV505. | | | | | |

A Newcastle disease virus Fusion Protein (NDV-F) corresponding to genotype VIId sequence (SEQ ID NO:2 encoded by SEQ ID NO:3) was chemically synthesized (GenScript, Piscataway, NJ, USA). The F protein cleavage site of this synthetic gene was altered to match with a lentogenic F cleavage site sequence and the resultant NDV-F gene sequence has 99% nucleotide as well as 99% amino acid sequence identity to NDV-F sequence deposited in GenBank under accession number AY337464 (for DNA) and AAP97877.1 (for protein), respectively .

### Donor plasmid SB-1 US10mFwt SbfI construction

A fragment containing the synthetic NDV-F gene was excised from pUC57 NDV-F VIId wt plasmid (synthesized by GeneScript) using NotI and inserted into the same site of pCD046 plasmid containing mCMV promoter and SV40 polyA tail. The resultant plasmid, pCD046+NDV-F wt was digested with *EcoR*I and *Sal*I and blunt ended with Klenow. A 3.3kb fragment was gel extracted and ligated to a *Sma*I digested and dephosphorylated (CIPed) vector (SB1 US10-SORF4 SbfI pUC57) containing flanking arms. Ligated material was transformed using Top10 Oneshot kit (Invitrogen, CA, USA). Bacterial colonies were grown in LBamp broth, plasmid extracted by using Qiagens MiniSpin Prep kit, and screened for insert orientation using *Pst*I digestion. The correct donor plasmid was designated SB-1 10mFwt SbfI. Large scale cultures were grown and plasmid extraction was done using Qiagens Maxi Prep kit. Transient expression of the maxi preps was verified using Fugene Transfection Reagent in Chicken Embryo Fibroblast Cells (CEF's) and chicken polyclonal sera against NDV.

### Recombinant generation

A standard homologous recombination procedure was followed by co-electroporation of secondary CEF cells using SB-1 US10mFwt SbfI donor plasmid and viral DNA isolated from vaccine strain of SB-1 virus. Co-electroporation was performed using 1x10⁷ 2° CEF in 300 µl Opti-MEM and shocked at 150 volts with 950 capacitance in a 2 mm electroporation cuvette. The transfected cells were seeded into 96-well plate and incubated for 5-7 days. The cells grown in the 96-well plate were then treated with trypsin and transferred into two "sisters" 96-well plates and incubated for 5 more days. One set of 96-well plates was used for IFA using chicken polyclonal sera against NDV-F to identify positive wells containing recombinants and another set of 96-well plates was used for recovering the infected cells from the positive wells.

The recombinant viral purification methods were performed first by 96-well plate duplication and IFA selection for the wells containing the most IFA positive plaques with the least amount of IFA negative plaques. Wells matching those criteria were then harvested and adjusted to 1ml in DMEM+2% FBS. From the 1ml stock, 5-20µl (depending on the number of visible plaques) were removed and mixed with 1x10⁷ CEFs in 10ml DMEM+2% FBS and aliquoted onto a new 96-well plate to have single SB-1 plaques per well. The 96-well plates were duplicated after 5 days of incubation and wells that contained plaques were tested for the presence of recombinant SB-1 and absence of parental virus by IFA and PCR. Again the wells that appeared to have more recombinant virus, by comparing the PCR banding results, were harvested and adjusted to 1ml and aliquoted onto new 96-well plates. After three to five rounds of purification of virus infected cells, recombinant SB-1 expressing NDV-F protein was isolated and the purity of the recombinant virus was tested by IFA and PCR to confirm the absence of parental virus. Selected recombinant virus was then passed from one well of a 96-well plate (P0) to 2xT-25 flasks (P1), then 2xT-75 flasks (P2), 2xT-175 flasks (P3), and finally 2x850 cm² roller bottles (pre-MSV stock or P4). Vials with 2 ml aliquot were stored in liquid nitrogen. Titrations were performed in triplicate on CEFs and a titer of 1x10⁵ pfu/ml was obtained for SB1-004.

### Expression analysis

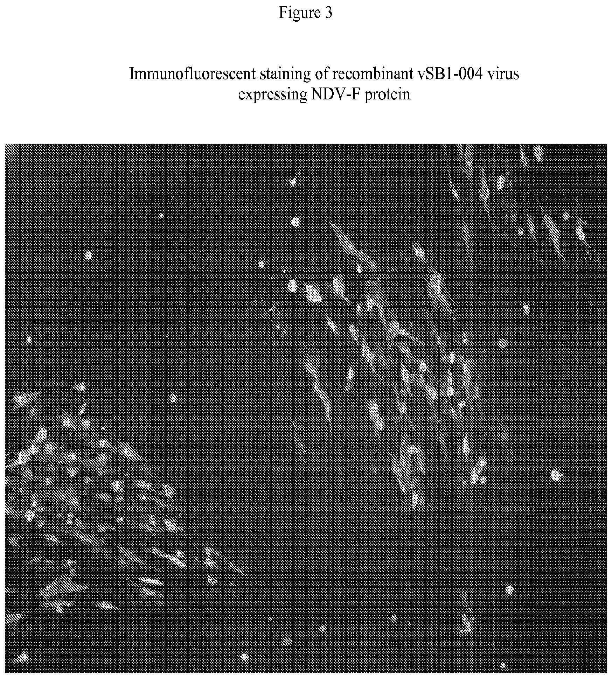

For immunofluorescence testing, the P3 material was diluted 1:100 in media. Approximately 50 µl of the diluted virus was added to 10 ml of DMEM+2% FBS with 1x10⁷ CEFs and then aliquoted onto a 96 well plate (100 µl/well). The plates were incubated for 5 days at 37°C+5%CO₂ until viral plaques were visible. The plates were fixed with 95% ice-cold acetone for three minutes and washed three times with PBS. Chicken anti-sera against Newcastle Disease Virus (lot#C0139, Charles Rivers Laboratory) at 1:1000 was added and the plates were incubated at 37°C for 1 hour. After one hour incubation, the plates were washed three times with PBS and FITC anti-chicken (cat# F8888, Sigma) was added at 1:500. Again the plates were incubated at 37°C for 1 hour. After one hour incubation the cells were rinsed three times with PBS and visualized with a fluorescent microscope using fluorescein isothiocyanate (FITC) filter. All examined plaques of vSB1-004 were found to express NDV-F protein (Fig. 3).

### Analysis of recombinant by PCR

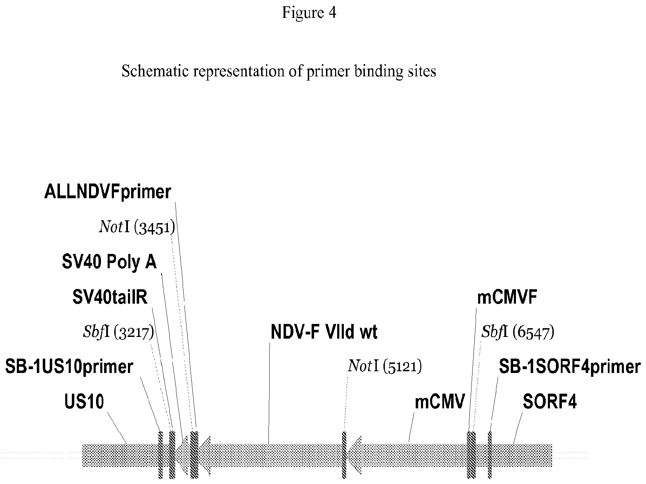

DNA was extracted from a stock virus by phenol/chloroform extraction, ethanol precipitated, and resuspended in 20mM HEPES. PCR primers were designed to specifically identify the NDV-F VIId gene, the promoter, the SV40 poly A and the SB-1 flanking arms (see Fig. 4). Primers, specific to HVT (strain FC126), MDV serotype 3 (MB080 +MB081) were also included in the analysis to check the purity of the recombinant virus from SB-1 parental virus. PCR was performed using 200 µg of DNA template along with the specified primers pairs.

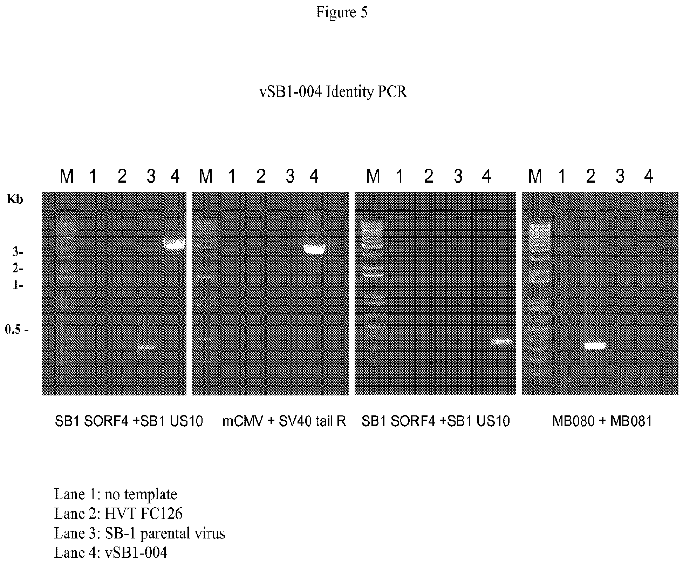

The PCR reactions with all primer pairs resulted in the expected PCR products and banding patterns. The PCR results demonstrate that recombinant virus vSB 1-004 carries the intended expression cassette and the virus stock is free from detectable amounts of parental SB-1 virus (Fig. 5).

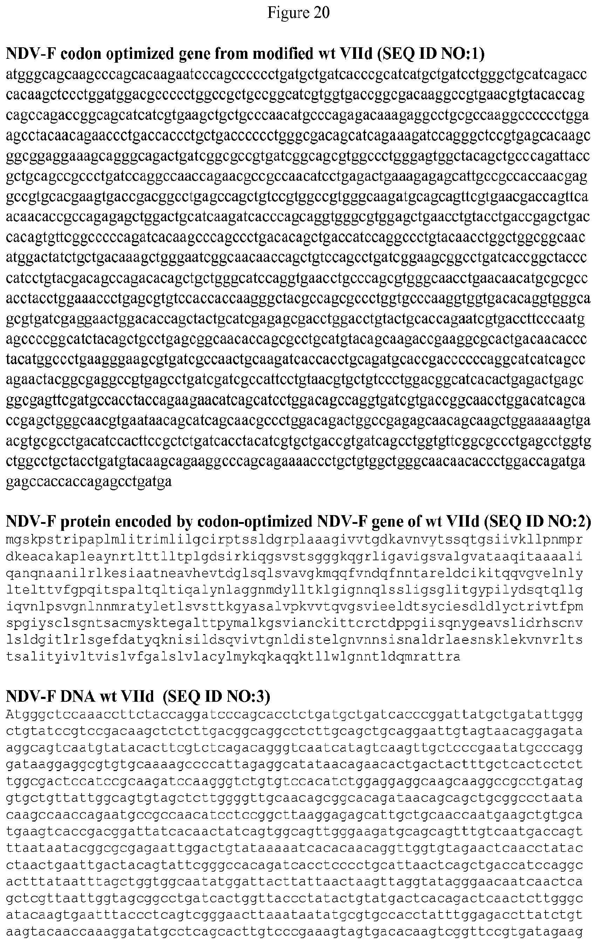

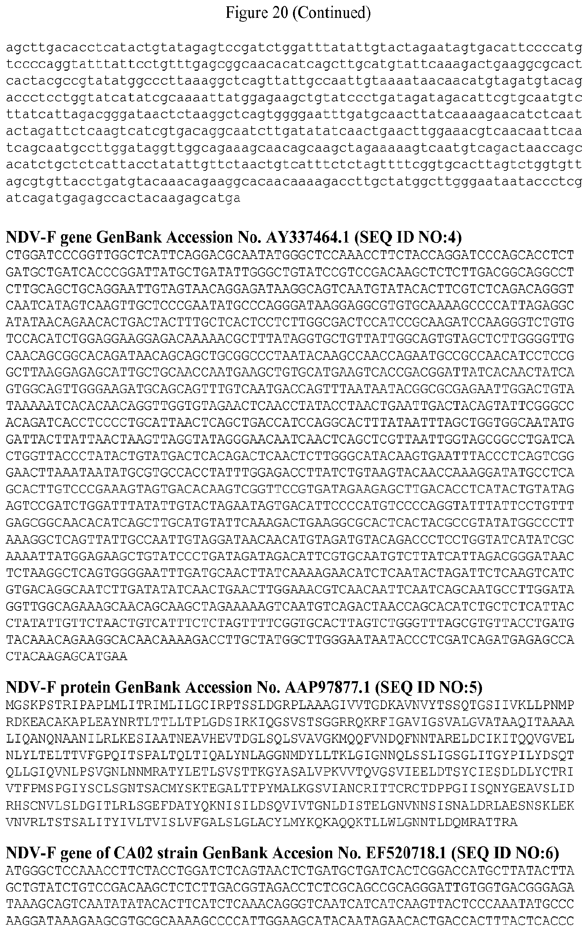

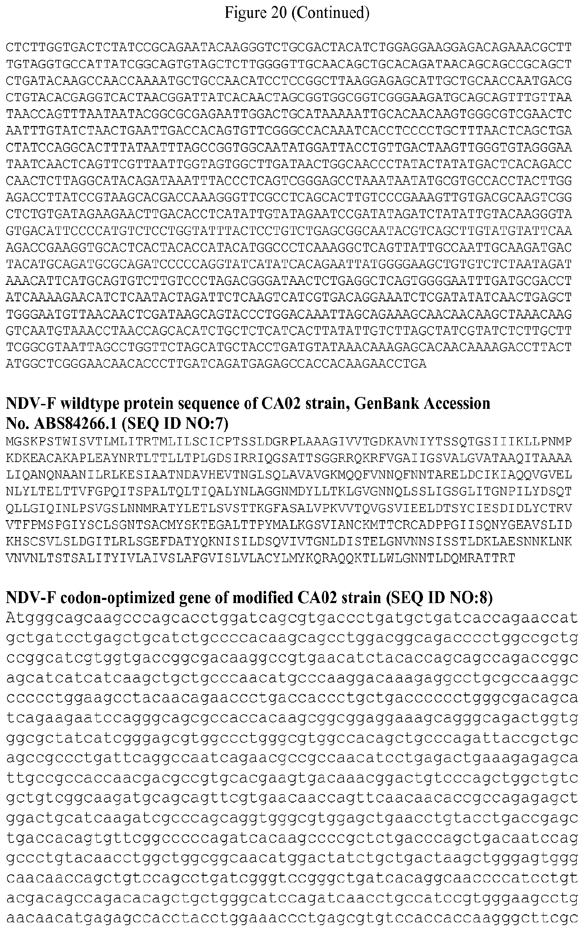

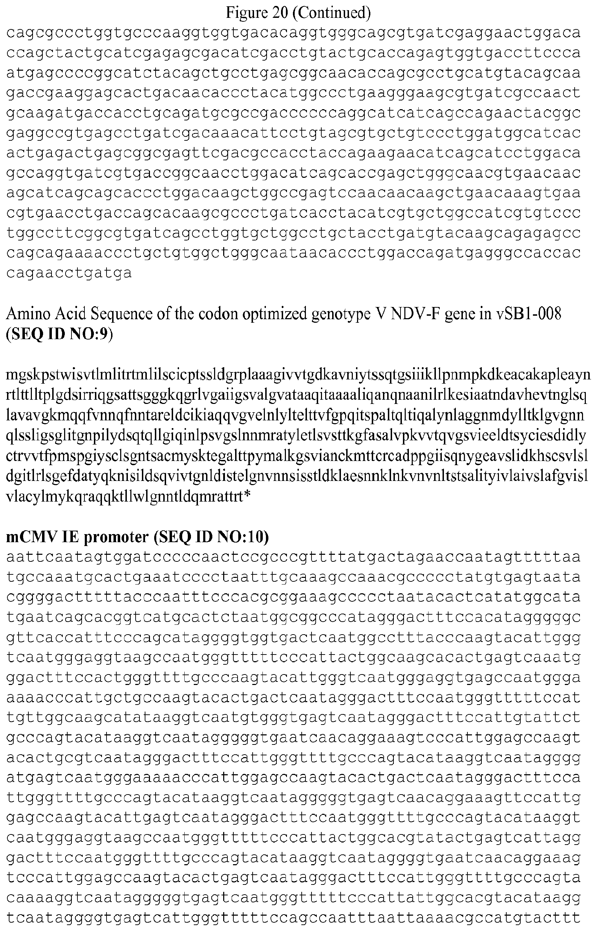

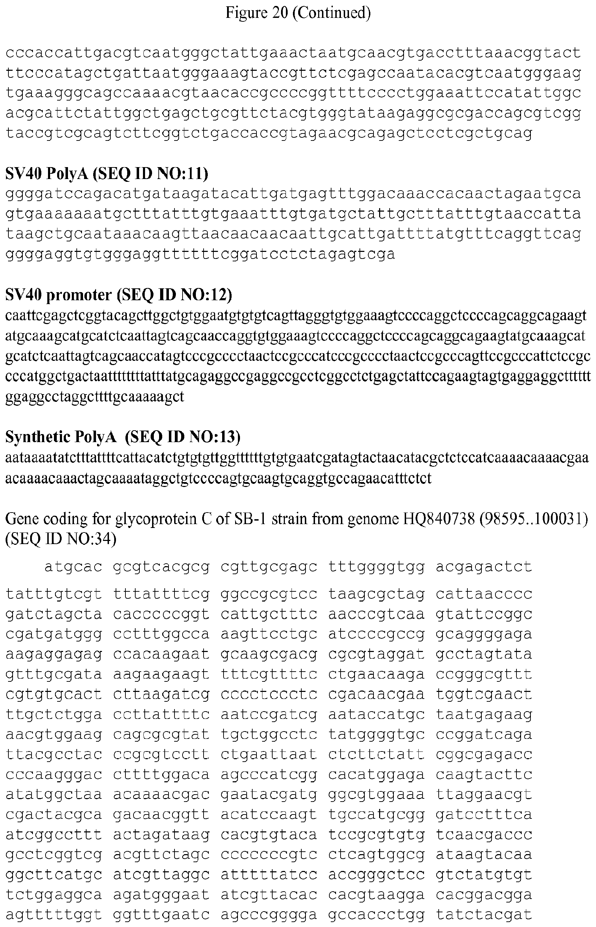

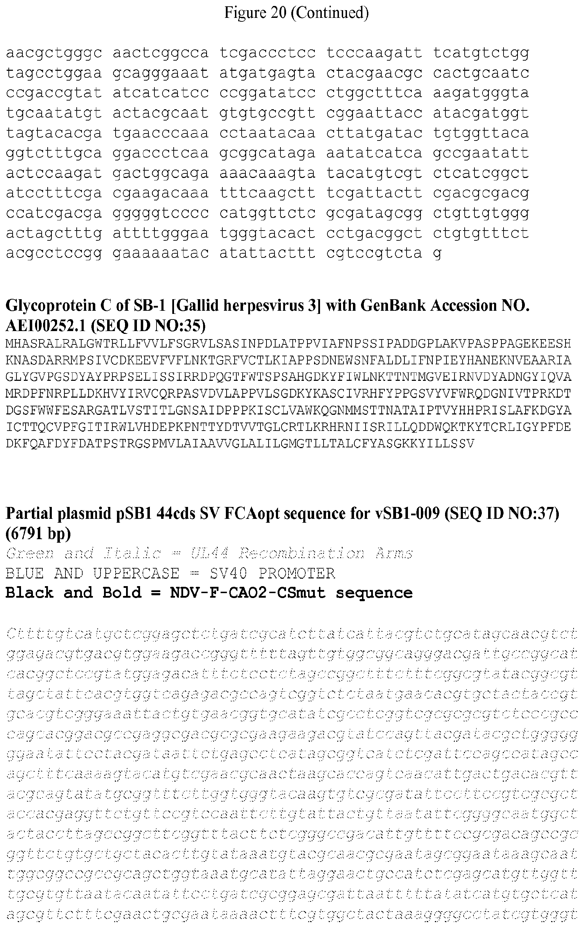

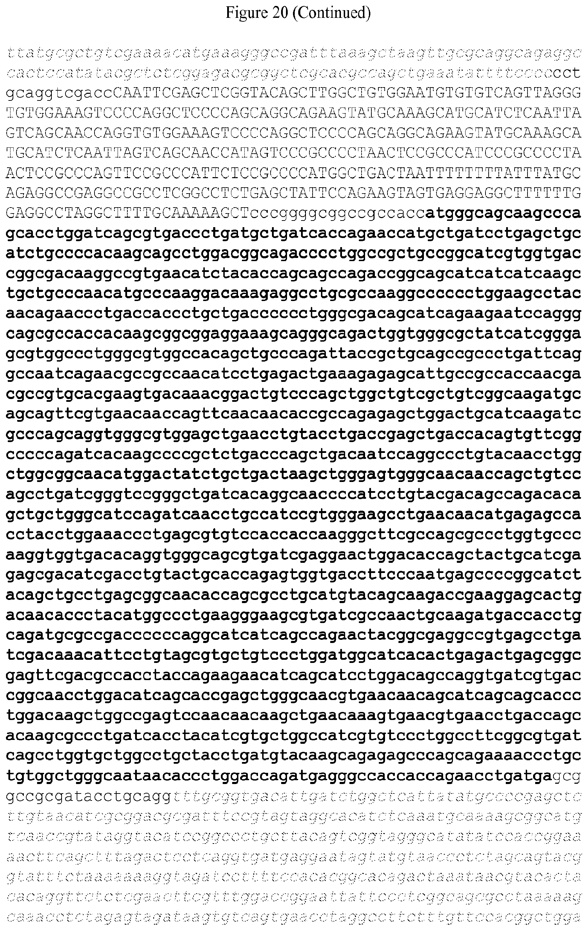

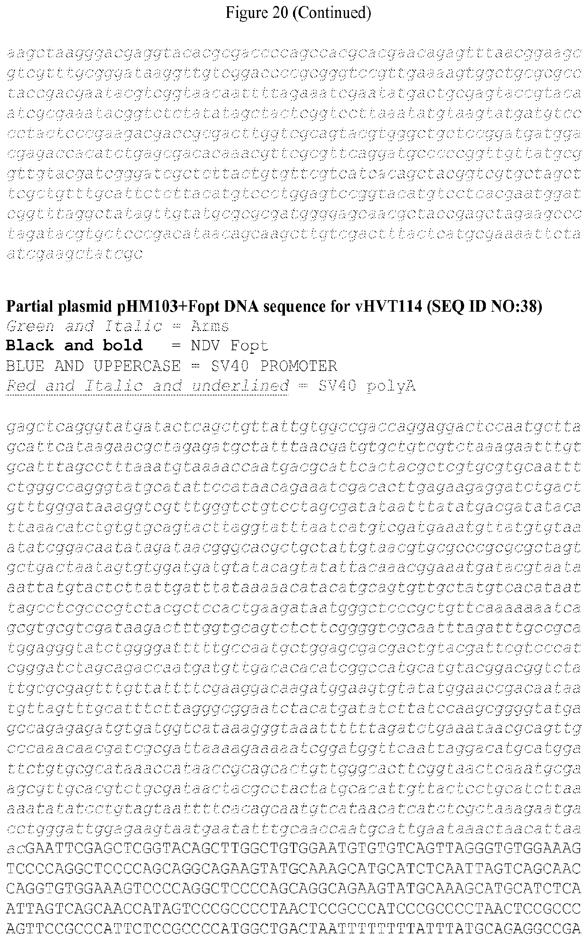

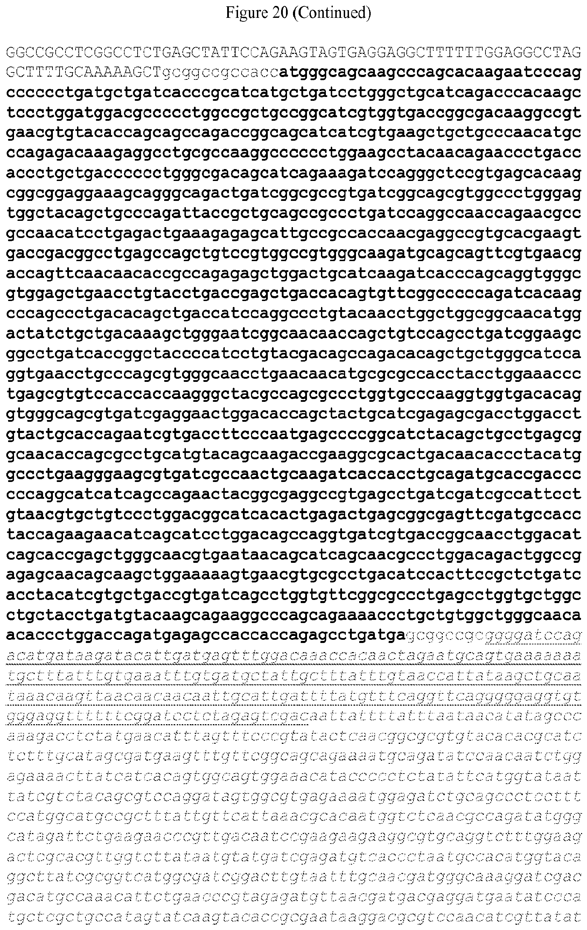

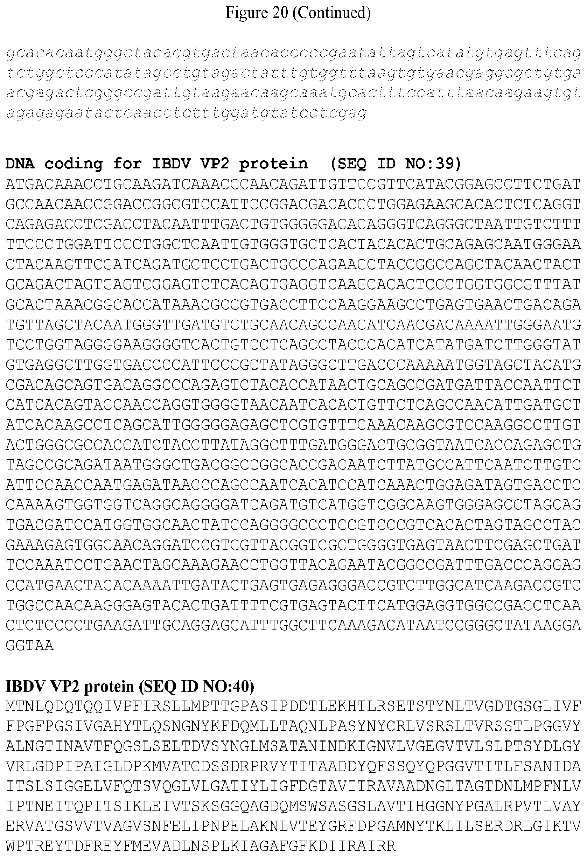

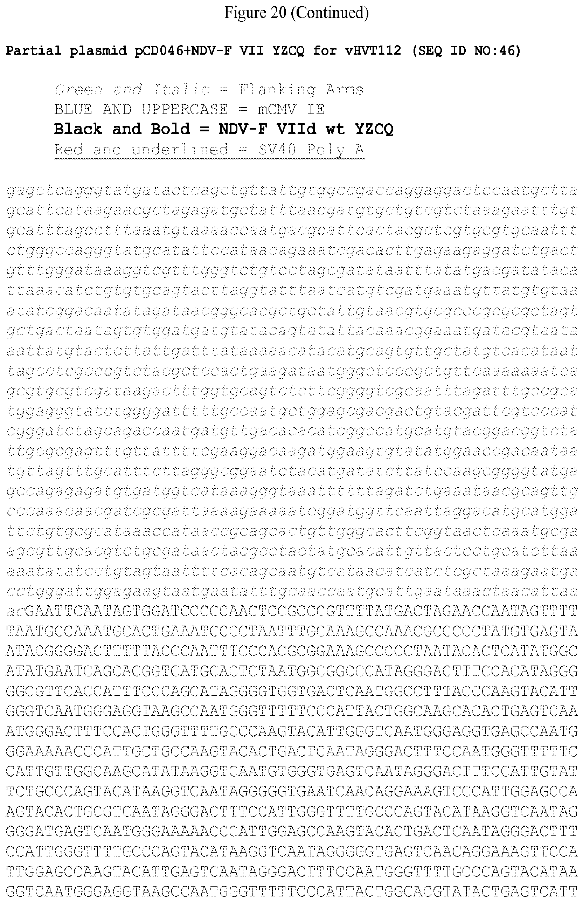

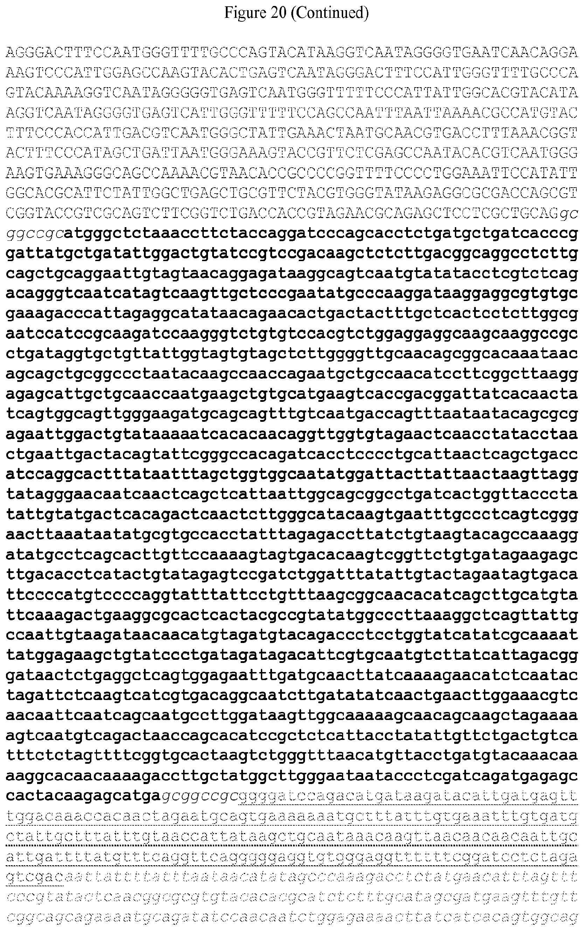

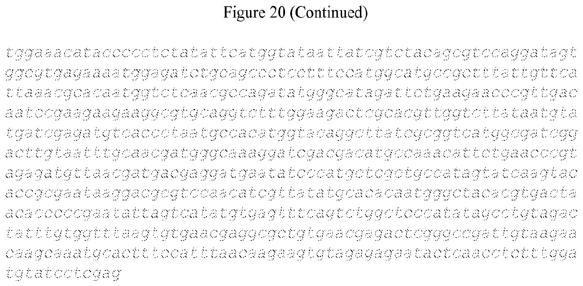

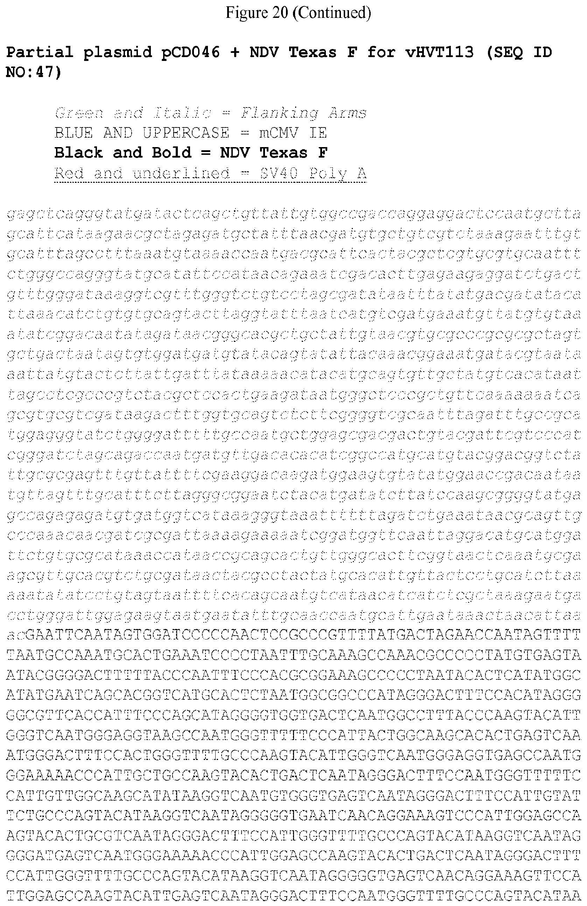

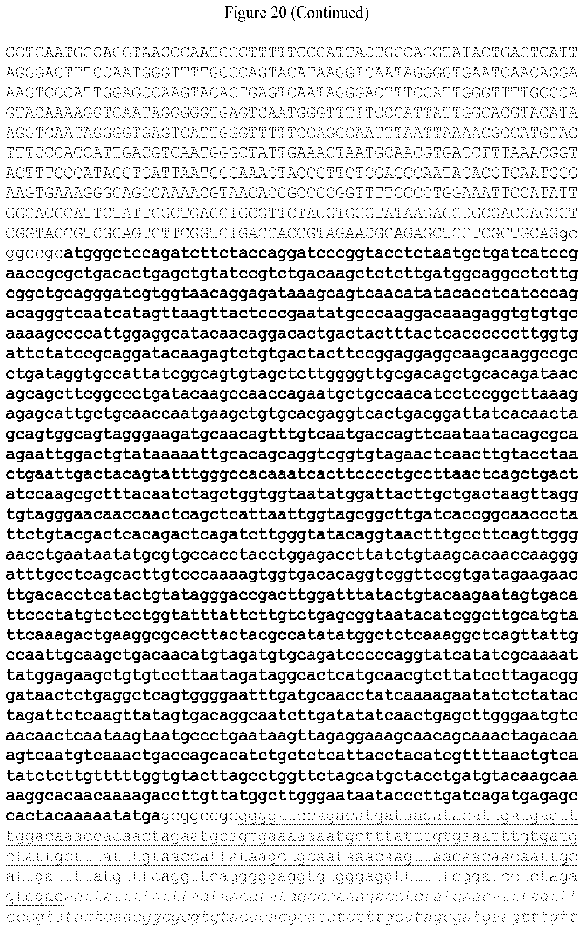

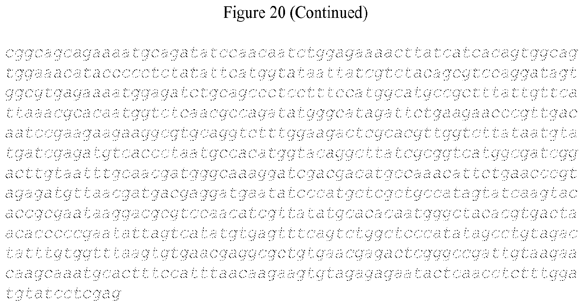

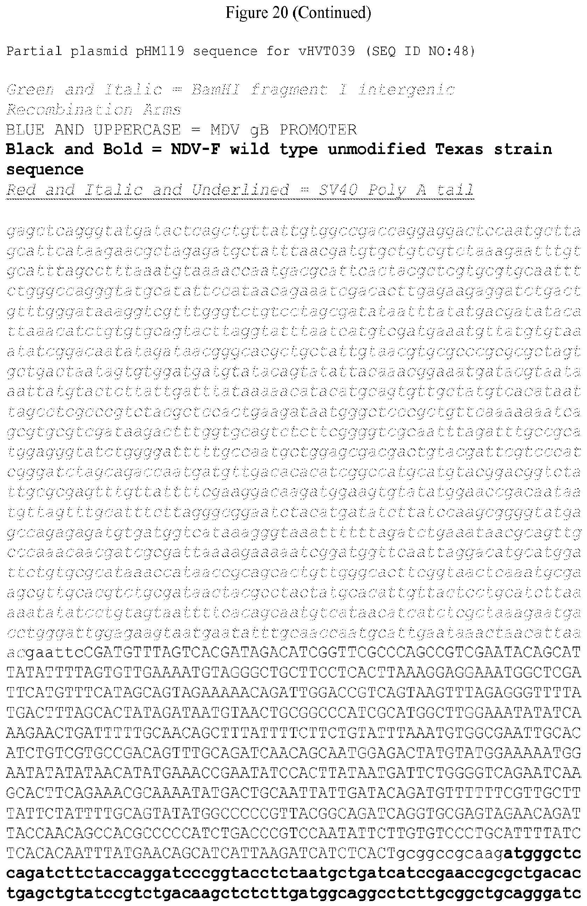

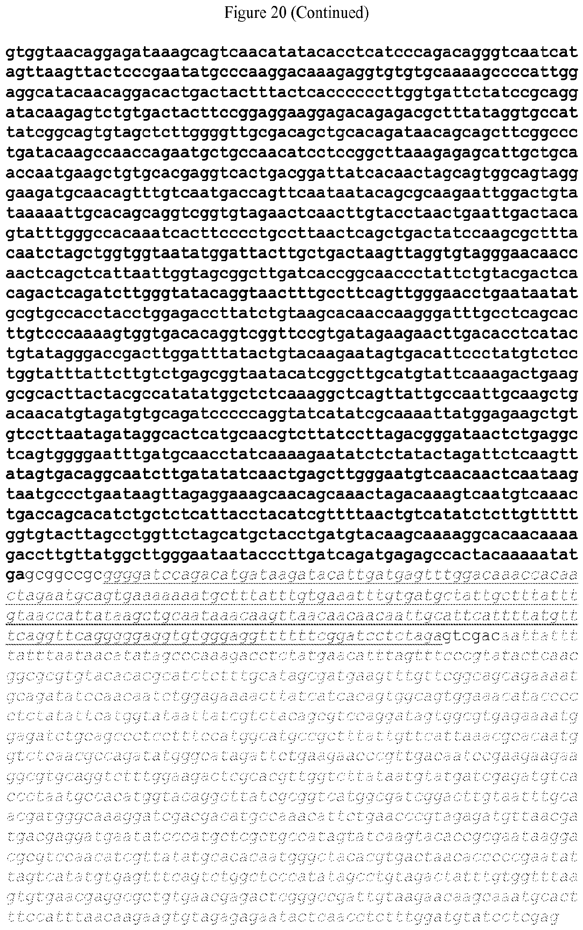

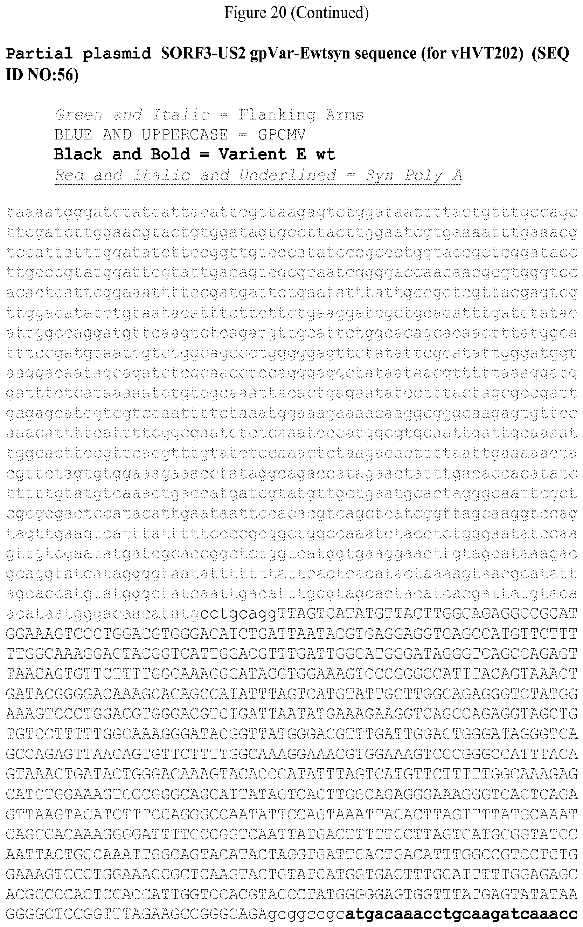

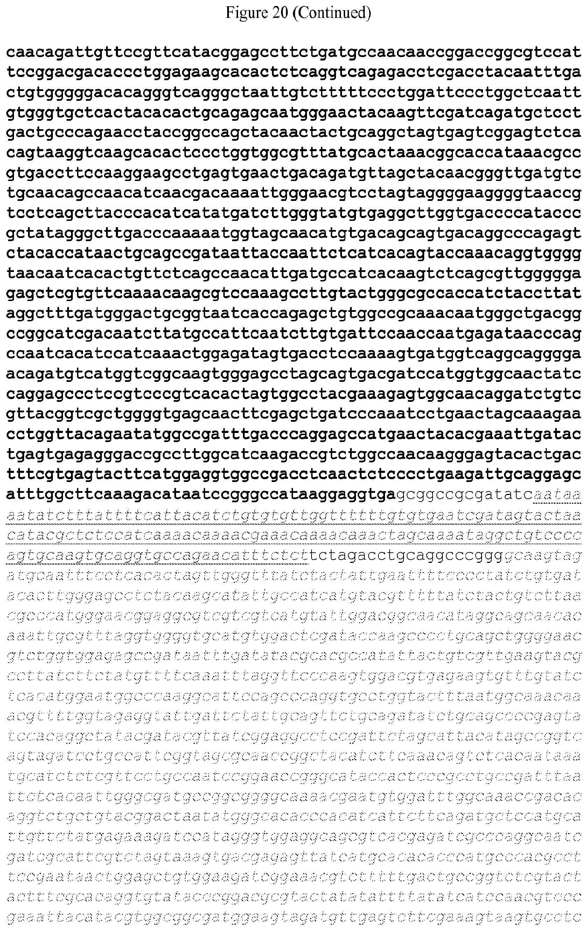

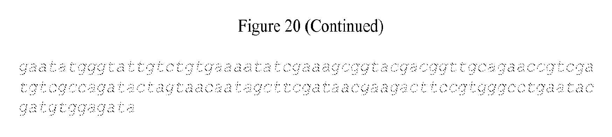

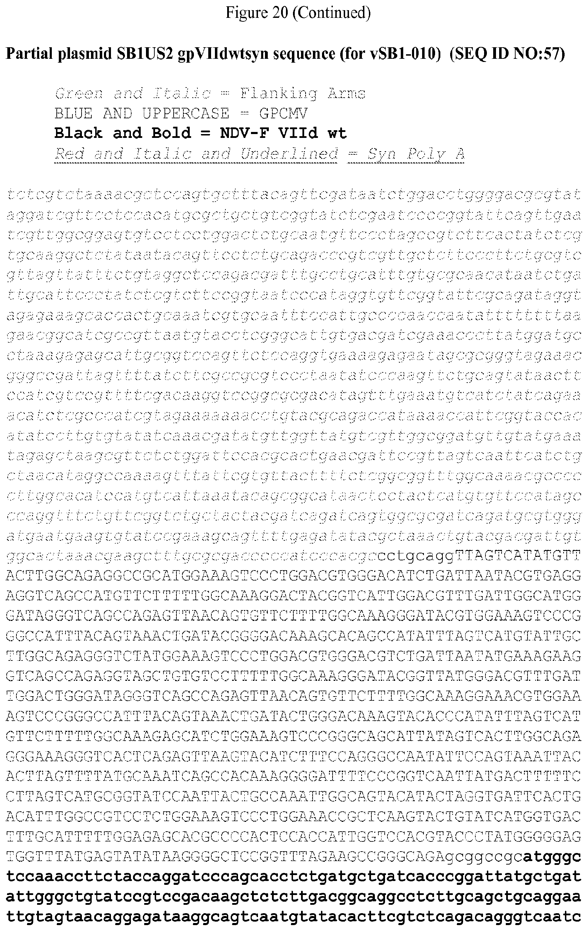

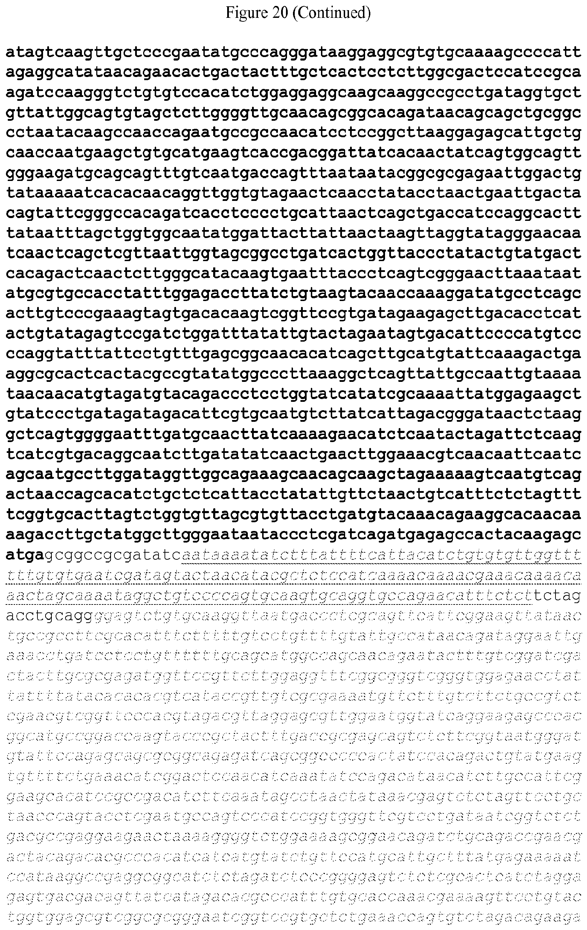

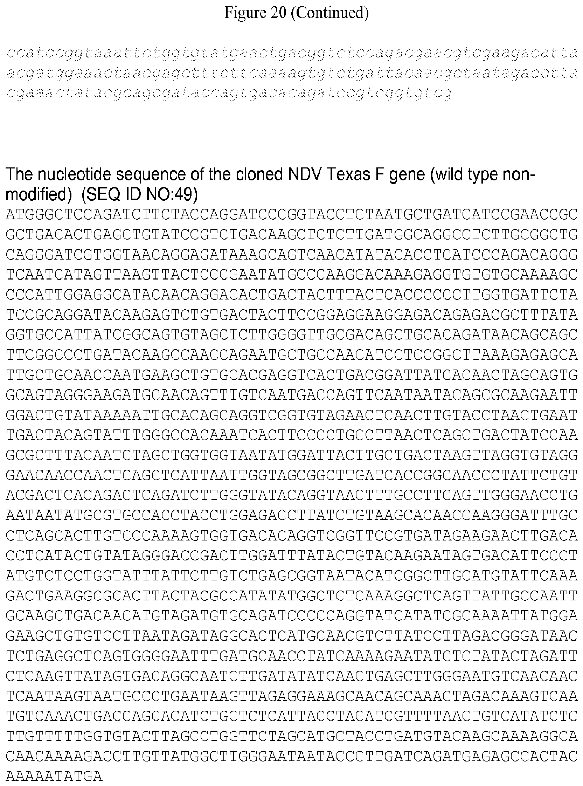

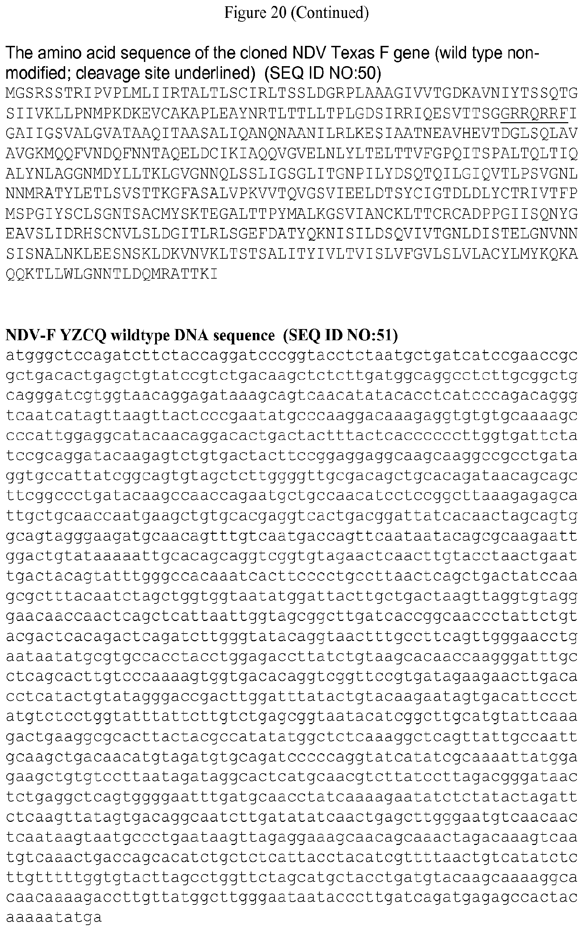

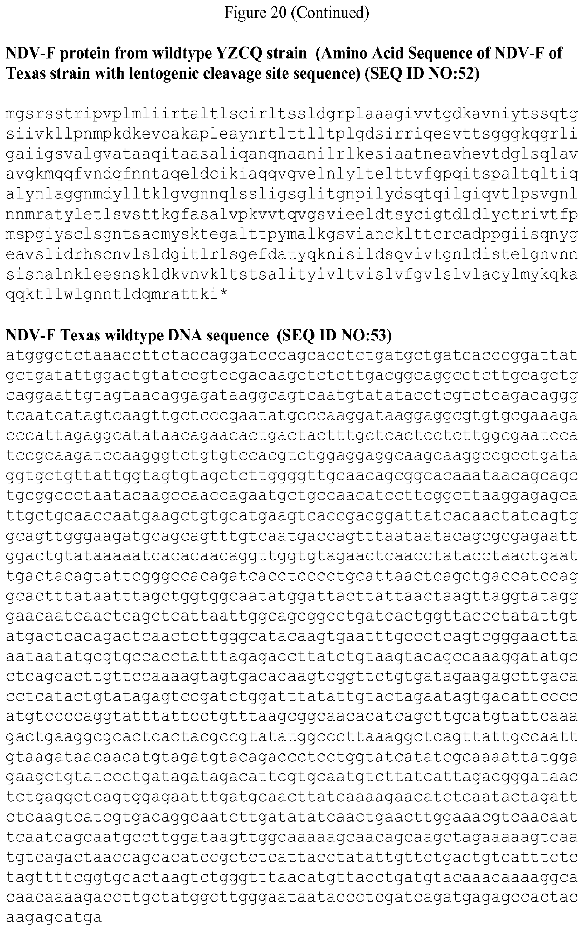

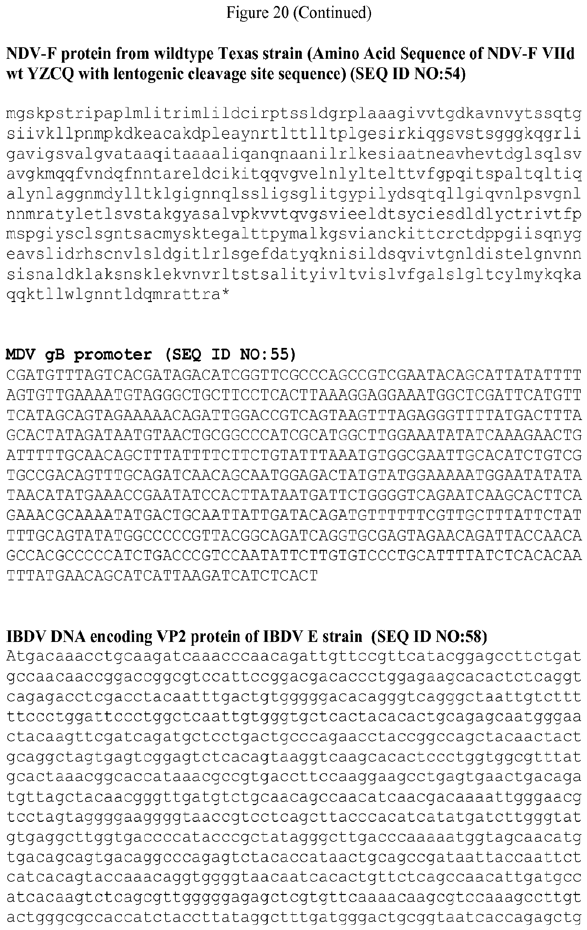

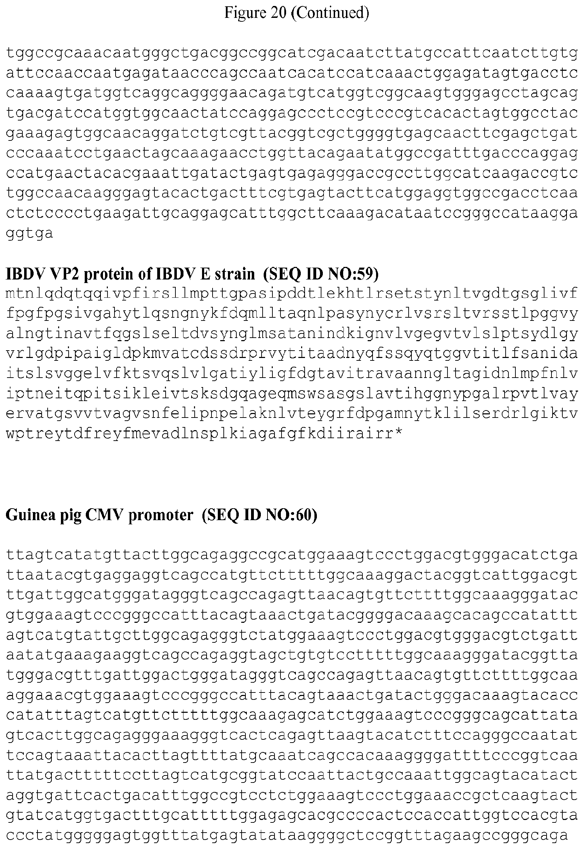

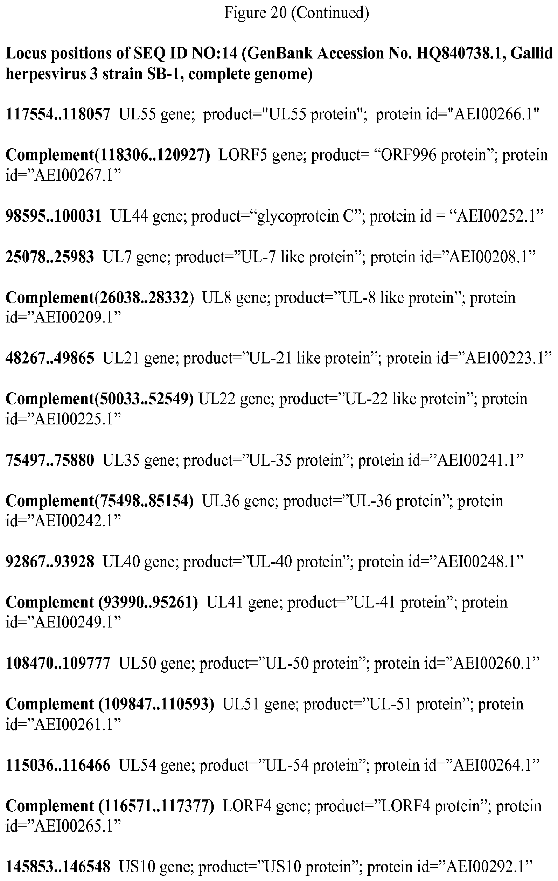

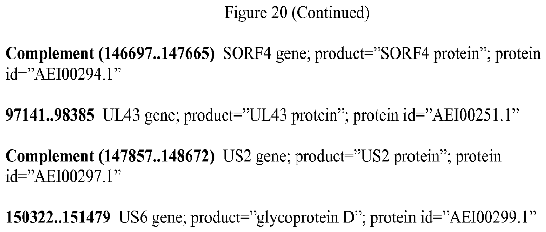

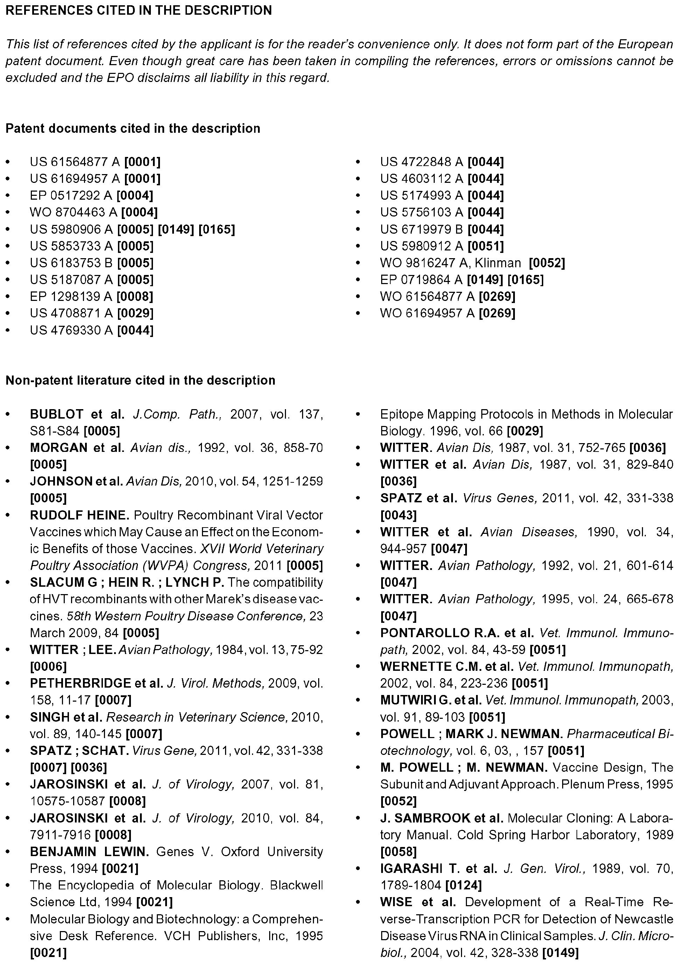

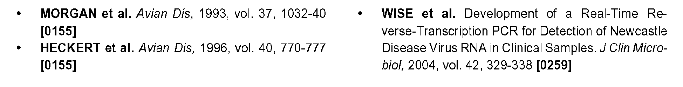

The nucleotide sequence of the donor plasmid SB-1 US10mFwt SbfI (SEQ ID NO:41) is shown in FIG. 20.

Based on PCR testing and immunofluorescence analysis, vSB 1-004 is a recombinant SB-1 expressing a NDV-F gene under the control of mCMV promoter. Recombinant vector vSB 1-004 is free of any detectable parental SB-1 virus or potential HVT contaminant.

### Example 2 Construction of recombinant vSB1-006 expressing NDV-F

The aim of the work is to construct a recombinant SB-1 virus in which an expression cassette containing SV40 promoter, Newcastle disease virus fusion protein (NDV-F), and synthetic polyA tail is inserted between the UL55 and LORF5 site of SB-1 virus (Table 2).

**Table 2 Characteristics of vSB1-006**

| Name | Parental virus | Promoter | gene | Poly-A | Locus |
|---|---|---|---|---|---|
| vSB 1-006 | SB-1 | SV40 | Opt-NDV-F of VIId | Syn | UL55/LORF5 |

A Newcastle disease virus Fusion Protein (NDV-F) corresponding to a consensus codon-optimized genotype VIId sequence (SEQ ID NO:2 encoded by SEQ ID NO:1) was chemically synthesized (GeneArt).

### Donor plasmid SB-1 UL55 SV Fopt syn tail SbfI construction

A synthetic SB-1 UL55-LOrf5 Sbfl plasmid covering approximately 1 kb sequence on each side of the insertion site (GenScript) was digested with *Sbf*I and dephosphorylated. A synthetic SV OptF syn tail pUC57 plasmid (Genscript) was digested with *Sbf*I and a 2239 base pair fragment was gel extracted and ligated to the *Sbf*I digested vector to create the new SB1 UL55 SVFopt syn tail Sbfl donor plasmid.

### Recombinant generation, expression analysis and PCR testing

A standard homologous recombination procedure was followed by co-electroporation of secondary CEF cells using donor plasmid SB1 UL55 SV Fopt syn tail SbfI and viral DNA isolated from vaccine strain of SB-1 virus. Essentially the procedure described in example 1 for vSB 1-004 was followed to generate, plaque purify and characterize recombinants by immunofluorescence and PCR.

The nucleotide sequence of the donor plasmid SB1 UL55 SVFopt syn tail SbfI (SEQ ID NO:42) is shown in FIG. 20.

### Recombinant Generation and Expression Analyses

Genomic DNA of SB-1 virus was co-electroporated with SB-1 UL55 SV Fopt syn tail Sbfl donor plasmid to generate recombinant SB-1 using homologous recombination technique. Recombinant virus was separated from parental SB-1 virus by immunofluorescent positive well selection and PCR screening in multiple rounds of plaque purification. A plaque purified recombinant SB-1 virus expressing the NDV-F protein, designated vSB 1-006, was scaled up from tissue culture flasks to 2 x 850 cm² roller bottles. After about 72 hrs post infection in roller bottles, the infected CEFs were harvested. Aliquots were frozen in liquid nitrogen containing 10% FBS and 10% DMSO. Titrations were performed in triplicate on CEFs and a titer of 8x10⁵ pfu/ml was obtained for SB1-006.

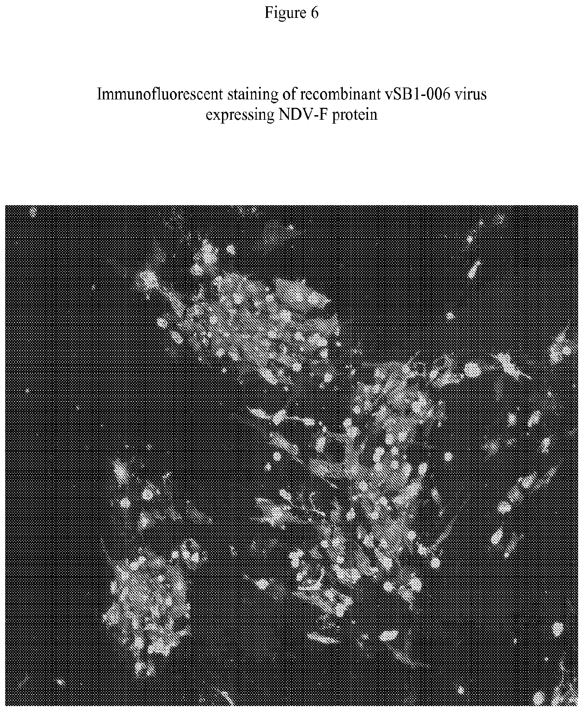

Immunofluorescence was preformed using chicken anti-sera (lot# C0139, Charles Rivers Laboratories) followed by a FITC labeled anti-chicken IgG (cat# 02-24-06, KPL). All examined plaques of vSB 1-006 were found to express NDV-F protein (Fig. 6).

### PCR analysis of vSB1-006

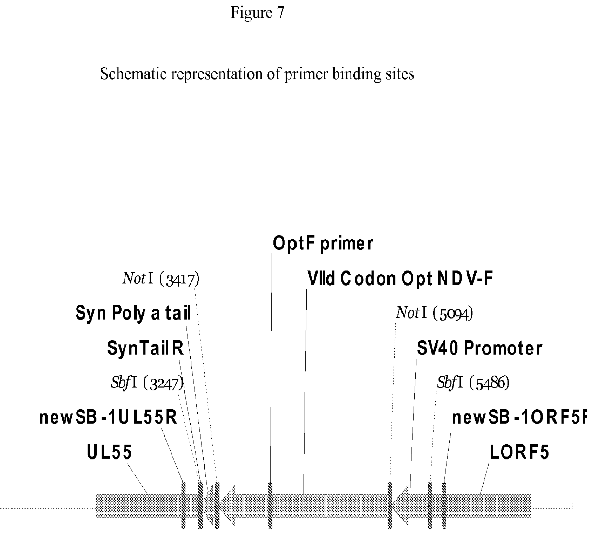

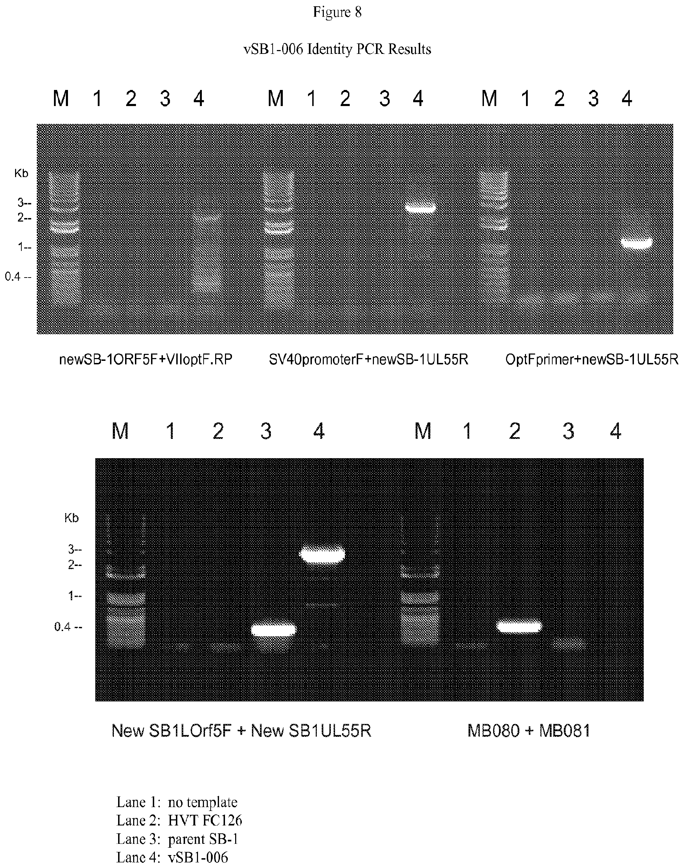

Purity of recombinant virus was verified by PCR using primer pairs that are specific to the SB-1 flanking arms, codon-optimized NDV-F VIId, SV40 promoter as well as primer pairs specific to HVT (see Fig. 7). PCR reactions with all primer pairs resulted in the expected PCR products and banding patterns. In addition, there was no evidence of the parental SB-1 virus in vSB 1-006 (Fig. 8).

Based on PCR testing and immunofluorescence analysis, it is confirmed that vSB1-006 is a recombinant SB-1 expressing a codon-optimized NDV-F gene under the control of SV40 promoter. Recombinant vector vSB 1-006 is free of any detectable amount of parental SB-1 virus and potential HVT contaminant.

### Example 3 Construction of recombinant vSB1-007 expressing NDV-F

The aim of the work is to construct a recombinant SB-1 virus in which an expression cassette containing SV40 promoter, NDV-F gene corresponding to the F sequence of genotype VIId of NDV is used to replace the coding sequence of glycoprotein C (gC or UL44) of SB-1 virus (Table 3).

**Table 3 Characteristics of vSB1-007**

| Name | Parental virus | Promoter | gene | Poly-A | Locus |
|---|---|---|---|---|---|
| vSB 1-007 | SB-1 | SV40 | Opt-NDV-F of VIId | (endogeneous from gC gene) | gC |

A Newcastle disease virus Fusion Protein (NDV-F) corresponding to a consensus codon-optimized genotype VIId sequence (SEQ ID NO:2 encoded by SEQ ID NO:1) was chemically synthesized (GeneArt).

### Donor plasmid pSB1 44 cds SVOptF construction

A synthetic pSB1 44 cds plasmid containing flanking arms was generated by gene synthesis (GenScript). The pSB1 44 cds was digested with SbfI, dephosphorylated. Another plasmid named SV-OptF-syn no polyA tail-pUC57 was digested with SbfI and 2.1 kb fragment containing SV40 promoter and NDV-F gene was gel extracted, ligated into the SbfI digested vector and transformed using the Top10 Oneshot kit (Invitrogen). Bacterial colonies were grown in LB-ampicillin media (100ug/ml), and plasmids were extracted by using Qiagen Mini Spin Prep kit, and screened for insertions by EcoRI and NcoI digestion. The resultant donor plasmid was designated pSB1 44 cds SVOptF.

The synthetic plasmid pSB1 44 cds (SEQ ID NO:36 in FIG. 20) can also be used as a donor plasmid without further modification (without inserting NDV-F expression cassette) to generate a recombinant SB-1 lacking the glycoprotein (gC) gene.

### Recombinant Generation and Expression Analyses

A standard homologous recombination procedure was followed by co-electroporation of secondary CEF cells using donor plasmid pSB1 44 cds SVOptF and viral DNA isolated from vaccine strain of SB-1 virus. Essentially the procedure described in example 1 for vSB 1-004 was followed to generate, plaque purify and characterize recombinants by immunofluorescence. A plaque purified recombinant SB-1 virus expressing the NDV-F protein, designated vSB 1-007, was scaled up from T-25 tissue culture flasks to 10xT-150 cm² flasks. Infected CEF cells were harvested and aliquots were frozen in liquid nitrogen containing 10% FBS and 10% DMSO. Titrations were performed in triplicate on CEFs and a titer of 7.2x10⁴ pfu/ml was obtained for SB1-007.

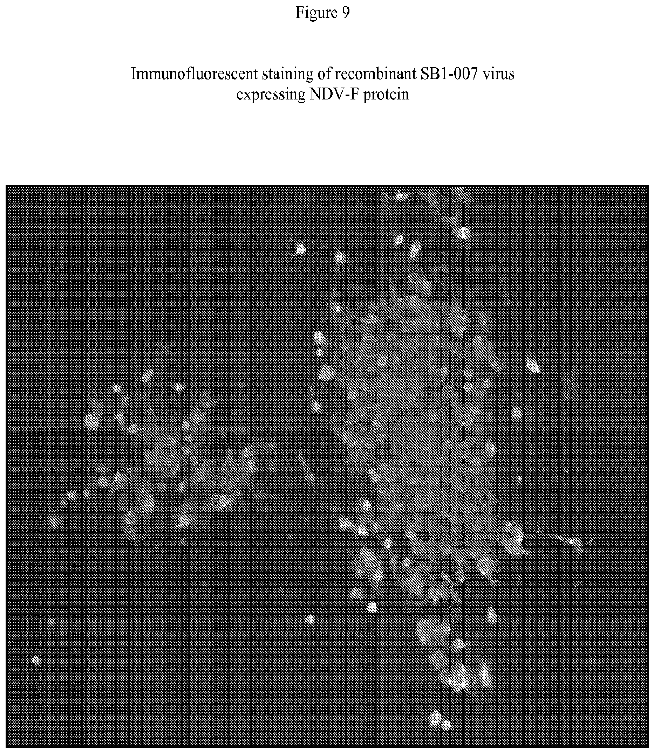

Immunofluorescents was performed using chicken anti-sera (lot# C0139, Charles Rivers Laboratories) followed by a FITC labeled anti-chicken IgG (cat# 02-24-06, KPL). All examined plaques of vSB 1-007 were found to express NDV-F protein (Fig. 9).

### PCR analysis of vSB1-007

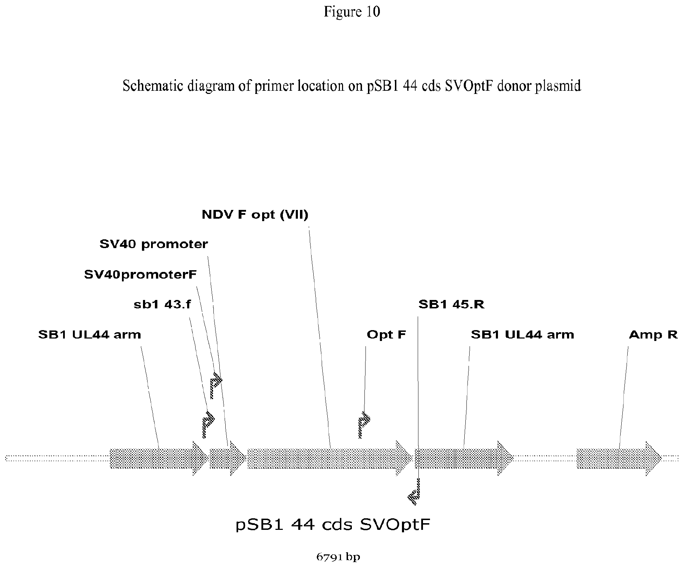

Viral DNA was extracted from SB1-007 from P.1 through P.6 by QIA DNeasy Blood & Tissue Kit (Qiagen). PCR primers were designed to specifically identify the presence of NDV F (codon-optimized), the SV40 promoter and the flanking arms of UL44 (see Fig. 10). PCR amplifications were preformed using 200ng of DNA template along with the specified primer pairs.

Similarly, a standard homologous recombination procedure using synthetic plasmid pSB1 44 cds and viral DNA isolated from vaccine strain of SB-1 virus will generate a recombinant SB-1 in which the coding region of gC gene is deleted. Two PCR primers (SB1 43.F and SB1 45.R, Table 4) will produce a PCR product of 103 nucleotides for a gC-deleted recombinant SB-1 versus a 1540 nucleotides for the parent SB-1 virus.

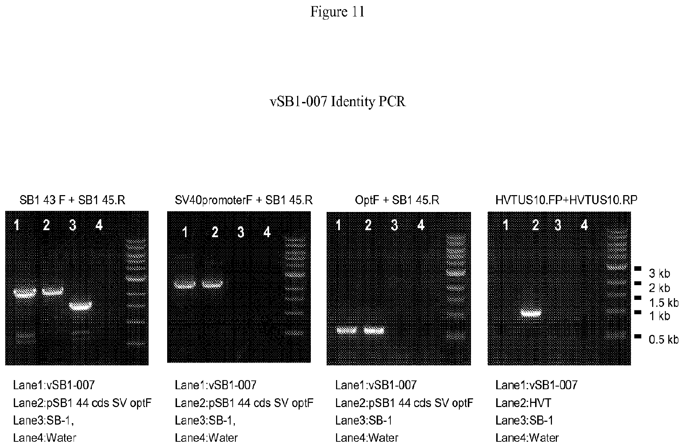

Purity of recombinant virus was verified by PCR using primer pairs that are specific to the SB-1 flanking arms, codon-optimized NDV-F VIId, SV40 promoter as well as primer pairs (MB080 +MB081) specific to HVT. PCR reactions with all primer pairs resulted in the expected PCR products and banding patterns. In addition, there is no evidence of the parental SB-1 virus in vSB 1-007 (Tables 4-5 and Fig. 11).

**Table 4 PCR primers**

| Primer | SEQ ID NO: | Sequence (5' to 3') |
|---|---|---|
| SB1 43.F | 27 | GCTCTCGGAGACGCGGCTCGC |
| SB1 45.R | 28 | GCTCTTGTAACATCGCGGACG |
| SV40 promoter.F | 29 | AGCTTGGCTGTGGAATGT |
| Opt F | 24 | ACTGACAACACCCTACATGGC |
| HVTUS10.FP | 30 | CCGGCAACATACATAATGTG |
| HVTUS10.RP | 31 | GGCACTATCCACAGTACG |

**Table 5 Expected amplicon size**

| Primer pairs | Expected amplicon size (bp) | |
|---|---|---|
| | SB-1 | vSB1-007/pSB 1 44 cds SVOptF |
| SB1 43.F+SB1 45.R | 1540 | 2188 |
| SV40promoterF + SB1 45.R | None | 2113 |
| Opt F + SB1 45.R | None | 611 |
| HVTUS 10.FP + HVTUS 10.RP | None | None |

Based on PCR testing and immunofluorescence analysis, it is confirmed that vSB 1-007 is a recombinant SB-1 expressing a codon-optimized NDV-F gene under the control of SV40 promoter. The NDV-F expression cassette was successfully used to replace the gC gene of SB1, demonstrating that gC is dispensable for *in vitro* propagation of SB-1 virus. Recombinant vector vSB 1-007 is free of any detectable amount of parental SB-1 virus or HVT.

The nucleotide sequence of the donor plasmid pSB1 44 cds SVOptF (SEQ ID NO:43) is shown in FIG. 20.

### Example 4 Construction of recombinant vSB1-008 expressing NDV-F

The aim of the work is to construct a recombinant SB-1 virus in which an expression cassette containing SV40 promoter, NDV-F gene corresponding to the F sequence of CA02 strain of NDV, and synthetic polyA tail is inserted between the UL55 and LORF5 site of SB-1 virus (Table 6).

**Table 6 Characteristics of vSB1-008**

| Name | Parental virus | Promoter | gene | Poly-A | Locus |
|---|---|---|---|---|---|
| vSB1-008 | SB-1 | SV40 | Opt-NDV-F of CA02 | Syn | UL55/LORF5 |

An NDV-F corresponding to a codon-optimized genotype V (CA02 strain) sequence (SEQ ID NO:9 encoded by SEQ ID NO:8) was chemically synthesized (GeneArt). The F protein cleavage site of this synthetic gene was altered to match a lentogenic F cleavage site sequence and the resultant NDV-F gene sequence has 99% amino acid sequence identity to NDV-F sequence deposited in GenBank (ABS84266).

### Donor plasmid SB1 UL55 SV CaFopt syn tail SbfI construction

A synthetic SB-1 UL55-LOrf5 SbfI plasmid (Genscript) containing approximately 1 kb sequence of each side of the insertion site was digested with *Sbf*I and dephosphorylated. A synthetic SV OptF syn tail pUC57 plasmid (Genscript) was digested with *Sbf*I and a 2239 base pair fragment containing syn tail was gel extracted and ligated to the *Sbf*I digested vector to create the new SB1 UL55 SVFopt syn tail SbfI donor plasmid. This donor plasmid was then digested with *Not*I, CIPed, and a 5196 base pair fragment was gel extracted. A synthetic NDV-F CAO2 CSmut 0813005 pVR101 donor plasmid (GeneArt) was digested with *Not*I and a 1677 base pair fragment was gel extracted and ligated to the *Not*I digested and CIPed UL55 vector resulting in donor plasmid SB1 UL55 SV CaFopt syn tail SbfI.

### Recombinant generation and expression analysis

A standard homologous recombination procedure was followed by co-electroporation of secondary CEF cells using donor plasmid SB-1 UL55 SV CaFopt syn tail SbfI and viral DNA isolated from vaccine strain of SB-1 virus. Essentially the procedure described in example 1 was followed to generate and characterize recombinants by immunofluorescence and PCR.

Recombinant virus was separated from parental SB-1 virus by immunofluorescent positive well selection and PCR screening in multiple rounds of plaque purification. A plaque purified recombinant SB-1 virus expressing the NDV-F protein, designated vSB1-008, was scaled up from tissue culture flasks to 2 x 850 cm² roller bottles. After about 72 hrs post infection in roller bottles, the infected CEFs were harvested. Aliquots were frozen in liquid nitrogen containing 10% FBS and 10% DMSO.

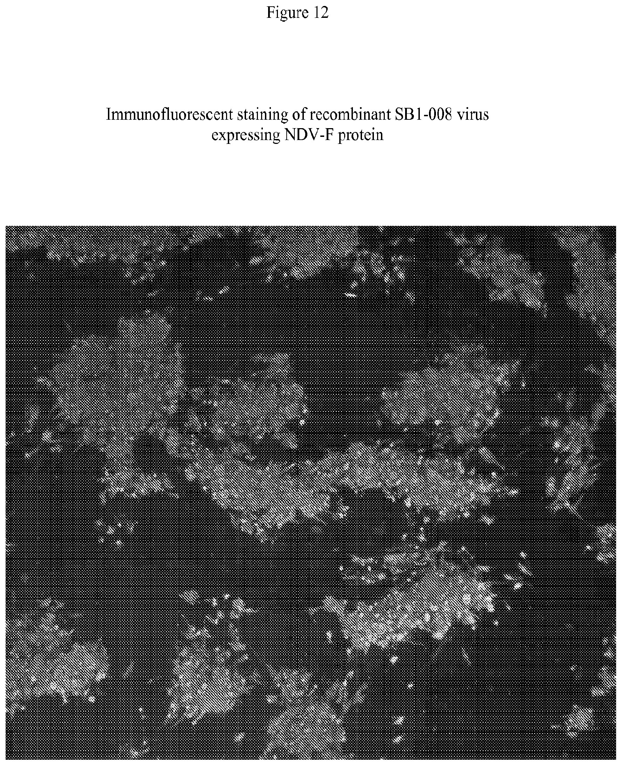

Immunofluorescence was performed using chicken anti-sera (Charles Rivers Laboratories) followed by a FITC labeled anti-chicken IgG (KPL) (Fig. 12).

### PCR analysis of vSB1-008

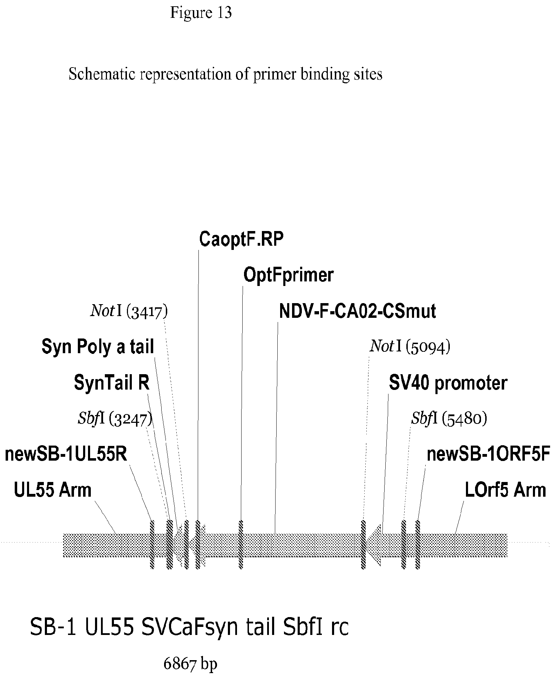

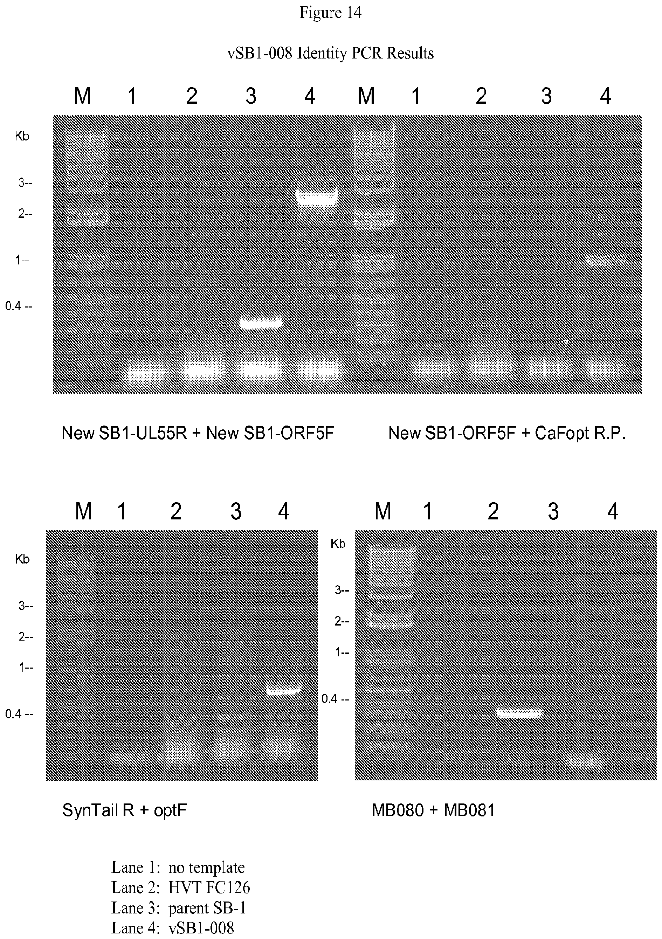

Purity of recombinant virus was verified by PCR using primer pairs that are specific to the SB-1 flanking arms, codon-optimized NDV-F VIId, SV40 promoter (see Fig. 13) as well as primer pairs (MB080 +MB081) specific to HVT, MDV serotype 3. PCR reactions with all primer pairs resulted in the expected PCR products and banding patterns. In addition, there is no evidence of the parental SB-1 virus in vSB 1-008 (Fig. 14).

The nucleotide sequence of the donor plasmid SB-1 UL55 CaFopt syn tail SbfI (SEQ ID NO:44) is shown in Fig. 20.

Based on PCR testing and immunofluorescence analysis, it is confirmed that vSB 1-008 is a recombinant SB-1 expressing a codon-optimized NDV-F gene under the control of SV40 promoter. Recombinant vector vSB 1-008 is free of any detectable parental SB-1 virus or HVT.

### Example 5 Construction of recombinant vSB1-009 and vSB1-010 expressing NDV-F

The aim of the study is to construct a recombinant SB-1 viral vector vSB 1-009 in which an expression cassette containing SV40 promoter and Newcastle disease virus fusion (NDV-F) gene is inserted to replace UL44 coding (gC) sequence of SB-1 and to construct a recombinant SB-1 viral vector vSB1-010 in which an additional expression cassette containing guinea pig CMV promoter and NDV-F gene is inserted in SORF-US2 locus of SB1-009 vector backbone.

### Example 5.1 Construction of vSB1-009

A donor plasmid pSB1 44 cds SV FCAopt was constructed containing UL44 flanking arms of SB1 virus, SV40 promoter and NDV F codon optimized gene sequence (SEQ ID NO:8, coding for SEQ ID NO:9) (Table 7).

**Table 7 Characteristics of vSB1-009**

| Name | Parental virus | Promoter | F gene | Poly-A | Locus |
|---|---|---|---|---|---|
| vSB1-009 | SB1 | SV40 | Opt-NDV-F of CA02 | (endogeneous from gC gene) | UL44 (gC) |

### Generation of recombinant virus

A standard homologous recombination procedure was followed by co-electroporation of secondary CEF cells using donor plasmid pSB1 44 cds SV FCAopt and viral DNA isolated from SB-1 virus infected CEFs. Essentially the procedure described in example 1 was followed to generate, plaque purify and characterize recombinants by immunofluorescence.

After two rounds of plaque purification, pure recombinant virus (vSB1-009) was isolated and the purity of vSB 1-009 was tested by IFA and PCR to validate the appropriate insertion as well as no remnant parental virus.

### PCR analysis

Viral DNA was extracted from vSB 1-009 pre-master seed virus (pre-MSV) stock by QIA DNeasy Blood & Tissue Kit (Qiagen). PCR primers were designed to identify the presence of the NDV F optimized, the NDV F wild type, the SV40 promoter, the mCMV promoter, the UL44 flanking arms of SB-1 virus and HVT virus. PCR amplifications were performed using approximately 200ng of DNA template along with the primer pairs.

PCR amplification with various primers confirmed that the vSB1-009 has the expected amplification patterns and amplicons.

### Expression analysis

Indirect immunofluorescent assay (IFA) was performed on the vSB 1-009 pre-MSV stock to examine the expression of NDV F gene and SB-1 virus antigen. The CEFs that were inoculated with vSB1-009 were fixed with ice-cold 95% acetone for three minutes at room temperature and air-dried for 10 min. The plates were washed with PBS, then two primary antibodies, chicken anti-Newcastle Disease Virus sera (Charles Rivers Laboratories cat#10100641, lot#C0117A) at 1:500 dilution and Y5.9 monoclonal antibody against SB-1 virus (Merial Select, Gainesville, GA) at 1:3000 dilution were added and the plates were incubated for 45 min at 37°C. After three washes with PBS, two secondary antibodies, goat anti-chicken IgG-fluorescein (KPL) at 1:500 dilution and donkey anti-mouse IgG-Alexa Fluor 568 (Molecular Probe) at 1:250 dilution were added. The plates were incubated at 37°C for 45 min and followed by three washes with PBS. The wells were screened for IFA positive plaques with a fluorescent microscope using fluorescein isothiocyanate (FITC) and tetramethylrhodamine isothiocyanate (TRITC)-filters of Nikon Eclipse Ti inverted microscope. Similarly, reactivity of vSB1-009 with NDV F Mab was examined by Dual IFA using anti-MDV serum (Charles River Laboratories (1/300 dilution) and anti-NDV F monoclonal antibody (1/300 dilution) as primary antibody. The goat anti-chicken IgG-fluorescein (KPL) (1:500 dilution) and donkey anti-mouse IgG-Alexa Fluor 568 (Molecular Probe) (1:250 dilution) were used as secondary antibodies. The wells were observed to identify the IFA positive plaques with a fluorescent microscope using FITC- and TRITC-filters of Nikon Eclipse Ti inverted microscope.

IFA results indicate that vSB1-009 expresses the NDV F protein in virus-infected CEF. Over 500 vSB1-009 plaques were counted for NDV F protein expression as well as SB-1 virus specific protein expression with dual IFA. The expression of NDV F protein completely matched with SB-1 virus antigen expression in each virus plaque (Table 8).

**Table 8 Dual IFA of vSB1-009**

| Virus | Dual IFA plate#1 (total 189 plaques) | | Dual IFA plate#2 (total 361 plaques) | |
|---|---|---|---|---|
| | Anti-NDV serum positive plaques | Anti-SB-1 Mab positive plaques | Anti-NDV serum positive plaques | Anti-SB-1 Mab positive plaques |
| vSB1-009 | 189 | 189 | 361 | 361 |

NDV F Mab reactivity was confirmed by Dual IFA. Over 200 vSB 1-009 plaques were examined for NDV F Mab reactivity as well as anti-MDV serum reactivity. The reactivity with NDV F Mab completely matched with anti-MDV serum reactivity in each virus plaque (Table 9).

**Table 9 Reactivity of vSB1-009 with anti-NDV F Mab**

| Virus | Dual IFA (total 254 plaques) | |
|---|---|---|
| | Anti-MDV serum positive plaques | Anti-NDV F Mab positive plaques |
| vSB 1-009 | 254 | 254 |

### Southern Blot analysis

Total genomic DNA was extracted from vSB1-009 pre-MSV stock infected CEFs. The genomic DNA of vSB 1-009, SB-1 virus (negative control), pSB1 44 cds SV FCA opt donor plasmid were digested at 37°C with EcoRI, NcoI, and KpnI restriction endonucleases separately. The restriction fragments were separated by a 0.8 % agarose gel electrophoresis and transferred onto a positively charged Nylon membrane. After transfer, the membrane was treated with 0.4M NaOH and then neutralized with 2XSSC-HCl buffer. The membrane was then air dried and UV crosslinked.

Following the North2South Chemiluminescent Hybridization and Detection Kit (Thermo Scientific cat#89880) manufacturers' instructions, the membrane was pre-hybridized for 1 hr and then hybridized with the probe at 55°C for overnight. For hybridization, two probes were used; 1) the SbfI fragment of pSB1 44 cds SV FCA opt as NDV F cassette probe, 2) the SmaI-EcoRI fragment of pUC57 SB1 44 arm (GenScript) as recombination arm probe. After the overnight hybridization, several stringency washes were conducted until the membrane was placed in blocking buffer with the addition of Streptavidin-HRP. After rinsing the membrane of any unbound Streptavidin-HRP, the substrate solution of Luminal and peroxide were added. The membrane was then exposed to X-ray film and the film was developed.

The Southern blot results were as expected based on Vector NTI map analysis. The NDV F cassette (SV40 promoter, NDV-F CA02 codon optimized gene) replaced the UL44 coding sequences of SB-1 virus.

### Genomic analysis

The genomic DNA of vSB1-009 pre-MSV stock was conducted by nucleotide sequence determination of the region of recombination arm as well as inserted gene cassette. Primers were designed and used to amplify the entire NDV-F gene cassette including the recombination arms.

The vSB1-009 sequence (donor plasmid pSB1 44 cds SV FCAopt) containing the recombinant arms, SV40 promoter and NDV F codon-optimized gene was confirmed to be correct as shown in SEQ ID NO:37 (FIG. 20).

### Western blot analysis

The CEF monolayer was infected with vSB1-009 pre-MSV at MOI ∼ 0.1. After a 5-day incubation, the CEFs were pelleted and washed with PBS followed by lysis with IP Lysis/Wash buffer of Pierce Classic IP Kit (Thermo Scientific cat#26146) according to the manufacturers' protocols. The lysate was pre-cleared and incubated with 100 ul of anti-NDV F monoclonal antibody to make the immune complex. The immune complex was captured by Protein A/G Plus Agarose and after removing of the un-bounded immune complex by washing steps, the 50 ul of sample buffer was used to elute under non-reducing conditions. The uninfected CEFs were included as a control. The 20 ul of eluted samples were separated in 10% Bis-Tris gels by electrophoresis. After the electrophoresis, the separated proteins in a gel were transferred onto PVDF membrane. The Protein Detection TMB Western Blot Kit (KPL cat#54-11-50) was used to detect the NDV antigens onto PVDF membrane with chicken anti-NDV serum (Charles River Laboratories Laboratories cat#10100641, lot#C0117A), and goat anti-chicken IgG-peroxidase conjugate (KPL cat#14-24-06) following the manufacturers' protocols.

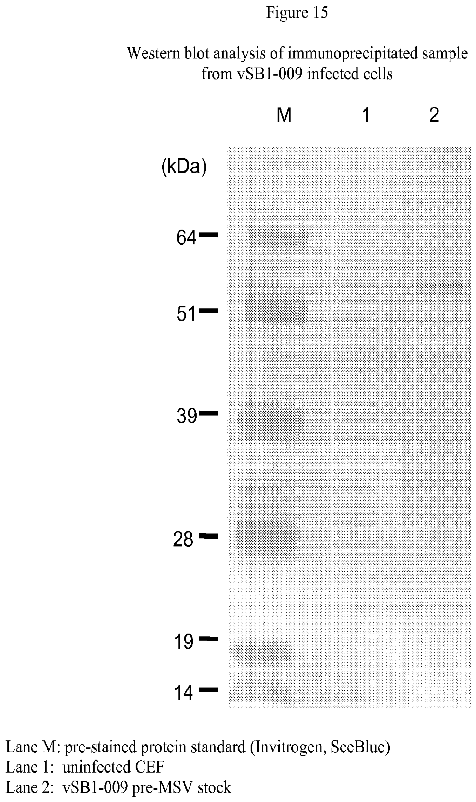

The NDV F protein expression of vSB1-009 was confirmed by two-step immunodetection. First, the expressed NDV F proteins from vSB1-009 infected CEF lysate were captured by the immunoprecipitation using anti-NDV F monoclonal antibody 001C3. Subsequently Western blot analysis using anti-NDV polyclonal serum (Charles River Laboratories cat#10100641, lot#C0117A) was applied to detect the NDV F protein in the captured samples (NDV F protein-monoclonal antibody complex) (Fig. 15). An approximately 55 kDa protein in vSB 1-007 pre-MSV lysates was detected by anti-NDV serum that corresponding the expected size of NDV F1 fusion protein (Fig.15).

### Example 5.2 Construction of vSB1-010

### Donor plasmid SB1US2 gpVIIdwtsyn construction

Using the plasmid HVT SOrf3-US2 gpVar-Ewt Syn, the gpCMV, Varient E, Syn tail was removed by SbfI digestion. This fragment was ligated into the SB1 US2 donor plasmid. The Varient E gene was cut out by *Not*I and replaced by NDV-F VIId wt. The synthetic NDV-F VIId wild type gene (SEQ ID NO:3 encoding SEQ ID NO:2) was excised from pUC57 NDV-F VIId wt plasmid (synthesized by GeneScript) using NotI digestion. Ligated material was transformed using Top10 Oneshot kit (cat#C404002, Invitrogen). Bacterial colonies were grown in LBamp broth, plasmid extracted by using Qiagens MiniSpin Prep kit, and screened for insert orientation using *NcoI*+*SalI* digestion. The correct donor plasmid was designated pSB1 US2 gpVIIdwt Syn. Table 10.1 shows the features unique to the construct around the expression cassettes, including the respective sequences. Large scale cultures were grown and plasmid extraction was done by using Qiagens Maxi Prep kit. Transient expression of the maxi preps was verified using Fugene Transfection Reagent in Chicken Embryo Fibroblast Cells (CEF's) and chicken polyclonal sera against NDV-F.

### Recombinant generation

A standard homologous recombination procedure was followed by co-electroporation of secondary CEF cells using pSB1 US2 gpVIIdWt Syn donor plasmid and viral DNA isolated from vSB1-009 (vSB1-009 is already a recombinant virus expressing CA02 F gene of NDV). Essentially the procedure described in example 1 for was followed to generate, plaque purify and characterize recombinants by immunofluorescence.

After five rounds of plaque purification, pure recombinant virus (vSB1-010) was isolated and the purity of vSB1-010 was tested by IFA and PCR to validate the appropriate insertion as well as no remnant parental virus.

**Table 10.1 Characteristics of vSB1-010**

| Name | Parental virus | Promoter | F gene | Poly-A | Locus |
|---|---|---|---|---|---|
| vSB1-010 | vSB1-009 | Guinea pig CMV | NDV-F VIId | Synthetic | SORF4-US2 |

Sequencing of the insert region confirmed that vSB1-010 contains the correct sequences of guinea pig CMV promoter and the NDV-F VIId wt gene as shown in the sequence of the donor plasmid SB1US2 gpVIIdwtsyn (SEQ ID NO:57).

### Analysis of recombinant by PCR

DNA was extracted from a stock virus by phenol/chloroform extraction, ethanol precipitated, and resuspended in 20mM HEPES. PCR primers were designed to specifically identify the NDV-F VIId wt gene, the promoter, the polyA, as well as, the purity of the recombinant virus from SB1 parental virus. PCR was performed using 200 µg of DNA template along with the specified primers pairs indicted in Table 1. PCR cycling conditions are as follows (unless otherwise noted): 94°C - 2 min; 30 cycles of 94°C - 30 sec, 55°C - 30 sec, 68°C - 3 min; 68°C - 5 min.

Purity of recombinant virus was verified by PCR using primer pairs that are specific to the SB1 flanking arms, the gpCMV promoter, the NDV-F VIId wt gene and the syn tail. Primers, specific to HVT, MDV serotype 3 (MB080 +MB081) were also included in the analysis. The PCR results demonstrate that recombinant virus vSB1-010 carries the intended expression cassette and the virus stock is free from detectable amounts of parental SB1-009 virus.

### Immunofluorescent staining of recombinant vSB1-010 virus expressing two NDV-F proteins

For immunofluorescence testing, the P3 material was diluted 1:100 in media. Approximately 50 µl of the diluted virus was added to 10 ml of DMEM+2% FBS with 1x10⁷ CEFs and then aliquoted onto a 96 well plate (100 µl/well). The plates were incubated for 5 days at 37°C+5% CO₂ until viral plaques were visible. The plates were fixed with 95% ice-cold acetone for three minutes and washed three times with PBS. Chicken anti-sera against Newcastle Disease Virus (lot#C0139, Charles Rivers Laboratory) at 1:1000 was added and the plates were incubated at 37°C for 1 hour. After one hour incubation, the plates were washed three times with PBS and FITC anti-chicken (cat# F8888, Sigma) was added at 1:500. Again the plates were incubated at 37°C for 1 hour. After one hour incubation the cells were rinsed three times with PBS and visualized with a fluorescent microscope using fluorescein isothiocyanate (FITC) filter.

The immunofluorescent staining results indicate that vSB1-010 exhibited a very strong expression of the NDV-F protein when the polyclonal sera against both CA02 and VIId F proteins of NDV were used.

### Conclusion

Based on PCR testing and immunofluorescence analysis, vSB1-010 is a recombinant SB-1 in which VIId-F gene of NDV under the control of gpCMV promoter was successfully inserted into a vSB 1-009, which already expresses the CA02-F gene of NDV. Consequently vSB1-010 carries both VIId and CA02 F genes of NDV genotypes and it is free of any detectable parental vSB 1-009.

### Example 6 Construction of recombinant vHVT vectors expressing NDV-F

### Preparation of donor plasmid pHM103+Fopt for vHVT114

The plasmid pHM103 (Merial Limited) containing the Intergenic I arms of HVT FC126, SV40 promoter and SV40 poly A was digested with NotI, dephosphorylated, and the 5.6kb fragment was gel extracted. A NotI flanked 1.7 kb fragment of a chemically synthesized codon-optimized genotype VIId NDV-F gene (SEQ ID NO:1, coding for SEQ ID NO:2) was also NotI digested and the 1.7kb fragment was gel extracted. The 5.6 and 1.7kb fragments were ligated to create pHM103+Fopt (Table 10.2).

**Table 10.2 Characteristics of vHVT114**

| Name | Parental virus | Promoter | F gene | Poly-A | Locus |
|---|---|---|---|---|---|
| vHVT114 | HVT FC126 strain | SV40 | Opt-VIId | SV40 | IG1 |

### Generation of recombinant HVT viral vector

A standard homologous recombination procedure was followed by co-electroporation of secondary CEF cells using donor plasmid pHM103+Fopt and viral DNA isolated from the HVT strain FC126 (Igarashi T. et al., J. Gen. Virol. 70, 1789-1804, 1989). Essentially the procedure described in example 1 was followed to generate, plaque purify and characterize recombinants by immunofluorescence.

After five rounds of plaque purification, a recombinant virus designated as vHVT114 was isolated and the purity was tested by IFA and PCR to confirm NDV-F expression and the absence of parental virus.

### PCR analysis of recombinant vHVT114

DNA was extracted from vHVT114 by phenol/chloroform extraction, ethanol precipitated, and was resuspended in 20mM HEPES. PCR primers were designed to specifically identify the presence of the codon optimized NDV-F, the SV40 promoter, as well as, the purity of the recombinant virus from FC126 CL2 parental virus.

The PCR results showed that the sizes of PCR products after gel electrophoresis correspond well with the expected sizes and the banding patterns.

### Sequence analysis of the inserted region in recombinant vHVT114

Analysis of vHVT114 genomic DNA region was performed by PCR amplification. Total of 10 primers were used to amplify the entire cassette, as well as, beyond the flanking BamHI-I arms used in the donor plasmid. The 4.727 kb PCR product was gel purified and the entire fragment was sequenced using the sequencing primers. The sequence result confirmed that the vHVT114 contains the correct SV40 promoter, the codon-optimized NDV-F and the SV40 polyA sequences that match exactly the sequence described for the donor plasmid pHM103+Fopt in SEQ ID NO:38 (see Fig. 20).

### Western blot analysis of recombinant vHVT114

Approximately 2x10⁶ chicken fibroblast cells were infected at ∼0.1 MOI with vHVT114 Pre-MSV. After two days of incubation at 37°C, infected as well as uninfected cells were harvested using a cell scraper after removing the media and rinsing with PBS. The cells were harvested with 1ml of PBS and centrifuged. The cell pellets were lysed by following the Pierce Classic IP Kit (Thermo Scientific). 100 µl of the anti-NDV-F monoclonal antibody 001C3 (Merial Limited) was used to form the immune complex. The antibody/lysate sample was added to Protein A/G Plus Agarose to capture the immune complex. The immune complex was washed three times to remove non-bound material and then eluted in 50ul volume using sample buffer elution under non-reducing condition. After boiling for 5 minutes, 10 µl of the samples were loaded into a 10% Acrylamide gel (Invitrogen). The PAGE gel was run in MOPS buffer (Invitrogen) at 200volts for 1 hour. Then the gel was transferred onto a PVDF membrane.

The Protein Detector Western Blot Kit TMB System (KPL, cat# 54-11-50) was used for blotting the PVDF membrane by using the reagents and following manufacturer's directions. After blocking the membrane for 1 hour at room temperature, the membrane was then rinsed three times in IX Wash Buffer, five minutes each and then soaked in blocking buffer containing 1:1000 dilution of chicken serum raised against NDV virus (Lot # C0139, Charles River Laboratories). After washing three times in a washing buffer, the membrane was incubated with a peroxidase labeled goat anti-chicken IgG (KPL, cat# 14-24-06) at a dilution of 1:2000 for 1 hour at room temperature. The membrane was then rinsed three times in 1X Wash Buffer, five minutes each. 5ml of TMB membrane peroxidase substrate was added to the membrane and gently rocked for about 1 minute. The developing reaction was stopped by placing the membrane into water.

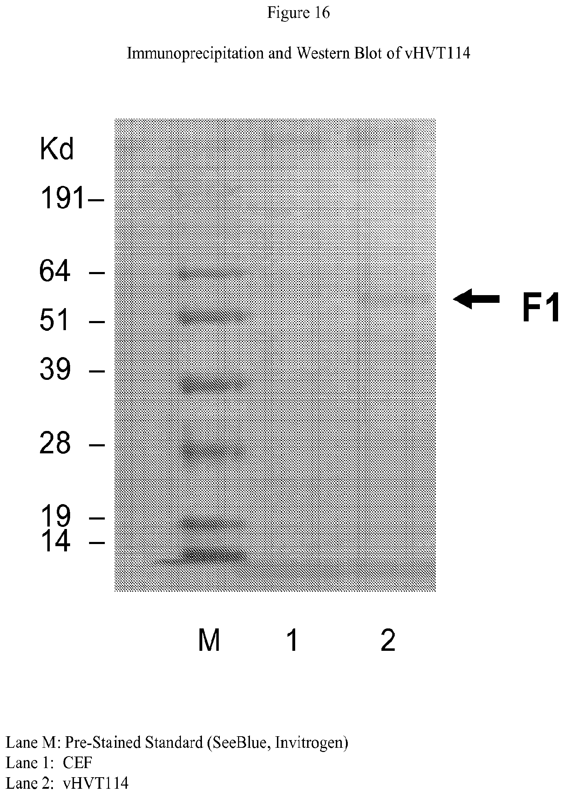

The immunoprecipitation and Western blot technique detected an approximately 55 kD protein in vHVT114 sample that corresponds to the expected size of F1 component of the NDV-F protein (Fig. 16).

### Generation and characterization of other HVT recombinants

Generation and characterization of other HVT recombinants, such as vHVT039, vHVT110, vHVT111, vHVT112, vHVT113, and vHVT116 were essentially done in the same way as for vHVT114 described above. Table 11 shows the features unique to each construct around the expression cassettes, including the respective sequences.

**Table 11 Characteristics of the expression cassettes of single HVT recombinants**

| Name | Parental virus | Promoter | F gene | Poly-A | Locus |
|---|---|---|---|---|---|
| vHVT039 | HVT | MDV gB | Wtnm-Texas | SV40 | IG1 |
| vHVT110 | HVT | mCMV IE | Wt-VIId | SV40 | IG1 |
| vHVT111 | HVT | SV40 | Wt-VIId | SV40 | IG1 |
| vHVT112 | HVT | MCMV IE | Wt-YZCQ | SV40 | IG1 |
| vHVT113 | HVT | MCMVIE | Wt-Texas | SV40 | IG1 |
| vHVT114 | HVT | SV40 | Opt-VIId | SV40 | IG1 |
| vHVT116 | HVT | SV40 | Opt-NDV-F of CA02 | SV40 | IG1 |

### Example 7 Construction of double HVT vectors expressing NDV-F and IBDV VP2,and double HVT vectors expressing IBDV VP2 variants

### Preparation of donor plasmid pHVT US2 SV- Fopt-synPA for vHVT306

The donor plasmid pHVT US2 SV- Fopt-synPA was constructed containing SV40 promoter, synthetic NDV F codon optimized VII gene, synthetic polyA tail flanked by the SORF3 and US2 arm sequences of HVT FC126.

### Generation of recombinant virus

A standard homologous recombination procedure was followed by co-electroporation of secondary CEF cells using donor plasmid pHVT US2 SV-Fopt-synPA and viral DNA isolated from vHVT13 (an HVT vector expressing the IBDV VP2 gene, Merial Limited). Essentially the procedure described in example 1 was followed to generate, plaque purify and characterize recombinants by immunofluorescence.

After two rounds of plaque purification, pure recombinant virus (vHVT306) was isolated and the purity of vHVT306 was tested and confirmed by IFA and PCR.

### PCR analysis

Viral DNA was extracted from vHVT306 pre-master seed virus (pre-MSV) stock by QIA DNeasy Blood & Tissue Kit (Qiagen). PCR primers were designed to identify the presence of the NDV F optimized, the NDV F wild type, the SV40 promoter, the mCMV promoter, the flanking arms of US2 HVT virus and SB-1 virus.

PCR amplification with various primers confirmed that the vHVT306 had the expected amplification patterns and amplicons.

### Genomic analysis

The genomic DNA of vHVT306 pre-MSV stock was sequenced to verify the sequence of the recombination arm region as well as inserted gene cassette,

Primers were designed to amplify the entire inserted gene cassette including recombination arm used in donor plasmid. Analysis of vHVT306 genomic DNA was performed by PCR amplification and followed by nucleotide sequence determination.

The vHVT306 (donor plasmid pHVT US2 SV- Fopt-synPA) containing the recombinant arms, SV40 promoter and NDV F codon-optimized gene was confirmed to be correct as shown in SEQ ID NO:45 (Fig. 20).

### Western blot analysis

The NDV F protein expression of vHVT306 was confirmed by two-step immunodetection. First, the expressed NDV F proteins from vHVT306 infected CEF were captured by the immunoprecipitation using anti-NDV F monoclonal antibody 001C3 (Merial Limited). Subsequently Western blot analysis using anti-NDV polyclonal serum (Charles River Laboratories) was applied to detect the NDV F protein in the captured samples (NDV F protein-monoclonal antibody complex). A 55 kDa protein in vHVT306 pre-MSV lysates was detected by anti-NDV serum which corresponds to the expected size of NDV F1 fusion protein.

### Generation and characterization of other double HVT recombinants

Generation and characterization of double HVT recombinants, such as vHVT301, vHVT302, vHVT303, vHVT304, vHVT202, and vHVT307 were essentially done in the same way as for vHVT306 described above. Table 12 shows the features unique to each construct around the expression cassettes, including the respective sequences.

**Table 12 Characteristics of the expression cassettes of double HVT recombinants**

| Name | Parental virus | Promoter | NDV-F gene or IBDV VP2 gene | Poly-A | Locus |
|---|---|---|---|---|---|
| vHVT301 | vHVT13 | SV40 | Wt-VIId NDV-F | SV40 | IG2 |
| vHVT302 | vHVT13 | US10 | Opt-VIId NDV-F | US10 | US10 |
| vHVT303 | vHVT13 | US10 | Opt-V (CA02) NDV-F | US10 | US10 |
| vHVT304 | vHVT13 | SV40 | Opt-VIId NDV-F | Synthetic | IG2 |
| vHVT306 | vHVT13 | SV40 | Opt-VIId NDV-F | Synthetic | SORF3-US2 |
| vHVT307 | vHVT13 | SV40 | Opt-V (CA02) NDV-F | Synthetic | SORF3-US2 |
| vHVT202 | vHVT306 | Guinea pig CMV | IBDV E VP2 | Synthetic | SORF3-US2 |

### Example 8 Lack of horizontal transmission of gC-deleted SB-1 mutant

The objective of the study was to compare the level of viremia and horizontal transmission induced by the parental SB-1 with that of a recombinant SB-1 virus in which the gC gene was deleted (see example 3).

Two groups (A and B) of thirty one-day-old specific pathogen free (SPF) white Leghorn chicks were randomly constituted. Twenty birds from groups A were vaccinated (D0) by the subcutaneous route (nape of the neck; 0.2ml/bird) with 2000 PFU of parental SB-1 and twenty from groups B with 2000 PFU of the SB-1 gC-deleted mutant. Ten birds were kept unvaccinated in the same isolator as the vaccinated birds (groups Ac and Bc). At 2-weeks-of-age (D14), the spleen as well as 2 feathers of twenty vaccinated birds of groups A and B were removed after euthanasia. At 4-weeks-of-age (D28) the spleen of the 10 contact birds of groups Ac and Bc were also removed for viral isolation. White blood cells were collected from the buffy coat of ground spleens which had added to lymphocyte separation medium and centrifuged. For each bird, 10⁶ leucocytes were added to a 60mm tissue culture dish that contained confluent monolayers of primary chicken embryo fibroblasts (CEF) prepared the day before. Five days post-infection, MDV plaques were counted on each dish and the number of positive birds and mean number of plaques was calculated. For feather follicles samples, the feather pulp was added to SPGA medium and sonicated for 10 seconds before placing on confluent monolayers of primary CEF from which the media had been removed. The pulp suspension was allowed to absorb for 45 minutes prior to adding fresh media with 1% calf serum.

Results of virus isolation from spleen and from feather follicles of vaccinated birds at D14 are reported in Table 13. All birds from both groups were positive for virus isolation from spleen with a similar mean number of plaques of 142.5 and 176.0 for groups A and B, respectively. Virus could be isolated from feather follicles of all birds in group A and from 90% of birds in group B.

Results of virus isolation from spleen of unvaccinated contact birds at D28 are reported in Table 14. Seven out of ten birds from group Ac were positive for virus isolation from spleen indicating that the parental SB-1 spread horizontally to contact birds. Virus could not be isolated from birds of group Bc suggesting that the gC-deleted mutant did not spread to contact birds.

**Table 13 Results of viral isolation from spleen buffy coat (BC) and from feather follicles (FF) of vaccinated birds from groups A and B at D14**

| **Sample No.** | **Group A -SB1** | | **Group B - SB-1 gC deleted** | |
|---|---|---|---|---|
| | **Spleen BC*** | **FF**** | **Spleen BC*** | **FF** |
| 1 | 46 | + | 179 | + |
| 2 | 92 | + | 129 | + |
| 3 | 80 | + | 108 | + |
| 4 | 135 | + | 111 | + |
| 5 | 18 | + | 38 | + |
| 6 | 55 | + | 109 | - |
| 7 | 187 | + | 83 | - |
| 8 | 233 | + | 383 | + |
| 9 | 51 | + | 31 | + |
| 10 | 213 | + | 251 | + |
| 11 | 100 | + | 345 | + |
| 12 | 50 | + | 44 | + |
| 13 | 271 | + | 331 | + |
| 14 | 128 | + | 106 | + |
| 15 | 155 | + | 80 | + |
| 16 | 226 | + | TNTC (563) | + |
| 17 | 145 | + | 145 | + |
| 18 | 114 | + | 224 | + |
| 19 | 88 | + | 181 | + |
| 20 | TNTC*** (462) | + | 78 | + |
| Mean or positive/total | 142.5 | 20/20 | 176.0 | 18/20 |
| Standard deviation | 103.3 | - | 137.6 | - |
| * Average plaque counts from spleen buffy coat (BC) | | | | |
| ** positive sample from feather follicles | | | | |
| *** TNTC too numerous to count | | | | |

**Table 14 Results of viral isolation from spleen buffy coat (BC) of unvaccinated contact birds from groups Ac and Bc at D28**

| **Sample No.** | **Group Ac - SB-1** | **Group Bc - SB-1 gC deleted** |
|---|---|---|
| | **Spleen BCE*** | **Spleen BCE*** |
| 1 | 0 | 0 |
| 2 | 0 | 0 |
| 3 | 0 | 0 |
| 4 | 8 | 0 |
| 5 | 129 | 0 |
| 6 | 3 | 0 |
| 7 | 25 | 0 |
| 8 | 1 | 0 |
| 9 | 108 | 0 |
| 10 | 1 | 0 |
| * Average plaque counts | | |

This study indicates that the level of viremia of the gC-deleted SB-1 mutant measured at D14 post-vaccination was similar to that of the parental SB-1 virus suggesting that the gC deletion did not impair the ability of the SB-1 virus to replicate in vaccinated birds. The level of virus at the feather follicle was slightly lower with the gC-deleted mutant since 2/20 birds did not have detectable amount of virus. Horizontal transmission could be detected in 7/10 birds in contact with birds vaccinated with the parental SB-1. In contrast, no virus could be detected from the birds in contacts with birds vaccinated with the gC-deleted mutant indicating that the gC deletion severely impaired horizontal transmission.

### Example 9 ND efficacy induced by SB-1 recombinant alone or in combination with an HVT-IBD vector vaccine in one day-old SPF chickens

The objective of the study was to evaluate the efficacy of the vSB1-004 recombinant expressing NDV F gene against an ND challenge performed at 4 week-of-age in SPF chicks vaccinated with vSB1-004 alone or in combination with an HVT-IBD vector vaccine.

Three groups (1, 2 and 3) of fifteen one-day-old specific pathogen free (SPF) white Leghorn chicks were randomly constituted. Two vectored vaccines were used: the vSB1-004 described in example 1 and vHVT13, an herpesvirus of turkey (HVT) vector expressing the VP2 gene of infectious bursal disease virus Faragher 52/70 strain (active ingredient of the Merial licensed VAXXITEK^{®} HVT+IBD vaccine, US 5,980,906 and EP 0 719 864). Birds from groups 1, 2 and 3 received vHVT13 only (control group), vSB1-004 only and a mix of vHVT13 and vSB1-004, respectively (see Table 6). All birds were vaccinated by the subcutaneous route (nape of the neck) with 2000 PFU of vSB1-004 and/or vHVT13 (D0). Twenty seven days after vaccination (D27), birds of each group were challenged with the genotype V Mexican Chimalhuacan (Mex V) velogenic NDV strain. The challenge was performed by the intramuscular (IM) route using 10⁵ Egg Infectious Dose 50 (EID50) diluted in 0.2 ml of physiological sterile water. Birds were observed daily during 14 days after challenge for clinical signs and mortality. Oropharyngeal swabs were also sampled from 10 birds per group 5, 7 and 9 days after challenge. The viral RNA load was evaluated in these swabs after RNA extraction by using a quantitative reverse transcriptase real time polymerase chain reaction (qRT-PCR) based on the M gene and described by Wise et al. (2004; Development of a Real-Time Reverse-Transcription PCR for Detection of Newcastle Disease Virus RNA in Clinical Samples; J. Clin. Microbiol. 42, 328-338). Shedding levels were expressed as log10 egg infectious dose 50% (EID50) per mL. Blood was also sampled at the time of challenge (D27). The serums were tested with the anti-IBD ELISA (Synbiotics ELISA ProFlok PLUS IBD) to evaluate the impact of vSBA-004 on the vHVT13-induced IBDV antibodies.

Results of protection and serology are summarized in Table 15. All control birds died within 5 days after ND challenge. The vSB 1-004 recombinant virus induced full clinical protection either alone or when combined with vHVT13. The number of birds shedding detectable amount of challenge ND virus was very low in both vaccinated groups. The mean IBD ELISA titers in groups 1 and 3 were nearly identical indicating the lack of vSB 1-004 interference on vHVT13-induced IBDV antibodies.

**Table 15 Results of ND protection induced by SB-1 recombinants expressing NDV F gene in SPF day-old chicks (15/group) challenged at D27**

| Group | Vaccine (D0) | ND protection | IBD ELISA titer (log10±SD*) | Shedding in oropharyngeal swabs** | | |
|---|---|---|---|---|---|---|
| | | | | D5*** | D7 | D9 |
| 1 | vHVT13 | 0% | 4.04±0.15 | **** | - | - |
| 2 | vSB1-004 | 100% | 0.26±0.50 | 1/10 (2.2) | 0/10 | 0/10 |
| 3 | vSB 1-004 + vHVT13 | 100% | 4.02±0.08 | 3/10 (4.1) | 2/10 (2.8) | 1/9(3.4) |
| * Standard deviation | | | | | | |
| ** number of birds shedding/total (mean log10 EID50 equivalent/mL) | | | | | | |
| *** day post-challenge | | | | | | |
| **** all birds of group 1 died before D5 and therefore, shedding was not evaluated in this group | | | | | | |

The ND challenge model with the genotype V Chimalhuacan velogenic NDV is very severe. In these severe challenge conditions, vSB 1-004 induced full clinical protection and excellent protection against shedding of challenge virus by the oropharyngeal route. It is worth noting that the F gene inserted in vSB 1-004 is from a genotype VIId NDV strain and the challenge strain used here is a genotype V. It shows therefore that the genotype VIId F gene inserted into the SB-1 vector is cross-protecting birds against a genotype V challenge. The addition of vHVT13 did not impair the ND protection induced by vSB 1-004 and the vSB1-004 did not interfere on vHVT13-induced IBD antibody titers, demonstrating compatibility of SB-1 vector with HVT vector.

### Example 10 ND early efficacy induced by SB-1 recombinant in one-day-old SPF chickens

The objective of the study was to evaluate the efficacy of the vSB1-004 recombinant expressing NDV F gene against an early (D14) ND challenge in SPF chicks performed with two different NDV challenge strains.

Two groups (1 and 2) of twenty one-day-old specific pathogen free (SPF) white Leghorn chicks were randomly constituted. Birds from group 2 were vaccinated by the subcutaneous route (nape of the neck) with 2000 PFU of vSB 1-004. Chicks from group 1 were not vaccinated and were kept as control birds. At 2 week-of-age, half of the birds of each group were challenged with the genotype V Mexican Chimalhuacan (Mex V) velogenic NDV strain and the other half with the genotype VIId Malaysia 04-1 (Mal VIId) velogenic NDV strain. The challenge was performed by the intramuscular (IM) route using 10⁵ Egg Infectious Dose 50 (EID50) diluted in 0.2 ml of physiological sterile water. Birds were observed daily during 14 days after challenge for clinical signs and mortality.

Results of protection are summarized in Table 16. All control birds died within 5 days after ND challenges. The vSB1-004 recombinant virus induced partial protection against mortality (70% and 40% protection after challenge with Mal VIId and Mex V, respectively) and against morbidity (50% and 30% protection after challenge with Mal VIId and Mex V, respectively) in these severe early challenge conditions.

**Table 16 Results of early ND protection induced by SB-1 recombinants expressing NDV F gene in SPF day-old chicks**

| Group | Vaccine | Challenge strain | Protection against mortality | Protection against morbidity |
|---|---|---|---|---|
| 1 | - | Mal VIId | 0/10 | 0/10 |
| | | Mex V | 0/9 | 0/9 |
| 2 | vSB1-004 | Mal VIId | 7/10 | 5/10 |
| | | Mex V | 4/10 | 3/10 |

The early ND challenge model that was used to evaluate the efficacy of vSB1-004 recombinant was chosen because Marek's disease virus vectors expressing NDV F gene do not generally provide full protection in this model. Indeed, their onset of immunity is delayed compared to live NDV vaccines (Morgan et al. (1993) Avian Dis 37, 1032-40; Heckert et al. (1996) Avian Dis 40, 770-777). It is therefore a good model to evaluate and compare the vaccine candidates. In these severe early challenge conditions, vSB1-004 recombinant induced partial protection that was only slightly higher against the Malaysian genotype VIId challenge than against the Mexican Chimalhuacan genotype V one indicating a broad protection against the 2 most prevalent genotypes circulating in the Americas and Eurasia/Africa, respectively.

### Example 11 ND efficacy induced by SB-1 recombinant alone or in combination with an HVT-IBD vector vaccine in 1 day-old broiler chickens with maternal antibodies

The objective of the study was to evaluate the efficacy of the vSB1-004 recombinant expressing NDV F gene against two ND challenges performed at 4 week of age in broiler chicks vaccinated with vSB 1-004 alone or in combination with an HVT-IBD vector vaccine.

Six groups (1a, 1b, 2a, 2b, 3a, 3b) of twelve one-day-old broilers (Hubbard JA957 line) were randomly constituted. Two vectored vaccines were used: the vSB1-004 described in example 1 and vHVT13, an herpesvirus of turkey (HVT) vector expressing the VP2 gene of infectious bursal disease virus Faragher 52/70 strain (active ingredient of the Merial licensed VAXXITEK^{®} HVT+IBD vaccine). Birds from groups 1 (1a & 1b) were vaccinated with vHVT13 only (control group); those from groups 2 with vSB1-004 only and those from groups 3 with a mix of vHVT13 and vSB1-004 (see Table 17). All birds were vaccinated by the subcutaneous route (nape of the neck) with 2000 PFU of vSB1-004 and/or vHVT13 (D0). Twenty eight days after vaccination (D28), all birds of each subgroup "a" were challenged with the genotype VIId Malaysia 04-1 (Mal VIId) velogenic NDV strain and all birds of each subgroup "b" with the genotype V Mexican Chimalhuacan (Mex V) velogenic NDV strain. The challenge was performed by the intramuscular (IM) route using 10⁵ Egg Infectious Dose 50 (EID50) diluted in 0.2 ml of physiological sterile water. Birds were observed daily during 14 days after challenge for clinical signs and mortality. Blood was also sampled from 5 birds in each group at the time of challenge (D28). The serums were tested with the anti-IBD ELISA (Synbiotics ELISA ProFlok PLUS IBD) to evaluate the impact of vSB1-004 on the vHVT13-induced IBDV antibodies in broilers.

Results of protection and serology are summarized in Table 17. All control birds died within 5 days after ND challenges. The vSB 1-004 recombinant virus induced significant level of clinical protection when combined or not with vHVT13. The number of birds shedding detectable amount of virus was very low in both vaccinated groups. The mean IBD antibody titers in groups 2 was still high (3 log10) at D27 indicating a high level of maternally-derived IBD antibodies; nevertheless, vHVT13 induced a clear IBD antibody response which was not affected when mixed with vSB 1-004.

**Table 17 Results of ND protection induced by SB-1 recombinants expressing NDV F gene in broiler day-old chicks (12 per group except group 1b: 11) challenged at D28**

| Group | Vaccine (D0) | ND challenge | ND protection | IBD ELISA titer (log10±SD*) |
|---|---|---|---|---|
| 1a | vHVT13 | Mal VIId | 0% | 3.94±0.24 |
| 1b | vHVT13 | Mex V | 0% | |
| 2a | vSB1-004 | Mal VIId | 83% | 3.03±0.44 |
| 2b | vSB 1-004 | Mex V | 75% | |
| 3a | vSB1-004+vHVT13 | Mal VIId | 75% | 4.02±0.23 |
| 3b | vSB1-004+vHVT13 | Mex V | 83% | |
| *Standard deviation | | | | |

Results of this study indicated significant levels of protection induced by vSB1-004 in broilers with NDV MDA. The addition of vHVT13 did not have negative impact on vSB1-004-induced ND protection indicating the lack of vHVT13 interference. Furthermore, vSB1-004 did not interfere on vHVT13-induced IBD antibodies, confirming in broilers the compatibility between these two vectors.

### Example 12 Lack of interference of vSB1-004 on IBD early efficacy induced by an HVT-IBD vector vaccine in 1 day-old SPF chicks

The objective of the study was to evaluate the potential interference of the vSB1-004 recombinant on the IBD efficacy induced by an HVT-IBD vector vaccine (vHVT13) in an early (D14) IBD challenge model in SPF chicks.

Three groups (1 to 3) of ten one-day-old specific pathogen free (SPF) white Leghorn chicks were randomly constituted. Birds from group 1 were vaccinated by the subcutaneous route (nape of the neck) with 2000 PFU of vSB 1-004 (control group). Chicks from group 2 were vaccinated with 2000 PFU of vHVT13 and birds from group 3 were vaccinated with 2000 PFU of vHVT13 and 2000 PFU of vSB 1-004. At 2 week of age, all birds of each group were challenged by the ocular route with 50µL containing 2.5 log10 EID50 of the IBDV classical strain Faragher 52/70. Birds were observed daily during 10 days after challenge for clinical signs and mortality. All birds were euthanized 10 days after challenge and body and bursa of Fabricius weights were recorded in order to evaluate the bursa/body weight ratio. Their bursa was also checked for histological lesions typical of IBD. A score was assigned to each bursa based on the severity of the lesions as shown in Table 18. The number of affected birds (non-protected) in each group was calculated. A bird was considered as affected if it died and/or showed notable sign of disease and/or intermediate or severe lesions of the bursa of Fabricius (i.e., histology score ≥3).

**Table 18 Scoring scale of histological lesions of the bursa of Fabricius**

| Score | Histology observation/lesions |
|---|---|
| 0 | No lesion, normal bursa |
| 1 | 1% to 25% of the follicles show lymphoid depletion (i.e., less than 50% of depletion in 1 affected follicle), influx of heterophils in lesions |
| 2 | 26% to 50% of the follicles show nearly complete lymphoid depletion (i.e., with more than 75% of depletion in 1 affected follicle), the affected follicles show necrosis lesions and severe influx of heterophils may be detected |
| 3 | 51% to 75% of the follicles show lymphoid depletion; affected follicles show necrosis lesions and a severe influx of heterophils is detected |
| 4 | 76% to 100% of the follicles show nearly complete lymphoid depletion; hyperplasia and cyst structures are detected; affected follicles show necrosis lesions and severe influx of heterophils is detected |
| 5 | 100% of the follicles show nearly complete lymphoid depletion; complete loss of follicular structure; thickened and folded epithelium; fibrosis of bursal tissue |

Results of protection are summarized in Table 19. All control birds became sick and one died after challenge whereas all vaccinated birds remained healthy. The bursal body weight ratios of groups 2 and 3 were similar and significantly higher than that of group 1. All 8 birds that survived challenge from group 1 had bursa lesion scores of 4 or 5.

**Table 19 Results of early (D14) IBD protection induced by vHVT13 alone or in combination with vSB 1-004 recombinant expressing NDV F gene in SPF day-old chicks.**

| Group | Vaccine | Mortality | Morbidity | Bursal/Body weight ratio* 100 | Bursa with score ≥3 | Protection |
|---|---|---|---|---|---|---|
| 1 | vSB 1-004 | 1/9* | 9/9 | 0.14±0.02 | 8/8 | 0% |
| 2 | vHVT13 | 0/10 | 0/10 | 0.47±0.10 | 0/10 | 100% |
| 3 | vHVT13+ vSB 1-004 | 0/9* | 0/9 | 0.46±0.20 | 1/9 | 89% |
| * One bird in these groups died before challenge. | | | | | | |

The early IBD challenge model that was used to evaluate the lack of interference of vSB 1-004 recombinant on vHVT 13-induced IBD protection was chosen because it is very sensitive to detect interference on vHVT13 protection. Results obtained with vSB1-004+vHVT13 indicated an excellent level of IBD protection (89%) indicating compatibility between vSB 1-004 and vHVT13 even when measured in an early IBD challenge.

### Example 13 Efficacy of vHVT114, vHVT116, vSB1-007, vSB1-008 (alone or with vHVT13) and vHVT 304 against challenges with NDV ZJ1 (genotype VIId) and California/02 (genotype V) at 21 days of age in SPF chickens

The aim of the study was to assess the efficacy of 2 single HVT recombinant constructs (vHVT1 14 and vHVT1 16), 2 SB1 recombinant constructs (vSB1-007 & vSB1-008) expressing the NDV F gene and a double HVT recombinant (vHVT304) against Newcastle disease challenge with NDV ZJ1 (genotype VIId) and California/02 (genotype V) performed at 21 days of age in SPF chickens.

The characteristics of these 5 vaccine candidates are described in Table 20 below.

**Table 20 Characteristics of the vectors used in the challenge study**

| Name | Parental virus | Promoter | F gene | Poly-A | Locus |
|---|---|---|---|---|---|
| vHVT114 | HVT | SV40 | Opt-VIId | SV40 | IG1 |
| vHVT116 | HVT | SV40 | Opt-V | SV40 | IG1 |
| vSB 1-007 | SB-1 | SV40 | Opt-VIId | gC | gC |
| vSB1-008 | SB-1 | SV40 | Opt-V | SV40 | IG1 |
| vHVT304 | vHVT13* | SV40 | Opt-VIId | Synth | IG2 |
| *vHVT13 is the active ingredient of the licensed Vaxxitek HVT-IBD vaccine based on an HVT vector expressing the IBDV VP2 gene (see US 5,980,906 and EP 0 719 864). | | | | | |

On D0, 158 one-day-old SPF chickens were randomly allocated into 6 groups of 24 birds (vaccinated) and 1 group of 12 birds (non-vaccinated controls). The birds were injected by subcutaneous injection in the neck at D0 with 0.2 mL of recombinant vaccines containing a target dose of 1000 pfu as described in Table 21 below. The birds were then separated into two sub-groups, each sub-group being challenged by the intramuscular route on D21 with 5 log10 EID50 of either NDV ZJ1 (genotype VIId) or California/02 (genotype V) velogenic strain.

**Table 21 Results of efficacy**

| **Group** | **Vaccine at day-old (D0)** | **% clinical protection** | |
|---|---|---|---|
| | | **CA/02 (genotype V)** | **ZJ1 (genotype Vlld)** |
| **G1** | - | 0% | 0% |
| **G2** | vHVT114 | 100% | 100% |
| **G3** | vHVT116 | 100% | 90% |
| **G4** | vSB1-007 | 92% | 100% |
| **G5** | vSB1-008 | 100% | 100% |
| **G6** | vSB1-008+vHVT13 | 100% | 83% |
| **G7** | vHVT304 | 92% | 75% |

Each group was monitored before and after challenge. Technical problems observed with isolators reduced the number of birds in group 2 (vHVT1 14: from 24 to 14) and in group 3 (vHVT1 16: from 24 to 20). NDV clinical signs were recorded after challenge. Serum was collected from blood samples taken from birds of groups 2 and 7 before challenge (D21) for NDV serology by HI test using each challenge strains as antigen.

Percentages of protection against mortality and morbidity are reported in the table above. Full susceptibility was observed in the non-vaccinated challenged control group G1 thus validating the high severity of both challenges. All vaccines induced high levels (≥75%) of protection against both challenges. Full clinical protection against both challenges was induced by vHVT114 and vSB 1-008.

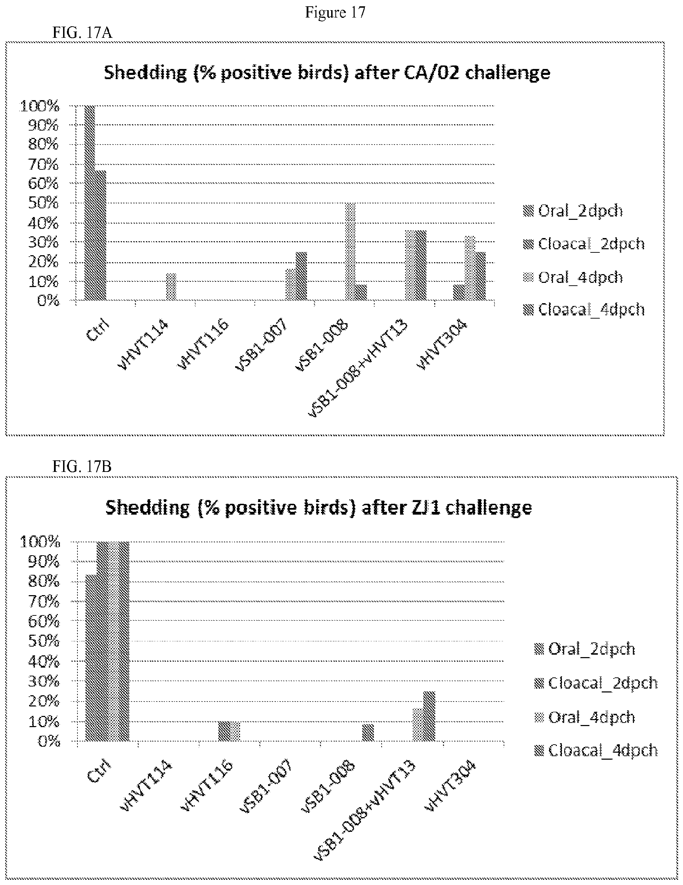

The shedding was evaluated after challenge by real time RT-PCR in oral and cloacal swabs taken 2 and 4 days post-challenge. Percentage of positive (Ct<40) birds are shown for both challenges in Figs. 17A and 17B. Note that all 6 birds were dead at 4 dpch in the control group challenged with the CA/02 isolate and only one bird (out of 6) was still alive at 4 dpch in the control group challenged with ZJ1. Shedding was detected in all control birds. Reduction of the percentage of birds positive for shedding was observed in all vaccinated groups.

In conclusion, the results of this study showed the very good ND protection at 3 weeks of age induced by tested Marek's disease vector vaccines.

### Example 14 Efficacy of vHVT114, vSB1-007, vSB1-009, vHVT306 and vHVT307 vaccines against challenges with NDV Texas GB strain at 28 days of age in SPF chickens

The aim of the study was to assess the efficacy of combinations of different Marek's disease vector vaccines expressing the NDV F and/or the IBDV VP2 gene against Newcastle disease challenge (Texas GB strain, genotype II) performed at 28 days of age in SPF chickens.

The characteristics of the 5 recombinant vaccine candidates tested in this study are described in Table 22 below.

**Table 22 Characteristics of the vectors used in the challenge study**

| Name | Parental virus | Promoter | F gene | Poly-A | Locus |
|---|---|---|---|---|---|
| vHVT114 | HVT | SV40 | Opt-VIId | SV40 | IG1 |
| vSB 1-007 | SB-1 | SV40 | Opt-VIId | gC | gC |
| vSB 1-009 | SB-1 | SV40 | Opt-V (CA02) | gC | gC |
| vHVT306 | vHVT13 | SV40 | Opt-VIId | Synth | SORF3-US2 |
| vHVT307 | vHVT13 | SV40 | Opt-V (CA02) | Synth | SORF3-US2 |

The Marek's disease virus serotype 1 (CVI988 (or Rispens) strain; Gallid herpesvirus 2) and serotype 2 (SB-1 strain; gallid herpesvirus 3) vaccines were used also in combination with recombinant viruses in some of the groups.

On D0, 135 one-day-old SPF chickens were randomly allocated into 9 groups of 15 birds. The birds were injected by subcutaneous injection in the neck at D0 with 0.2 mL containing a target dose of 2000 pfu for recombinant vaccines (vSB1-007, vSB 1-009, vHVT13, vHVT306, vHVT307, vHVT114), and 1000 pfu for parental Marek's disease vaccine strains (SB-1 and CVI988). The design of the study is shown in Table 23 below. The birds were challenged by the intramuscular route on D28 with 4.0 log10 EID50 velogenic ND Texas GB (genotype II) strain.

**Table 23 Results of efficacy**

| **Group** | **Vaccine at day-old (D0)** | **% ND protection after Newcastle disease challenge at 28 days of age** |
|---|---|---|
| **G1** | - | 0% |
| **G2** | vSB1-007+vHVT13 | 80% |
| **G3** | vSB1-009 | 100% |
| **G4** | vSB1-009+vHVT13 | 86% |
| **G5** | vSB1-009+vHVT13+CVI988 | 93% |
| **G6** | vHVT306+SB-1 | 100% |
| **G7** | vHVT307 | 100% |
| **G8** | vHVT307+SB-1 | 93% |
| **G9** | vHVT114+vHVT13+SB-1 | 100% |

Each group was monitored before and after challenge. NDV clinical signs after challenge were recorded.

Percentages of protection against mortality and morbidity are reported in the table 23 above. Full susceptibility was observed in the non-vaccinated challenged control group G1 thus validating the high severity of challenge. Excellent levels of protection were observed in all vaccinated groups. Birds from G3, G6, G7 and G9 were fully protected. This study shows that the vSB1-ND candidates can be co-administered with vHVT13 and CVI988 and still provide a very good ND protection. Similarly, double HVT-IBD+ND are compatible with SB-1 and vHVT-ND (vHVT1 14) is compatible with vHVT13 and SB-1.

In conclusion, the results of this study showed the lack of interference on ND protection induced by the tested Marek's disease parental and vector vaccines.

### Example 15 Efficacy of vHVT114, vHVT307, vSB1-007 and vSB1-009 in combination with vHVT13 against challenges with NDV Chimalhuacan strain (genotype V) at D28 in SPF chickens

The aim of the study was to assess the efficacy of one HVT recombinant construct (vHVT114) and two SB1 recombinant constructs (vSB1-007 and vSB 1-009) expressing the NDV F gene in combination with vHVT-IBD (vHVT13), as well as a double HVT vHVT307 expressing both NDV F and IBDV VP2 against Newcastle disease challenge (Chimalhuacan, genotype V) performed at 28 days of age in SPF chickens.

The characteristics of these 4 vaccine candidates are described in Table 24 below.

**Table 24 Characteristics of the vectors used in the challenge study**

| Name | Parental virus | Promoter | F gene | Poly-A | Locus |
|---|---|---|---|---|---|
| vHVT114 | HVT | SV40 | Opt-VIId | SV40 | IG1 |
| vSB1-007 | SB-1 | SV40 | Opt-VIId | gC | gC |
| vSB1-009 | SB-1 | SV40 | Opt-V (CA02) | gC | gC |
| vHVT307 | vHVT13* | SV40 | Opt-V (CA02) | Synth | SORF3-US2 |

On D0, 45 one-day-old SPF chickens were randomly allocated into 4 groups of 10 birds and 1 group of 5 birds (unvaccinated control group). The birds were injected by subcutaneous injection in the neck at D0 with 0.2 mL of recombinant vaccines containing a target dose of 2000 pfu as described in Table 25 below. The birds were challenged by the intramuscular route on D28 with 5.0 log10 EID50 velogenic Chimalhuacan (genotype V) strain.

**Table 25 Study design and results of ND efficacy**

| **Group** | **Vaccine at day-old (D0)** | **% protection against mortality** | **% protection against morbidity** |
|---|---|---|---|
| **G1** | - | 0% | 0% |
| **G2** | vHVT114+vHVT13 | 100% | 100% |
| **G3** | vHVT307 | 80% | 80% |
| **G4** | vSB1-007+vHVT13 | 90% | 90% |
| **G5** | vSB1-009+vHVT13 | 90% | 90% |

Each group was monitored before and after challenge. NDV clinical signs were recorded after challenge. Oropharyngeal swabs were taken in the vaccinated groups at 5 and 7 days post-challenge to evaluate the viral load by real time RT-PCR.

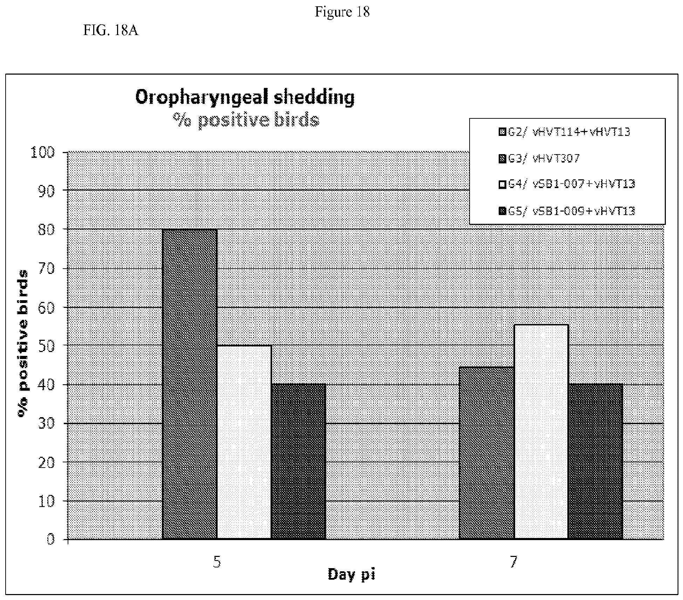

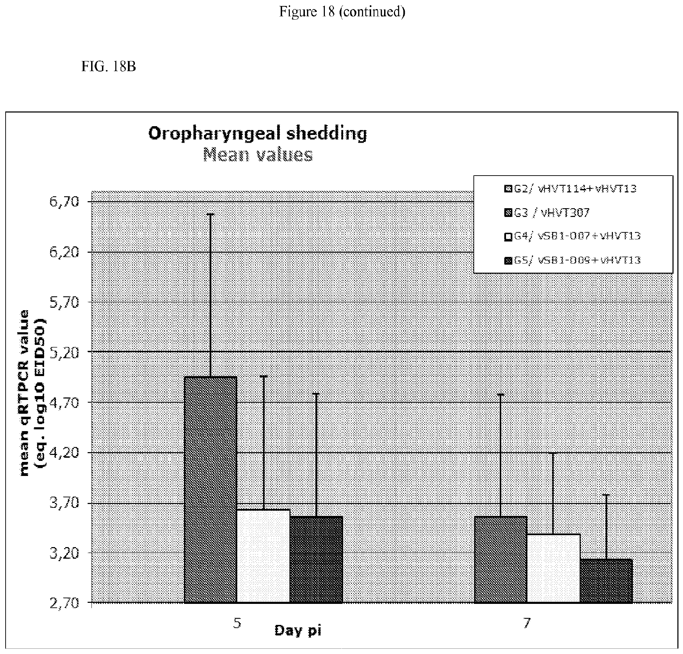

Percentages of protection against mortality and morbidity are reported in the table above. Full susceptibility was observed in the non-vaccinated challenged control group G1 thus validating the high severity of challenge. Very good protection was observed in all 4 vaccinated groups, a full clinical protection being induced by vHVT114+vHVT13. The percentage of positive birds and the mean shedding titer (expressed as log 10 EID50 equivalent per mL) are shown in Figs. 18A and 18B. Surprisingly, no shedding was detected in G2 indicating a complete (against both clinical signs and shedding) ND protection induced by vHVT114 even if co-administered with vHVT13, in the tested conditions. The shedding levels detected in the other vaccinated groups were low with a slightly higher level detected in G3 (vHVT307) at 5 days post-infection (pi) only.

In conclusion, this example further illustrates the excellent ND protection induced by double HVT-IBD+ND recombinant or a combination of SB1-ND or HVT-ND and HVT-IBD (vHVT13) recombinant viruses. Contrary to the general belief in the field that a second HVT vaccine (regular HVT vaccines or recombinant HVT vaccines) interferes with the immunity to the foreign genes inserted into the first recombinant HVT vaccine, the present invention showed surprising result that vHVT114 in combination with vHVT13 offered excellent protection against NDV and no interference effect was observed.

### Example 16 Efficacy of vHVT306, vSB1-008 in combination with vHVT13 administered by SC or in ovo route against challenge with NDV Chimalhuacan strain (genotype V) at D28 in SPF chickens

The aim of the study was to assess the efficacy of the vHVT306 double HVT expressing both NDV F and IBDV VP2 genes, and the vSB1-008 SB1 recombinant expressing the NDV F gene in combination with vHVT-IBD (vHVT13), administered by the *in ovo* or by the subcutaneous route against Newcastle disease challenge (Chimalhuacan, genotype V) performed at 28 days of age in SPF chickens.

The design of the groups is shown on Table 26. Sixty SPF embryonated eggs (after approximately 18 days and 18 hours of incubation; D-3) were used for the *in ovo* administration (20 per group for G1, G2 and G3). Fifty microliters of vaccine containing 2000 PFU were administered by the *in ovo* route using the IntelliLab System device from AviTech LLC (Salisbury, MD, USA). Hatchability and survival were recorded after *in ovo* administration. On D0, 20 one-day-old SPF chickens were randomly allocated into 2 groups of 10 birds (G4 and G5). The birds were injected by subcutaneous (SC) injection in the neck at D0 with 0.2 mL of recombinant vaccines containing a target dose of 2000 pfu as described in Table 26 below. Ten birds per group were challenged by the intramuscular route on D28 with 5.0 log10 EID50 velogenic Chimalhuacan (genotype V) strain.

**Table 26 Study design and results of ND efficacy**

| **Group** | **Vaccine at day-old (D0)** | **Admin. route** | **% protection against mortality** | **% protection against morbidity** |
|---|---|---|---|---|
| **G1** | vHVT13 | *In ovo* | 0% | 0% |
| **G2** | vHVT306 | *In ovo* | 100% | 100% |
| **G3** | vSB1-008+vHVT13 | *In ovo* | 78% | 68% |
| **G4** | vHVT306 | SC | 100% | 100% |
| **G5** | vSB1-008+vHVT13 | SC | 100% | 70% |

Each group was monitored before and after challenge. NDV clinical signs were recorded after challenge. Oropharyngeal swabs were taken in the vaccinated groups at 5 and 7 days post-challenge to evaluate the viral load by real time RT-PCR.

Full hatchability and viability were recorded up to D28 (challenge day) for birds of groups G1 and G2. Hatchability in G3 was 85% and one additional bird died after hatching in this group. The lower hatchability of that group may be due to egg incubator problems. Body weights of males and females in G1, G2 and G3 were similar at D1 and at D28.

Percentages of protection against mortality and morbidity are reported in the table 26. Full susceptibility was observed in the non-vaccinated challenged control group G1 thus validating the high severity of challenge. Very good protection was observed in all 4 vaccinated groups, a full clinical protection being induced by vHVT306 administered by both routes.

The percentage of positive birds and the mean shedding titer (expressed as log 10 EID50 equivalent per mL) are shown in Table 27. Absence of detectable or very low shedding was observed in G2 and G4 vaccinated with vHVT306. The shedding levels detected in the groups vaccinated with vSB1-008+vHVT13 were higher especially at 5 days post-infection (pi).

**Table 27 Results of protection against shedding (percentage of birds with detectable shedding and mean viral load in log10) evaluated at D5 and D7 after NDV challenge**

| **Group** | **Vaccine at day-old (D0)** | **Admin. Route** | **Percent of positive birds (D5/D7 pi)** | **Mean viral load* (D5/D7 pi)** |
|---|---|---|---|---|
| **G2** | vHVT306 | ***In ovo*** | 0/0% | 2.7/2.7 |
| **G3** | vSB1-008+vHVT13 | ***In ovo*** | 100/38% | 5.2/3.2 |
| **G4** | vHVT306 | SC | 20/10% | 3.2/2.9 |
| **G5** | vSB1-008+vHVT13 | SC | 80/50% | 4.6/3.4 |
| * Mean quantitative real time PCR value expressed in equivalent log10 EID50; the threshold is set at 2.7 log10. | | | | |

In conclusion, this example shows excellent ND protection induced by vHVT306 double HVT recombinant administered either by *in ovo* or by SC routes. The performance of vSB1-008+vHVT13 was slightly lower especially after *in ovo* administration, but it may be at least partially due to egg incubator problems. Indeed, the *in ovo* safety testing of another SB1-ND recombinant (vSB1-009) at 1000 or 4000 PFU associated with 6000 PFU of vHVT13 did not show any difference in hatchability and early survival with a group receiving 6000 PFU of vHVT13 only.

### Example 17 Efficacy of vHVT304, vHVT306, vSB1-007 and vSB1-008 in combination with vHVT13 against challenge with NDV Chimalhuacan strain (genotype V) at D42 in commercial broiler chickens

The aim of the study was to assess the efficacy of two double HVT (vHVT304 and vHVT306) expressing both NDV F and IBDV VP2 genes, and two SB1 recombinants (vSB1-007 and vSB 1-008) expressing the NDV F gene in combination with vHVT-IBD (vHVT13) against Newcastle disease challenge (Chimalhuacan, genotype V) performed at 42 days of age in commercial broiler chickens.

The design of the groups is shown on Table 28. On D0, 55 one-day-old commercial broiler chickens were randomly allocated into 5 groups of 11 birds. The birds were injected by subcutaneous (SC) injection in the neck at D0 with 0.2 mL of recombinant vaccines containing a target dose of 2000 pfu as described in Table 28 below. Ten birds per group were challenged by the intramuscular route on D42 with 5.0 log10 EID50 velogenic Chimalhuacan (genotype V) strain.

**Table 28 Study design and results of ND efficacy**

| **Group** | **Vaccine at day-old (D0)** | **% protection against mortality** | **% protection against morbidity** |
|---|---|---|---|
| **G1** | vHVT13 | 0% | 0% |
| **G2** | vHVT304 | 82% | 82% |
| **G3** | vHVT306 | 100% | 100% |
| **G4** | vSB1-007+vHVT13 | 100% | 100% |
| **G5** | vSB 1-008+vHVT13 | 91% | 91% |

Each group was monitored before and after challenge. NDV clinical signs were recorded during 14 days after challenge. Oropharyngeal swabs were taken in the vaccinated groups at 5 and 7 days post-challenge to evaluate the viral load by real time RT-PCR.

Percentages of protection against mortality and morbidity are reported in the table 28. Full susceptibility was observed in the non-vaccinated challenged control group G1 thus validating the high severity of challenge. Very good protection was observed in all 4 vaccinated groups, a full clinical protection being induced by vHVT306 and by vSB1-007+vHVT13.

The percentage of positive birds and the mean shedding titer (expressed as log10 EID50 equivalent per mL) are shown in Table 29. The best reduction of shedding was induced by vHVT306 and vSB1-007+vHVT13, which were also the best candidates for clinical protection.

**Table 29 Results of protection against shedding (percentage of birds with detectable shedding and mean viral load in log10) evaluated at D5 and D7 after NDV challenge (pi)**

| **Group** | **Vaccine at day-old (D0)** | **Percent of positive birds (D5/D7 pi)** | **Mean viral load* (D5/D7 pi)** |
|---|---|---|---|
| **G2** | vHVT304 | 100/100% | 5.4/4.6 |
| **G3** | vHVT306 | 40/50% | 3.5/3.7 |
| **G4** | vSB1-007+vHVT13 | 80/70% | 3.8/4.8 |
| **G5** | vSB 1-008+vHVT13 | 100/100% | 4.8/4.3 |
| * Mean quantitative real time PCR value expressed in equivalent log10 EID50; the threshold is set at 2.7 log10. | | | |

The vSB1-007+vHVT13 performed better than vSB1-008+vHVT13. The vSB1-007 genomic structure differs from that of vSB1-008 in different aspects: locus of insertion, promoter, poly-adenylation signal and F gene origin. The combination of these foreign sequences and locus of insertion in vSB 1-007 were likely responsible for its better ND protection performances.

In summary, this example illustrates the importance of the locus of insertion and other regulatory sequences of the NDV expression cassette in the ND protection induced by HVT and MDV serotype 2 vectors.

### Example 18 Efficacy of double HVT-ND+IBD (vHVT304 and vHVT306) or SB1-ND (vSB1-008) in combination with vHVT13 recombinant vaccines, against challenge with a classical IBDV isolate on D14 in SPF chickens

The aim of the study was to assess the early IBD efficacy of double HVT recombinants vHVT304 and vHVT306 as well as that of vHVT13 co-administered with a SB1-ND (vSB1-008) recombinant constructs against a virulent infectious bursal disease virus (vIBDV) challenge (Faragher 52/70 strain) performed at 14 days of age in SPF chickens.

On D0, 95 one-day-old SPF chickens were randomly allocated into 9 groups of 10 birds and 1 group of 5 birds (unvaccinated unchallenged control group). The birds were injected by subcutaneous injection in the neck at D0 with 0.2 mL of recombinant vaccines containing a target dose of 300 or 1000 pfu as described in the Table 30 below. On D14, blood sample was collected from 5 birds per group for serological testing with the Kit ProFLOK^{®} plus IBD (Synbiotics Corp). The birds (10 birds per group except for group 7 in which 1 bird died before challenge) were challenged by the eye drop (0.05 mL per bird) with 2.5 log10 EID50.

**Table 30 Study design and results of IBD efficacy**

| **Group** | **Vaccine at day-old (dose in PFU)** | **IBD+ ELISA titer at D14¹** | **Number Dead/Sick²** | **% protecttion³** | **Mean bursal/body weight ratio⁴** |
|---|---|---|---|---|---|
| **G1** | vSB1-008 (1000) | 0.2 | 7/10 | 0% | 0.0013 |
| **G2** | vHVT13 (300) | 2.7 | 0/0 | 100% | 0.0051 |
| **G3** | vHVT13 (1000) | 2.7 | 0/0 | 90% | 0.0049 |
| **G4** | vHVT13+vSB1-008 (300) | 1.9 | 1/1 | 60% | 0.0041 |
| **G5** | vHVT13+vSB1-008 (1000) | 2.4 | 0/0 | 70% | 0.0041 |
| **G6** | vHVT304 (300) | 2.9 | 0/0 | 60% | 0.0037 |
| **G7** | vHVT304 (1000) | 2.2 | 0/0 | 67% | 0.0047 |
| **G8** | vHVT306 (300) | 2.4 | 0/0 | 80% | 0.0033 |
| **G9** | vHVT306 (1000) | 2.7 | 0/0 | 40% | 0.0026 |
| ¹ Mean IBD+ ELISA titers expressed in log10 in the serum of 5 birds per group sampled at D14 before challenge; | | | | | |
| ² Birds sick for more than 2 days or still sick on D25 were considered as sick. | | | | | |
| ³ Protection against clinical signs and severe bursal lesion (bursal score <3) | | | | | |
| ⁴ The bursal/body weight ratio of the unvaccinated/unchallenged group was 0.0047. | | | | | |

Each group was monitored before and after challenge. IBDV clinical signs were recorded for 11 days after challenge (from D15 to D25). At the end of the post-challenge observation period (D25), all the surviving birds were euthanized and necropsied. Body and bursal weights were recorded. Each bursa of Fabricius (BF) was weighted then stored in individual recipients containing 4% formaldehyde for histology. Histological lesions of the bursa were scored according to the scale presented in Table 31.

**Table 31 Scoring scale of histological lesions of the bursa of Fabricius***

| **Score** | **Histology observation/lesions** |
|---|---|
| 0 | No lesion, normal bursa |
| 1 | 1% to 25% of the follicles show lymphoid depletion (i.e. less than 50% of depletion in 1 affected follicle), influx of heterophils in lesions |
| 2 | 26% to 50% of the follicles show nearly complete lymphoid depletion (i.e. more than 75% of depletion in 1 affected follicle), affected follicles show necrosis and severe influx of heterophils may be detected |
| 3 | 51 % to 75% of the follicles show lymphoid depletion; affected follicles show necrosis lesions and a severe influx of heterophils is detected |
| 4 | 76% to 100% of the follicles show nearly complete lymphoid depletion; hyperplasia and cyst structures are detected; affected follicles show necrosis and severe influx of heterophils is detected |
| 5 | 100% of the follicles show nearly complete lymphoid depletion; complete loss of follicular structure, thickened and folded epithelium, fibrosis of bursal tissue |
| * sourced from Monograph No. 01/2008:0587 of EU Pharmacopoeia "Avian Infectious Bursal Disease vaccine (live) | |

A bird was considered as affected if it died and/or showed notable sign of disease and/or severe lesions of the bursa of Fabricius (*i.e*., histology score ≥3).

The mean ELISA IBD+ antibody titer expressed in log10 before challenge is shown in Table 30. Significant titers were detected in all vaccinated groups that were significantly higher than that of the control group G1. The serology titer was not dose-dependent.

Severe clinical signs were observed after challenge in all birds of the control group G1. Seven out of 10 birds of that group died within the 11 days observation period indicating the high severity of challenge. None of the vaccinated birds showed severe clinical signs after challenge except 1 bird of G4 that died. Percentages of protection against severe bursal lesions are shown in the table 30 above. Significant IBD protection was observed in all groups, the best protection being observed in G2 and G3 (vHVT13 alone). The co-administration of vSB1-008+vHVT13 and the double vHVT304 and vHVT306 constructs induced similar levels of IBD protection. The protection was not dose-dependent at the tested doses. The mean bursal/body weight ratios are also shown in Table 30. Ratios in all vaccinated groups were higher than those of the challenged control group.

In conclusion, these data indicate that both the combination of a SB1-ND vector with a single HVT-IBD or double HVT expressing both NDV-F and IBDV-VP2 induce IBD antibodies and early IBD protection in a severe IBDV challenge model.

### Example 19 Efficacy of single HVT-ND (vHVT114) or SB1-ND (vSB1-007 and vSB1-009) in combination with vHVT13 recombinant vaccines, against challenge with a very virulent IBDV isolate on D23 in commercial broiler chickens

The aim of the study was to assess the IBD efficacy of vHVT13 co-administered with an HVT-ND (vHVT1 14) or SB1-ND (vSB1-007 and vSB 1-009) recombinant constructs against a very virulent infectious bursal disease virus (wIBDV) challenge (91-168/980702 isolate) performed at 23 days of age in commercial broiler chickens.

On D0, 90 one-day-old broiler chickens were randomly allocated into 7 groups of 12 birds and 1 group of 6 birds (unvaccinated unchallenged control group). The birds were injected by subcutaneous injection in the neck at D0 with 0.2 mL of recombinant vaccines containing a target dose of 3000 pfu as described in the Table 32. On D14, blood sample was collected from 5 birds per group for serological testing with the Kit ProFLOK^{®} plus IBD (Synbiotics Corp). The serum of 10 extra one-day-old broiler chickens was tested at D0 with the same kit to evaluate the level of IBDV maternal antibody. The birds (10 birds per group) were challenged by the eye drop (0.05 mL per bird) on D23 with 4.3 log10 EID50 of the vvIBDV 91-168 isolate.

Each group was monitored before and after challenge. IBDV clinical signs were recorded for 11 days after challenge (from D23 to D33). At the end of the post-challenge observation period (D33), all the surviving birds were euthanized and necropsied. Body and bursal weights were recorded. Each bursa of Fabricius (BF) was weighted then stored in individual recipients containing 4% formaldehyde for histology. Histological lesions of the bursa were scored according to the scale presented in Table 31.

A bird was considered as affected if it died and/or showed notable signs of disease and/or severe lesions of the bursa of Fabricius (*i.e*., histology score ≥3).

**Table 32 Study design and serology results**

| **Group** | **Vaccine at day-old (D0)** | **IBD+ ELISA titer at D23¹** | **Mean bursal/body weight ratio²** |
|---|---|---|---|
| **G1** | - | 3.9 | 0.0007 |
| **G2** | vHVT13 | 4.0 | 0.0015 |
| **G3** | vHVT114+vHVT13 | 4.1 | 0.0015 |
| **G4** | vSBI-007+vHVT13 | 3.8 | 0.0018 |
| **G5** | vSBI-009+vHVT13 | 4.0 | 0.0019 |
| ¹ Mean IBD+ ELISA titers expressed in log10 in the serum of 5 birds per group sampled at D23 before challenge; | | | |
| ² The bursal/body weight ratio of the unvaccinated/unchallenged group was 0.0047 | | | |

The mean ELISA IBD+ serological titer at D0 was 4.36±0.01 log10 indicating a very high level of IBD maternal antibody at hatch. At D23, the mean ELISA IBD+ titer was still high (3.9) in the control G1. ELISA mean titers in the vaccinated groups were not significantly different from those of the control group.

Neither morbidity nor mortality was observed in any of the groups after challenge. Percentages of protection against severe bursal lesions are shown in Table 32 above. The result showed that co-administration of vHVT114, vSB 1-007 or vSB1-009 did not interfere with vHVT13-induced IBD protection indicating a lack of interference. Similarly, the mean bursal/body weight ratios of the vaccinated groups were similar and clearly higher than that of the control group, indicating IBD protection and no difference between the vaccination regimens.

In conclusion, the data indicate the compatibility between vHVT114, vSB1-007 or vSB 1-009 and vHVT13 for IBD protection.

### Example 20 Efficacy of double HVT-ND+IBD (vHVT304 and vHVT306) associated or not with SB-1 and of SB1-ND (vSB1-007 and vSB1-008) in combination with vHVT13 recombinant vaccines, against challenge with a variant E IBDV isolate on D28 in SPF chickens

The aim of the study was to assess the efficacy of two double HVT (HVT-ND+IBD: vHVT304 and vHVT306) or two vSB-1-NDV in combination with vHVT13 (vSB1-007+vHVT13, vSB1-008+vHVT13) vectored vaccines administered subcutaneously (SC) to day-old SPF chicks and challenged with IBDV-Variant (VAR-E) 28 days post-vaccination.

On D0, 105 one-day-old SPF chickens were randomly allocated into 7 groups of 15 birds including a group of challenged controls (G6) and unchallenged controls (G7). The birds of groups G1 to G5 were injected by subcutaneous injection in the neck at D0 with 0.2 mL of recombinant and/or SB-1 vaccines containing each a target dose of 2000 pfu. The design of the study is shown in Table 33 below. On D28, all birds from groups G1 to G6 were challenged by the eye drop (0.03 mL containing 3 log10 EID50 per bird) of the IBDV variant E isolate from University of Delaware (USA). Each group was monitored before and after challenge. Eleven days post-challenge, birds were weighed and necropsied. The bursa were collected and weighed. The bursal/body weight ratio (bursa weight/body weight ratio X 100) was calculated.

**Table 33 Study design and results of IBD efficacy**

| **Group** | **Vaccine at day-old** | **Mean bursal/body weight ratio (*100)** |
|---|---|---|
| **G1** | vHVT304 | 0.33 |
| **G2** | vHVT304+SB-1 | 0.33 |
| **G3** | vHVT306 | 0.29 |
| **G4** | vHVT13+vSB 1-007 | 0.49 |
| **G5** | vHVT13+vSB1-008 | 0.47 |
| **G6** | - (challenged) | 0.13 |
| **G7** | - (unchallenged) | 0.46 |

The mean bursal/body weight ratios are shown in Table 33. The challenged control birds had a severe bursal atrophy compared to unchallenged ones. The vSB1-007 and vSB 1-008 vaccines did not interfere on vHVT13-induced protection (G4 and G5). The bursal/body weight ratios of birds vaccinated with the double HVT (HVT-ND+IBD) were slightly lower than the unchallenged control group but were clearly higher than the challenged control groups. Furthermore, the SB-1 serotype 2 Marek's disease vaccine did not interfere with vHVT304-induced IBD protection.

In conclusion, these data indicate that both the combination of a SB1-ND vector with a single HVT-IBD or double HVT expressing both NDV-F and IBDV-VP2 induce IBD protection in a variant E IBDV challenge model.

### Example 21 Lack of interference of vHVT114, vSB1-009 and/or SB-1 on vHVT13 induced variant E IBD protection in SPF chickens

The aim of the study was to assess the IBD efficacy of vHVT13 when administered by SC or in ovo route concomitantly with vHVT114, vSB 1-009 and/or SB-1 in SPF chicks in an IBDV-Variant (VAR-E) at D28 challenge model.

75 one-day-old SPF chickens and 75 SPF 18 to 19 day-old chicken embryo were randomly allocated into 5 groups (G1 to G5 and G6 to G10, respectively) including a group of challenged controls (G4 and G9, respectively) and unchallenged controls (G5 and G10, respectively). The birds of groups G1 to G3 were injected by subcutaneous injection in the neck at D0 with 0.2 mL of vaccines containing each a target dose of 3000 pfu except for SB-1 which had a target dose of 1000 PFU. Birds from G6 to G8 received the same vaccine doses but in 0.05 mL volume by the *in ovo* route 2-3 days before hatch. The design of the study is shown in Table 34 below. At 28 days of age, all birds from groups G1 to G4 and G6 to G9 were challenged by the eye drop (0.03 mL containing 3 log10 EID50 per bird) of the IBDV variant E isolate from University of Delaware (USA). Each group was monitored before and after challenge. Eleven days post-challenge, birds were weighed and necropsied. The bursa were collected and weighed. The bursal/body weight ratio (bursa weight/body weight ratio X 100) was calculated.

**Table 34 Study design and results of IBD efficacy**

| **Group** | **Vaccine at day-old** | **Administration route** | **Mean bursal/body weight ratio (*100)** |
|---|---|---|---|
| **G1** | vHVT13+vHVT114+SB-1 | SC | 0.56 |
| **G2** | vHVT13+vHVT114+vSB1-009 | SC | 0.58 |
| **G3** | vHVT13+vSB1-009 | SC | 0.52 |
| **G4** | - (challenged) | SC | 0.13 |
| **G5** | - (unchallenged) | SC | 0.51 |
| **G6** | vHVT13+vHVT114+SB-1 | *In ovo* | 0.54 |
| **G7** | vHVT13+vHVT114+vSB1-009 | *In ovo* | 0.47 |
| **G8** | vHVT13+vSB1-009 | *In ovo* | 0.53 |
| **G9** | - (challenged) | *In ovo* | 0.14 |
| **G10** | - (unchallenged) | *In ovo* | 0.58 |

The mean bursal/body weight ratios are shown in Table 34. The challenged control birds (G4 and G9) had a severe bursal atrophy compared to unchallenged ones. The bursal/body weight ratios of the vaccinated groups (G1 to G3 and G6 to G8) were similar to those of the unchallenged control groups (G5 and G10) and well above those of the challenged control groups (G4 and G9). The lack of interference of vHVT114 on vHVT 13-induced IBD protection after both SC or *in ovo* routes was surprising and confirmed data obtained in examples 15 and 19.

In conclusion, these data indicate clearly the compatibility of vHVT114+vSB1-009 or +SB-1 and of vSB 1-009 with vHVT13 when administered by SC or *in ovo* route in a variant E IBDV challenge model.

### Example 22 Efficacy of vHVT114 and vHVT13 and SB1 or vSB1-009 vectors against very virulent plus Marek's disease challenge

The aim of this study was to evaluate the Marek's disease efficacy induced by different combinations of vaccines including vHVT114, vHVT13, SB-1 and/or vSB1-009 administered by the SC route to one-day-old SPF chicks and challenged 4 days later with the very virulent plus Marek's disease virus (vv+MDV) T-King isolate.

On D0, 100 one-day-old SPF chickens were randomly allocated into 5 groups of 20 birds. The birds from groups 1 to 3 were injected by subcutaneous injection in the neck at DO with 0.2 mL of vaccines containing a target dose of 2000 pfu for each vaccine except for SB-1 for which the target dose was 1000 pfu. Birds from groups 4 and 5 were non-vaccinated and were used as sham controls challenged (group 4) or unchallenged (group 5). The study design is shown in the Table 35. On D4, All birds from groups 1 to 4 were challenged with 0.2 mL of the vv+MDV T-King isolate using the intraperitoneal route of administration.

**Table 35 Study design and MD protection results**

| **Group** | **Vaccine at day-old (D0)** | **Number of MD positive/total** | **Percentage of protection** |
|---|---|---|---|
| **G1** | vHVT13+SB-1 | 7/20 | 65% |
| **G2** | vHVT114+SB-1 | 7/20 | 65% |
| **G3** | vHVT13+vHVT114+vSB1-009 | 7/20 | 65% |
| **G4** | - (challenged) | 20/20 | 0% |
| **G5** | - (unchallenged) | 0/20 | 100% |

Each group was monitored daily for any unfavourable reactions before and after challenge. At day 49, all live birds were terminated and necropsied to examine for gross lesions associated with Marek's disease. Chickens were classified as positive for infection with Marek's disease if nervous signs, such as paralysis, locomotive signs attributable to the disease, and severe emaciation or depression are observed, if mortality directly attributable to Marek's Disease occurs, or if gross lesions are observed at necropsy. Lesions might include, but not be limited to, the following: liver, heart, spleen, gonads, kidneys, and muscle lesions

Results of protection are shown in the Table 35 above. All vaccinated groups (G1 to G3) performed equally, inducing a partial (65%) MD protection as expected in this very severe and early challenge model. These results indicated that the vector vaccine candidates retain their ability to protect against Marek's disease.

### Example 23 Evaluation of Marek's disease efficacy of the SB1-ND vector combined with HVT-IBD vector

The synergy between parental HVT and SB-1 in inducing a protection against Marek's disease is well known. The SB-1 vector expressing a foreign gene can therefore be mixed with either parental HVT or vectored HVT expressing another foreign gene in order to get a bivalent or a trivalent vaccine solution, respectively. An example of evaluation of Marek's disease efficacy induced by a combination of vSB1-009 with vHVT114 and vHVT13 is shown above (example 22). Marek's disease (MD) efficacy is also demonstrated for Marek's disease vectored recombinants either alone or in combination in other MD challenge models including virulent Marek's disease (vMD) challenge such as GA22, very virulent Marek's disease (vvMD) challenge such as RB1B and/or very virulent plus Marek's disease (vv+MD) challenge such as the T. King virus. One-day-old chickens are inoculated subcutaneously or 18-19-day-old embryonated eggs are inoculated with a 0.2 ml dose or 0.05 ml dose, respectively, of the test viruses. At five days of age the vaccinated chickens and naive controls are challenged with the relevant Marek's challenge virus (v, vv, or vv+ MDV). The challenged birds are observed until seven weeks of age. All birds are terminated and necropsied to observe for grossly visible lesions associated with Marek's disease as described in Example 22.

### Example 24 Efficacy of vSB1-004, vSB1-006, vSB1-007, vSB1-008, SB1-vectored ND vaccine alone or in association with vHVT13 HVT-vectored IBD vaccine, and the vHVT302 and vHVT304 vaccines against challenges with NDV Texas GB strain at 14 and/or 28 days of age in SPF chickens

The aim of the study was to assess the efficacy of combinations of different Marek's disease vector vaccines expressing the NDV F and/or the IBDV VP2 gene against Newcastle disease challenge (Texas GB strain, genotype II) performed at 14 and/or 28 days of age in SPF chickens.

The characteristics of the 6 NDV recombinant vaccine candidates tested in this study are described in the Table 36 below.

**Table 36 characteristics of the 6 NDV recombinant vaccine candidates tested in this study**

| Name | Parental virus | Promoter | F gene | Poly-A | Locus |
|---|---|---|---|---|---|
| vSB1-004 | SB-1* | mCMV IE | Wt-VIId | SV40 | SORF4/US10 |
| vSB1-006 | SB-1 | SV40 | Opt-VIId | Synthetic | UL55/LORF5 |
| vSB 1-007 | SB-1 | SV40 | Opt-VIId | (endogeneous from gC gene) | gC |
| vSB1-008 | SB-1 | SV40 | Opt-CA02 | Synthetic | UL55/LORF5 |
| vHVT302 | vHVT13 | US10 | Opt-VIId | US10 | US10 |
| vHVT304 | vHVT13 | SV40 | Opt-VIId | Synthetic | IG2 |

On D0, 225 one-day-old SPF chickens were randomly allocated into 9 groups of 15 birds (G1a to G9a challenged at D14) and 6 groups of 15 birds (G1b, G3b, G4b, G5b, G8b, G9b challenged at D28). The birds were injected by subcutaneous injection in the neck at D0 with 0.2 mL containing a target dose of 2000 pfu for recombinant vaccines. The design of the study is shown in Table 37 below. The birds were challenged by the intramuscular route on D14 or D28 with 4.3 and 4.2 log10 EID50 (0.1 mL) velogenic ND Texas GB (genotype II) strain, respectively.

**Table 37 Results of ND efficacy**

| **Group** | **Vaccine at day-old (D0)** | **%ND protection after ND challenge at 14 days of age** | **%ND protection after ND challenge at 28 days of age** |
|---|---|---|---|
| **G1a & 1b** | - | 0% | 0% |
| **G2a** | vSB1-004 | 20% | ND* |
| **G3a & 3b** | vSB1-006 | 26.6% | |
| **G4a & 4b** | vSB1-007 | 33.3% | 93.3% |
| **G5a & 5b** | vSB1-008 | 46.6% | 86.6% |
| **G6a** | vSB1-006+vHVT13 | 14% | ND |
| **G7a** | vSB1-008+vHVT13 | 21.4% | ND |
| **G8a & 8b** | vHVT302 | 13.3% | 80% |
| **G9a & 9b** | vHVT304 | 33.3% | 93.3% |
| *ND = not done | | | |

Each group was monitored before and after challenge. NDV clinical signs after challenge were recorded. One bird died in G6 and G7 before challenge reducing the number of birds from 15 to 14 in these groups.

Percentages of clinical protection (including protection against both mortality and morbidity) are reported in Table 37 above. Full susceptibility was observed in the non-vaccinated challenged control group G1a and G1b thus validating the high severity of challenge. Partial protections ranging from 13.3 to 46.6% were observed after challenge at D14, the highest levels of protection being induced by vSB1-008, vSB1-007 and vHVT304. Protection levels after ND challenge at D28 were much higher for all vaccinated groups and were again slightly higher in the groups vaccinates with vSB1-008, vSB1-007 or vHVT304. These results indicated that ND protection levels were dependent on the date of challenge and on the construct. The vSB 1-008 and vSB1-007 constructs performed slightly better than vSB 1-004 and vSB 1-006, and the vHVT304 performed slightly better than vHVT302, indicating that different characteristics of the constructs are playing a role in the performances of MDV-based vector vaccines.

In conclusion, the results of this study showed that ND protection levels induced by Marek's disease vectors expressing NDV F gene may depend on different parameters including the vector, the locus of insertion, the F gene, the promoter, the poly-adenylation site and the challenge conditions.

### Example 25 Efficacy of double HVT-ND+IBD vHVT304 and vHVT306 vaccines against challenges with NDV Texas GB strain at 14 and/or 28 days of age in SPF chickens

The aim of the study was to assess the efficacy of HVT-vectored vaccine expressing both NDV F and IBDV VP2 genes against Newcastle disease challenge (Texas GB strain, genotype II) performed at 14 and/or 28 days of age in SPF chickens.

The characteristics of the 2 recombinant vaccine candidates tested in this study are described in the Table 38 below.

**Table 38 Characteristics of the recombinant vaccine candidates used in this study**

| Name | Parental virus | Promoter | F gene | Poly-A | Locus |
|---|---|---|---|---|---|
| vHVT304 | vHVT13 | SV40 | Opt-VIId | Synthetic | IG2 |
| vHVT306 | vHVT13 | SV40 | Opt-VIId | Synthetic | SORF3-US2 |

On D0, 90 one-day-old SPF chickens were randomly allocated into 3 groups of 15 birds (G1a to G3a challenged at D14) and 3 groups of 15 birds (G1b to G3b challenged at D28). The birds were injected by subcutaneous injection in the neck at D0 with 0.2 mL containing a target dose of 2000 pfu for recombinant vaccines. The design of the study is shown in Table 39 below. The birds were challenged by the intramuscular route on D14 or D28 with a target dose of 4.0 log10 EID50 (0.1 mL) velogenic ND Texas GB (genotype II) strain.

**Table 39 Results of ND efficacy**

| **Group** | **Vaccine at day-old (D0)** | **% ND protection after ND challenge at 14 days of age** | **% ND protection after ND challenge at 28 days of age** |
|---|---|---|---|
| **G1a & 1b** | - | 0% | 0% |
| **G2a & 2b** | vHVT304 | 26.7% | 92.9% |
| **G3a & 3b** | vHVT306 | 33.3% | 86.7% |

Each group was monitored before and after challenge. NDV clinical signs after challenge were recorded. One bird died in G2b before challenge reducing the number of birds from 15 to 14 in this group.

Percentages of clinical protection (including protection against both mortality and morbidity) are reported in Table 39 above. Full susceptibility was observed in the non-vaccinated challenged control group G1a and G1b thus validating the high severity of challenge. Protections levels after challenge at D14 were much lower than those obtained after challenge at D28. These vaccine candidates had the same NDV F expression cassette inserted into 2 different loci of vHVT13 genome. They performed equally in terms of ND protection in the tested conditions, indicating that both insertion loci (IG2 and SORF3-US2) are equally suitable for NDV F cassette insertion.

In conclusion, the results of this study showed that ND protection levels induced by Marek's disease vectors expressing NDV F gene depend on different parameters including the vector, the locus of insertion, the F gene, the promoter, the poly-adenylation site and the challenge conditions.

### Example 26 ND early efficacy induced by double HVT-ND+IBD (vHVT302, vHVT303, and vHVT304) or SB1-vectors (vSB1-006 and vSB1-007) in one day-old SPF chickens against a velogenic genotype V NDV challenge

The objective of the study was to evaluate the efficacy of three double HVT-ND+IBD (vHVT302, vHVT303, and vHVT304) and two SB1-ND vectors (vSB1-006 and vSB1-007) in one day-old SPF chickens against a velogenic genotype V (Chimalhuacan) NDV challenge performed at D14.

The characteristics of the 5 recombinant vaccine candidates tested in this study are described in the Table 40 below.

**Table 40 Characteristics of the recombinant vaccine candidates used in this study**

| Name | Parental virus | Promoter | F gene | Poly-A | Locus |
|---|---|---|---|---|---|
| vHVT302 | vHVT13 | US10 | Opt-VIId | US10 | US10 |
| vHVT303 | vHVT13 | US10 | Opt-V (CA02) | US10 | US10 |
| vHVT304 | vHVT13 | SV40 | Opt-VIId | Synthetic | IG2 |
| vSB 1-006 | SB-1 | SV40 | Opt-VIId | Synthetic | UL55/LORF5 |
| vSB 1-007 | SB-1 | SV40 | Opt-VIId | (endogeneous from gC gene) | gC |

Six groups (1 and 2) of ten one-day-old specific pathogen free (SPF) white Leghorn chicks were randomly constituted. Birds from groups 2 to 6 were vaccinated by the subcutaneous route (nape of the neck) with a target dose of 2000 PFU as shown in the Table 41 below. Chicks from group 1 were not vaccinated and were kept as control birds. At 2 week-of-age, all birds were challenged with the genotype V Mexican Chimalhuacan (Mex V) velogenic NDV strain. The challenge was performed by the intramuscular (IM) route using 10⁵ Egg Infectious Dose 50 (EID50) diluted in 0.2 ml of physiological sterile water. All birds were monitored until 14 days post-challenge. After challenge, health status of each bird was scored daily as follows: healthy / with specific symptoms (ruffled feathers, prostration, torticollis, tremor) / dead. Any bird that showed specific symptoms for more than 2 days or was noted sick on D28 was taken into account for calculation of morbidity.

**Table 41 Results of early ND protection induced by different MDV vectored candidates expressing NDV F gene in SPF day-old chicks**

| Group | Vaccine | Target dose (PFU) under 0.2 mL (actual dose) | Protection against mortality | Protection against morbidity |
|---|---|---|---|---|
| G1 | - | - | 0% | 0% |
| G2 | vHVT302 | 2000(4427) | 50% | 10% |
| G3 | vHVT303 | 2000 (ND) | 10% | 0% |
| G4 | vHVT304 | 2000 (1169) | 80% | 60% |
| G5 | vSB1-006 | 2000 (1720) | 60% | 40% |
| G6 | vSB 1-007 | 2000(1564) | 80% | 50% |

Results of protection are summarized in Table 41. All control birds died after ND challenge. Variable levels of ND protection were induced by the different tested vaccines ranging from 10% to 80% and from 0% and 60% in terms of protection against mortality and morbidity, respectively. The vHVT304 candidate induced a better protection than the vHVT303 and vHVT302 candidates; this may be due to the exogenous SV40 promoter placed in front of the NDV F gene. The vSB1-007 performed slightly better than the vSB 1-006. Furthermore, performances obtained with vHVT304 were comparable to those obtained with vSB1-007 indicating that different Marek's disease vectors can reach the same level of ND protection.

In conclusion, this study demonstrates that both double HVT-ND+IBD and SB1-ND vectored vaccines can reach significant levels of ND protection in a very severe and early NDV challenge model.

### Example 27 ND efficacy induced by the double HVT-ND+IBD vHVT306 administered by in ovo or SC route to one day-old SPF chickens against a velogenic genotype V NDV challenge performed at D28

The objective of the study was to evaluate the efficacy of one double HVT-ND+IBD (vHVT306) administered by the in ovo or SC route to SPF chickens against a velogenic genotype V (Chimalhuacan) NDV challenge performed at 28 days of age.

The characteristics of the vHVT306 recombinant vaccine candidate tested in this study are described in Table 42 below. The single HVT-IBD vector vaccine vHVT13 was used as a control.

**Table 42 Characteristics of the recombinant vaccine candidate used in this study**

| Name | Parental virus | Promoter | F gene | Poly-A | Locus |
|---|---|---|---|---|---|
| vHVT306 | vHVT13 | SV40 | Opt-VIId | Synthetic | SORF3-US2 |

On day -3, 40 SPF embryonated eggs aged around 18 days and 18 hours of incubation were randomly allocated into 2 groups of 20 eggs each. On D0, one group of 12 day-old SPF chicks was added. The definition of groups is given in Table 43 below. The vaccination was performed on D-3 (*in ovo* route) or on D0 (SC route, in the back of the neck) and the target dose of vHVT306 and vHVT13 was 2000PFU/bird. For the *in ovo* route, hatchability, viability (until D28) and growth of the birds (between hatching and D28) were monitored.

On D28, 10 birds per group were challenged with virulent ND Chimalhuacan strain. The challenge was performed by the intramuscular (IM) route using 10⁵ Egg Infectious Dose 50 (EID50) diluted in 0.2 ml of physiological sterile water. Birds were monitored until 14 days post-challenge. Specific clinical signs and mortality were recorded. Any bird that showed specific symptoms for more than 2 days or was noted sick on D42 was taken into account for calculation of morbidity. Five and seven days post-challenge (i.e. on D33 and D35), oropharyngeal swab was taken from each surviving bird. All the swabs were analyzed by specific NDV qRT-PCR.

**Table 43 Results of ND protection induced by vHVT306 MDV vectored candidate expressing both NDV F and IBDV VP2 genes administered by the SC or in ovo route into SPF chicks**

| Group | Vaccine/route | Hatchability/ viability (%) | Protection against mortality/morbidi ty | % birds shedding at 5 dpi/7 dpi (mean log10 titer*) |
|---|---|---|---|---|
| G1 | vHVT13/*in ovo* | 100%/100% | 0%/0% | (not tested) |
| G2 | vVHT306/*in ovo* | 100%/100% | 100%/100% | 0% (2.7)/0% (2.7) |
| G3 | vHVT306/SC | - | 100%/100% | 20% (3.2)/10% (2.9) |
| * The threshold titer of the real time RT PCR was set at 2.7 log 10 equivalent EID50 | | | | |

Full hatchability was recorded after *in ovo* vaccination in groups 1 and 2 and all hatched birds survived up to D28. No difference in body weights was detected between the two groups at both D0 and D28 confirming the perfect safety of vHVT306 when administered *in ovo.* Results of protection are summarized in Table 43. All vHVT13-vaccinated control birds died by 4 days after ND challenge. Full clinical ND protection was induced by vHVT306 administered by both routes. Furthermore, no shedding was detected after *in ovo* administration whereas only a few birds shed detectable amount of challenge virus after SC administration.

In conclusion, this study demonstrates that the double HVT-ND+IBD vHVT306 induce excellent level of ND protection by SC or *in ovo* administration routes in a very severe heterologous NDV challenge model.

### Example 28 Efficacy of double HVT-ND+IBD (vHVT302, vHVT303 and vHVT304) recombinant vaccines, against challenge with a classical IBDV isolate on D15 in SPF chickens

The aim of the study was to assess the early IBD efficacy of double HVT recombinants vHVT302, vHVT303 and vHVT304 recombinant constructs against a virulent infectious bursal disease virus (vIBDV) challenge (Faragher 52/70 strain) performed at 15 days of age in SPF chickens.

The characteristics of the 3 double HVT-ND+IBD recombinant vaccine candidates tested in this study are described in the Table 44 below.

**Table 44 Characteristics of the expression cassettes of double HVT recombinants**

| Name | Parental virus | Promoter | F gene | Poly-A | Locus |
|---|---|---|---|---|---|
| vHVT302 | vHVT13 | US10 | Opt-VIId | US10 | US10 |
| vHVT303 | vHVT13 | US10 | Opt-V (CA02) | US10 | US10 |
| vHVT304 | vHVT13 | SV40 | Opt-VIId | Synthetic | IG2 |

On D0, 40 one-day-old SPF chickens were randomly allocated into 4 groups of 10 birds including one control groups (G1) that was vaccinated with vSB 1-004, a SB-1 vector expressing NDV F gene. Five other SPF birds were kept unvaccinated and unchallenged for bursal/body weights evaluation. The birds were injected by subcutaneous injection in the neck at D0 with 0.2 mL of recombinant vaccines containing a target dose of 2000 pfu as described in the Table 45 below. On D15, blood sample was collected from all birds per group (10 birds per group except for groups 1 and 3 in which 1 bird died before blood sampling) for serological testing with the Kit ProFLOK^{®} plus IBD (Synbiotics Corp). On D15, birds from all 4 groups were challenged by the eye drop (0.05 mL per bird) with 2.5 log10 EID50.

**Table 45 Study design and results of IBD efficacy**

| **Group** | **Vaccine at day-old** | **ELISA IBD+ titer (log10)** | **Number Dead /Sick (total)¹** | **% protection²** | **Mean bursal/body weight ratio⁴** |
|---|---|---|---|---|---|
| **G1** | vSB 1-004 | 0.25 | 1/9 (9) | 0% | 0.0014 |
| **G2** | vHVT302 | 2.6 | 0/1 (10) | 80% | 0.0043 |
| **G3** | vHVT303 | 3.0 | 0/0 (9) | 100% | 0.0053 |
| **G4** | vHVT304 | 2.4 | 0/0 (10) | 80% | 0.0034 |
| ¹ Birds sick for more than 2 days or still sick on D25 were considered as sick. The number in brackets is the total number of birds in the group that were challenged. | | | | | |
| ² Protection against clinical signs and severe bursal lesion (bursal score <3) | | | | | |
| ⁴ The bursal/body weight ratio of the unvaccinated/unchallenged group was 0.0043. | | | | | |

Each group was monitored before and after challenge. IBDV clinical signs were recorded for 11 days after challenge (from D15 to D25). At the end of the post-challenge observation period (D25), all the surviving birds were euthanized and necropsied. Body and bursal weights were recorded. Each bursa of Fabricius (BF) was weighted then stored in individual recipients containing 4% formaldehyde for histology. Histological lesions of the bursa were scored according to the scale presented in Table 46.

**Table 46 Scoring scale of histological lesions of the bursa of Fabricius***

| **Score** | **Histology observation/lesions** |
|---|---|
| 0 | No lesion, normal bursa |
| 1 | 1% to 25% of the follicles show lymphoid depletion (i.e. less than 50% of depletion in 1 affected follicle), influx of heterohils in lesions |
| 2 | 26% to 50% of the follicles show nearly complete lymphoid depletion (i.e. more than 75% of depletion in 1 affected follicle), affected follicles show necrosis and severe influx of heterophils be detected |
| 3 | may 51% to 75% of the follicles show lymphoid depletion; affected follicles show necrosis lesions and a severe influx of heterophils is detected |
| 4 | 76% to 100% of the follicles show nearly complete lymphoid depletion; hyperplasia and cyst structures are detected; affected follicles show necrosis and severe influx of heterophils is detected |
| 5 | 100% of the follicles show nearly complete lymphoid depletion; complete loss of follicular structure, thickened and folded epithelium, fibrosis of bursal tissue |
| * sourced from Monograph No. 01/2008:0587 of EU Pharmacopoeia "Avian Infectious Bursal Disease vaccine (live) | |

A bird was considered as affected if it died and/or showed notable sign of disease and/or severe lesions of the bursa of Fabricius (*i.e*., histology score ≥3).

The mean ELISA IBD+ antibody titer expressed in log 10 before challenge is shown in Table 45. Significant titers were detected in all vaccinated groups that were significantly higher than that of the control group G1. The serology titer was slightly higher in G3 (vHVT303).

Severe clinical signs were observed after challenge in all 9 birds of the control group G1, which lead to the death of 1 bird. Only one vaccinated bird in G2 (vHVT302) showed clinical signs after challenge. Percentages of protection against severe bursal lesions are shown in Table 45 above. Significant IBD protection was observed in all vaccinated groups, a full protection being observed in G3 (vHVT303). The mean bursal/body weight ratios are also shown in Table 45. Ratios in all vaccinated groups were higher than those of the challenged control group G1 and not significantly different from the unvaccinated and unchallenged control group.

In conclusion, these data indicate that the three double HVT-IBD+ND tested in this study induced IBD antibodies and early IBD protection in a severe IBDV challenge model.

### Example 29 Efficacy of five different HVT-ND vaccine candidates against challenges with velogenic NDV ZJ1 (genotype VIId) isolate at 14 days of age in SPF chickens

The aim of the study was to assess the efficacy of 5 single HVT recombinant constructs (vHVT39, vHVT110, vHVT111, vHVT112 and vHVT113) expressing the NDV F gene against Newcastle disease challenge with velogenic NDV ZJ1 (genotype VIId) isolate performed at 14 days of age in SPF chickens.

The characteristics of these 5 vaccine candidates are described in Table 47 below.

**Table 47 Characteristics of the HVT-ND recombinant viruses used in the challenge study**

| Name | Parental virus | Promoter | F gene* | Poly-A | Locus |
|---|---|---|---|---|---|
| vHVT039 | HVT | MDV gB | Wtnm-Texas | SV40 | IG1 |
| vHVT110 | HVT | MCMV IE | Wt-VIId | SV40 | IG1 |
| vHVT111 | HVT | SV40 | Wt-VIId | SV40 | IG1 |
| vHVT112 | HVT | MCMV IE | Wt-YZCQ | SV40 | IG1 |
| vHVT113 | HVT | MCMV IE | Wt-Texas | SV40 | IG1 |
| *Wt means that the wild type velogenic F gene sequence was used but the cleavage site was modified to that of a lentogenic virus. Wtnm means that the cleavage site of the wild type sequence was not modified. The Texas velogenic strain belongs to genotype IV and YZCQ to the genotype VIId. | | | | | |

On D0, 72 one-day-old SPF chickens were randomly allocated into 5 groups of 12 birds (vaccinated) and 1 group of 12 birds (non-vaccinated controls). The birds were injected by subcutaneous injection in the neck at D0 with 0.2 mL of recombinant vaccines containing a target dose of 6000 pfu as described in Table 48 below. The birds were challenged by the intramuscular route on D14 with 5 log 10 EID50 of NDV ZJ1/2000 (genotype VIId) velogenic strain.

**Table 48 Results of ND efficacy**

| **Group** | **Vaccine at day-old (D0)** | **% clinical protection** | |
|---|---|---|---|
| | | **Protection against mortality/morbidity** | **Mean shedding titer (log10) at 2/4 dpi** |
| **G1** | - | 0%/0% | 3.5/- (all dead) |
| **G2** | vHVT039 | 25%/8% | 2.5/4.8 |
| **G3** | vHVT110 | 100%/83% | 1.8/2.0 |
| **G4** | vHVT111 | 100%/67% | 1.8/2.8 |
| **G5** | vHVT1 12 | 75%/42% | 1.7/3.4 |
| **G6** | vHVT113 | 83%/25% | 1.4/3.3 |

Each group was monitored before and after challenge. NDV clinical signs and mortality were recorded after challenge. Oropharyngeal swabs were taken at 2 and 4 days post-infection (dpi) for evaluation of viral load by real time RT-PCR using the method described by Wise et al. (2004; Development of a Real-Time Reverse-Transcription PCR for Detection of Newcastle Disease Virus RNA in Clinical Samples. J Clin Microbiol 42, 329-338).

Percentages of protection against mortality and morbidity are reported in Table 48 above. Full susceptibility was observed in the non-vaccinated challenged control group G1 thus validating the high severity of the challenge. Vaccines induced variable levels of protection against mortality (25-100%) or against morbidity (8%-83%). The best protection level was induced by vHVT110 whereas the lowest one was induced by vHVT039, the other candidates giving intermediate results. Results of oropharyngeal shedding at 2 and 4 dpi are also shown in Table 48 above and are in line with those of clinical protection. These vaccine candidates differ in their promoter and F gene sequence. These results show that both of these parameters are important for the design of optimal HVT-ND vaccine candidate.

In conclusion, the results of this study showed the importance of promoter and F gene sequence in the ND efficacy induced by HVT-vectored ND vaccine candidates.

### Example 30 Evaluation of the Newcastle disease efficacy induced by double SB1 constructs expressing IBDV VP2 and NDV F

The aim of the study is to assess the efficacy of double SB1 constructs expressing IBDV VP2 and NDV F against Newcastle disease challenge.

On D0, one-day-old SPF chickens are randomly allocated into several groups of 10-20 birds, including vaccinated and non-vaccinated groups. The birds of the vaccinated groups are injected by subcutaneous injection in the neck at D0 with 0.2 mL containing a target dose of 1000 to 5000 pfu of recombinant vaccines. Alternatively, the same dose in 0.05 mL may be administered *in ovo* 2 or 3 days before hatch. The birds (at least one vaccinated and one non vaccinated group) are challenged by the intramuscular route at different time after vaccination: for instance, D14, D28 or D42 with about 4.0 log10 EID50 (0.1 mL) of a velogenic NDV strain such as Texas GB (genotype II), ZJ1 (genotype VIId), Chimalhuacan (genotype V) strain.

Each group is monitored clinically before and after challenge. NDV clinical signs (morbidity) and mortality are recorded after challenge. Percentages of clinical protection in all groups are calculated. At least 90% of non-vaccinated challenged SPF birds should die or be severely sick after challenge to validate the severity of challenge. Oropharyngeal and cloacal swabs can be samples at different times after challenge such as 3, 5, 7 and 9 days post-challenge and the viral load can be estimated by real-time RT-PCR. The best candidates will be those who induced the highest level of clinical protection and the lowest level of viral load in the swabs. A similar study can be performed in broilers containing NDV maternal antibodies; however, these maternal antibodies may potentially protect the non-vaccinated birds if the challenge is performed early. The double SB1 construct may also be tested in combination with other Marek's disease vaccine or vector vaccines.

### Example 31 Evaluation of the infectious bursal disease efficacy induced by double SB1 constructs expressing IBDV VP2 and NDV F

The aim of the study is to assess the IBD efficacy of double SB1 expressing both the IBDV VP2 and the NDV F.

One-day-old SPF chickens are randomly allocated into several groups of 10 to 20 birds including vaccinated and non-vaccinated controls. Non-vaccinated controls will be separated into 2 subgroups including challenged and unchallenged birds. The birds of vaccinated groups are injected by subcutaneous injection in the neck at D0 with 0.2 mL of vaccines containing each a target dose of 1000 to 5000 pfu. Alternatively, the same dose in 0.05 mL may be administered *in ovo* 2 or 3 days before hatch. At different times after vaccination such as 14, 21, 28 or 42 days post-vaccination, all birds from vaccinated groups and the challenged controls are challenged by the eye drop (0.03 mL containing 2 to 4 log 10 EID50 per bird) of a virulent IBDV (such as the Faragher or the US standard strain), a very virulent IBDV such as the 91-168 isolate or a variant IBDV isolate such as the US Delaware variant E isolate. Each group is clinically monitored before and after challenge. Birds can be necropsied 4 or 5 days post-challenge for bursal gross lesions evaluation. They can also be necropsied 10 to 11 days post-challenge. Gross and/or histological lesions can be evaluated. Furthermore, birds and bursa are weighed the bursal/body weight ratios (bursa weight/body weight ratio X 100) are calculated compared to those of the non-vaccinated unchallenged group. Control SPF challenged birds must show clinical signs and/or have significant gross and/or histological lesions, and/or should have a bursal/body weight ratio significantly lower than the unvaccinated unchallenged control birds to validate the severity of challenge. The efficacy of the vaccine is evaluated by comparing these parameters with unvaccinated/challenged and unvaccinated/unchallenged groups. Such study may be performed in broiler chickens containing IBDV maternal antibodies; however, these maternal antibodies may potentially protect the non-vaccinated birds if the challenge is performed early. The double SB1 construct may also be tested in combination with other Marek's disease vaccine or vector vaccines.

### Example 32 Evaluation of the Marek's disease efficacy induced by double SB1 constructs expressing IBDV VP2 and NDV F

The aim of the study is to evaluate Marek's disease efficacy induced by the SB1 vectors expressing both IBDV VP2 and NDVF.

One-day-old SPF chickens are randomly allocated into several groups of 20 to 50 birds including vaccinated and non-vaccinated controls. Non-vaccinated controls may be separated into 2 subgroups including challenged and unchallenged birds. The birds of vaccinated groups are injected by subcutaneous injection in the neck at D0 with 0.2 mL of vaccines containing each a target dose of 1000 to 5000 pfu. Alternatively, the same dose in 0.05 mL may be administered *in ovo* 2 or 3 days before hatch. At different times after vaccination such as 3 to 10 days post-vaccination, all birds from vaccinated groups and the challenged controls are challenged by the intraperitoneal route with 0.2 mL of a Marek's disease virus (MDV) strain. MDV strain may be of several pathotypes such as virulent MDV (vMDV) including the JM or GA22 isolate, very virulent MDV (vvMDV) such as the RB-1B or Md5 isolate, very virulent plus (vv+MDV) such as the T-King or 648A isolate. MDV challenge strain inoculum are prepared by infecting chickens, harvesting and freezing their blood cells into liquid nitrogen in presence of a cryopreservative such as DMSO. The chicken infectious dose 50 (CID50) is established for each challenge batch before performing vaccination/challenge studies. Each group is clinically monitored before and after challenge. Birds are necropsied after at least 7 weeks post-vaccination and the presence Marek's disease gross lesions is checked in each bird. Lesions might include, but not be limited to, the following: liver, heart, spleen, gonads, kidneys, nerve and muscle lesions. Such study may be performed in broiler chickens containing MDV maternal antibodies. The double SB1 construct may also be tested in combination with other Marek's disease vaccine (for insance HVT and or CVI988 Rispens strains) or MD vector vaccines. MD challenge may also be performed by contact between vaccinated birds and MDV infected non-vaccinated SPF chicks.

## Claims

1. A composition or vaccine for use in a method of inducing an immunogenic or protective response in an animal against Newcastle Disease Virus (NDV), said composition or vaccine comprising a recombinant Gallid herpesvirus 3 (MDV-2) SB-1 strain vector, said vector comprising one or more heterologous polynucleotides coding for and expressing at least one antigen of an avian pathogen, wherein the heterologous polynucleotide encodes the Newcastle Disease Virus protein NDV-F, wherein said method comprises at least one administration of said composition or vaccine.

2. The composition or vaccine for use according to claim 1, further comprising one or more compositions or vaccines selected from the group consisting of a recombinant HVT vector (or MDV-3 or Meleagrid herpesvirus-1) comprising heterologous polynucleotides coding for and expressing at least one antigen of an avian pathogen, wild type HVT (MDV-3), recombinant MDV-1 vector (or Gallid herpesvirus-2) comprising heterologous polynucleotides coding for and expressing at least one antigen of an avian pathogen, wild type MDV-1, and a combination thereof.

3. The composition or vaccine for use according to claim 2, wherein the recombinant HVT vector is vHVT13.

4. The composition or vaccine for use according to claims 1-2, wherein the NDV-F protein sequence has at least 90% sequence identity to the sequence as set forth in SEQ ID NOs: 2, 9, 50, 52 or 54.

5. The composition or vaccine for use according to claims 1-2, wherein the polynucleotide encoding NDV-F has at least 90% sequence identity to SEQ ID NOs: 1, 8, 49, 51 or 53.

6. The composition or vaccine for use according to any one of claims 1-5, wherein the heterologous polynucleotide in Gallid herpesvirus 3 is inserted into the region coding for glycoprotein c (UL44), the region between ORF UL55 and ORF LORF5 in the unique long (UL) region, the region between ORF SORF4 and ORF US 10, or within the US2 region of the Gallid herpesvirus 3 vector.

7. The composition or vaccine for use according to any one of claims 1-6, wherein the recombinant Gallid herpesvirus 3 vector comprises a promoter.

8. The composition or vaccine for use according to claim 7, wherein the promoter is selected from the group consisting of an immediate early CMV promoter, guinea pig CMV promoter, SV40 promoter, Pseudorabies Virus glycoprotein X promoter, Herpes Simplex Virus-1 alpha 4 promoter, Marek's Disease Virus glycoprotein A (or gC) promoter, Marek's Disease Virus glycoprotein B promoter, Marek's Disease Virus glycoprotein E promoter, Infectious Laryngotracheitis Virus glycoprotein B, Infectious Laryngotracheitis Virus glycoprotein E, Infectious Laryngotracheitis Virus glycoprotein D or Infectious Laryngotracheitis Virus glycoprotein I promoter, and Bovine Herpesvirus 1.1 VP8 promoter.

9. The composition or vaccine for use according to any one of claims 1-8, further comprising a pharmaceutically or veterinarily acceptable carrier, excipient, vehicle or adjuvant.

10. A recombinant Gallid herpesvirus 3 (MDV-2) SB-1 strain vector comprising a heterologous polynucleotide encoding a Newcastle Disease Virus protein NDV-F, a promoter, and a polyadenylation signal; wherein
(a) the heterologous polynucleotide is a wild-type NDV-F VIId polynucleotide, the promoter is a mouse cytomegalovirus IE (mCMV IE) promoter and the polyadenylation signal is an Simian virus 40 (SV40) polyadenylation signal, further wherein the heterologous polynucleotide encoding NDV-F is inserted into the region between ORF SORF4 and ORF US10 of the Gallid herpesvirus 3 (MDV-2) SB-1 strain vector; or
(b) the heterologous polynucleotide is a codon-optimized NDV-F VIId polynucleotide, the promoter is a SV40 promoter and the polyadenylation signal is SEQ ID NO: 13, further wherein the heterologous polynucleotide encoding NDV-F is inserted into the region between ORF UL55 and ORF LORF5 in the unique long (UL) region of the Gallid herpesvirus 3 (MDV-2) SB-1 strain vector; or
(c) the heterologous polynucleotide is a codon-optimized NDV-F VIId polynucleotide, the promoter is a SV40 promoter and the polyadenylation signal is endogenous originating from the glycoprotein C (gC) gene, further wherein the heterologous polynucleotide encoding NDV-F is inserted into the region coding for glycoprotein C (UL44) of the Gallid herpesvirus 3 (MDV-2) SB-1 strain vector; or
(d) the heterologous polynucleotide is a codon-optimized NDV-F V (CA02 strain) polynucleotide, the promoter is a SV40 promoter and the polyadenylation signal is SEQ ID NO: 13, further wherein the heterologous polynucleotide encoding NDV-F is inserted into the region between ORF UL55 and ORF LORF5 in the unique long (UL) region of the Gallid herpesvirus 3 (MDV-2) SB-1 strain vector; or
(e) the heterologous polynucleotide is a codon-optimized NDV-F V (CA02 strain) polynucleotide, the promoter is a SV40 promoter and the polyadenylation signal is endogenous originating from the glycoprotein C (gC) gene, further wherein the heterologous polynucleotide encoding NDV-F is inserted into the region coding for glycoprotein C (UL44) of the Gallid herpesvirus 3 (MDV-2) SB-1 strain vector.

11. The vector according to claim 10 for use in a method of inducing an immunogenic or protective response in an animal against NDV, wherein said method comprises at least one administration of said composition or vaccine.

12. The composition or vaccine for use according to any one of claims 1-9 or the vector for use according to claim 11, further wherein said composition, vaccine or vector is for use in a method of vaccinating an animal, the method comprising at least one administration of the composition, vaccine or vector.

13. The composition, vaccine or vector for use according to claim 12, wherein the method comprises a prime-boost administration regime.

14. The composition, vaccine or vector for use according to any one of claims 1 to 9 or 11-13, wherein the animal is avian.

## Patentansprüche

1. Zusammensetzung oder Impfstoff zur Verwendung in einem Verfahren zum Induzieren einer Immunantwort oder Schutzantwort in einem Tier gegen das Newcastle-Krankheit-Virus (Newcastle Disease Virus; NDV), wobei die Zusammensetzung oder der Impfstoff einen Vektor des rekombinanten Gallid-Herpesvirus 3 (MDV-2) SB-1-Stamms umfasst, wobei der Vektor ein oder mehrere heterologe Polynucleotide umfasst, die mindestens ein Antigen eines Vogelpathogens codieren und dieses exprimieren, wobei das heterologe Polynucleotid das Newcastle-Krankheit-Virus-Protein (Newcastle Disease Virus protein) NDV-F codiert, wobei das Verfahren mindestens eine Verabreichung der Zusammensetzung oder des Impfstoffs umfasst.

2. Zusammensetzung oder Impfstoff zur Verwendung nach Anspruch 1, des Weiteren umfassend eine oder mehrere Zusammensetzungen oder Impfstoffe, die ausgewählt sind aus der Gruppe bestehend aus einem rekombinanten HVT-Vektor (oder MDV-3 oder Meleagrid-Herpesvirus-1), der heterologe Polynucleotide umfasst, die mindestens ein Antigen eines Vogelpathogens codieren und dieses exprimieren, einem Wildtyp-HVT (MDV-3), einem rekombinanten MDV-1-Vektor (oder Gallid-Herpesvirus-2), der heterologe Polynucleotide umfasst, die mindestens ein Antigen eines Vogelpathogens codieren und dieses exprimieren, einem Wildtyp-MDV-1 und einer Kombination davon.

3. Zusammensetzung oder Impfstoff zur Verwendung nach Anspruch 2, wobei der rekombinante HVT-Vektor vHVT13 ist.

4. Zusammensetzung oder Impfstoff zur Verwendung nach den Ansprüchen 1 bis 2, wobei die NDV-F-Proteinsequenz mindestens 90% Sequenzidentität mit der wie in SEQ ID NOs:2, 9, 50, 52 oder 54 gezeigten Sequenz aufweist.

5. Zusammensetzung oder Impfstoff zur Verwendung nach den Ansprüchen 1 bis 2, wobei das Polynucleotid, das NDV-F codiert, mindestens 90% Sequenzidentität mit SEQ ID NOs:1, 8, 49, 51 oder 53 aufweist.

6. Zusammensetzung oder Impfstoff zur Verwendung nach einem der Ansprüche 1 bis 5, wobei das heterologe Polynucleotid im Gallid-Herpesvirus 3 in die Region, die Glycoprotein c (UL44) codiert, die Region zwischen ORF UL55 und ORF LORF5 in der Unique Long (UL)-Region, die Region zwischen ORF SORF4 und ORF US10 oder in die US2-Region des Gallid-Herpesvirus 3-Vektors inseriert ist.

7. Zusammensetzung oder Impfstoff zur Verwendung nach einem der Ansprüche 1 bis 6, wobei der rekombinante Gallid-Herpesvirus 3-Vektor einen Promotor umfasst.

8. Zusammensetzung oder Impfstoff zur Verwendung nach Anspruch 7, wobei der Promotor ausgewählt ist aus der Gruppe bestehend aus einem Immediate Early CMV-Promotor, Meerschweinchen-CMV-Promotor, SV40-Promotor, Pseudorabies-Virus-Glycoprotein-X-Promotor, Herpes-Simplex-Virus-1 Alpha 4-Promotor, Mareksche Krankheit-Virus-Glycoprotein-A (oder gC)-Promotor, Mareksche Krankheit-Virus-Glycoprotein-B-Promotor, Mareksche Krankheit-Virus-Glycoprotein-E-Promotor, infektiösem Laryngotracheitis-Virus-Glycoprotein-B, infektiösem Laryngotracheitis-Virus-Glycoprotein-E, infektiösem Laryngotracheitis-Virus-Glycoprotein-D oder infektiösem Laryngotracheitis-Virus-Glycoprotein-I-Promotor und bovinem Herpesvirus 1.1 VP8-Promotor.

9. Zusammensetzung oder Impfstoff zur Verwendung nach einem der Ansprüche 1 bis 8, des Weiteren umfassend einen pharmazeutisch oder veterinärmedizinisch verträglichen Träger, Exzipienten, ein pharmazeutisch oder veterinärmedizinisch verträgliches Vehikel oder Adjuvans.

10. Vektor des rekombinanten Gallid-Herpesvirus 3 (MDV-2) SB-1-Stammes, umfassend ein heterologes Polynucleotid, das ein Newcastle-Krankheit-Virus-Protein NDV-F codiert, einen Promotor und ein Polyadenylierungssignal, wobei
(a) das heterologe Polynucleotid ein Wildtyp-NDV-F VIId-Polynucleotid ist, der Promotor ein Maus-Cytomegalovirus-IE(mCMV IE)-Promotor ist und das Polyadenylierungssignal ein Simian-Virus 40 (SV40)-Polyadenylierungssignal ist, des Weiteren wobei das heterologe Polynucleotid, das NDV-F codiert, in die Region zwischen ORF SORF4 und ORF US10 des Vektors des Gallid-Herpesvirus 3 (MDV-2) SB-1- Stammes inseriert ist; oder
(b) das heterologe Polynucleotid ein Codon-optimiertes NDV-F VIId-Polynucleotid ist, der Promotor ein SV40-Promotor ist und das Polyadenylierungssignal SEQ ID NO:13 ist, des Weiteren wobei das heterologe Polynucleotid, das NDV-F codiert, in die Region zwischen ORF UL55 und ORF LORF5 in der Unique Long (UL)-Region des Vektors des Gallid-Herpesvirus 3 (MDV-2) SB-1-Stammes inseriert ist; oder
(c) das heterologe Polynucleotid ein Codon-optimiertes NDV-F VIId-Polynucleotid ist, der Promotor ein SV40-Promotor ist und das Polyadenylierungssignal endogen ist und von dem Glycoprotein C (gC)-Gen stammt, des Weiteren wobei das heterologe Polynucleotid, das NDV-F codiert, in die Region, die das Glycoprotein C (UL44) des Vektors des Gallid-Herpesvirus 3 (MDV-2) SB-1-Stammes codiert, inseriert ist; oder
(d) das heterologe Polynucleotid ein Codon-optimiertes NDV-F V (CA02-Stamm)-Polynucleotid ist, der Promotor ein SV40-Promotor ist und das Polyadenylierungssignal SEQ ID NO:13 ist, des Weiteren wobei das heterologe Polynucleotid, das NDV-F codiert, in die Region zwischen ORF UL55 und ORF LORF5 in der Unique Long (UL)-Region des Vektors des Gallid-Herpesvirus 3 (MDV-2) SB-1-Stammes inseriert ist; oder
(e) das heterologe Polynucleotid ein Codon-optimiertes NDV-F V (CA02-Stamm)-Polynucleotid ist, der Promotor ein SV40-Promotor ist und das Polyadenylierungssignal endogen ist und von dem Glycoprotein C (gC)-Gen stammt, des Weiteren wobei das heterologe Polynucleotid, das NDV-F codiert, in die Region, die das Glycoprotein C (UL44) des Vektors des Gallid-Herpesvirus 3 (MDV-2) SB-1-Stammes codiert, inseriert ist.

11. Vektor nach Anspruch 10 zur Verwendung in einem Verfahren zum Induzieren einer Immunantwort oder einer Schutzantwort in einem Tier gegen NDV, wobei das Verfahren mindestens eine Verabreichung der Zusammensetzung oder des Impfstoffs umfasst.

12. Zusammensetzung oder Impfstoff zur Verwendung nach einem der Ansprüche 1 bis 9 oder Vektor zur Verwendung nach Anspruch 11, des Weiteren wobei die Zusammensetzung, der Impfstoff oder der Vektor zur Verwendung in einem Verfahren zum Impfen eines Tiers ist, wobei das Verfahren mindestens eine Verabreichung der Zusammensetzung, des Impfstoffs oder des Vektors umfasst.

13. Zusammensetzung, Impfstoff oder Vektor zur Verwendung nach Anspruch 12, wobei das Verfahren ein Prime-Boost-Verabreichungsschema umfasst.

14. Zusammensetzung, Impfstoff oder Vektor zur Verwendung nach einem der Ansprüche 1 bis 9 oder 11 bis 13, wobei das Tier ein Vogel ist.

## Revendications

1. Composition ou vaccin destiné(e) à être utilisé(e) dans un procédé d'induction d'une réponse immunogène ou protectrice chez un animal contre le virus de la maladie de Newcastle (NDV), ladite composition ou ledit vaccin comprenant un vecteur souche SB-1 de gallid herpèsvirus 3 (MDV-2) recombinant, ledit vecteur comprenant un ou plusieurs polynucléotides hétérologues codant et exprimant au moins un antigène d'un agent pathogène aviaire, où le polynucléotide hétérologue code la protéine NDV-F du virus de la maladie de Newcastle, où ledit procédé comprend au moins une administration de ladite composition ou dudit vaccin.

2. Composition ou vaccin destiné(e) à être utilisé(e) selon la revendication 1, comprenant en outre une ou plusieurs compositions ou un ou plusieurs vaccins choisi(e)s dans le groupe consistant en un vecteur HVT (ou MDV-3 ou meleagrid herpèsvirus 1) recombinant comprenant des polynucléotides hétérologues codant et exprimant au moins un antigène d'un agent pathogène aviaire, HVT (MDV-3) de type sauvage, un vecteur MDV-1 (ou gallid herpèsvirus 2) recombinant comprenant des polynucléotides hétérologues codant et exprimant au moins un antigène d'un agent pathogène aviaire, MDV-1 de type sauvage, et une combinaison de ceux-ci.

3. Composition ou vaccin destiné(e) à être utilisé(e) selon la revendication 2, où le vecteur HVT recombinant est vHVT13.

4. Composition ou vaccin destiné(e) à être utilisé(e) selon les revendications 1-2, où la séquence de la protéine NDV-F a au moins 90 % d'identité de séquence avec la séquence telle que présentée dans SEQ ID NO: 2, 9, 50, 52 ou 54.

5. Composition ou vaccin destiné(e) à être utilisé(e) selon les revendications 1-2, où le polynucléotide codant NDV-F a au moins 90 % d'identité de séquence avec SEQ ID NO: 1, 8, 49, 51 ou 53.

6. Composition ou vaccin destiné(e) à être utilisé(e) selon l'une quelconque des revendications 1-5, où le polynucléotide hétérologue dans le gallid herpèsvirus 3 est inséré dans la région codant la glycoprotéine c (UL44), la région entre l'ORF UL55 et l'ORF LORF5 dans la longue région unique (UL), la région entre l'ORF SORF4 et l'ORF US10, ou dans la région US2 du vecteur gallid herpèsvirus 3.

7. Composition ou vaccin destiné(e) à être utilisé(e) selon l'une quelconque des revendications 1-6, où le vecteur gallid herpèsvirus 3 recombinant comprend un promoteur.

8. Composition ou vaccin destiné(e) à être utilisé(e) selon la revendication 7, où le promoteur est choisi dans le groupe consistant en le promoteur précoce immédiat de CMV, le promoteur de CMV du cobaye, le promoteur de SV40, le promoteur de la glycoprotéine X du virus de la pseudo-rage, le promoteur alpha 4 du virus herpès simplex 1, le promoteur de la glycoprotéine A (ou gC) du virus de la maladie de Marek, le promoteur de la glycoprotéine B du virus de la maladie de Marek, le promoteur de la protéine E du virus de la maladie de Marek, le promoteur de la glycoprotéine B du virus de la laryngotrachéite infectieuse, de la glycoprotéine E du virus de la laryngotrachéite infectieuse, de la glycoprotéine D du virus de la laryngotrachéite infectieuse ou de la glycoprotéine I du virus de la laryngotrachéite infectieuse, et le promoteur VP8 de l'herpèsvirus bovin 1.1.

9. Composition ou vaccin destiné(e) à être utilisé(e) selon l'une quelconque des revendications 1-8, comprenant en outre un vecteur, excipient, véhicule ou adjuvant acceptable du point de vue pharmaceutique ou vétérinaire.

10. Vecteur souche SB-1 de gallid herpèsvirus 3 (MDV-2) recombinant comprenant un polynucléotide hétérologue codant une protéine NDV-F du virus de la maladie de Newcastle, un promoteur et un signal de polyadénylation; où
(a) le polynucléotide hétérologue est un polynucléotide NDV-F VIId de type sauvage, le promoteur est un promoteur IE de cytomégalovirus de souris (mCMV IE) et le signal de polyadénylation est un signal de polyadénylation du virus simien 40 (SV40), en outre où le polynucléotide hétérologue codant NDV-F est inséré dans la région entre l'ORF SORF4 et l'ORF US10 du vecteur souche SB-1 du gallid herpèsvirus 3 (MDV-2); ou
(b) le polynucléotide hétérologue est un polynucléotide NDV-F VIId optimisé quant aux codons, le promoteur est un promoteur de SV40 et le signal de polyadénylation est SEQ ID NO: 13, en outre où le polynucléotide hétérologue codant NDV-F est inséré dans la région entre l'ORF UL55 et l'ORF LORF5 dans la longue région unique (UL) du vecteur souche SB-1 du gallid herpèsvirus 3 (MDV-2); ou
(c) le polynucléotide hétérologue est un polynucléotide NDV-F VIId optimisé quant aux codons, le promoteur est un promoteur de SV40 et le signal de polyadénylation est endogène provenant du gène de la glycoprotéine C (gC), en outre où le polynucléotide hétérologue codant NDV-F est inséré dans la région codant la glycoprotéine C (UL44) du vecteur souche SB-1 du gallid herpèsvirus 3 (MDV-2); ou
(d) le polynucléotide hétérologue est un polynucléotide NDV-F V (souche CA02) optimisé quant aux codons, le promoteur est un promoteur de SV40 et le signal de polyadénylation est SEQ ID NO: 13, en outre où le polynucléotide hétérologue codant NDV-F est inséré dans la région entre l'ORF UL55 et l'ORF LORF5 dans la longue région unique (UL) du vecteur souche SB-1 du gallid herpèsvirus 3 (MDV-2); ou
(e) le polynucléotide hétérologue est un polynucléotide NDV-F V (souche CA02) optimisé quant aux codons, le promoteur est un promoteur de SV40 et le signal de polyadénylation est endogène provenant du gène de la glycoprotéine C (gC), en outre où le polynucléotide hétérologue codant NDV-F est inséré dans la région codant la glycoprotéine C (UL44) du vecteur souche SB-1 du gallid herpèsvirus 3 (MDV-2).

11. Vecteur selon la revendication 10 destiné à être utilisé dans un procédé d'induction d'une réponse immunogène ou protectrice chez un animal contre NDV, où ledit procédé comprend au moins une administration de ladite composition ou dudit vaccin.

12. Composition ou vaccin destiné(e) à être utilisé(e) selon l'une quelconque des revendications 1-9 ou vecteur destiné à être utilisé selon la revendication 11, en outre où ladite composition, ledit vaccin ou ledit vecteur est destiné(e) à être utilisé(e) dans un procédé de vaccination d'un animal, le procédé comprenant au moins une administration de la composition, du vaccin ou du vecteur.

13. Composition, vaccin ou vecteur destiné(e) à être utilisé(e) selon la revendication 12, où le procédé comprend un schéma d'administration primo-immunisation-rappel.

14. Composition, vaccin ou vecteur destiné(e) à être utilisé(e) selon l'une quelconque des revendications 1 à 9 ou 11-13, où l'animal est un aviaire.
